(19) Europäisches Patentamt
European Patent Office
Office européen des brevets

(11) EP 0 934 303 B1

(12) **EUROPEAN PATENT SPECIFICATION**

(45) Date of publication and mention of the grant of the patent:
**29.12.2004 Bulletin 2004/53**

(21) Application number: **97946875.8**

(22) Date of filing: **11.09.1997**

(51) Int Cl.7: **C07D 401/00**

(86) International application number:
**PCT/US1997/019976**

(87) International publication number:
**WO 1998/011091 (19.03.1998 Gazette 1998/11)**

(54) **COMPOUNDS USEFUL FOR INHIBITION OF FARNESYL PROTEIN TRANSFERASE**

TRIZYKLISCHE VERBINDUNGEN MIT FARNESYL PROTEIN TRANSFERASE INHIBIERENDER WIRKUNG

COMPOSES SERVANT A INHIBER LA FARNESYL PROTEINE TRANSFERASE

(84) Designated Contracting States:
**AT BE CH DE DK ES FI FR GB GR IE IT LI LU NL PT SE**
Designated Extension States:
**LT LV RO**

(30) Priority: **13.09.1996 US 713297**
**17.06.1997 US 877453**

(43) Date of publication of application:
**11.08.1999 Bulletin 1999/32**

(73) Proprietor: **SCHERING CORPORATION**
**Kenilworth, New Jersey 07033-0530 (US)**

(72) Inventors:
- **TAVERAS, Arthur, G.**
  **Rockaway, NJ 07866 (US)**
- **MALLAMS, Alan, K.**
  **Hackettstown, NJ 07840 (US)**
- **AFONSO, Adriano**
  **West Caldwell, NJ 07006 (US)**
- **REMISZEWSKI, Stacy, W.**
  **Washington Township, NJ 07675 (US)**
- **NJOROGE, F., George**
  **Union, NJ 07083 (US)**
- **DOLL, Ronald, J.**
  **Maplewood, NJ 07040 (US)**
- **LALWANI, Tarik**
  **Forest Hills, NY 11375 (US)**
- **ALVAREZ, Carmen**
  **Roselle Park, NJ 07204 (US)**

(74) Representative: **Ritter, Stephen David et al**
**Mathys & Squire**
**100 Gray's Inn Road**
**London WC1X 8AL (GB)**

(56) References cited:
**EP-A- 0 270 818**
**EP-A- 0 396 083**
**EP-A- 0 495 484**
**WO-A-95/10514**
**WO-A-95/10515**
**WO-A-95/10516**
**WO-A-96/30362**
**WO-A-96/30363**
**WO-A-96/31478**
**WO-A-97/23478**

- **BISHOP,W.R. ET AL.: "Novel Tricyclic Inhibitors of Farnesyl Protein Tranferase" J.BIOL.CHEM., vol. 270, no. 51, 22 December 1995, ROCKVILLE PIKE, pages 30611-30618, XP002050604**
- **BUSS,J.E. ET AL.: "Farnesyl transferase inhibitors : the successes and surprises of a new class of potential cancer chemotherapeutics" CHEM.BIOL., vol. 2, December 1995, pages 787-791, XP002056549**
- **NJOROGE,F.G. ET AL.: "Novel Tricyclic Aminoacetyl and Sulfonamide inhibitors of RAS farnesyl protein transferase" BIOORG.&MED.CHEM.LETT., vol. 6, no. 24, December 1996, OXFORD, pages 2977-2982, XP002056550**
- **NJOROGE,F.G. ET AL.: "Discovery of a novel Nonpeptide Tricyclic Inhibitors of RAS Farnesyl Transferase " BIOORG.&MED.CHEM.LETT., vol. 5, no. 1, 1997, OXFORD, pages 101-113, XP002056551**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 934 303 B1

Printed by Jouve, 75001 PARIS (FR)

## Description

The present invention relates to inhibitors of farnesyl protein transferase.

### BACKGROUND

Bishop *et al*., J. Biol. Chem. (1995) 270, pp 30611-30618 and *Buss et al*., Chem. and Biol. (1995), 2, pp 787-791 discloses the 8-chloro, piperidyl-substituted compound SCH 44342 as an inhibitor of farnesyl protein transferase:

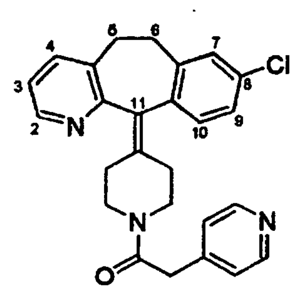

Tricyclic aminoacetyl and sulfonamide inhibitors of farnesyl protein transferase are disclosed in Njoroge *et al*., Bioorg. Med. Chem. Lett., (1996) 6, pp 2977-2982.

Tricyclic compounds useful for the inhibition of farnesyl protein transferase are also disclosed in WO 95/10516, WO 95/10515, WO 95/10514 and Njoroge *et. al*., Bioorg. Med. Chem. Lett., (1997), 5, pp 101-113. WO 95/10516 and WO 95/10514 each disclose, in the specific examples, 3,4,8-tri-halo substituted compounds. In the general formulae disclosed in WO 95/10516, the compounds can contain the group - (O)$CH_2$-(N-substituted piperidyl), said group being attached at the N-atom of the 11-piperidyl/piperazinyl ring. The substituent on the nitrogen atom of the terminal N-substituent can be alkyl, alkoxycarbonyl, haloalkyl or alkylcarbonyl or -CONH$R^{10}$ wherein $R^{10}$ is H or alkyl.

Further tricyclic compounds useful for the inhibition of farnesyl protein transferase are disclosed in WO 96/30363, WO 96/30362, WO 96/31478 and WO 96/23478.

In view of the current interest in inhibitors of farnesyl protein transferase, a welcome contribution to the art would be further compounds useful for the inhibition of farnesyl protein transferase. Such a contribution is provided by this invention.

### SUMMARY OF THE INVENTION

This invention provides compounds useful for the inhibition of farnesyl protein transferase (FPT). The compounds of this invention are represented by the formula:

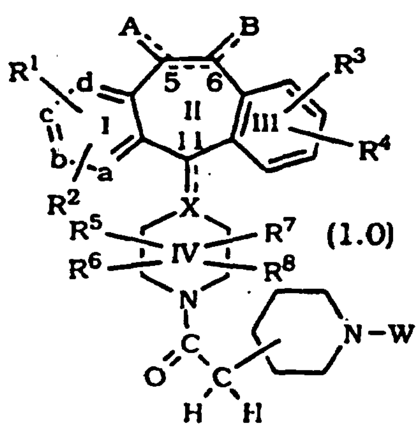

(1.0)

or a pharmaceutically acceptable salt or solvate thereof, wherein: one of a, b, c and d represents N or $NR^9$ wherein $R^9$ is $O^-$, $-CH_3$ or $-(CH_2)_nCO_2H$ wherein n is 1 to 3, and the remaining a, b, c and d groups represent $CR^1$ or $CR^2$; or

each of a, b, c, and d are independently selected from $CR^1$ or $CR^2$;

$R^2$ is H and $R^1$, $R^3$ and $R^4$ are halo;

$R^5$, $R^6$, $R^7$ and $R^8$ each independently represents H, $-CF_3$, - $COR^{10}$, alkyl or aryl, said alkyl or aryl optionally being substituted with $-OR^{10}$, $-SR^{10}$, $-S(O)_tR^{11}$, $-NR^{10}COOR^{11}$, $-N(R^{10})_2$, $-NO_2$, - $COR^{10}$, $-OCOR^{10}$, $-OCO_2R^{11}$, $-CO_2R^{10}$, $OPO_3R^{10}$, or $R^5$ is combined with $R^6$ to represent =O or =S and/or $R^7$ is combined with $R^8$ to represent =O or =S;

$R^{10}$ represents H, alkyl, aryl, or aralkyl (e.g., benzyl);

$R^{11}$ represents alkyl or aryl;

X represents N, CH or C, which C may contain an optional double bond (represented by the dotted line) to carbon atom 11;

the dotted line between carbon atoms 5 and 6 represents an optional double bond, such that when a double bond is present. A and B independently represent $-R^{10}$, halo, $-OR^{11}$, - $OCO_2R^{11}$ or $-OC(O)R^{10}$, and when no double bond is present between carbon atoms 5 and 6, A and B each independently represent $H_2$, $-(OR^{11})_2$; H and halo, dihalo, alkyl and H, $(alkyl)_2$, -H and $-OC(O)R^{10}$, H and $-OR^{10}$, =O, aryl and H, $=NOR^{10}$ or $-O-(CH_2)_p-O-$ wherein p is 2, 3 or 4; and

W is selected from the group consisting of:

(1) cyano (i.e., CN);

(2) $-C(O)R^{12}$ wherein $R^{12}$ is selected from:

(a) a heteroaryl group, for example, pyridyl (e.g., 3-pyridyl), indolyl (e.g., 2-indolyl), pyrrolyl (e.g., 2-pyrrolyl) and N-substituted pyrrolyl (e.g., N-alkylpyrrolyl such as N-alkylpyrrol-2-yl, such as, N-methylpyrrol-2-yl);

(b) H;

(c) alkyl (e.g., $-CH_3$); or

(d) a substituent of the formula:

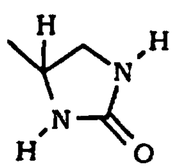 , 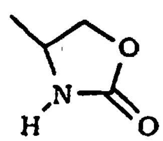 , 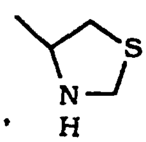 , 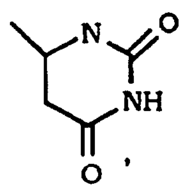 , 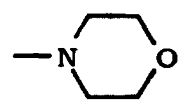 ,

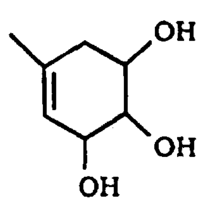 or 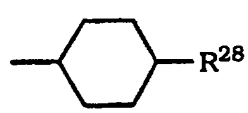 ,

wherein $R^{28}$ is selected from $-OC(O)R^{29}$, -OH, $-OC(O)NHC(O)CCl_3$. or $-OC(O)NH_2$, wherein $R^{29}$ is alkyl (e.g., $-CH_3$);

(3) an imidate represented by the formula:

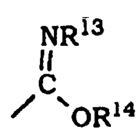

wherein $R^{13}$ is selected from the group consisting of: (a) H, (b) CN, (c) $-SO_2$-alkyl (e.g., $-SO_2CH_3$), (d) -C(O)-aryl (e.g., $-C(O)C_6H_5$, i.e., -C(O)phenyl), (e) $-SO_2NR^{10}R^{15}$ (e.g., $-SO_2NH_2$), (f) $-C(O)NR^{10}R^{15}$ (e.g., $-C(O)NH_2$), (g) $-OR^{10}$ (e.g., -OH and $-OCH_3$); and (h) $-C(O)NR^{10}C(O)NR^{10}R^{15}$ (e.g., $-C(O)NHC(O)NH_2$); $R^{14}$ is aryl; and $R^{10}$ and $R^{15}$ are independently selected from the group consisting of: H, alkyl, aryl and aralkyl;

(4) an imidamido (amidino) represented by the formula:

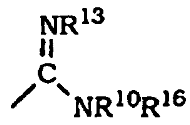

wherein $R^{13}$ is selected from the group consisting of (a)H, (b) CN, (c) $-SO_2$-alkyl (e.g., $-SO_2CH_3$), (d) -C(O)-aryl (e.g., $-C(O)C_6H_5$, i.e., -C(O)phenyl), (e) $-SO_2NR^{10}R^{15}$ (e.g., $-SO_2NH_2$), (f) $-C(O)NR^{10}R^{15}$ (e.g., $-C(O)NH_2$), (g) $-OR^{10}$ (e.g., -OH and $-OCH_3$); and (h) $-C(O)NR^{10}C(O)NR^{10}R^{15}$ (e.g., $-C(O)NHC(O)NH_2$); $R^{16}$ is selected from the group consisting of: alkyl, aralkyl, aryl, cycloalkyl, heteroaryl, heteroaralkyl and heterocycloalkyl; $R^{10}$ and $R^{15}$ are as defined above; and $R^{10}$ and $R^{16}$ are independently selected from the above defined groups;

(5) 1-amino-2-nitroethylene derivatives of the formula:

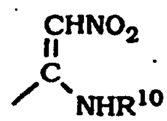

wherein $R^{10}$ is as defined above; and

(6) a substituent of the formula:

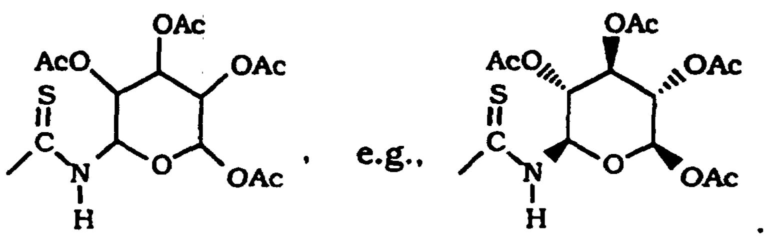

The compounds of this invention: (i) potently inhibit farnesyl protein transferase, but not geranylgeranyl protein transferase I, in vitro; (ii) block the phenotypic change induced by a form of transforming Ras which is a farnesyl acceptor but not by a form of transforming Ras engineered to be a geranylgeranyl acceptor; (iii) block intracellular processing of Ras which is a farnesyl acceptor but not of Ras engineered to be a geranylgeranyl acceptor; and (iv) block abnormal cell growth in culture induced by transforming Ras.

The compounds of this invention inhibit farnesyl protein transferase and the farnesylation of the oncogene protein Ras. Thus, this invention further provides a method of inhibiting farnesyl protein transferase, (e.g., ras farnesyl protein transferase) in mammals, especially humans, by the administration of an effective amount of the tricyclic compounds described above. The administration of the compounds of this invention to patients, to inhibit farnesyl protein transferase, is useful in the treatment of the cancers described below.

This invention provides compounds of formula (1.0) for inhibiting or treating the abnormal growth of cells, including transformed cells, by administering an effective amount of a compound of this invention. Abnormal growth of cells refers to cell growth independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

This invention also provides compounds of formula (1.0) for inhibiting or treating tumor growth by administering an effective amount of the tricyclic compounds, described herein, to a mammal (e.g., a human) in need of such treatment. In particular, this invention provides a method for inhibiting or treating the growth of tumors expressing an activated Ras oncogene by the administration of an effective amount of the above described compounds. Examples of tumors which may be inhibited or treated include, but are not limited to, lung cancer (e.g., lung adenocarcinoma), pancreatic cancers (e.g., pancreatic carcinoma such as, for example, exocrine pancreatic carcinoma), colon cancers (e.g., colorectal carcinomas, such as, for example, colon adenocarcinoma and colon adenoma), myeloid leukemias (for example, acute myelogenous leukemia (AML)), thyroid follicular cancer, myelodysplastic syndrome (MDS). bladder carcinoma, epidermal carcinoma, breast cancer and prostate cancer.

It is believed that this invention also provides compounds of formula (1.0) for inhibiting or treating proliferative diseases, both benign and malignant, wherein Ras proteins are aberrantly activated as a result of oncogenic mutation in other genes--i.e., the Ras gene itself is not activated by mutation to an oncogenic form--with said inhibition or treatment being accomplished by the administration of an effective amount of the tricyclic compounds described herein, to a mammal (e.g., a human) in need of such treatment. For example, the benign proliferative disorder neurofibromatosis, or tumors in which Ras is activated due to mutation or overexpression of tyrosine kinase oncogenes (e.g., neu, src, abl. lck, and fyn), may be inhibited or treated by the tricyclic compounds described herein.

The tricyclic compounds of this invention inhibit or treat the abnormal growth of cells. Without wishing to be bound by theory, it is believed that these compounds may function through the inhibition of G-protein function, such as ras p21, by blocking G-protein isoprenylation, thus making them useful in the treatment of proliferative diseases such as tumor growth and cancer. Without wishing to be bound by theory, it is believed that these compounds inhibit ras farnesyl protein transferase, and thus show antiproliferative activity against ras transformed cells.

DETAILED DESCRIPTION OF THE INVENTION

As used herein, the following terms are used as defined below unless otherwise indicated:

Ac-represents acetyl;
$MH^+$-represents the molecular ion plus hydrogen of the molecule in the mass spectrum;
$M^+$-represents the molecular ion of the molecule in the mass spectrum;
benzotriazol-1-yloxy represents

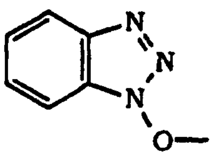
;

1-methyl-tetrazol-5-ylthio represents

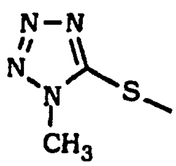
;

acyl-represents represents -C(O)-alkyl, -C(O)-alkenyl, -C(O)-alkynyl, -C(O)-cycloalkyl, -C(O)-cycloalkenyl or -C(O)-cycloalkynyl;
alkenyl-represents straight and branched carbon chains having at least one carbon to carbon double bond and containing from 2 to 12 carbon atoms, preferably from 2 to 6 carbon atoms and most preferably from 3 to 6 carbon atoms;
alkynyl-represents straight and branched carbon chains having at least one carbon to carbon triple bond and containing from 2 to 12 carbon atoms, preferably from 2 to 6 carbon atoms;
alkyl-(including the alkyl portions of alkoxy, aralkyl and heteroarylalkyl)-represents straight and branched carbon chains and contains from one to twenty carbon atoms, preferably one to six carbon atoms;
aralkyl-represents an aryl group, as defined below, bound to an alkyl group, as defined above, preferably the alkyl group is $-CH_2-$, (e.g., benzyl);
aryl (including the aryl portion of aralkyl, aryloxy and arylalkyloxy)-represents a carbocyclic group containing from 6 to 15 carbon atoms and having at least one aromatic ring (e.g., aryl is a phenyl ring), with all available substitutable carbon atoms of the carbocyclic group being intended as possible points of attachment, said carbocyclic group being optionally substituted (e.g., 1 to 3) with one or more of halo, alkyl, hydroxy, alkoxy, phenoxy, $CF_3$, amino, alkylamino, dialkylamino, $-COOR^{10}$ or $-NO_2$;
cycloalkyl-represents saturated carbocyclic rings branched or unbranched of from 3 to 20 carbon atoms, preferably 3 to 7 carbon atoms;
Et-represents ethyl;
halo-represents fluoro, chloro, bromo and iodo;
heteroaryl-represents cyclic groups, optionally substituted with $R^3$ and $R^4$, having at least one heteroatom selected from O, S or N, said heteroatom interrupting a carbocyclic ring structure and having a sufficient number of delocalized pi electrons to provide aromatic character, with the aromatic heterocyclic groups preferably containing from

2 to 14 carbon atoms, e.g., (1) thienyl (e.g., 2- or 3-thienyl), (2) imidazolyl (e.g., (2-, 4- or 5-) imidazolyl), (3) triazolyl (e.g., 3- or 5- [1,2,4-triazolyl]), (4) tetrazolyl, (5) substituted tetrazolyl, such as

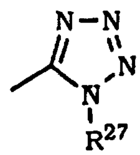

[wherein $R^{27}$ represents (a) aryl (e.g., phenyl), (b) alkyl (e.g., - $CH_3$) or (c) arylalkyl (aralkyl) (e.g., benzyl)], (6) furyl (e.g., 2- or 3-furyl). (7) thiazolyl (or thiazyl) (e.g., 2-, 4- or 5-thiazolyl), (8) pyrimidinyl (e.g., 2-, 4- or 5-pyrimidinyl), (9) pyrazinyl (e.g., 2-pyrazinyl), (10) pyridazinyl (e.g., 3- or 4-pyridazinyl), (11) triazinyl (e.g., 2-, 4- or 6-[1,3,5-triazinyl]), (12) 3- or 5-[1,2,4-thiadiazolyl], (13) 2-, 3-, 4-, 5-, 6- or 7-benzofuranyl, (14) benzoxazolyl (e.g., 2-, 4-, 5-, 6- or 7-benzoxazolyl), (15) indolyl (benzopyrrolyl) (e.g., 2-, 3-, 4-, 5-, 6- or 7-indolyl), (16) pyrazolyl (e.g., 3-, 4- or 5-pyrazolyl), (17) oxazolyl (e.g., 2-, 4- or 5-oxazolyl), (18) 2-, 3- or 4-pyridyl or pyridyl N-oxide (optionally substituted with $R^3$ and $R^4$), wherein pyridyl N-oxide can be represented as:

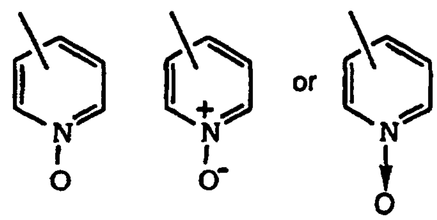
or
,

(19) benzisoxazolyl, (20) pyrrolyl, (21) benzimidazolyl, (22) isoquinolinyl, (23) quinolinyl, (24) pyridopyrazinyl, (25) naphthyridinyl, (26) pyranyl, (27) benzothienyl, (28) isobenzofuranyl, (29) isothiazolyl, (30) isoxazolyl and (31) an N-substituted pyrrolyl

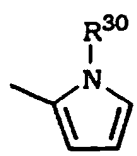

wherein $R^{30}$ is selected from: alkyl (e.g., $C_2H_5$), aralkyl (e.g., $-CH_2C_6H_5$), $-CH_2C(O)NH_2$, $-SO_2CH_3$, or $-CH_2C(O)OR^{31}$ wherein $R^{31}$ is H or alkyl (e.g., $-C(CH_3)_3$);

heteroarylalkyl (heteroaralkyl)-represents a heteroaryl group, as defined above, bound to an alkyl group, as defined above, preferably the alkyl group is $-CH_2-$, for example, $-CH_2$-(4-or 5-)imidazolyl;

heterocycloalkyl-represents a saturated, branched or unbranched carbocylic ring containing from 3 to 15 carbon atoms, preferably from 4 to 6 carbon atoms, which carbocyclic ring is interrupted by 1 to 3 hetero groups selected from -O-, -S-, $-NR^{10}-$ (wherein $R^{10}$ is as defined above); suitable heterocycloalkyl groups include: (1) tetrahydrofuranyl (e.g., 2- or 3-tetrahydrofuranyl), (2) tetrahydrothienyl e.g., (2- or 3-tetrahydrothienyl), (3) piperidinyl (e.g., 2-, 3- or 4-piperidinyl), (4) pyrrolidinyl (e.g., 2- or 3-pyrrolidinyl), (5) 2- or 3-piperizinyl, (6) 2- or 4-dioxanyl, (7) tetrahydopyranyl, and (8) substituted tetrahydropyranyl wherein said substituents are selected from hydroxy and hydroxyalkyl (e.g., hydroxymethyl), for example

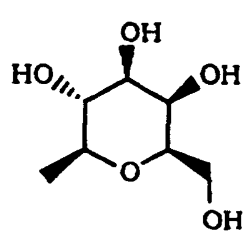
;

and

Ph-represents phenyl.

The following solvents and reagents are referred to herein by the abbreviations indicated: tetrahydrofuran (THF); isopropanol (iPrOH); ethanol (EtOH); methanol (MeOH); acetic acid (HOAc or AcOH); ethyl acetate (EtOAc); N,N-dimethylformamide (DMF); trifluoroacetic acid (TFA); trifluoroacetic anhydride (TFAA); 1-hydroxybenzo-triazole (HOBT); 1-(3-dimethylaminopropyl)-3-ethyl carbodiimide hydrochloride (DEC); diisobutylaluminum hydride (DIBAL); and 4-methylmorpholine (NMM).

Reference to the position of the substituents $R^1$, $R^2$, $R^3$, and $R^4$ is based on the numbered ring structure:

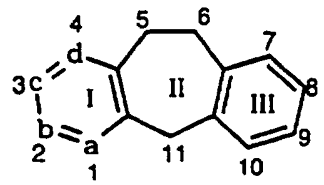

Those skilled in the art will also appreciate that the S and R stereochemistry at the C-11 bond are:

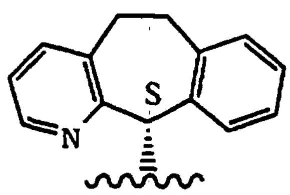

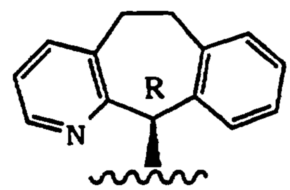

Compounds of Formula 1.0 include compounds wherein the bottom piperidinyl group is a 4- or 3-piperidinyl group, i.e.,

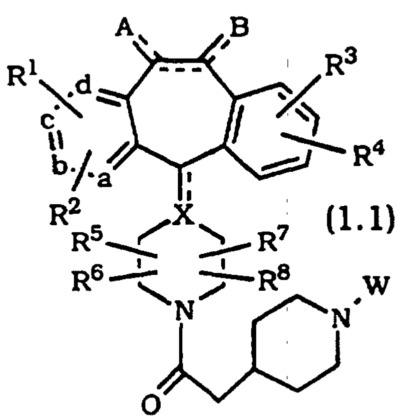

(1.1)

or

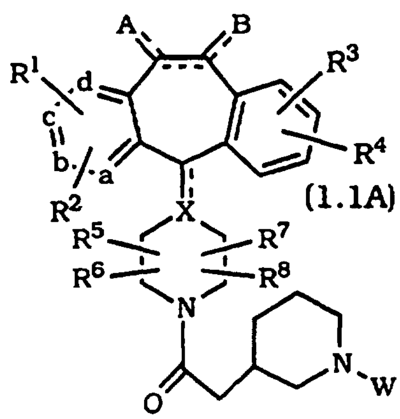

(1.1A)

Preferably, $R^1$, $R^3$ and $R^4$ are independently selected from Br and Cl.

Preferably, compounds of Formula 1.0 are represented by compounds of Formula 1.1:

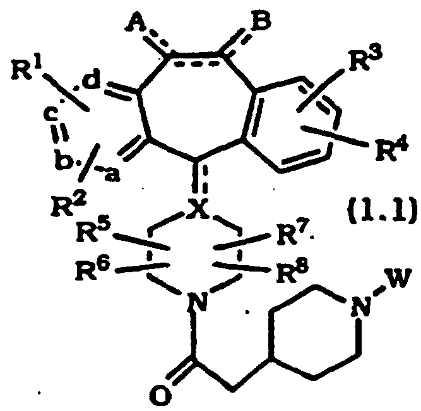

(1.1)

wherein all substituents are as defined for Formula 1.0.

Preferably, $R^2$ is H and $R^1$, $R^3$ and $R^4$ are halo: a is N and b, c and d are carbon; A and B are each $H_2$; the optional bond between C5 and C6 is absent; X is CH; and $R^5$, $R^6$, $R^7$ and $R^8$ are H. More preferably, $R^1$, $R^3$ and $R^4$ are independently selected from Br or Cl. Most preferably, $R^1$ is Br, and $R^3$ and $R^4$ are independently selected from Cl and Br.

More preferably, compounds of Formula 1.0 are represented by compounds of Formula 1.2 and Formula 1.3:

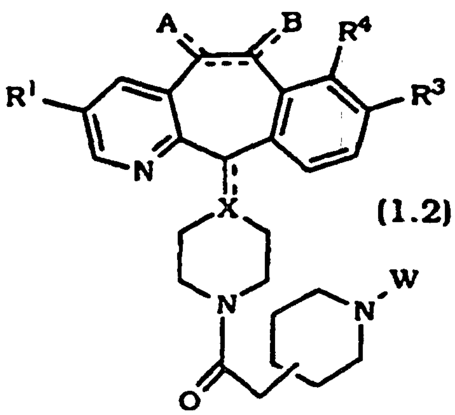

(1.2)

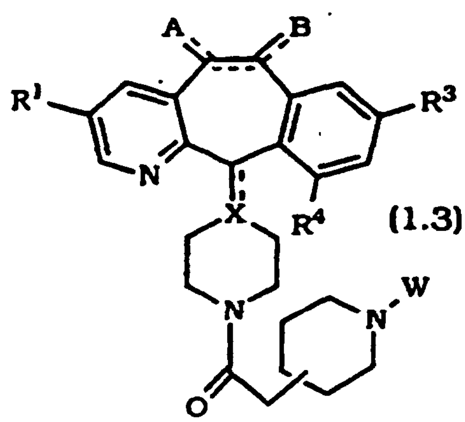

(1.3)

and most preferably, compounds of Formulas 1.4 and 1.5

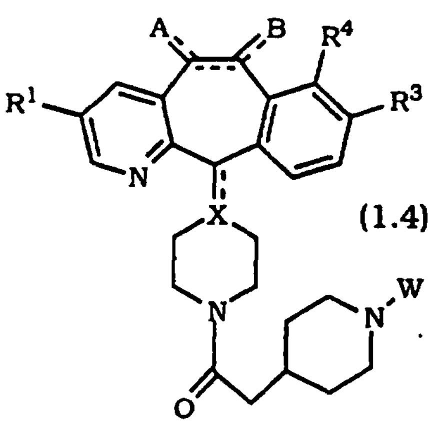

(1.4)

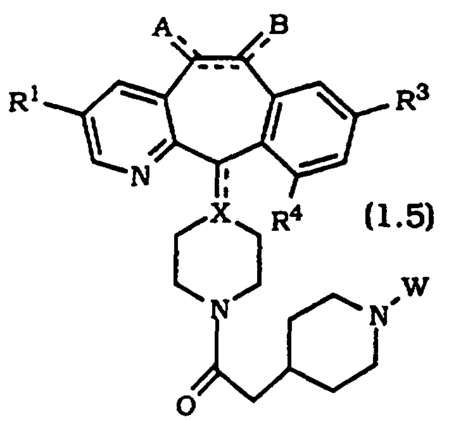

(1.5)

wherein $R^1$, $R^3$ and $R^4$ are each independently selected from halo, preferably, Br or Cl; and A, B, X and W are as defined for Formula 1.0. More preferably, A and B are each $H_2$; the optional bond between C5 and C6 is absent; and X is CH. Most preferably, $R^1$ is Br; $R^3$ and $R^4$ are independently Br or Cl, and still more preferably $R^3$ is Cl and $R^4$ is Br; A and B are each $H_2$; the optional bond between C5 and C6 is absent; X is CH; and $R^5$, $R^6$, $R^7$ and $R^8$ are H.

Examples of -C(O)$R^{12}$ substituents for W include groups wherein $R^{12}$ is selected from the group consisting of:

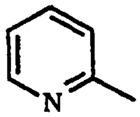, 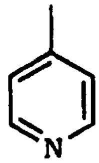, 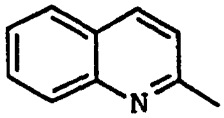, 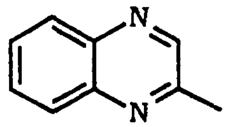, 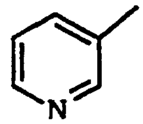,

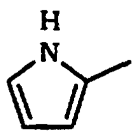, 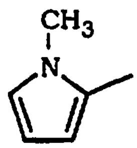, 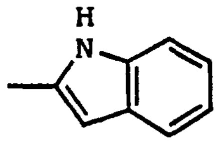, 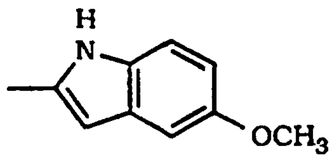,

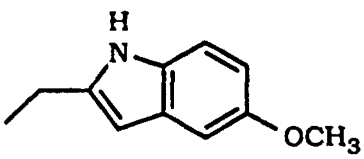, 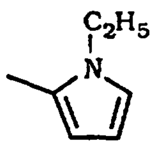, 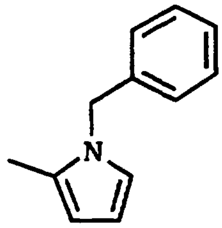, 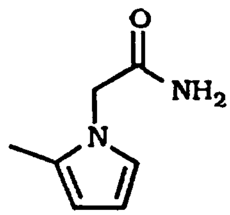,

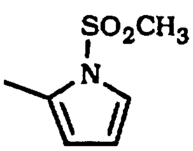, 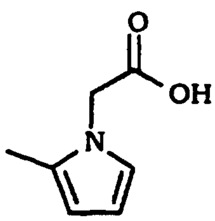, 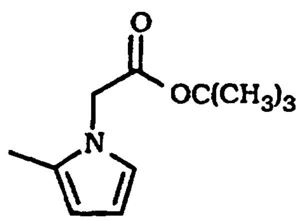, 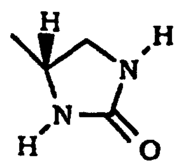,

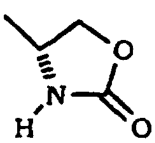, 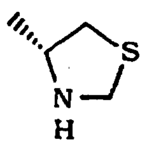, 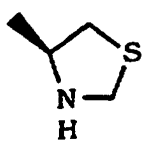, 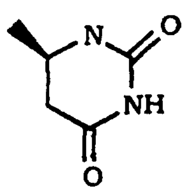, 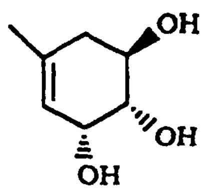,

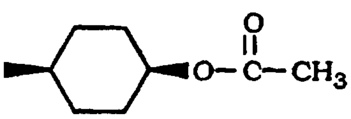, 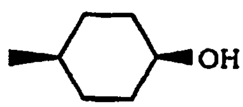,

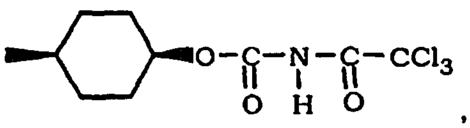, 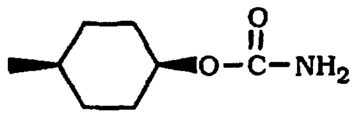, 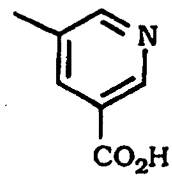,

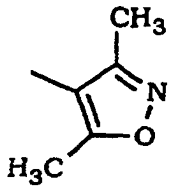, 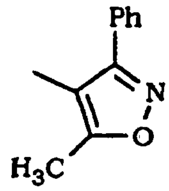, 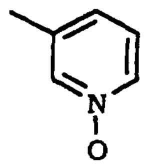, 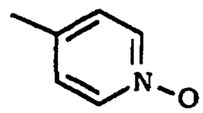, 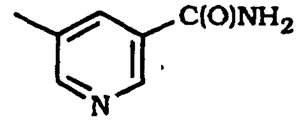,

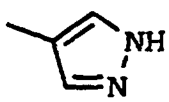, 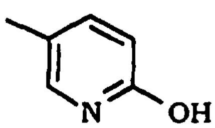, 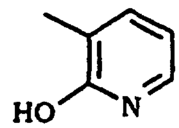, 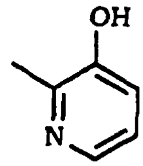 and 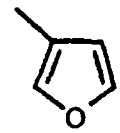.

Examples of imidates for substituent W include groups wherein $R^{13}$ is selected from the group consisting of: (1) CN; (2) H; (3) $-SO_2NR^{10}R^{15}$ wherein $R^{10}$ and $R^{15}$ are selected from the group consisting of: H and alkyl (e.g., methyl); (4) $-C(O)NR^{10}R^{15}$ wherein $R^{10}$ and $R^{15}$ are selected from the group consisting of: H and alkyl (e.g., methyl); (5) $-SO_2$-alkyl; and (6) -C(O)-aryl. Examples of imidates also include groups wherein $R^{14}$ is aryl (e.g., phenyl).

For example, imidates for substituent W include groups wherein $R^{13}$ is selected from the group consisting of: CN, - $C(O)NH_2$, H, $-SO_2NH_2$, $-SO_2NHCH_3$, $-SO_2N(CH_3)_2$, $-C(O)NHCH_3$, - $SO_2CH_3$ and $-C(O)C_6H_5$. Exampes of imidates also include groups wherein $R^{14}$ is phenyl; and $R^{13}$ is selected from the group consisting of: CN, $-C(O)NH_2$, H, $-SO_2NH_2$, $-SO_2NHCH_3$, - $SO_2N(CH_3)_2$, $-C(O)NH_2$, $-C(O)NHCH_3$, $-SO_2CH_3$ and $-C(O)C_6H_5$.

Examples of imidamidos for substituent W include groups wherein $R^{13}$ is selected from the group consisting of: (1) CN; (2) H; (3) $-OR^{10}$; (4) $-C(O)NR^{10}C(O)NR^{10}R^{15}$ wherein $R^{10}$ and $R^{15}$ are H; (5) $-SO_2NR^{10}R^{15}$ wherein $R^{10}$ and $R^{15}$ are independently selected from the group consisting of: H and alkyl (e.g., methyl), (6) $-C(O)NR^{10}R^{15}$ wherein $R^{10}$ and $R^{15}$ are independently selected from the group consisting of: H and alkyl (e.g., methyl); (7) $-SO_2$-alkyl; and (8) -C(O)-aryl. Examples of the imidamidos also include groups wherein $R^{10}$ and $R^{15}$ are selected from the group consisting of: H and alkyl (e.g., methyl); and wherein $R^{16}$ is selected from the group consisting of: H and heteroaralkyl (e.g., 3-pyridylmethyl).

For example, imidamidos for substituent W include groups wherein $R^{13}$ is selected from the group consisting of: CN, H, $-OCH_3$, -OH, $-C(O)NHC(O)NH_2$, $-SO_2NH_2$, $-SO_2NHCH_3$, $-SO_2N(CH_3)_2$, $-C(O)NH_2$, $-C(O)NHCH_3$, $-SO_2CH_3$ and $-C(O)C_6H_5$. Examples of imidamidos also include groups wherein $R^{10}$ and $R^{16}$ are selected from the group consisting of: H and

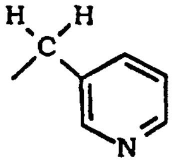

(i.e., 3-pyridylmethyl).

Examples of the imidamido substituents additionally include groups wherein: $R^{10}$ and $R^{16}$ are selected from the group consisting of: H and 3-pyridylmethyl; and $R^{13}$ is selected from the group consisting of: CN, H, $-OCH_3$, -OH, $-C(O)NHC(O)NH_2$, - $SO_2NH_2$, $-SO_2NHCH_3$, $-SO_2N(CH_3)_2$, $-C(O)NH_2$, $-C(O)NHCH_3$, - $SO_2CH_3$ and $-C(O)C_6H_5$.

In addition, examples of the imidamido substituents additionally include groups wherein: (1) $R^{13}$ and $R^{10}$ are H, and $R^{16}$ is 3-pyridylmethyl; and (2) $R^{10}$ and $R^{16}$ are H, and $R^{13}$ is selected from the group consisting of: CN, H,

$-OCH_3$, -OH, -C(O)NHC(O)$NH_2$, $-SO_2NH_2$, -and $-C(O)NHCH_3$, $-SO_2CH_3$ and $-C(O)C_6H_5$.

Examples of 1-amino-2-nitroethylene derivatives for substituent W include groups wherein $R^{10}$ is alkyl, e.g., methyl.

Compounds of Formulas 1.2A and 1.3A:

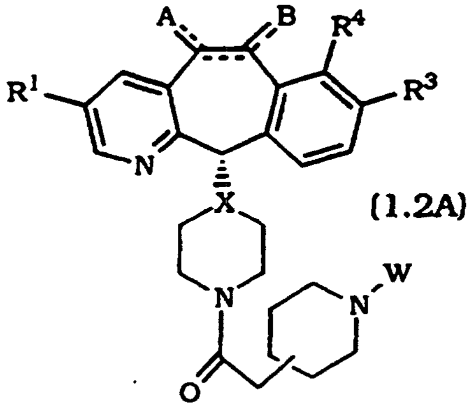

(1.2A)

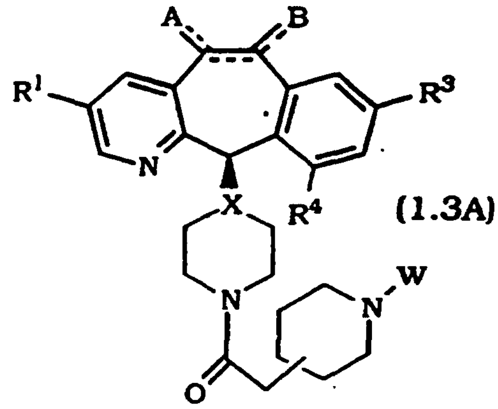

(1.3A)

are preferred when X is CH or N, and $R^1$, $R^3$ and $R^4$ are halo.

The preferred compounds of this invention are represented by the compounds of Formulas:

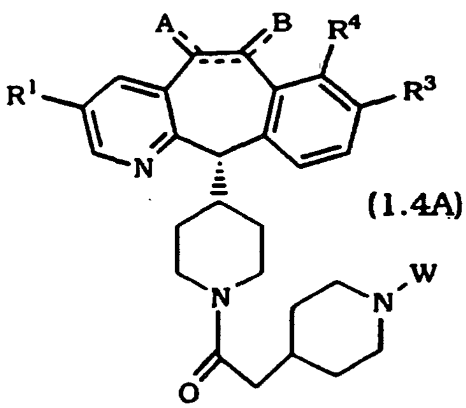

(1.4A)

and

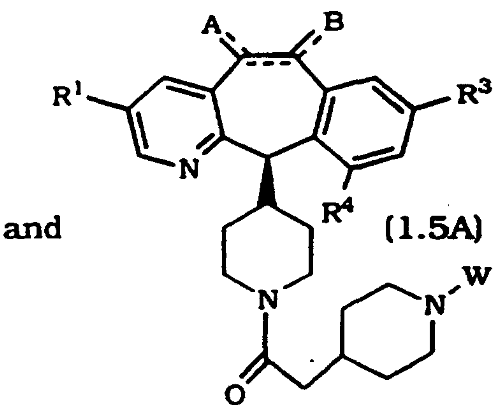

(1.5A)

wherein $R^1$, $R^3$ and $R^4$ are halo and the remaining substituents are as defined above, with the compounds of Formula 1.5A being more preferred.

Representative compounds of Formula 1.0 wherein W is an imidate include:

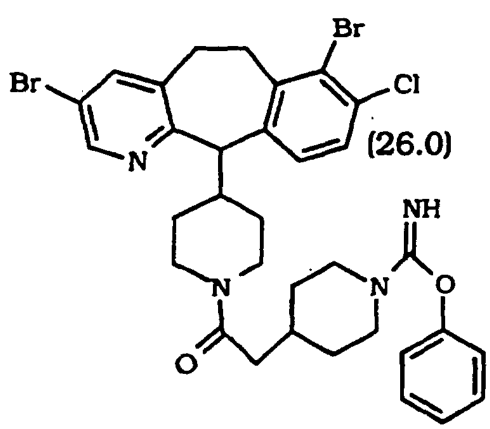

(26.0)

and

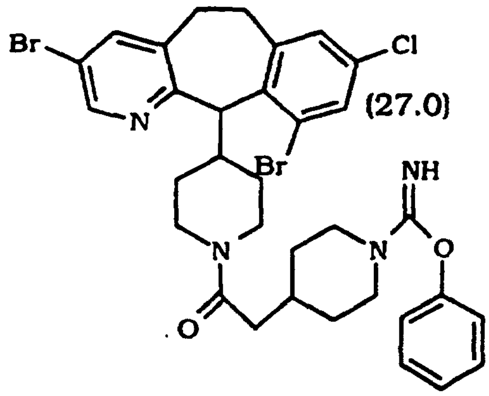

(27.0)

.

Representative compounds of formula 1.0 wherein W is an imidamido include:

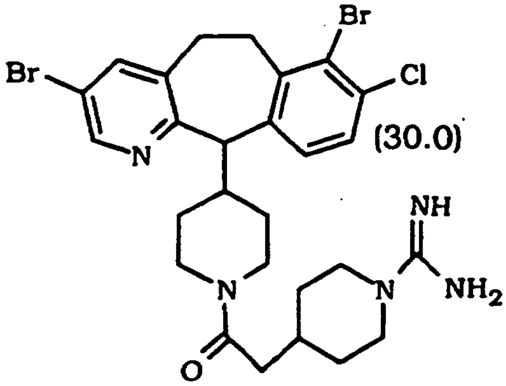

(30.0)

;

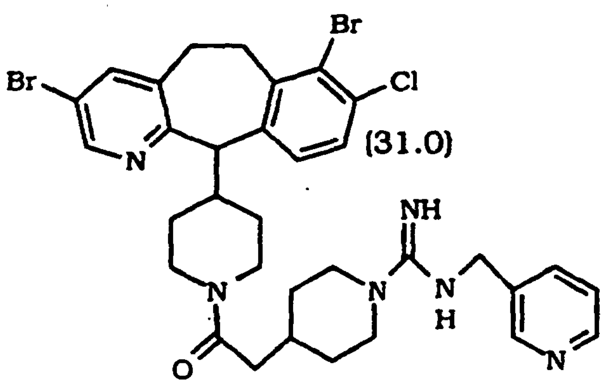

(31.0)

;

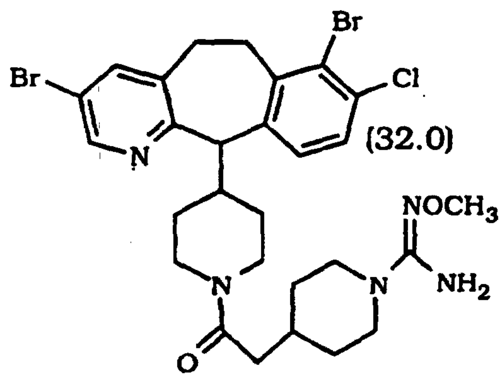

(32.0)

and

Representative compounds of Formula 1.0 wherein W is cyano include:

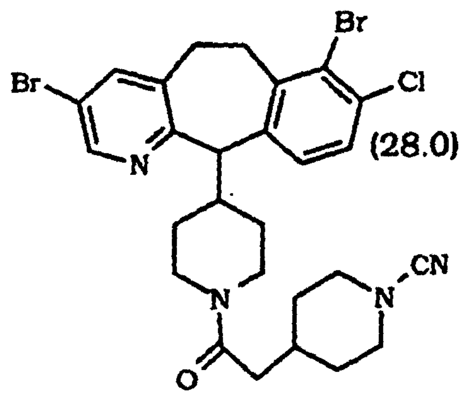

(28.0)

;

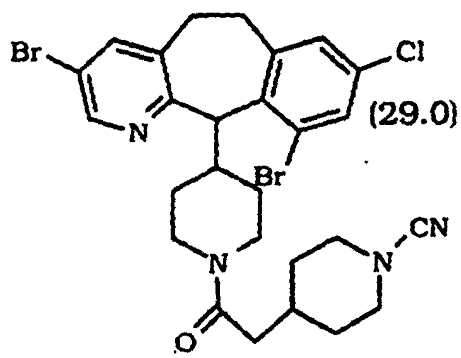

(29.0)

and

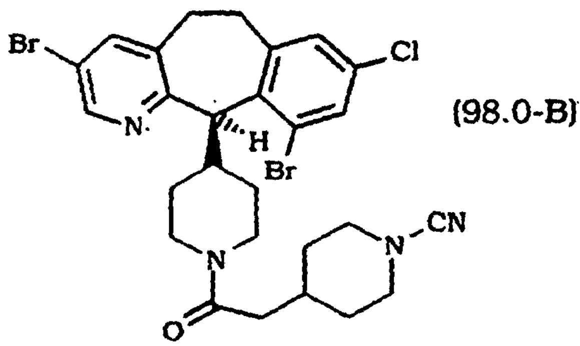

(98.0-B)

Representative compounds of Formula 1.0 wherein W is a 1-amino-2-nitroethylene derivative include:

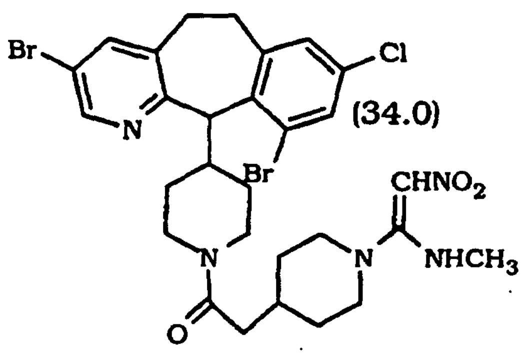

(34.0)

Compounds of Formula 1.0 wherein W is a -C(O)$R^{12}$ group include:

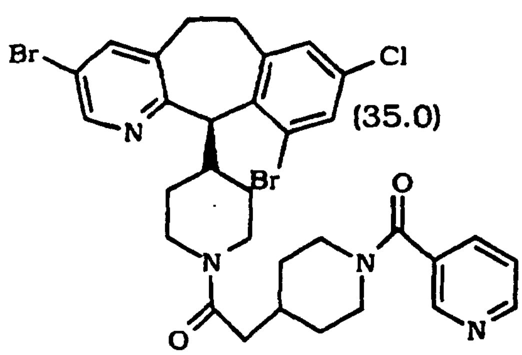

(35.0)

;

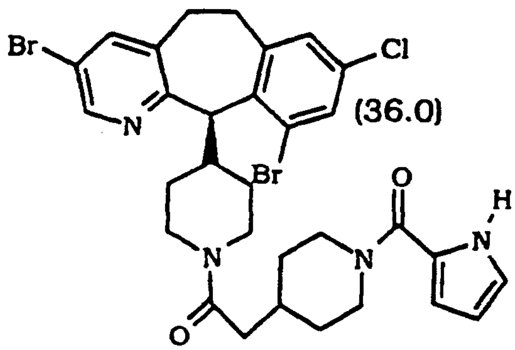

(36.0)

;

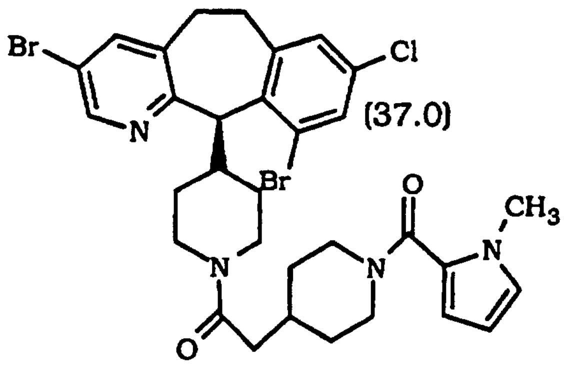

(37.0)

;

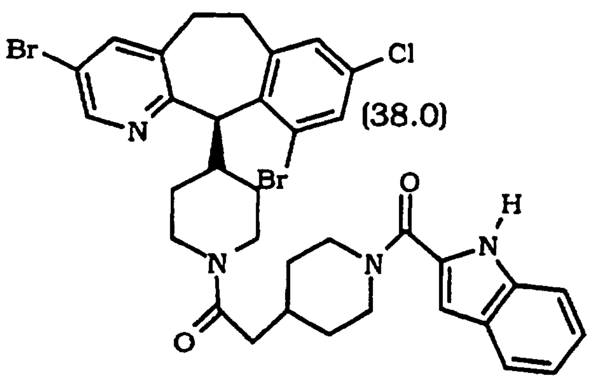

(38.0)

;

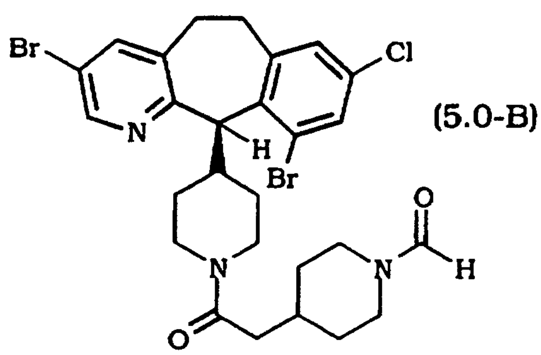

(5.0-B)

;

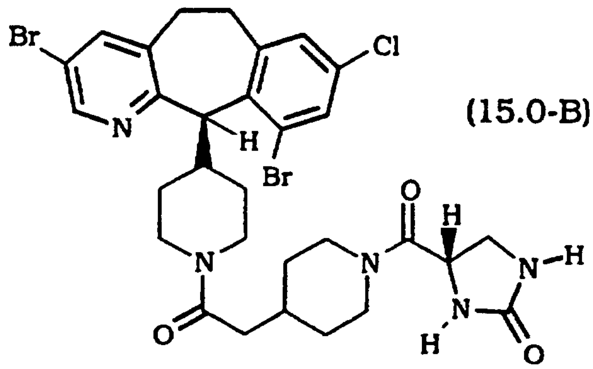

(15.0-B)

;

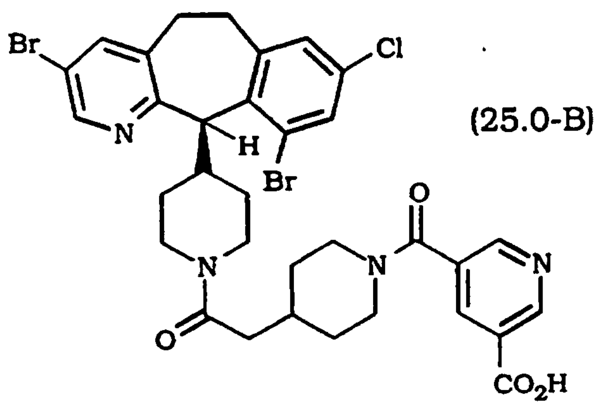

(25.0-B)

;

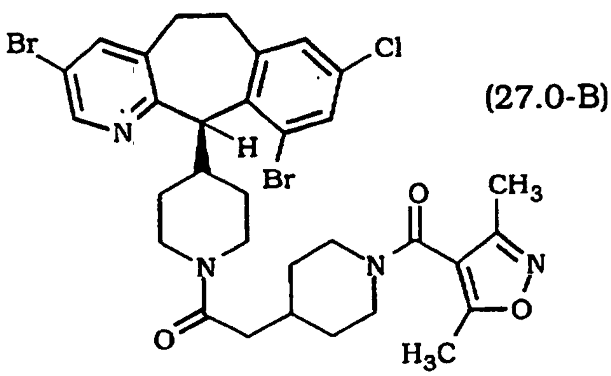

(27.0-B)

;

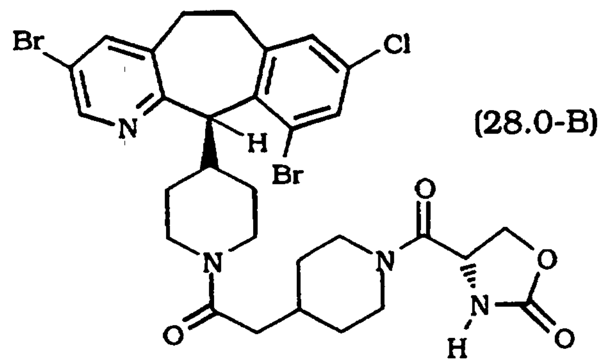

(28.0-B)

;

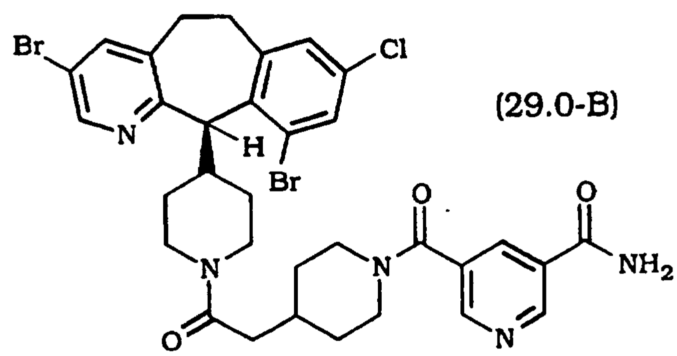

(29.0-B)

;

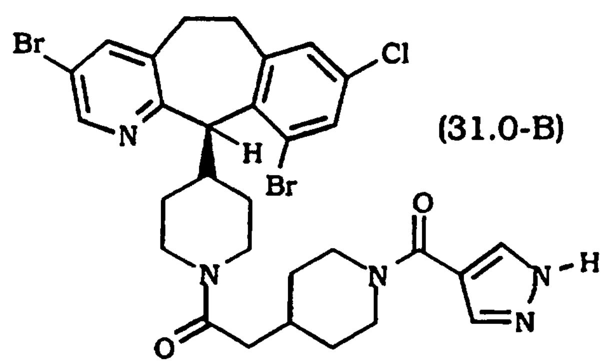

(31.0-B)

;

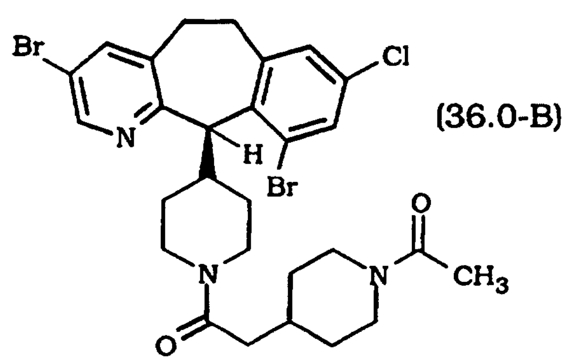

(36.0-B)

;

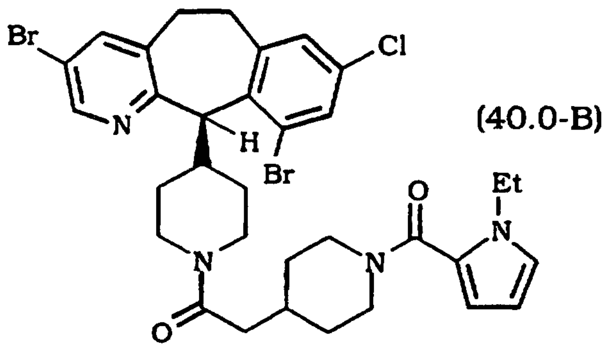

(40.0-B)

;

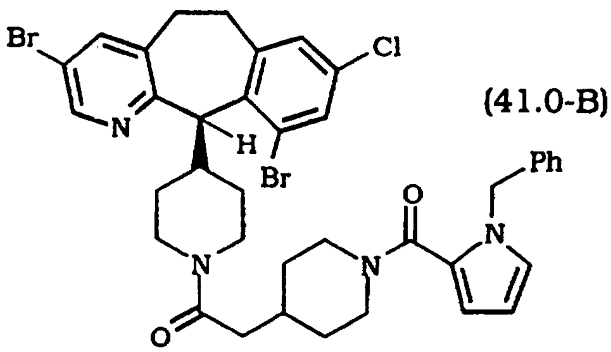

(41.0-B)

;

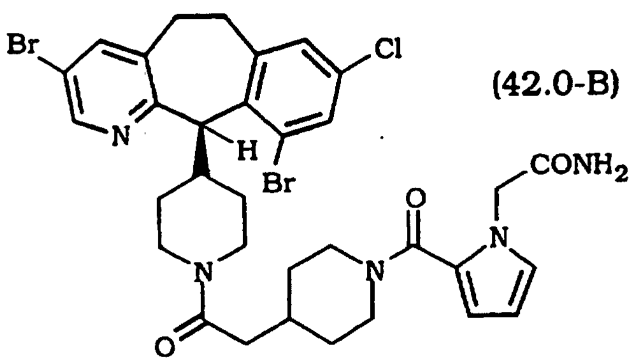

(42.0-B)

;

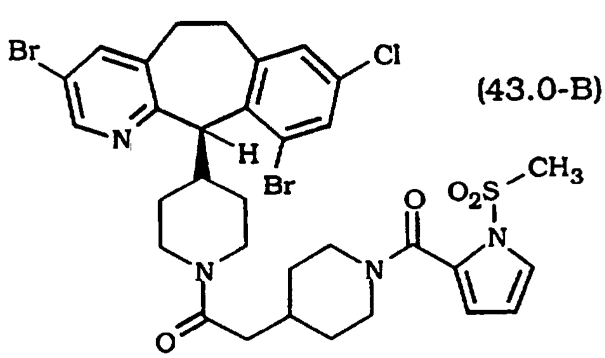

(43.0-B)

;

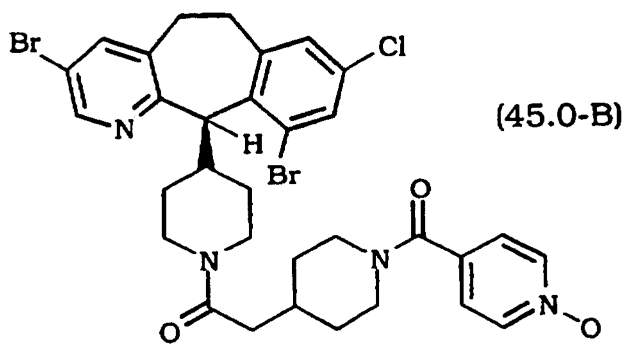

(45.0-B)

;

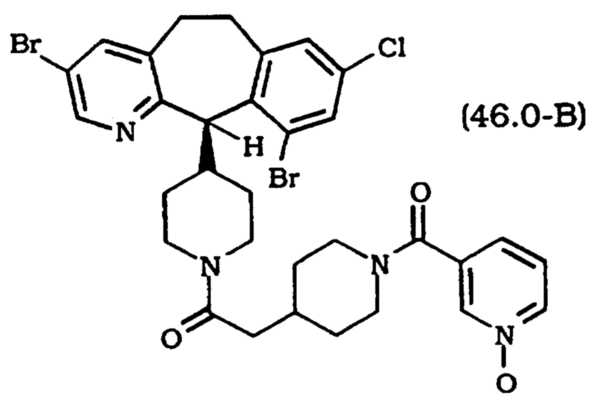

(46.0-B)

;

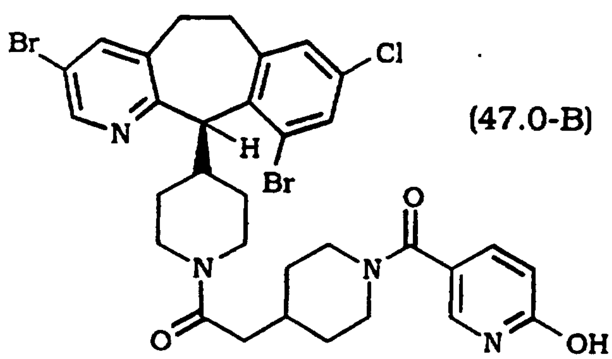

(47.0-B)

;

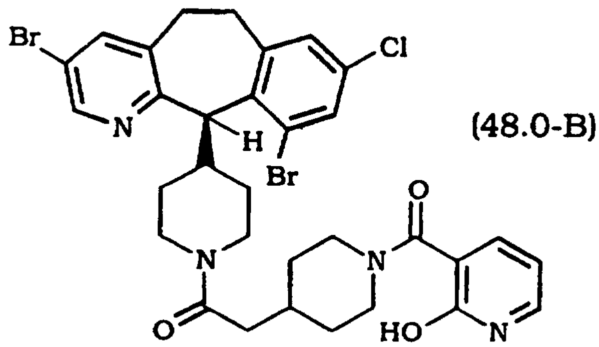

(48.0-B)

;

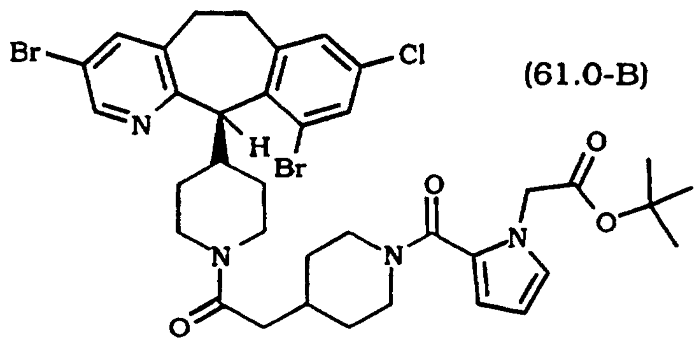

(61.0-B)

;

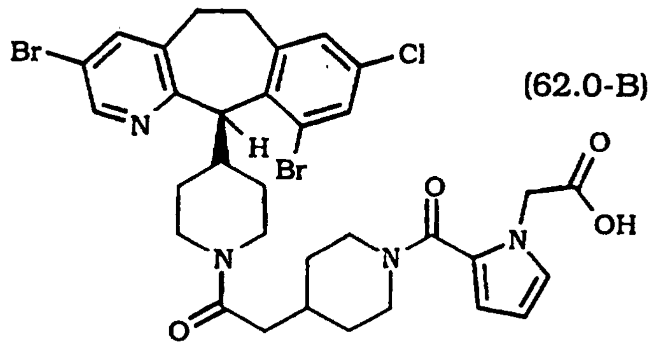

(62.0-B)

;

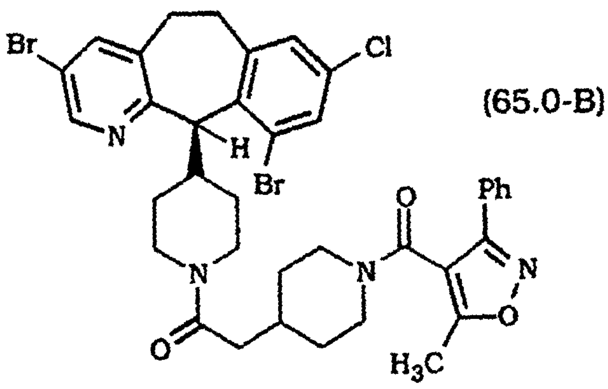

(65.0-B)

;

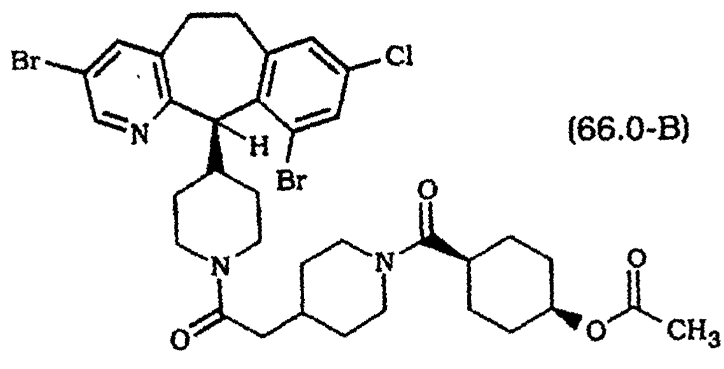

(66.0-B)

;

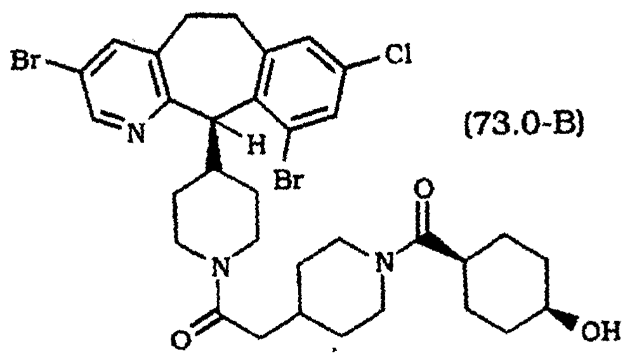

(73.0-B)

;

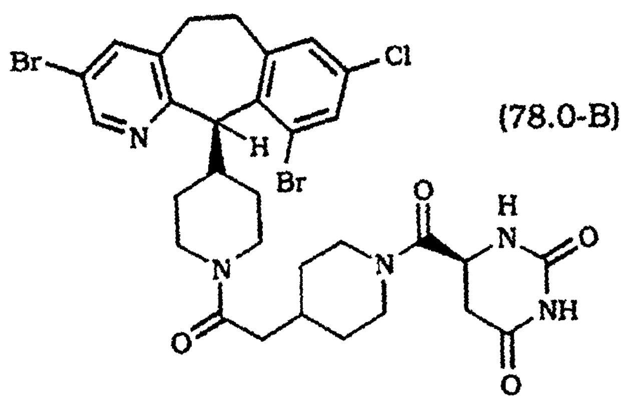

(78.0-B)

;

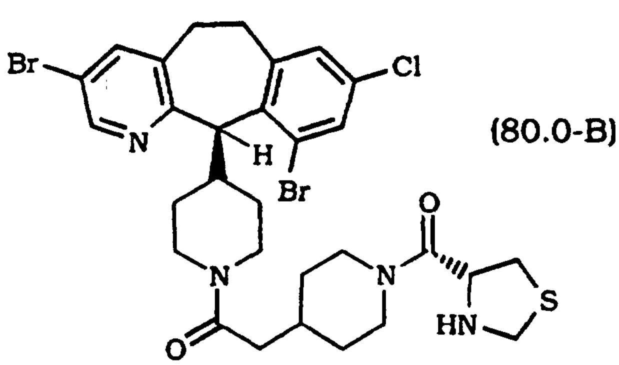

(80.0-B)

;

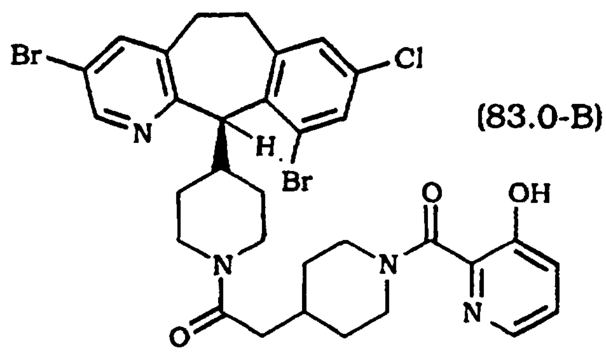

(83.0-B)

;

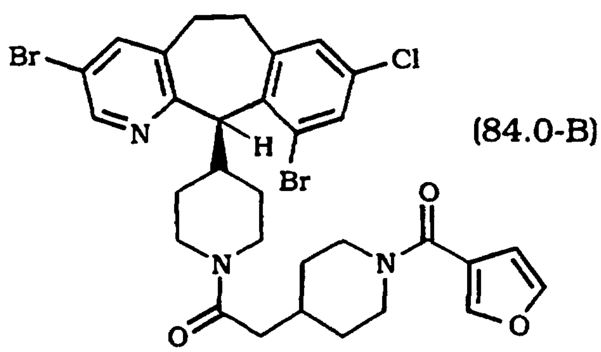

(84.0-B)

;

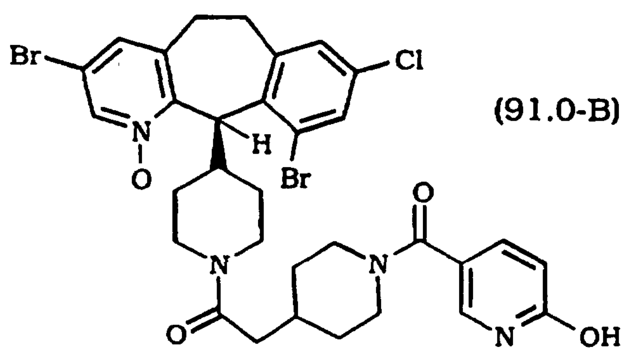

(91.0-B)

;

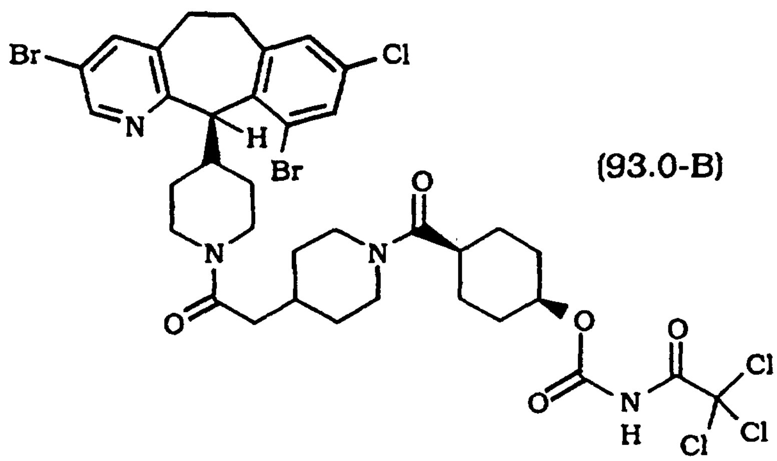

(93.0-B)

;

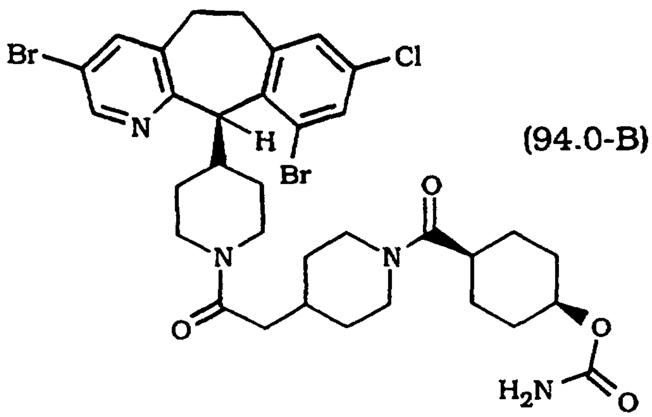

(94.0-B)

;

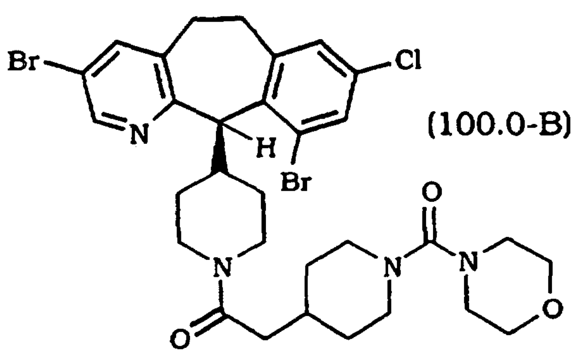

(100.0-B)

;

and

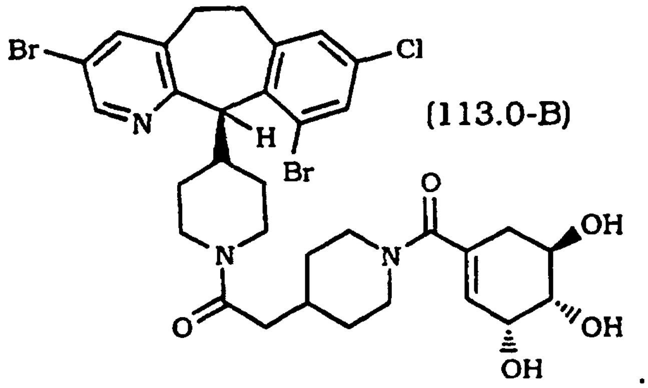

(113.0-B)

Compounds of Formula 1.0 also include a compound of the formula:

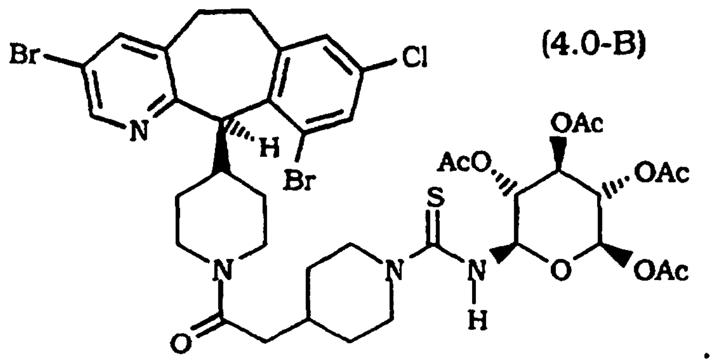

(4.0-B)

The compounds of this invention also include the 1-N-oxides--i.e, for example, compounds of the the formula:

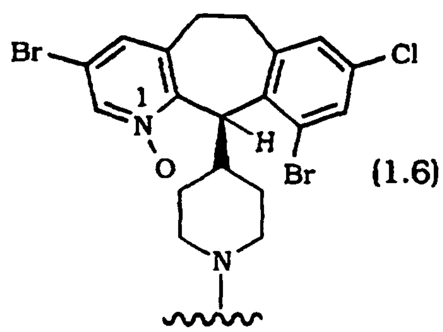

(1.6)

wherein ∿∿∿ represents the remainder of the compound, or pharmaceutically acceptable salts or solvates thereof.

Optical rotation of the compounds ((+)- or (-)-) are measured in methanol or ethanol at 25°C.

This invention includes the above compounds in the amorphous state or in the cyrstalline state.

Lines drawn into the ring systems indicate that the indicated bond may be attached to any of the substitutable ring carbon atoms.

Certain compounds of the present invention may exist in different isomeric forms (e.g., enantiomers or diastereoisomers) including atropisomers (i.e., compounds wherein the 7-membered ring is in a fixed conformation such that the 11-carbon atom is positioned above or below the plane of the fused beznene rings due to the presence of a 10-bromo substituent). The invention contemplates all such isomers both in pure form and in admixture, including racemic mixtures. Enol forms are also included.

Certain tricyclic compounds will be acidic in nature, e.g. those compounds which possess a carboxyl or phenolic hydroxyl group. These compounds may form pharmaceutically acceptable salts. Examples of such salts may

include sodium, potassium, calcium, aluminum, gold and silver salts. Also contemplated are salts formed with pharmaceutically acceptable amines such as ammonia, alkyl amines, hydroxyalkylamines, N-methylglucamine and the like.

Certain basic tricyclic compounds also form pharmaceutically acceptable salts, e.g., acid addition salts. For example, the pyrido-nitrogen atoms may form salts with strong acid, while compounds having basic substituents such as amino groups also form salts with weaker acids. Examples of suitable acids for salt formation are hydrochloric, sulfuric, phosphoric, acetic, citric, oxalic, malonic, salicylic, malic, fumaric, succinic, ascorbic, maleic, methanesulfonic and other mineral and carboxylic acids well known to those in the art. The salts are prepared by contacting the free base form with a sufficient amount of the desired acid to produce a salt in the conventional manner. The free base forms may be regenerated by treating the salt with a suitable dilute aqueous base solution such as dilute aqueous NaOH, potassium carbonate, ammonia and sodium bicarbonate. The free base forms differ from their respective salt forms somewhat in certain physical properties, such as solubility in polar solvents, but the acid and base salts are otherwise equivalent to their respective free base forms for purposes of the invention.

All such acid and base salts are intended to be pharmaceutically acceptable salts within the scope of the invention and all acid and base salts are considered equivalent to the free forms of the corresponding compounds for purposes of the invention.

Compounds of the invention may be prepared according to the procedures described in WO 95/10516 published April 20, 1995, Application Serial No. 08/410,187 filed March 24, 1995, Application Serial No. 08/577,951 filed December 22, 1995 (now abandoned), Application Serial No. 08/615,760 filed March 13, 1996 (now abandoned), WO 97/23478 published July 3, 1997 which discloses the subject matter of Serial No. 08/577,951 and 08/615,760, Application Serial No. 08/713297 filed September 13, 1996, and Application Serial No. 08/877,453 filed June 17, 1997, and according to the procedures described below.

Compounds of the invention can be prepared by reacting a compound of the formula:

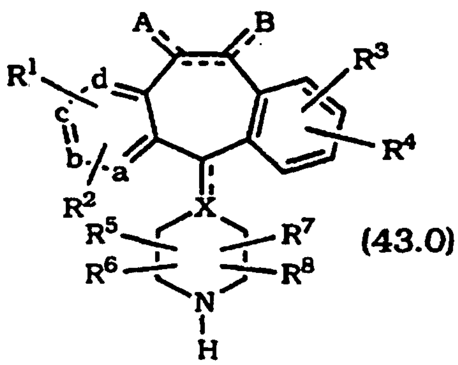

(43.0)

wherein all substituents are as defined for Formula 1.0, with the appropriate protected piperidinyl acetic acid (e.g., 1-N-t-butoxycarbonylpiperidinyl acetic acid) together with DEC/HOBT/NMM in DMF at 25°C for about 18 hours to produce a compound of the formula:

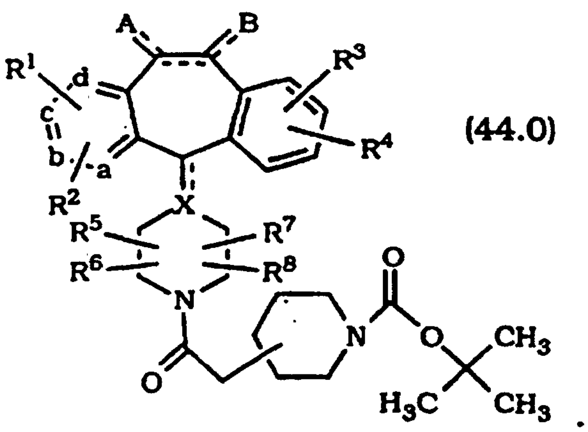

(44.0)

The compound of Formula 44.0 is then reacted either with TFA or 10% sulfuric acid in dioxane and methanol, followed by NaOH to produce the compound of formula:

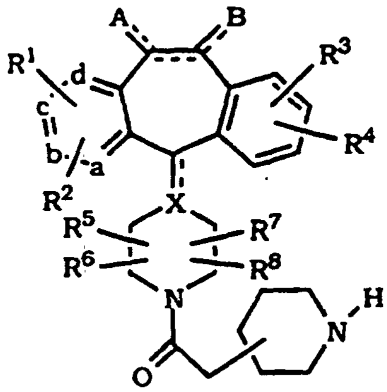

(45.0)

For example, the compound of formula

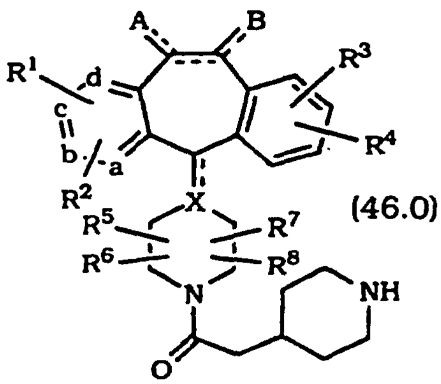

(46.0)

can be prepared by reaction of a compound of Formula 43.0 with 1-N-t-butoxy-carbonylpiperidinyl-4-acetic acid as described above.

For Example, compounds of Formula 46.0 include:

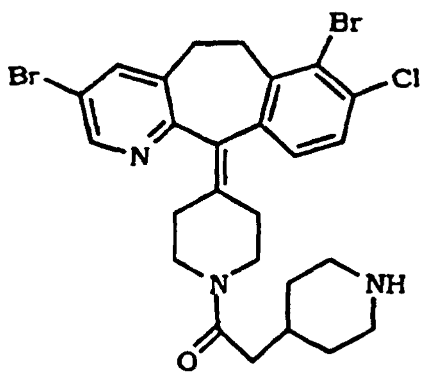

;

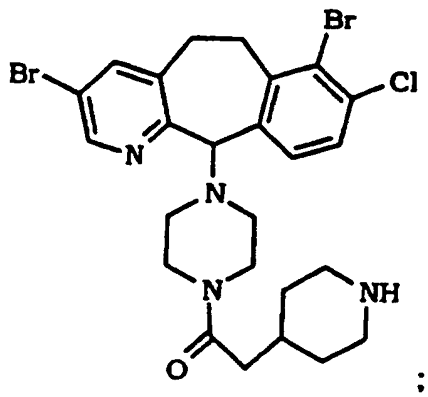

;

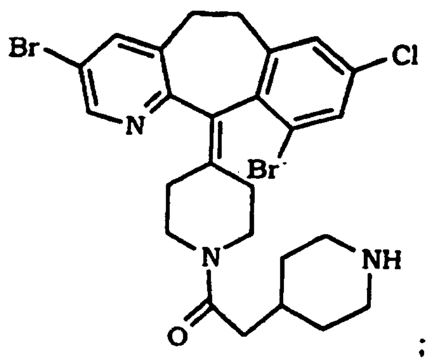

;

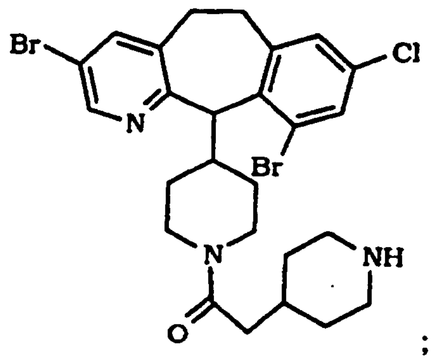

;

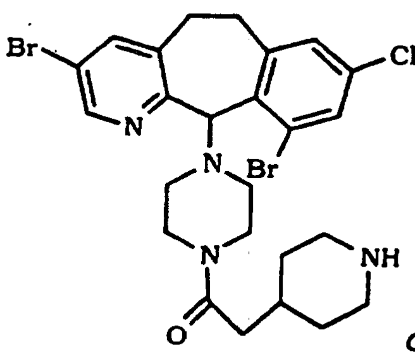

and

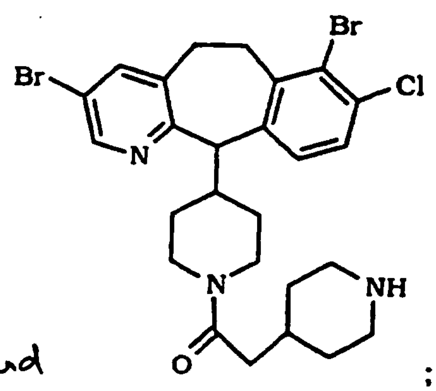

;

The preparation of these compounds are described in Preparative Examples 3, 4, 5, 6, 7, 8, 9, 10, 11, 12 and 13, respectively, below.

The compounds of the invention can be prepared by reacting a compound of the formula:

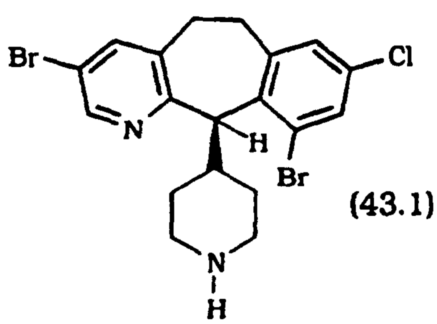

(43.1)

with the appropriate protected piperidinyl acetic acid (e.g., 1-N-t-butoxycarbonylpiperidinyl acetic acid together with DEC/HOBT/NMM in DMF at about 25°C for about 18 hours to produce a compound of the formula:

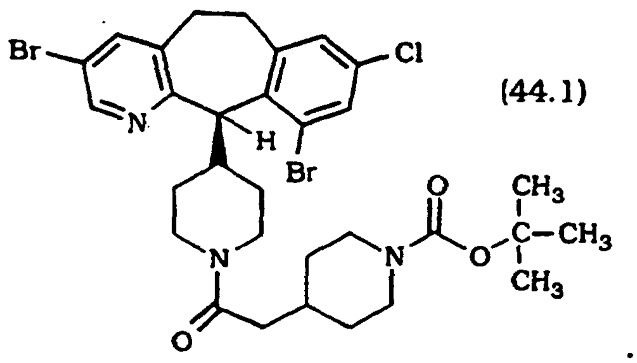

(44.1)

The compound of Formula 44.1 is then reacted either with TFA or 10% sulfuric acid in dioxane and methanol followed by NaOH to produce the compound of Formula 46.1

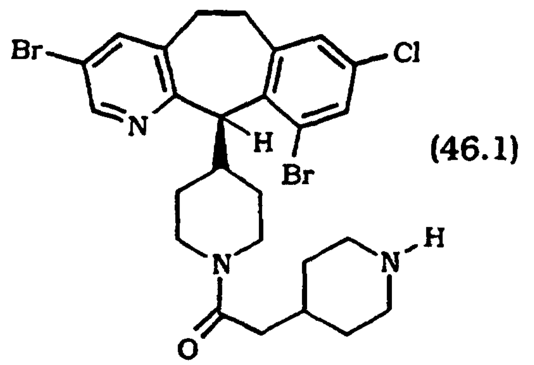

(46.1)

The amide compounds of this invention, represented by Formula 1.7

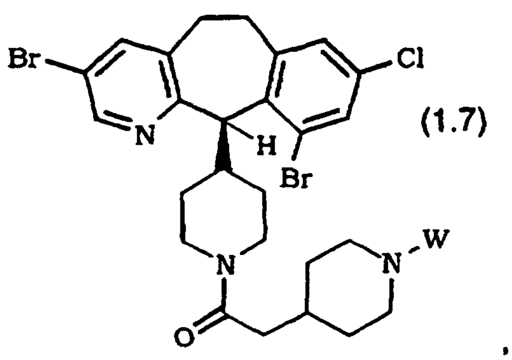

(1.7)

can be prepared by reacting the compound of Formula 46.1 with the appropriate carboxylic acid in the presence of a coupling agent such as DEC and HOBT in dimethylformamide. Alternatively, the compound of Formula 46.1 can be reacted with an acid chloride or anhydride in a solvent such as pyridine.

Compounds having an 1-N-O group:

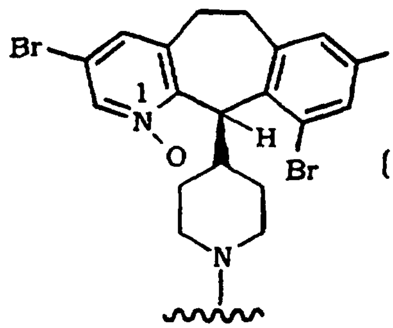

(1.6)

can be prepared from the corresponding pyridyl compounds:

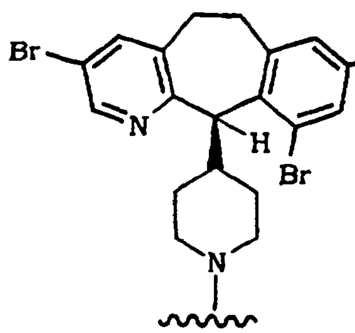

(1.8)

by oxidation with meta-chloroperoxybenzoic acid. This reaction is conducted in a suitable organic solvent, e.g., dichloromethane (usually anhydrous) or methylene chloride, at a suitable temperature, to produce the compounds of the invention having the N-O substituent at position 1 of Ring I of the tricyclic ring system.

Generally, the organic solvent solution of the starting tricyclic reactant is cooled to about 0°C before the m-chloroperoxybenzoic acid is added. The reaction is then allowed to warm to room temperature during the reaction period. The desired product can be recovered by standard separation means. For example, the reaction mixture can be washed with an aqueous solution of a suitable base, e.g., saturated sodium bicarbonate or NaOH (e.g., 1N NaOH), and then dried over anhydrous magnesium sulfate. The solution containing the product can be concentrated in vacuo. The product can be purified by standard means, e.g., by chromatography using silica gel (e.g., flash column chromatography).

Alternatively, N-O compounds can be made from intermediate:

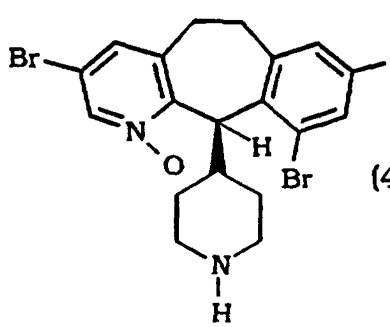

(43.1A)

by the above oxidation procedure with m-chloroperoxybenzoic acid and

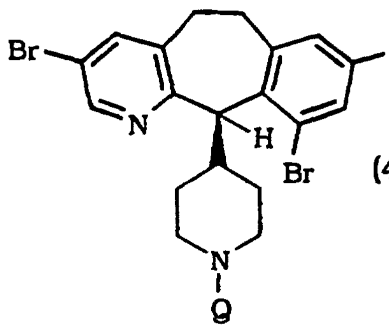

(43.1B)

wherein Q is a protecting group, e.g., BOC. After oxidation the protecting group is removed by techniques well known in the art. The N-O intermediate are then reacted further to produce the compounds of the invention.

Compounds of Formula 43.0 include the compound of Formula 43.1:

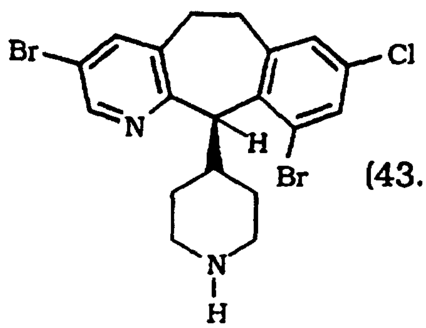

(43.1)

The compound of Formula 43.1 is prepared by methods known in the art, for example by methods disclosed in WO 95/10516, in U.S. 5,151,423 and those described below. The above intermediate compound can also be prepared by a procedure comprising the following steps:

(a) reacting an amide of the formula

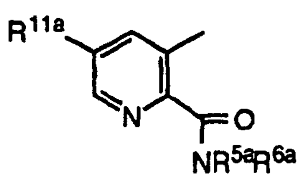

wherein $R^{11a}$ is Br, $R^{5a}$ is hydrogen and $R^{6a}$ is $C_1$-$C_6$ alkyl, aryl or heteroaryl; $R^{5a}$ is $C_1$-$C_6$ alkyl, aryl or heteroaryl and $R^{6a}$ is hydrogen; $R^{5a}$ and $R^{6a}$ are independently selected from the group consisting of $C_1$-$C_6$ alkyl and aryl; or $R^{5a}$ and $R^{6a}$, together with the nitrogen to which they are attached, form a ring comprising 4 to 6 carbon atoms or comprising 3 to 5 carbon atoms and one hetero moiety selected from the group consisting of -O- and -$NR^{9a}$-, wherein $R^{9a}$ is H, $C_1$-$C_6$ alkyl or phenyl;

with a compound of the formula

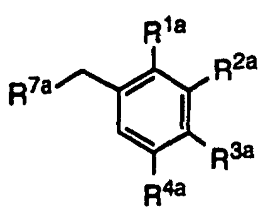

wherein $R^{1a}$, $R^{2a}$, $R^{3a}$ and $R^{4a}$ are are independently selected from the group consisting of hydrogen and halo

and $R^{7a}$ is Cl or Br, in the presence of a strong base to obtain a compound of the formula

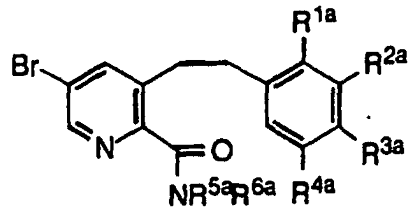

(b) reacting a compound of step (a) with

(i) $POCl_3$ to obtain a cyano compound of the formula

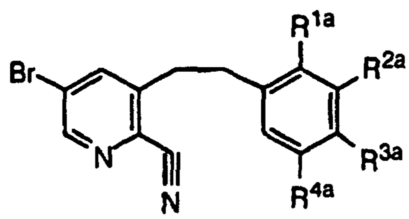

;

or
(ii) DIBALH to obtain an aldehyde of the formula

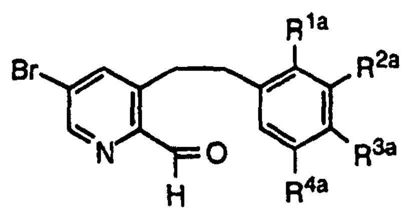

;

(c) reacting the cyano compound or the aldehyde with a piperidine derivative of the formula

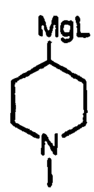

wherein L is a leaving group selected from the group consisting of Cl and Br, to obtain a ketone or an alcohol of the formula below, respectively:

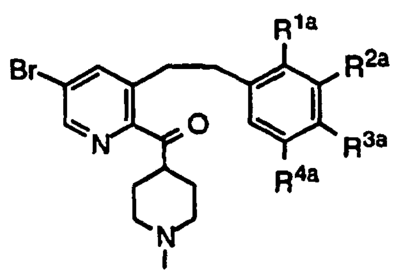

or

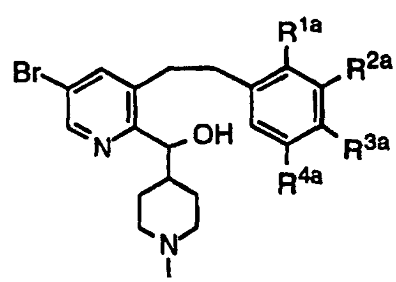

(d)(i) cyclizing the ketone with $CF_3SO_3H$ to obtain a compound of Formula 13.0a wherein the dotted line represents a double bond; or

(d)(ii) cyclizing the alcohol with polyphosphoric acid to obtain an Intermediate compound wherein the dotted line represents a single bond.

Methods for preparing the Intermediate compounds disclosed in WO 95/10516, U.S. 5,151,423 and described below employ a tricyclic ketone intermediate. Such intermediates of the formula

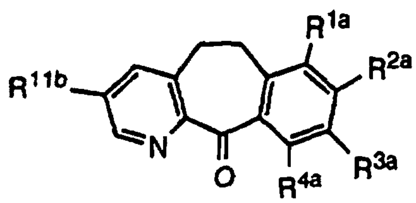

wherein $R^{11b}$, $R^{1a}$, $R^{2a}$, $R^{3a}$ and $R^{4a}$ are independently selected from the group consisting of hydrogen and halo, can be prepared by the following process comprising ;

(a) reacting a compound of the formula

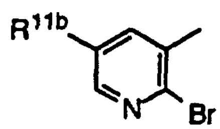

(i) with an amine of the formula $NHR^{5a}R^{6a}$, wherein $R^{5a}$ and $R^{6a}$ are as defined in the process above; in the presence of a palladium catalyst and carbon monoxide to obtain an amide of the formula:

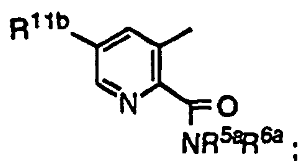

or

(ii) with an alcohol of the formula $R^{10a}OH$, wherein $R^{10a}$ is $C_1$-$C_6$ lower alkyl or $C_3$-$C_6$ cycloalkyl, in the presence of a palladium catalyst and carbon monoxide to obtain the ester of the formula

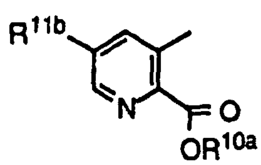

followed by reacting the ester with an amine of formula $NHR^{5a}R^{6a}$ to obtain the amide;

(b) reacting the amide with an iodo-substituted benzyl compound of the formula

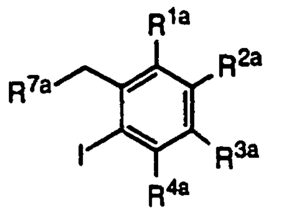

wherein $R^{1a}$, $R^{2a}$, $R^{3a}$, $R^{4a}$ and $R^{7a}$ are as defined above, in the presence of a strong base to obtain a compound of the formula

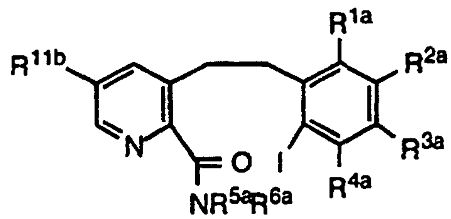

;

and

(c) cyclizing a compound of step (b) with a reagent of the formula $R^{8a}MgL$, wherein $R^{8a}$ is $C_1$-$C_8$ alkyl, aryl or heteroaryl and L is Br or Cl, provided that prior to cyclization, compounds wherein $R^{5a}$ or $R^{6a}$ is hydrogen are reacted with a suitable N-protecting group.

(+)-Isomers of compounds of Formula 43.2

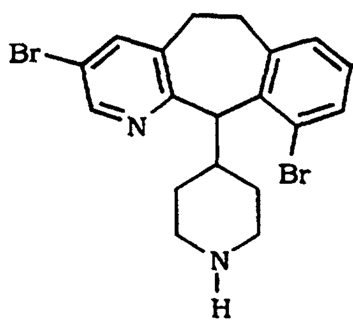

(43.2)

can be prepared with high enantioselectivity by using a process comprising enzyme catalyzed transesterification. Preferably, a racemic compound of Formula 43.3

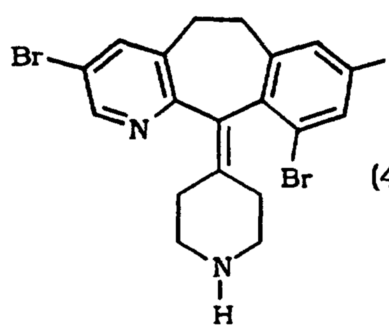

(43.3)

is reacted with an enzyme such as Toyobo LIP-300 and an acylating agent such as trifluoroethly isobutyrate; the resultant (+)-amide is then isolated from the (-)-enantiomeric amine by techniques well known in the art, and then the (+)-amide is hydrolyzed, for example by refluxing with an acid such as $H_2SO_4$, and the resulting compound is then reduced with DIBAL by techniques well known in the art to obtain the corresponding optically enriched (+)-isomer of Formula 43.2. Alternatively, a racemic compound of Formula 43.3, is first reduced to the corresponding

racemic compound of Formula 43.2 and then treated with the enzyme (Toyobo LIP-300) and acylating agent as described above to obtain the (+)-amide, which is hydrolyzed to obtain the optically enriched (+)-isomer.

Those skilled in the art will appreciate that compounds of Formula 1.0 having other $R^1$, $R^2$, $R^3$, and $R^4$ substituents may be made by the above enzyme process.

To produce compounds of Formula 1.0 wherein W is an imidate, a compound of Formula 45.0 or 46.0 is reacted with

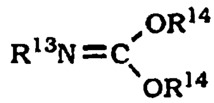

in the presence of a suitable solvent (e.g., isopropanol) and at a suitable temperature (e.g., about 80°C) for a suitable period of time (e.g., about 24 hours).

For example, a compound of Formula 46.0, wherein X is N, can be reacted with

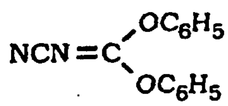

in isopropanol at 80°C for 24 hours to produce a compound of Formula 1.0 wherein $R^{13}$ is CN and $R^{14}$ is phenyl. Similarly, reaction with

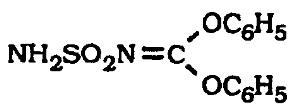

(M. Haake and B. Schummelfeder, Synthesis, pp. 753-758, September 1991) can produce compounds of Formula 1.0 wherein $R^{13}$ is $-SO_2NH_2$ and $R^{14}$ is phenyl.

Compounds of Formula 1.0 wherein $R^{13}$ is H and $R^{14}$ is phenyl can be produced by reacting a compound of Formula 45.0 or 46.0 with (e.g., wherein X is CH) with $CNOC_6H_5$ in diisopropylethylamine according to the procedure disclosed in Zweifel et al., Synthesis, p. 150 (1980).

Compounds of Formula 1.0 wherein W is -CN can be prepared by reacting a compound of Formula 1.0, wherein W is an imidate, with a strong base, such as NaH, in a suitable solvent, such as dichloromethane. For example, compounds of Formula 1.0 wherein W is an imidate and $R^{13}$ is H and $R^{14}$ is phenyl and X is CH can be reacted with NaH in dichloromethane to produce compounds of Formula 1.0 wherein W is -CN.

Compounds of Formula 1.0, wherein W is an imidamido group, can be produced by reacting a compound of Formula 1.0, wherein W is an imidate group, with concentrated $NH_4OH$ in a suitable solvent (e.g., isopropanol) at a suitable temperature (e.g., about 25°C) for a suitable amount of time (e.g., about 24 hours). For example, an imidate compound of Formula 1.0, wherein $R^{13}$ is -CN and $R^{14}$ is phenyl and X is N, can be reacted with concentrated $NH_4OH$ in isopropanol at 25°C for 24 hours to produce the corresponding imidamido wherein $R^{13}$ is -CN and both $R^{10}$ and $R^{16}$ are H.

Compounds of Formula 1.0, wherein W is an imidamido group, can also be produced as a hydrochloride salt by reacting a compound of Formula 1.0, wherein W is an imidate group, with concentrated $NH_4OH$, $NH_4Cl$, and water in a suitable pressure vessel at a suitable temperature (e.g., about 87°C). For example, an imidate of Formula 1.0 wherein X is CH, $R^{13}$ is H and $R^{14}$ is phenyl can be reacted with concentrated $NH_4OH$, $NH_4Cl$, and water in a suitable pressure vessel at about 87°C to produce the corresponding imidamido hydrochloride salt wherein $R^{13}$ is H and both $R^{10}$ and $R^{16}$ are H.

Compounds of Formula 1.0, wherein W is an imidamido group, can also be produced by reacting a compound of Formula 1.0, wherein W is an imidate group, with an amine (e.g., $HNR^{10}R^{16}$) in a suitable solvent (e.g., THF) at a suitable temperature (e.g., about 70°C). For example, an imidate of Formula 1.0 wherein X is CH and $R^{13}$ is H and $R^{14}$ is phenyl can be reacted with

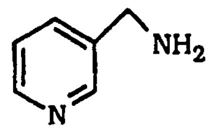

(3-aminomethylpyridine) in THF at about 70°C to produce the corresponding guanidine wherein $R^{13}$ is H and $R^{10}$ is H and $R^{16}$ is 3-methylpyridyl.

Compounds of Formula 1.0, wherein W is an imidamido group, can also be prepared by fusing an imidate of Formula 1.0 with sulfamide. For example, an imidate of Formula 1.0 wherein $R^{13}$ is H and $R^{14}$ is phenyl and X is N can be fused with sulfamide to produce the corresponding guanidine wherein $R^{17}$ is $-SO_2NH_2$ and both $R^{18}$ and $R^{19}$ are H.

Compounds of Formula 1.0, wherein W is an imidamido group, can also be prepared by fusing a cyanide of Formula 1.0 (e.g., X is N) with sulfamide to produce the corresponding imidamido wherein $R^{13}$ is $-SO_2NH_2$ and both $R^{10}$ and $R^{16}$ are H.

Compounds of Formula 1.0, wherein W is an imidate group and $R^{13}$ is an N-methylsulfamoyl group, can also be prepared by reacting an imidate of Formula 1.0, wherein $R^{13}$ is H, with a sulfamoyl chloride in a suitable solvent (e.g., acetonitrile, benzene or toluene) with a suitable base (e.g., triethylamine) at a suitable temperature (e.g., 0 to about 25°C). For example, an imidate of Formula 1.0 wherein $R^{13}$ is H and $R^{14}$ is phenyl and X is N can be reacted with N-methylsulfamoyl chloride or dimethylsulfamoyl chloride to produce the corresponding imidate wherein $R^{13}$ is $-SO_2NHCH_3$ or $-SO_2N(CH_3)_2$, respectively, and $R^{14}$ is phenyl.

Compounds of Formula 1.0, wherein W is an imidamido can also be prepared by reacting an imidate of Formula 1.0 with an aqueous solution of hydroxylamine hydrochloride and sodium hydroxide, or an aqueous solution of methoxylamine and sodium hydroxide, to produce an imidamido wherein $R^{13}$ is -OH or $-OCH_3$, respectively, and both $R^{10}$ and $R^{16}$ are H. The imidate of Formula 1.0 used, for example, can be one wherein X can be N, $R^{13}$ can be H and $R^{14}$ can be phenyl.

Compounds of Formula 1.0, wherein W is an imidate group and $R^{13}$ is $-C(O)NH_2$ or $-C(O)NHCH_3$, can also be prepared by reacting an imidate of Formula 1.0, wherein $R^{13}$ is H, with trimethylsilylisocyanate or methylisocyanate, respectively, in a suitable solvent (e.g., anhydrous dichloromethane) at a suitable temperature (e.g., about 25°C) for a suitable time period (about 48 hours). The imidate of Formula 1.0 used, for example, can be one wherein X can be N, $R^{13}$ can be H and $R^{14}$ can be phenyl.

Compounds of Formula 1.0, wherein W is an imidamido can also be prepared by fusing an imidate of Formula 1.0 with urea to produce an imidamido wherein $R^{13}$ is $-C(O)NH_2$, and both $R^{10}$ and $R^{16}$ are H. The imidate of Formula 1.0 used, for example, can be one wherein X can be N, $R^{13}$ can be H and $R^{14}$ can be phenyl.

The compounds of Formula 1.0 wherein W is 1-amino-2-nitroethylene can be prepared by reacting a compound of Formula 45.0 or 46.0 with 1-methylthio-1-methylamino-2-nitroethene, $N(C_2H_5)_3$ and CuCl according to the procedures disclosed in Canada Patent No. 1178289 (1984).

The compounds of Formula 1.0 wherein W is -amino-2-nitroethylene can also be prepared by reaction of a compound of

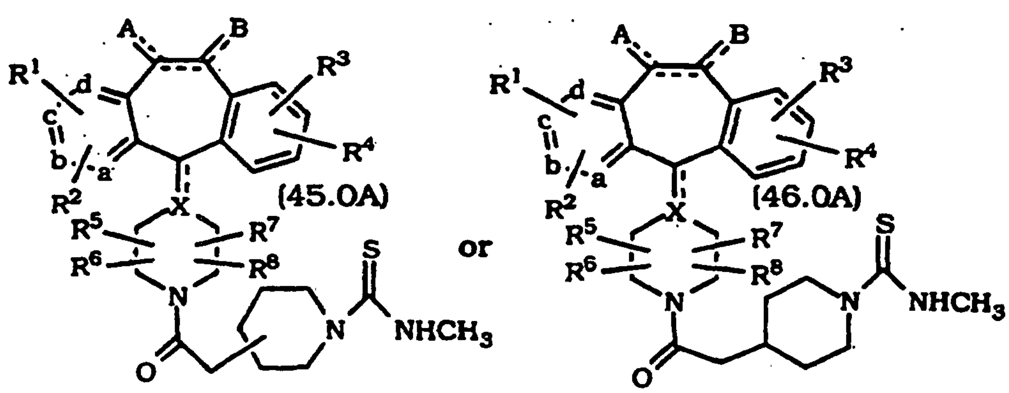

(45.0A) or (46.0A)

in methanol with methyl iodide at reflux, and then refluxing the resulting product with nitromethane (see Indian J. Chem., 15B, 297 (1977)).

The compounds of Formula 1.0 wherein W is -C(O)$R^{12}$ may be prepared by reacting a compound of formula 45.0 or 46.0 with the appropriate carboxylic acid, HOOC$R^{12}$, and HOBT/DEC/DMF.

The preparation of trihalo compounds of the invention is described in the following examples, together with the preparation of dihalo compounds which are not part of the present invention.

PREPARATIVE EXAMPLE 1

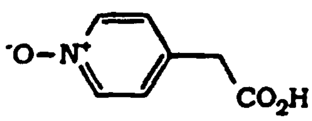

Step A:

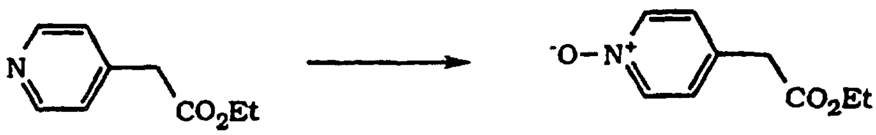

Combine 10 g (60.5 mmol) of ethyl 4-pyridylacetate and 120 mL of dry $CH_2Cl_2$ at -20°C, add 10.45 g (60.5 mmol) of MCPBA and stir at -20°C for 1 hour and then at 25°C for 67 hours. Add an additional 3.48 g (20.2 mmoles) of MCPBA and stir at 25°C for 24 hours. Dilute with $CH_2Cl_2$ and wash with saturated $NaHCO_3$ (aqueous) and then water. Dry over $MgSO_4$, concentrate in vacuo to a residue, and chromatograph (silica gel, 2%-5.5% (10% $NH_4OH$ in MeOH)/$CH_2Cl_2$)to give 8.12 g of the product compound. Mass Spec.: $MH^+$ = 182.15

Step B:

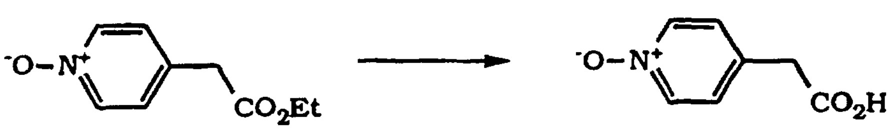

Combine 3.5 g (19.3 mmol) of the product of Step A, 17.5 mL of EtOH and 96.6 mL of 10% NaOH (aqueous) and heat the mixture at 67°C for 2 hours. Add 2 N HCl (aqueous) to adjust to pH = 2.37 and concentrate in vacuo to a residue. Add 200 mL of dry EtOH, filter through celite® and wash the filter cake with dry EtOH (2X50 ml). Concentrate the combined filtrates in vacuo to give 2.43 g of the title compound.

PREPARATIVE EXAMPLE 2

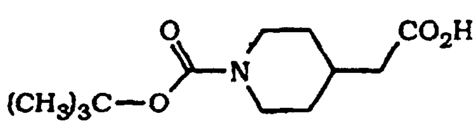

The title compound is prepared via the process disclosed in PCT International Publication No. WO95/10516.

PREPARATIVE EXAMPLE 3

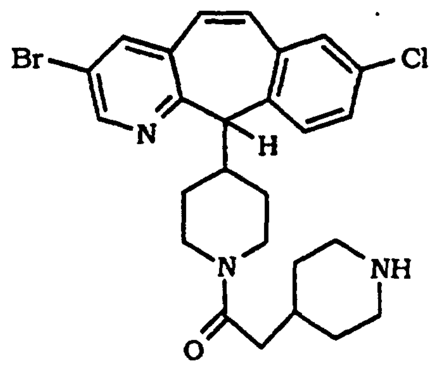

Step A:

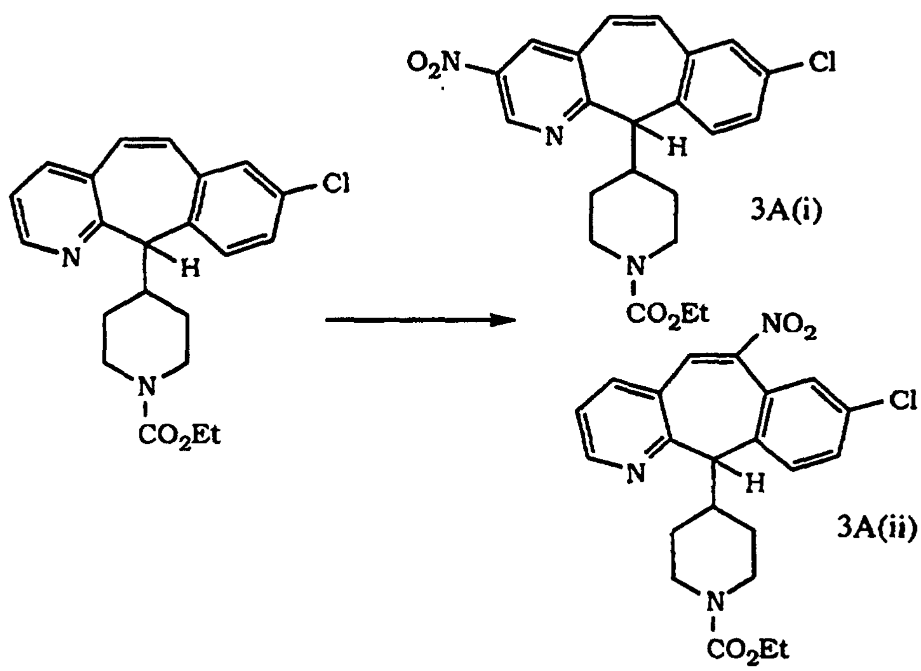

3A(i)

3A(ii)

Combine 14.95 g (39 mmol) of 8-chloro-11-(1-ethoxycarbonyl-4-piperidinyl)-11H-benzo[5,6]cyclohepta[1,2-b] pyridine and 150 mL of $CH_2Cl_2$, then add 13.07 g (42.9 mmol) of $(nBu)_4NNO_3$ and cool the mixture to 0°C. Slowly add (dropwise) a solution of 6.09 mL (42.9 mmol) of TFAA in 20 mL of $CH_2Cl_2$ over 1.5 hours. Keep the mixture at 0°C overnight, then wash successively with saturated $NaHCO_3$ (aqueous), water and brine. Dry the organic solution over $Na_2SO_4$, concentrate in vacuo to a residue and chromatograph the residue (silica gel, EtOAc/hexane gradient) to give 4.32 g and 1.90 g of the two product compounds 3A(i) and 3A(ii), respectively.

Mass Spec. for compound 3A(i): $MH^+$ = 428.2;

Mass Spec. for compound 3A(ii): $MH^+$ = 428.3.

Step B:

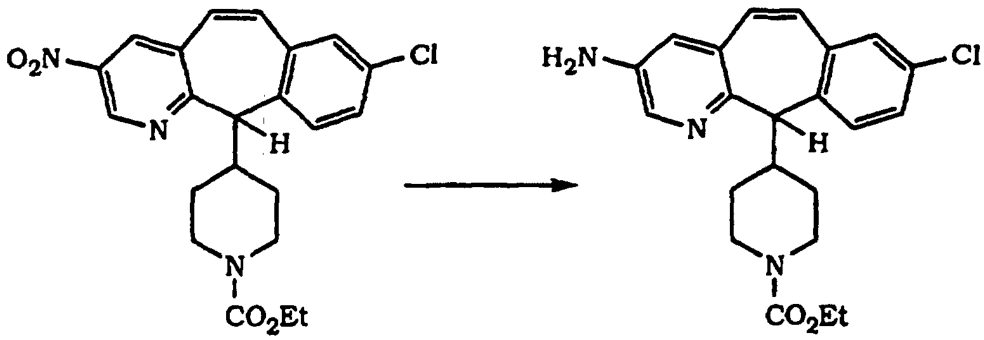

Combine 22.0 g (51.4 mmol) of the product 3A(i) from Step A, 150 mL of 85% EtOH (aqueous), 25.85 g (0.463 mole) of Fe powder and 2.42 g (21.8 mmol) of $CaCl_2$, and heat at reflux overnight. Add 12.4 g (0.222 mole) of Fe powder and 1.2 g (10.8 mmol) of $CaCl_2$ and heat at reflux for 2 hours. Add another 12.4 g (0.222 mole) of Fe powder and 1.2 g (10.8 mmol) of $CaCl_2$ and heat at reflux for 2 hours more. Filter the hot mixture through celite®, wash the celite® with 50 mL of hot EtOH and concentrate the filtrate in vacuo to a residue. Add 100 mL of anhydrous EtOH, concentrate to a residue and chromatograph the residue (silica gel, $MeOH/CH_2Cl_2$ gradient) to give 16.47 g of the product compound.

Step C:

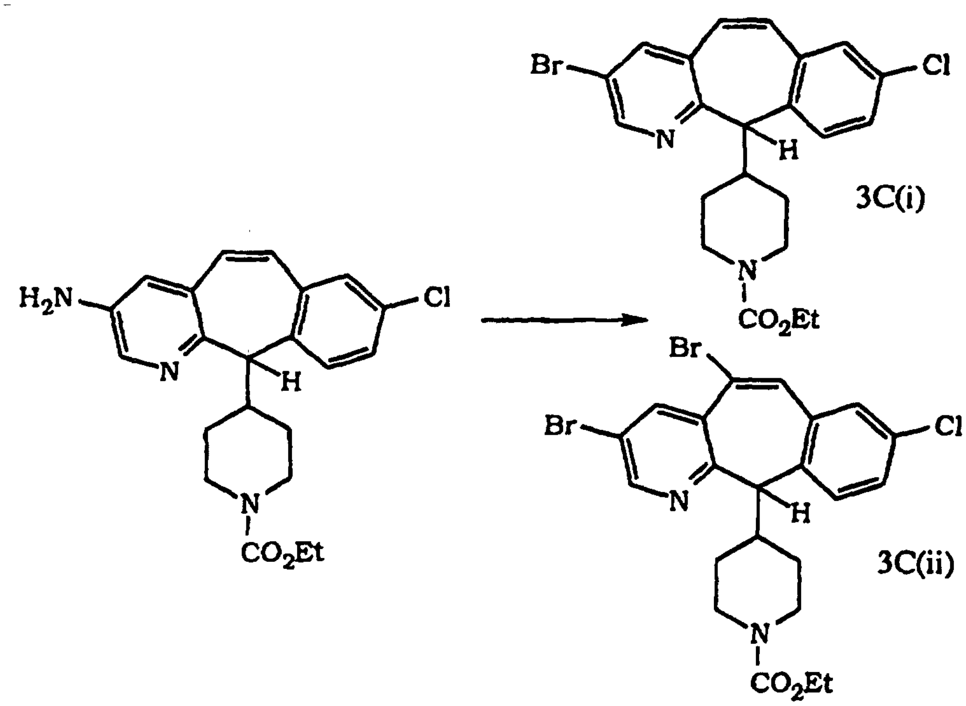

3C(i)

3C(ii)

Combine 16.47 g (41.4 mmol) of the product from Step B, and 150 mL of 48% HBr (aqueous) and cool to -3°C. Slowly add (dropwise) 18 mL of bromine, then slowly add (dropwise) a solution of 8.55 g (0.124 mole) of $NaNO_2$ in 85 mL of water. Stir for 45 minutes at -3° to 0°C, then adjust to pH = 10 by adding 50% NaOH (aqueous). Extract with EtOAc, wash the extracts with brine and dry the extracts over $Na_2SO_4$. Concentrate to a residue and chromatograph (silica gel, EtOAc/hexane gradient) to give 10.6 g and 3.28 g of the two product compounds 3C(i) and 3C(ii),

respectively.

Mass Spec. for compound 3C(i): $MH^+$ = 461.2;

Mass Spec. for compound 3C(ii): $MH^+$ = 539.

Step D:

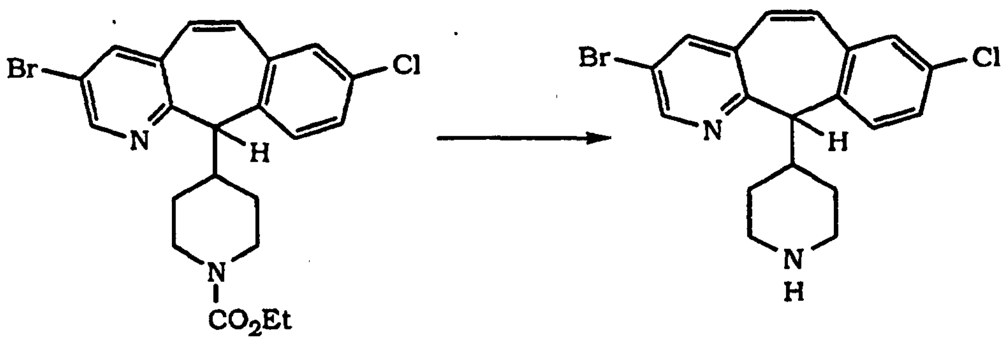

Hydrolyze the product 3C(i) of Step C by dissolving in concentrated HCl and heating to about 100°C for @ 16 hours. Cool the mixture, the neutralize with 1 M NaOH (aqueous). Extract with $CH_2Cl_2$, dry the extracts over $MgSO_4$, filter and concentrate *in vacuo* to the title compound.
Mass Spec.: $MH^+$ = 466.9.

Step E:

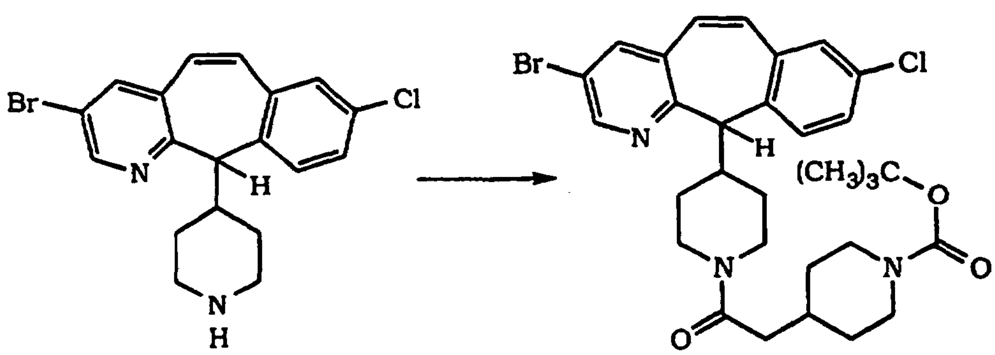

Dissolve 1.160 g (2.98 mmol) of the title compound from Step D in 20 mL of DMF, stir at room temperature, and add 0.3914 g (3.87 mmol) of 4-methyl-morpholine, 0.7418 g (3.87 mmol) of DEC, 0.5229 g (3.87 mmol) of HOBT, and 0.8795 g (3.87 mmol) of 1-N-t-butoxycarbonyl-piperidinyl-4-acetic acid. Stir the mixture at room temperature for 2 days, then concentrate *in vacuo* to s residue and partition the residue between $CH_2Cl_2$ and water. Wash the organic phase successively with saturated $NaHCO_3$ (aqueous), 10% $NaH_2PO_4$ (aqueous) and brine. Dry the organic phase over $MgSO_4$, filter and concentrate *in vacuo* to a residue. Chromatograph the residue (silica gel, 2% MeOH/ $CH_2Cl_2$ + $NH_3$) to give 1.72 g of the product. m.p. = 94.0-94.5°C, Mass Spec.: $MH^+$ = 616.3,

| | |
|---|---|
| elemental analysis | calculated - C, 60.54; H, 6.06; N, 6.83<br>found - C, 59.93; H, 6.62; N, 7.45. |

Step F:

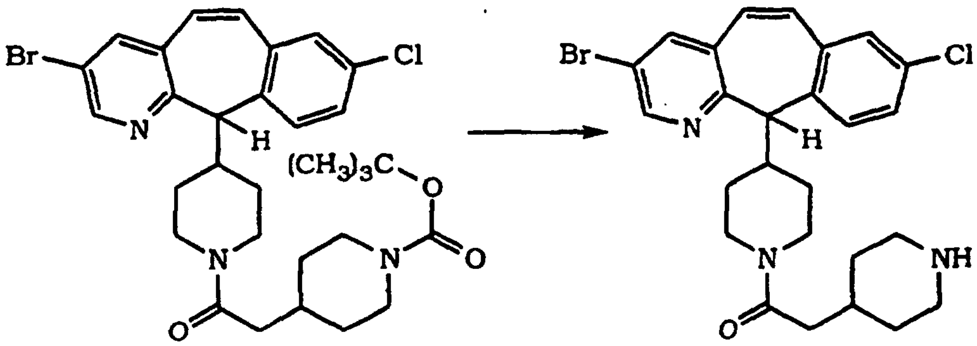

Combine 1.67 g (2.7 mmol) of the product of Step E and 20 mL of $CH_2Cl_2$ and stir at 0°C. Add 20 mL of TFA, stir the mixture for 2 hours, then basify the mixture with 1 N NaOH (aqueous). Extract with $CH_2Cl_2$, dry the organic phase over $MgSO_4$, filter and concentrate *in vacuo* to give 1.16 g of the product. m.p. = 140.2-140.8°C, Mass Spec.: $MH^+$ = 516.2.

PREPARATIVE EXAMPLE 4

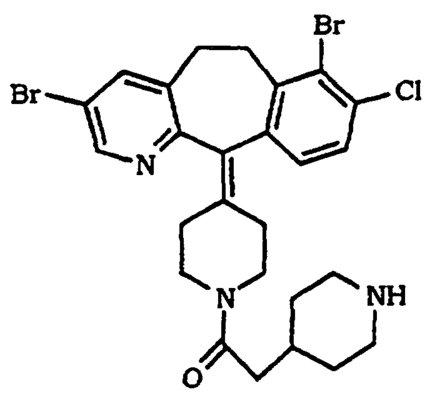

Step A:

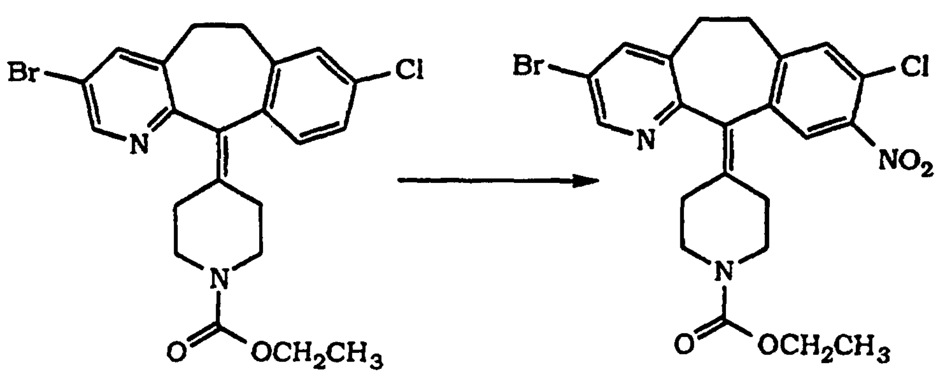

Combine 25.86 g (55.9 mmol) of 4-(8-chloro-3-bromo-5,6-dihydro-11H-benzo[5,6]cyclohepta[1,2-b]pyridin-11-ylidene)-1-piperidine-1-carboxylie acid ethyl ester and 250 mL of concentrated $H_2SO_4$ at -5°C, then add 4.8 g (56.4 mmol) of $NaNO_3$ and stir for 2 hours. Pour the mixture into 600 g of ice and basify with concentrated $NH_4OH$ (aqueous). Filter the mixture, wash with 300 mL of water, then extract with 500 mL of $CH_2Cl_2$. Wash the extract with 200 mL of water, dry over $MgSO_4$, then filter and concentrate *in vacuo* to a residue. Chromatograph the residue (silica gel, 10% EtOAc/ $CH_2Cl_2$) to give 24.4 g (86% yield) of the product. m.p. = 165-167°C, Mass Spec.: $MH^+$ = 506 (CI),

| | |
|---|---|
| elemental analysis | calculated - C, 52.13; H, 4.17; N, 8.29<br>found - C, 52.18; H, 4.51; N, 8.16. |

Step B:

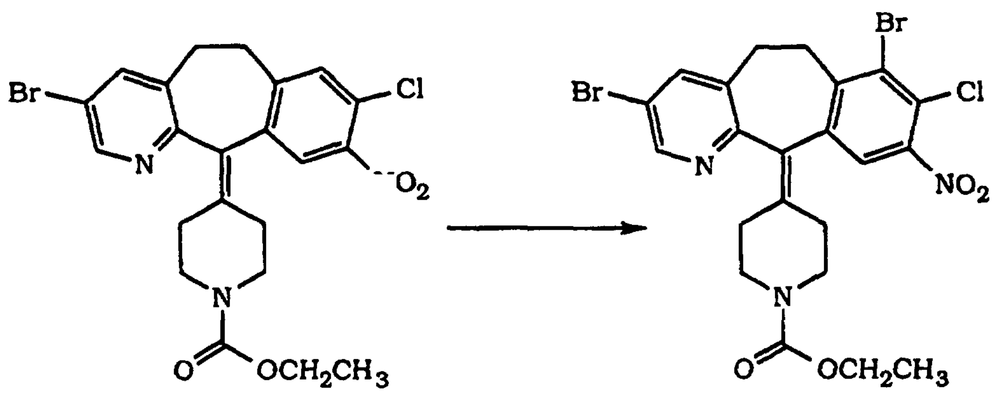

Combine 20 g (40.5 mmol) of the product of Step A and 200 mL of concentrated $H_2SO_4$ at 20°C, then cool the mixture to 0°C. Add 7.12 g (24.89 mmol) of 1,3-dibromo-5,5-dimethylhydantoin to the mixture and stir for 3 hours at 20°C. Cool to 0°C, add an additional 1.0 g (3.5 mmol) of the dibromohydantoin and stir at 20°C for 2 hours. Pour the mixture into 400 g of ice, basify with concentrated $NH_4OH$ (aqueous) at 0°C, and collect the resulting solid by filtration. Wash the solid with 300 mL of water, slurry in 200 mL of acetone and filter to provide 19.79 g (85.6% yield) of the product. m.p. = 236-237°C, Mass Spec.: $MH^+$ = 584 (CI),

| | |
|---|---|
| elemental analysis | calculated - C, 45.11; H, 3.44; N, 7.17<br>found - C, 44.95; H, 3.57; N, 7.16. |

Step C:

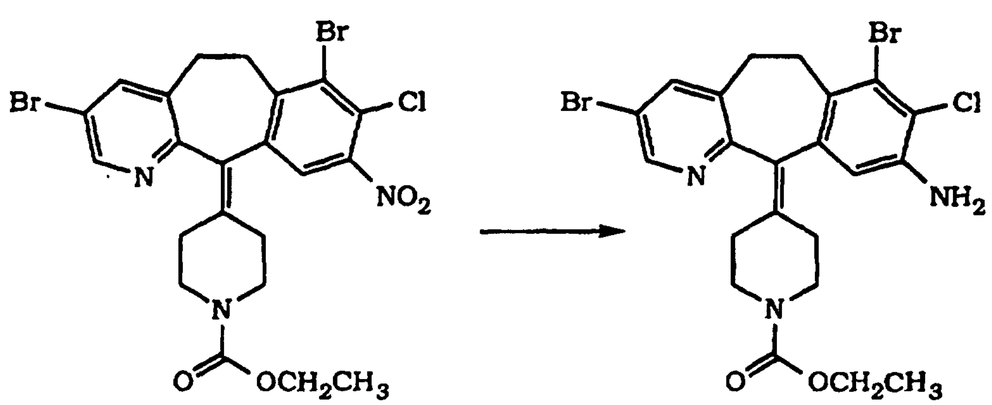

Combine 25 g (447 mmol) of Fe filings, 10 g (90 mmol) of $CaCl_2$ and a suspension of 20 g (34.19 mmol) of the product of Step B in 700 mL of 90:10 EtOH/water at 50°C. Heat the mixture at reflux overnight, filter through Celite®

and wash the filter cake with 2 X 200 mL of hot EtOH. Combine the filtrate and washes, and concentrate *in vacuo* to a residue. Extract the residue with 600 mL of $CH_2Cl_2$, wash with 300 mL of water and dry over $MgSO_4$. Filter and concentrate in vacuo to a residue, then chromatograph (silica gel, 30% EtOAc/$CH_2Cl_2$) to give 11.4 g (60% yield) of the product. m.p. = 211-212°C,
Mass Spec.: $MH^+$ = 554 (CI),

| | |
|---|---|
| elemental analysis | calculated - C, 47.55; H, 3.99; N, 7.56<br>found - C, 47.45; H, 4.31; N, 7.49. |

Step D:

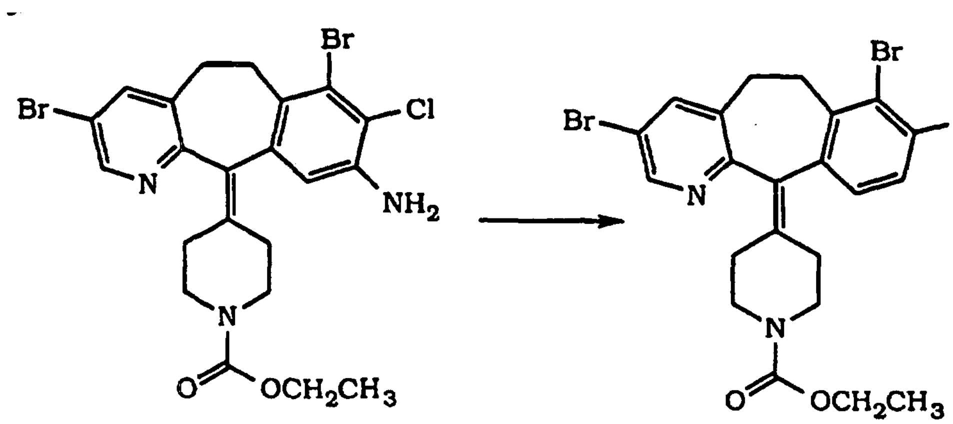

Slowly add (in portions) 20 g (35.9 mmol) of the product of Step C to a solution of 8 g (116 mmol) of $NaNO_2$ in 120 mL of concentrated HCl (aqueous) at -10°C. Stir the resulting mixture at 0°C for 2 hours, then slowly add (dropwise) 150 mL (1.44 mole) of 50% $H_3PO_2$ at 0°C over a 1 hour period. Stir at 0°C for 3 hours, then pour into 600 g of ice and basify with concentrated $NH_4OH$ (aqueous). Extract with 2 X 300 mL of $CH_2Cl_2$, dry the extracts over $MgSO_4$, then filter and concentrate *in vacuo* to a residue. Chromatograph the residue (silica gel, 25% EtOAc/hexanes) to give 13.67 g (70% yield) of the product. m.p. = 163-165°C, Mass Spec.: $MH^+$ = 539 (CI),

| | |
|---|---|
| elemental analysis | calculated - C, 48.97; H, 4.05; N, 5.22<br>found - C, 48.86; H, 3.91; N, 5.18. |

Step E:

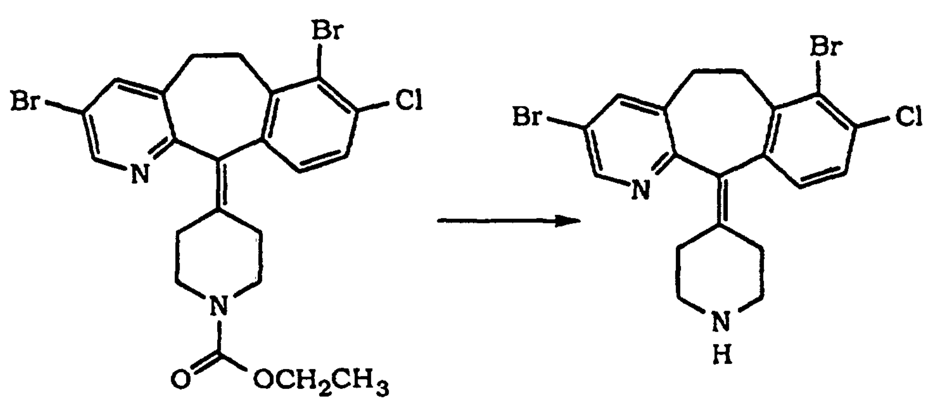

Combine 6.8 g (12.59 mmol) of the product of Step D and 100 mL of concentrated HCl (aqueous) and stir at

85°C overnight. Cool the mixture, pour it into 300 g of ice and basify with concentrated $NH_4OH$ (aqueous). Extract with 2 x 300 mL of $CH_2Cl_2$, then dry the extracts over $MgSO_4$. Filter, concentrate *in vacuo* to a residue, then chromatograph (silica gel, 10% MeOH/EtOAc + 2% $NH_4OH$ (aqueous)) to give 5.4 g (92% yield) of the title compound. m.p. = 172-174°C, Mass Spec.: $MH^+$ = 467 (FAB),

| | |
|---|---|
| elemental analysis | calculated - C, 48.69; H, 3.65; N, 5.97<br>found - C, 48.83; H, 3.80; N, 5.97. |

Step F:

Following essentially the same procedure as Step C of Preparative Example 5 below, the title compound from Step E above is reacted with 1-N-t-butoxycarbonylpiperidinyl-4-acetic acid to produce the compound

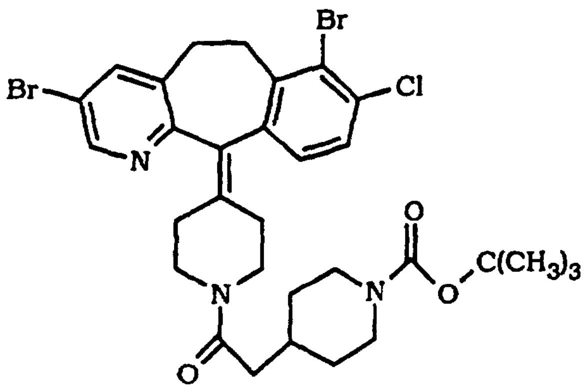

Step G:

Following essentially the same procedure as Step D of Preparative Example 5 below, the title compound from Step F above is deprotected to yield the title compound of Preparative Example 4.

PREPARATIVE EXAMPLE 5

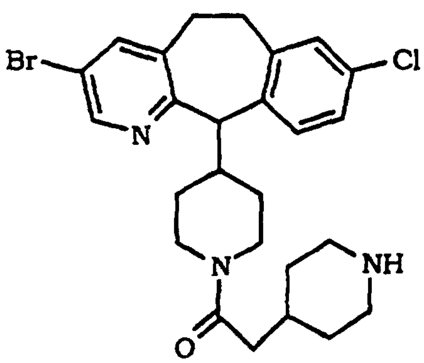

Step A:

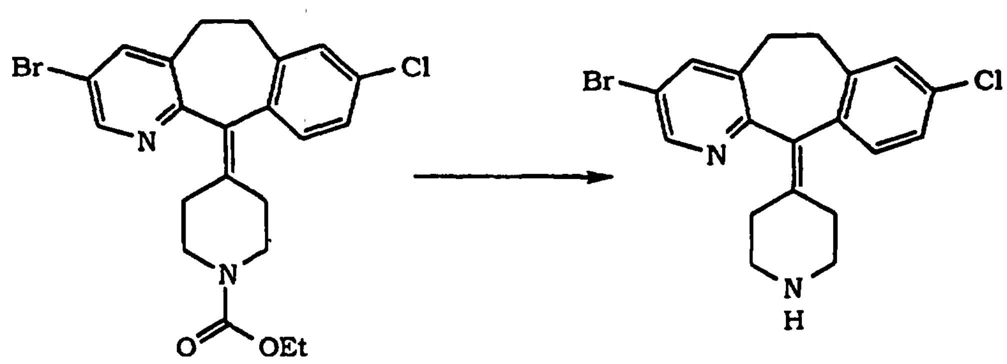

Hydrolyze 2.42 g of 4-(8-chloro-3-bromo-5,6-dihydro-11H-benzo[5,6]cyclohepta[1,2-b]pyridin-11-ylidene)-1-piperidine-1-carboxylic acid ethyl ester via substantially the same procedure as described in Preparative Example 3, Step D, to give 1.39 g (69% yield) of the product.

Step B:

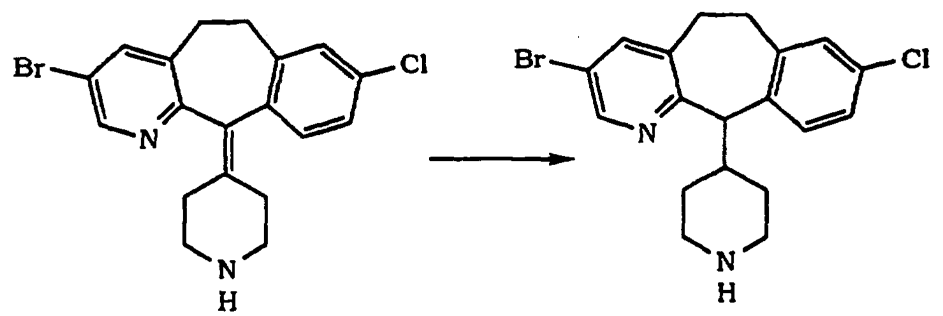

Combine 1 g (2.48 mmol) of the product of Step A and 25 mL of dry toluene, add 2.5 mL of 1 M DIBAL in toluene and heat the mixture at reflux. After 0.5 hours, add another 2.5 mL of 1 M DIBAL in toluene and heat at reflux for 1 hour. (The reaction is monitored by TLC using 50% $MeOH/CH_2Cl_2$ $+NH_4OH$ (aqueous).) Cool the mixture to room temperature, add 50 mL of 1 N HCl (aqueous) and stir for 5 min. Add 100 mL of 1 N NaOH (aqueous), then extract with EtOAc (3 X 150 mL). Dry the extracts over $MgSO_4$, filter and concentrate *in vacuo* to give 1.1 g of the title compound.

Step C:

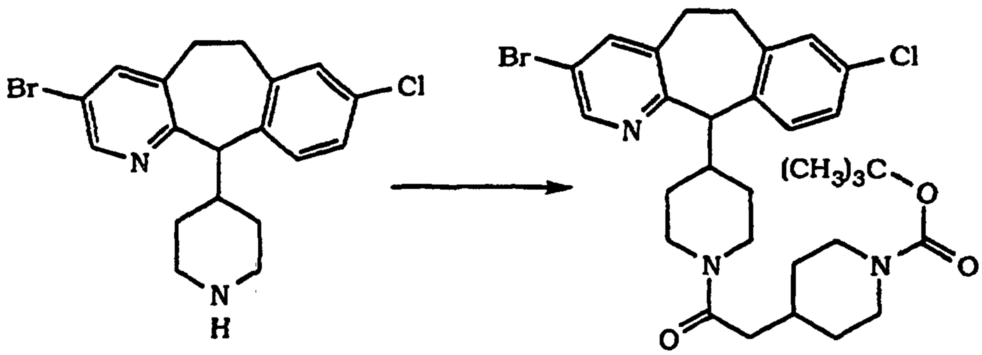

Combine 0.501 g (1.28 mmol) of the title compound of Step B and 20 mL of dry DMF, then add 0.405 g (1.664 mmol) of 1-N-t-butoxycarbonylpiperidinyl-4-acetic acid, 0.319 g (1.664 mmol) of DEC, 0.225 g (1.664 mmol) of HOBT, and 0.168 g (1.664 mmol) of 4-methylmorpholine and stir the mixture at room temperature overnight. Concentrate the mixture *in vacuo* to a residue, then partition the residue between 150 mL of $CH_2Cl_2$ and 150 mL of saturated $NaHCO_3$ (aqueous). Extract the aqueous phase with another 150 mL of $CH_2Cl_2$. Dry the organic phase over $MgSO_4$, and concentrate in vacuo to a residue. Chromatograph the residue (silica gel, 500 mL hexane, 1 L of 1% $MeOH/CH_2Cl_2$ + 0.1% $NH_4OH$ (aqueous), then 1 L of 2% $MeOH/CH_2Cl_2$ + 0.1 % $NH_4OH$ (aqueous)) to give 0.575 g of the product. m.p. = 115°-125°C; Mass Spec.: $MH^+$ = 616.

Step D:

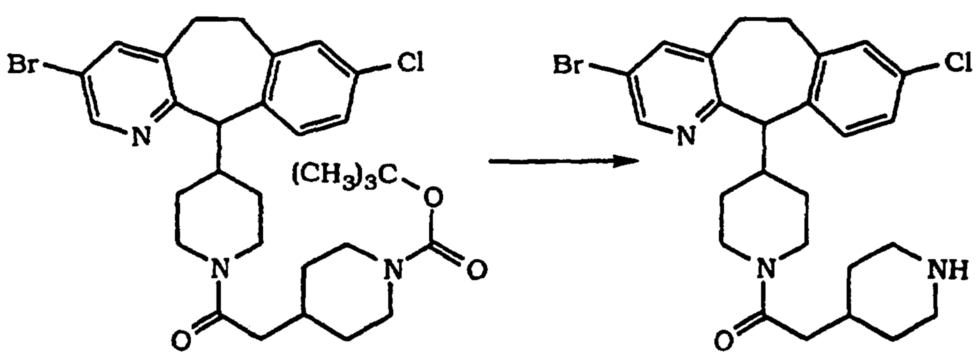

Combine 0.555 g (0.9 mmol) of the product of Step C and 15 mL of $CH_2Cl_2$ and cool the mixture to 0°C. Add 15 mL of TFA and stir at 0°C for 2 hours. Concentrate *in vacuo* at 40-45°C to a residue, then partition the residue between 150 mL of $CH_2Cl_2$ and 100 mL of saturated $NaHCO_3$ (aqueous). Extract the aqueous layer with 100 mL of $CH_2Cl_2$, combine the extracts and dry over $MgSO_4$. Concentrate *in vacuo* to give 0.47 g of the product.
m.p. = 140°-150°C; Mass Spec.: $MH^+$ = 516.

PREPARATIVE EXAMPLE 6

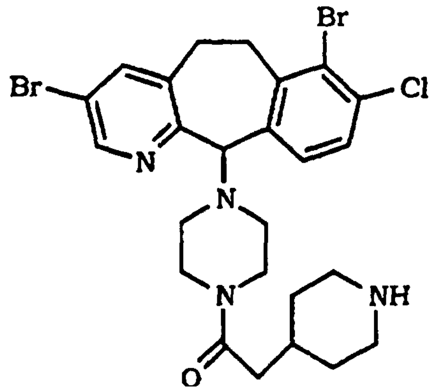

[racemic as well as (+)- and (-)-isomers]

Step A:

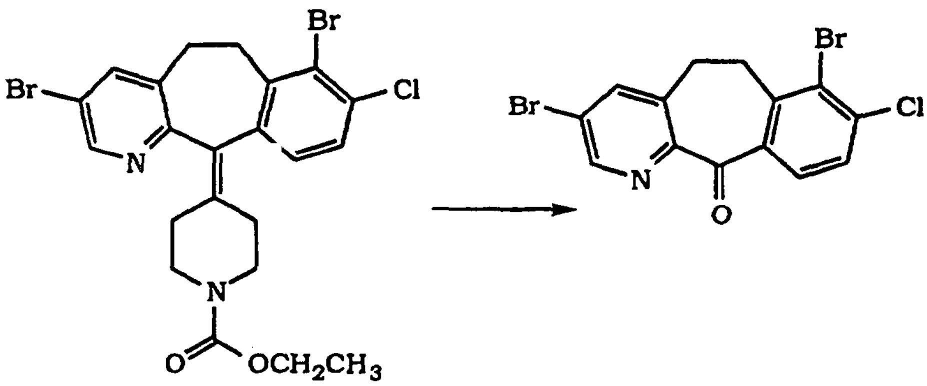

Combine 16.6 g (0.03 mole) of the product of Preparative Example 4, Step D, with a 3:1 solution of $CH_3CN$ and water (212.65 mL $CH_3CN$ and 70.8 mL of water) and stir the resulting slurry overnight at room temperature. Add 32.833 g (0.153 mole) of $NaIO_4$ and then 0.31 g (2.30 mmol) of $RuO_2$ and stir at room temperature give 1.39 g (69% yield) of the product. (The addition of RuO is accompanied by an exothermic reaction and the temperature climbs from 20° to 30°C.) Stir the mixture for 1.3 hrs. (temperature returned to 25°C after about 30 min.), then filter to remove the solids and wash the solids with $CH_2Cl_2$. Concentrate the filtrate *in vacuo* to a residue and dissolve the residue in $CH_2Cl_2$. Filter to remove insoluble solids and wash the solids with $CH_2Cl_2$. Wash the filtrate with water, concentrate to a volume of about 200 mL and wash with bleach, then with water. Extract with 6 N HCl (aqueous). Cool the aqueous extract to 0°C and slowly add 50% NaOH (aqueous) to adjust to pH = 4 while keeping the temperature <30°C. Extract twice with $CH_2Cl_2$, dry over $MgSO_4$ and concentrate *in vacuo* to a residue. Slurry the residue in 20 mL of EtOH and cool to 0°C. Collect the resulting solids by filtration and dry the solids *in vacuo* to give 7.95 g of the product. $^1H$ NMR ($CDCl_3$, 200 MHz): 8.7 (s, 1H); 7.85 (m, 6H); 7.5 (d, 2H); 3.45 (m, 2H); 3.15 (m, 2H).

Step B:

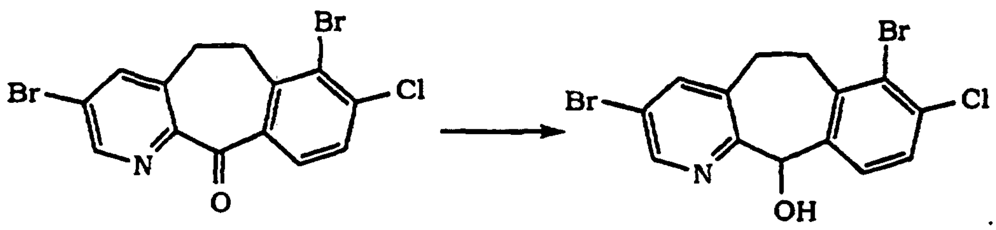

Combine 21.58 g (53.75 mmol) of the product of Step A and 500 mL of an anhydrous 1:1 mixture of EtOH and toluene, add 1.43 g (37.8 mmol) of $NaBH_4$ and heat the mixture at reflux for 10 min. Cool the mixture to 0°C, add 100 mL of water, then adjust to pH≈ 4-5 with 1 M HCl (aqueous) while keeping the temperature <10°C. Add 250 mL of EtOAc and separate the layers. Wash the organic layer with brine (3 X 50 mL) then dry over $Na_2SO_4$. Concentrate *in vacuo* to a residue (24.01 g) and chromatograph the residue (silica gel, 30 % hexane/$CH_2Cl_2$) to give the product. Impure fractions were purified by rechromatography. A total of 18.57 g of the product was obtained. $^1H$ NMR (DMSO-$d_6$, 400 MHz): 8.5 (s, 1H); 7.9 (s, 1H); 7.5 (d of d, 2H); 6.2 (s, 1H); 6.1 (s, 1H); 3.5 (m, 1H); 3.4 (m, 1H); 3.2 (m, 2H).

Step C:

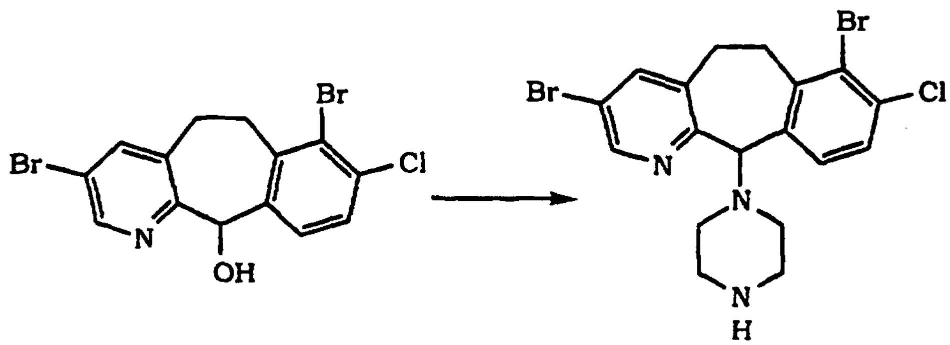

Combine 18.57 g (46.02 mmol) of the product of Step B and 500 mL of $CHCl_3$, then add 6.70 mL (91.2 mmol) of $SOCl_2$, and stir the mixture at room temperature for 4 hrs. Add a solution of 35.6 g (0.413 mole) of piperazine in 800 mL of THF over a period of 5 min. and stir the mixture for 1 hr. at room temperature. Heat the mixture at reflux overnight, then cool to room temperature and dilute the mixture with 1 L of $CH_2Cl_2$. Wash with water (5 X 200 mL), and extract the aqueous wash with $CHCl_3$ (3 X 100 mL). Combine all of the organic solutions, wash with brine (3 X 200 mL) and dry over $MgSO_4$. Concentrate *in vacuo* to a residue and chromatograph (silica gel, gradient of 5%, 7.5%, 10% MeOH/$CH_2Cl_2$ + $NH_4OH$) to give 18.49 g of the title compound as a racemic mixture.

Step D - Separation of Enantiomers:

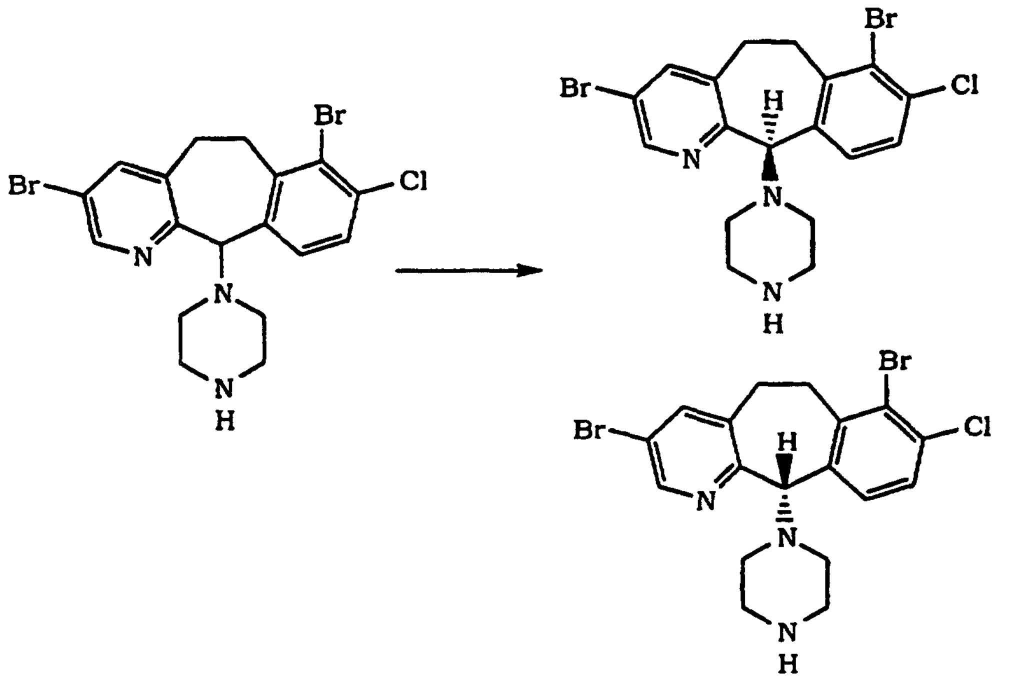

The racemic title compound of Step C is separated by preparative chiral chromatography (Chiralpack AD, 5 cm X 50 cm column, flow rate 100 mL/min., 20% iPrOH/hexane + 0.2% diethylamine), to give 9.14 g of the (+)-isomer and 9.30 g of the (-)-isomer.

Physical chemical data for (+)-isomer: m.p. = 74.5°-77.5°C; Mass Spec. $MH^+$ = 471.9; $[\alpha]_D^{25}$ = +97.4° (8.48 mg/ 2mL MeOH).

Physical chemical data for (-)-isomer: m.p. = 82.9°-84.5°C; Mass Spec. $MH^+$ = 471.8; $[\alpha]_D^{25}$ = -97.4° (8.32 mg/ 2mL MeOH).

Step E:

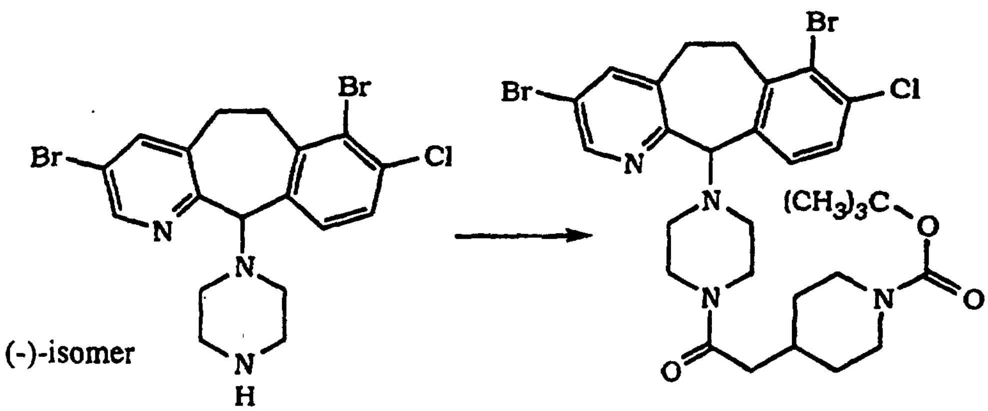

(-)-isomer

Combine 3.21 g (6.80 mmol) of the (-)-isomer product of Step D and 150 mL of anhydrous DMF. Add 2.15 g (8.8 mmol) of 1-N-t-butoxycarbonylpiperidinyl-4-acetic acid, 1.69 g (8.8 mmol) of DEC, 1.19 g (8.8 mmol) of HOBT and 0.97 mL (8.8 mmol) of N-methylmorpholine and stir the mixture at room temperature overnight. Concentrate *in vacuo* to remove the DMF and add 50 mL of saturated $NaHCO_3$ (aqueous). Extract with $CH_2Cl_2$ (2 X 250 mL), wash the

extracts with 50 mL of brine and dry over $MgSO_4$. Concentrate *in vacuo* to a residue and chromatograph (silica gel, 2% $MeOH/CH_2Cl_2$ + 10% $NH_4OH$) to give 4.75 g of the product. m.p. = 75.7°-78.5°C: Mass Spec.: $MH^+$ = 697; $[\alpha]_D^{25}$ = -5.5° (6.6 mg/2 mL MeOH).

Step F:

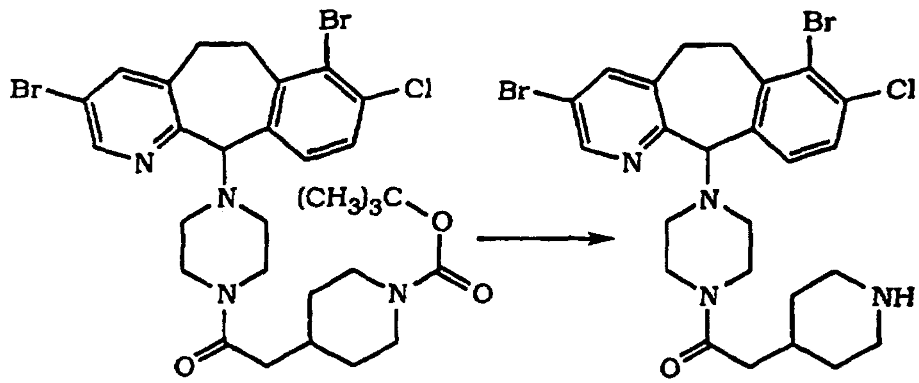

Combine 4.70 g (6.74 mmol) of the product of Step E and 30 mL of MeOH, then add 50 mL of 10% $H_2SO_4$/dioxane in 10 mL aliquots over a 1 hr. period. Pour the mixture into 50 mL of water and add 15 mL of 50% NaOH (aqueous) to adjust to pH≈ 10-11. Filter to remove the resulting solids and extract the filtrate with $CH_2Cl_2$ (2 X 250 mL). Concentrate the aqueous layer *in vacuo* to remove the MeOH and extract again with 250 mL of $CH_2Cl_2$. Dry the combined extracts over $MgSO_4$ and concentrate *in vacuo* to give the product, m.p. = 128.1°-131.5°C; Mass Spec.: $MH^+$ = 597; $[\alpha]_D^{25}$ = -6.02° (9.3 mg/2 mL MeOH).

PREPARATIVE EXAMPLE 7

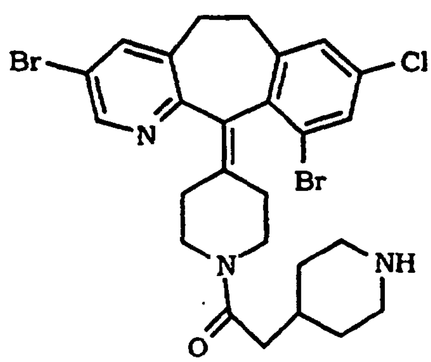

Step A:

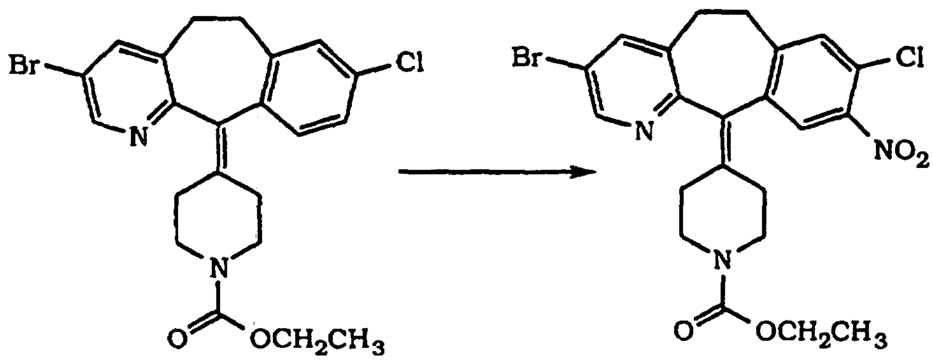

Combine 15 g (38.5 mmol) of 4-(8-chloro-3-bromo-5,6-dihydro-11H-benzo[5,6]cyclohepta[1,2-b]pyridin-11-ylidene)-1-piperidine-1-carboxylic acid ethyl ester and 150 mL of concentrated $H_2SO_4$ at -5°C, then add 3.89 g (38.5 mmol) of $KNO_3$ and stir for 4 hours. Pour the mixture into 3 L of ice and basify with 50% NaOH (aqueous). Extract with $CH_2Cl_2$, dry over $MgSO_4$, then filter and concentrate *in vacuo* to a residue. Recrystallize the residue from acetone to give 6.69 g of the product. $^1H$ NMR ($CDCl_3$, 200 MHz): 8.5 (s, 1H); 7.75 (s, 1H); 7.6 (s, 1H); 7.35 (s, 1H); 4.15 (q, 2H); 3.8 (m, 2H); 3.5-3.1 (m, 4H); 3.0-2.8 (m, 2H); 2.6-2.2 (m, 4H); 1.25 (t, 3H).

Step B:

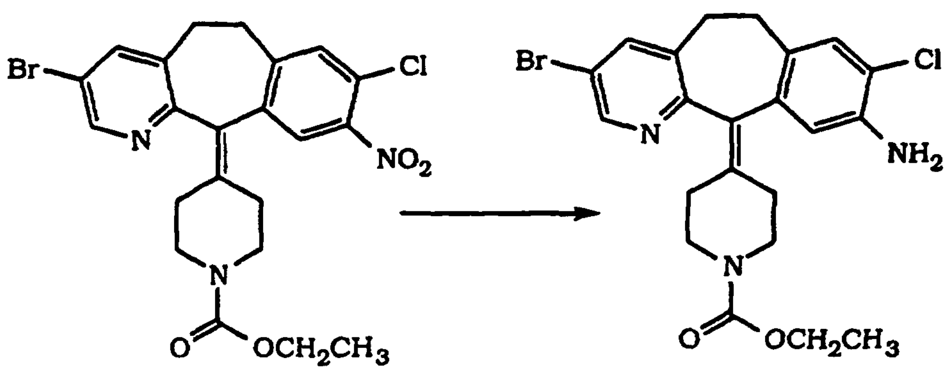

Combine 6.69 g (13.1 mmol) of the product of Step A and 100 mL of 85% EtOH/water, then add 0.66 g (5.9 mmol) of $CaCl_2$ and 6.56 g (117.9 mmol) of Fe and heat the mixture at reflux overnight. Filter the hot reaction mixture through celite ® and rinse the filter cake with hot EtOH. Concentrate the filtrate *in vacuo* to give 7.72 g of the product. Mass Spec.: $MH^+$ = 478.0.

Step C:

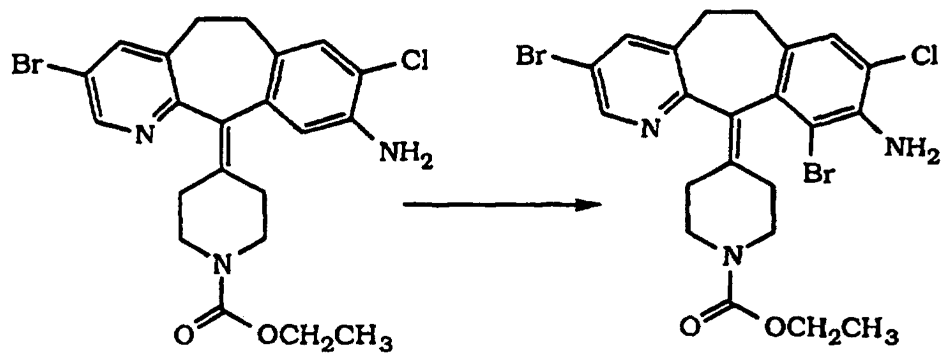

Combine 7.70 g of the product of Step B and 35 mL of HOAc, then add 45 mL of a solution of $Br_2$ in HOAc and stir the mixture at room temperature overnight. Add 300 mL of 1 N NaOH (aqueous), then 75 mL of 50% NaOH (aqueous) and extract with EtOAc. Dry the extract over $MgSO_4$ and concentrate *in vacuo* to a residue. Chromatograph the residue (silica gel, 20%-30% EtOAc/hexane) to give 3.47 g of the product (along with another 1.28 g of partially purified product). Mass Spec.: $MH^{+}$ = 555.9.
$^{1}H$ NMR ($CDCl_3$, 300 MHz): 8.5 (s, 1H); 7.5 (s, 1H); 7.15 (s, 1H); 4.5 (s, 2H); 4.15 (m, 3H); 3.8 (br s, 2H), 3.4-3.1 (m, 4H); 9-2.75 (m, 1H); 2.7-2.5 (m, 2H); 2.4-2.2 (m, 2H); 1.25 (m, 3H).

Step D:

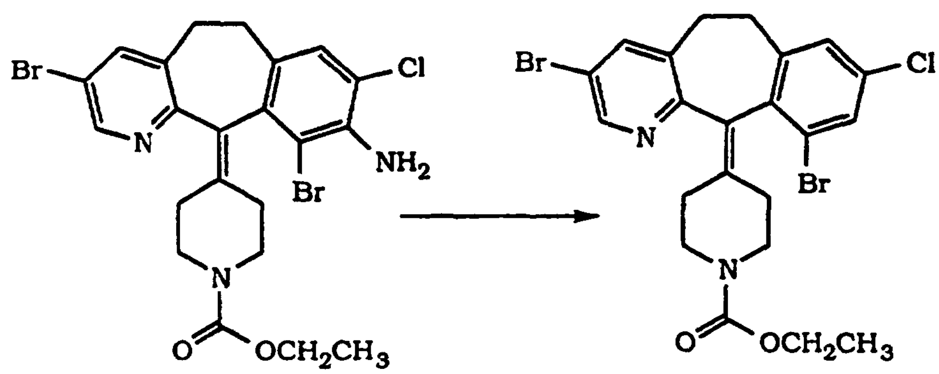

Combine 0.557 g (5.4 mmol) of t-butylnitrite and 3 mL of DMF, and heat the mixture at to 60°-70°C. Slowly add (dropwise) a mixture of 2.00 g (3.6 mmol) of the product of Step C and 4 mL of DMF, then cool the mixture to room temperature. Add another 0.64 mL of t-butylnitrite at 40°C and reheat the mixture to 60°-70°C for 0.5 hrs. Cool to room temperature and pour the mixture into 150 mL of water. Extract with $CH_2Cl_2$, dry the extract over $MgSO_4$ and concentrate *in vacuo* to a residue. Chromatograph the residue (silica gel, 10%-20% EtOAc/hexane) to give 0.74 g of the product. Mass Spec.: $MH^{+}$ = 541.0.
$^{1}H$ NMR (CDCl3, 200 MHz): 8.52 (s, 1H); 7.5 (d, 2H); 7.2 (s, 1H); 4.15 (q, 2H); 3.9-3.7 (m, 2H); 3.5-3.1 (m, 4H); 3.0-2.5 (m, 2H); 2.4-2.2 (m, 2H); 2.1-1.9 (m, 2H); 1.26 (t, 3H).

Step E:

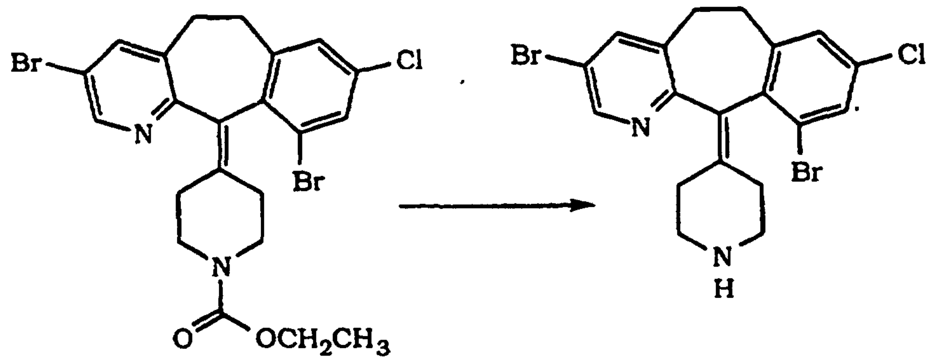

Combine 0.70 g (1.4 mmol) of the product of Step D and 8 mL of concentrated HCl (aqueous) and heat the mixture at reflux overnight. Add 30 mL of 1 N NaOH (aqueous), then 5 mL of 50% NaOH (aqueous) and extract with $CH_2Cl_2$. Dry the extract over $MgSO_4$ and concentrate *in vacuo* to give 0.59 g of the title compound. Mass Spec.: $M^+$ = 468.7. m.p. = 123.9°-124.2°C.

Step F:

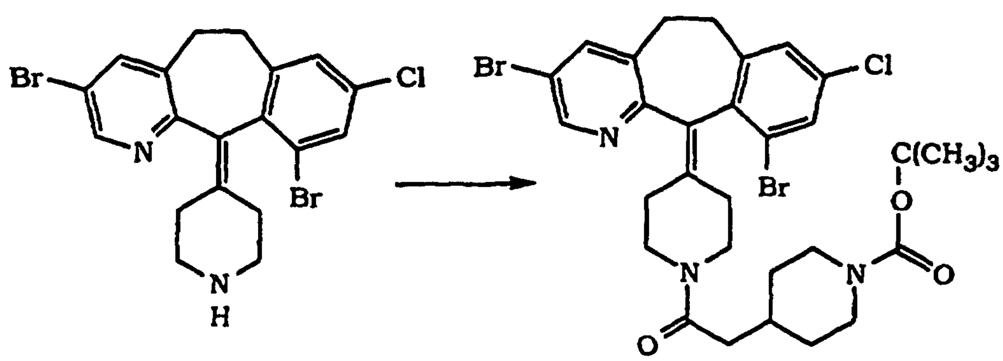

React 6.0 g (12.8 mmol) of the title compound from Step E and with 3.78 g (16.6 mmol) of 1-N-t-butoxycarbonylpiperidinyl-4-acetic acid using substantially the same procedures as described for Preparative Example 5, Step C, to give 8.52 g of the product. Mass Spec.: $MH^+$ = 694.0 (FAB). $^1H$ NMR ($CDCl_3$, 200 MHz): 8.5 (d, 1H); 7.5 (d, 2H); 7.2 (d, 1H); 4.15-3.9 (m, 3H); 3.8-3.6 (m, 1H); 3.5-3.15 (m, 3H); 2.9 (d, 2H); 2.8-2.5 (m, 4H); 2.4-1.8 (m, 6H); 1.8-1.6 (br d, 2H); 1.4 (s, 9H); 1.25-1.0 (m, 2H).

Step G:

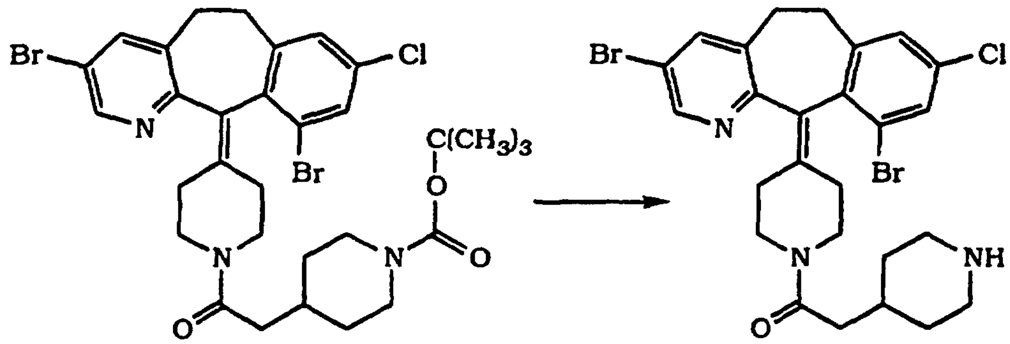

Combine 8.50 g of the product of Step F and 60 mL of $CH_2Cl_2$, then cool to 0°C and add 55 mL of TFA. Stir the mixture for 3 h at 0°C, then add 500 mL of 1 N NaOH (aqueous) followed by 30 mL of 50% NaOH (aqueous). Extract with $CH_2Cl_2$, dry over $MgSO_4$ and concentrate *in vacuo* to give 7.86 g of the product. Mass Spec.: $M^+$ = 593.9 (FAB). $^1H$ NMR ($CDCl_3$, 200 MHz): 8.51 (d, 1H); 7.52 (d of d, 2H); 7.20 (d, 1H); 4.1-3.95 (m, 2H); 3.8-3.65 (m, 2H); 3.5-3.05 (m, 5H); 3.0-2.5 (m, 6H); 2.45-1.6 (m, 6H);1.4-1.1 (m, 2H).

PREPARATIVE EXAMPLE 8

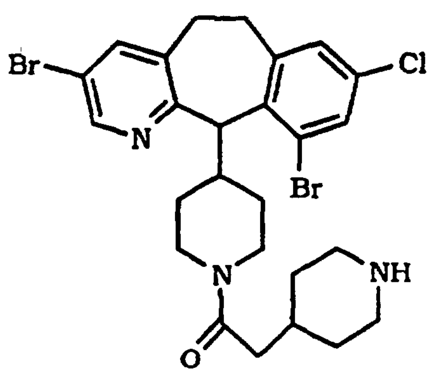

[racemic as well as (+)- and (-)-isomers]

Step A:

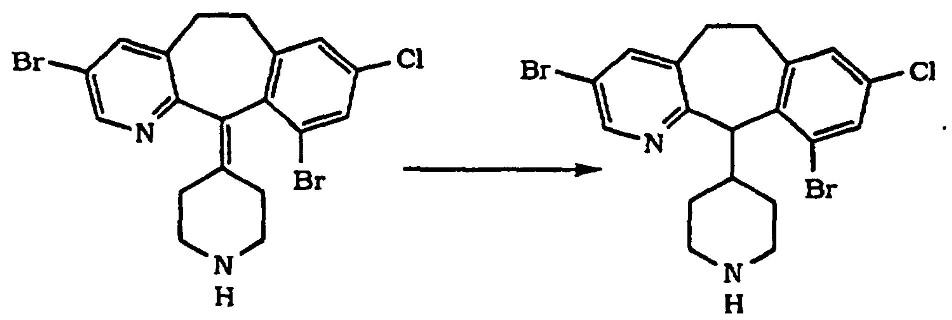

Prepare a solution of 8.1 g of the title compound from Preparative Example 7, Step E, in toluene and add 17.3 mL of a 1M solution of DIBAL in toluene. Heat the mixture at reflux and slowly add (dropwise) another 21 mL of 1 M DIBAL/toluene solution over a period of 40 min. Cool the reaction mixture to about 0°C and add 700 mL of 1 M HCl (aqueous). Separate and discard the organic phase. Wash the aqueous phase with $CH_2Cl_2$, discard the extract, then basify the aqueous phase by adding 50% NaOH (aqueous). Extract with $CH_2Cl_2$, dry the extract over $MgSO_4$ and concentrate *in vacuo* to give 7.30 g of the title compound, which is a racemic mixture of enantiomers.

Step B - Separation of Enantiomers:

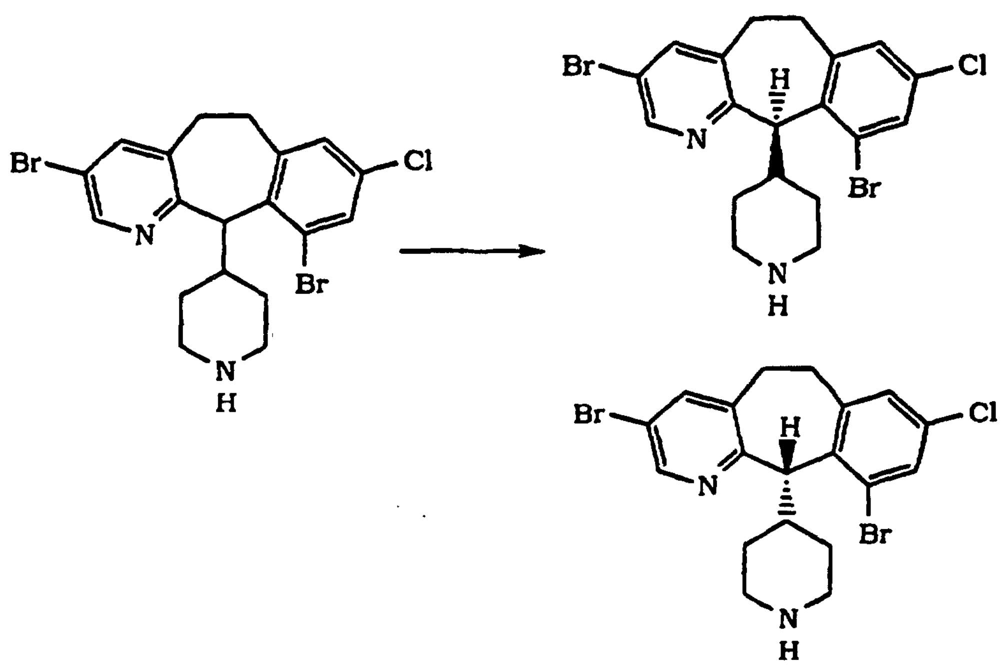

The racemic title compound of Step A is separated by preparative chiral chromatography (Chiralpack AD, 5 cm X 50 cm column, using 20% iPrOH/hexane + 0.2% diethylamine), to give the (+)-isomer and the (-)-isomer of the title compound.

Physical chemical data for (+)-isomer: m.p. = 148.8°C; Mass Spec. $MH^+$ = 469; $[\alpha]_D^{25}$ = +65.6° (12.93 mg/ 2mL MeOH).

Physical chemical data for (-)-isomer: m.p. = 112°C: Mass Spec. $MH^+$ = 469; $[\alpha]_D^{25}$ = -65.2° (3.65 mg/ 2mL MeOH).

Step C:

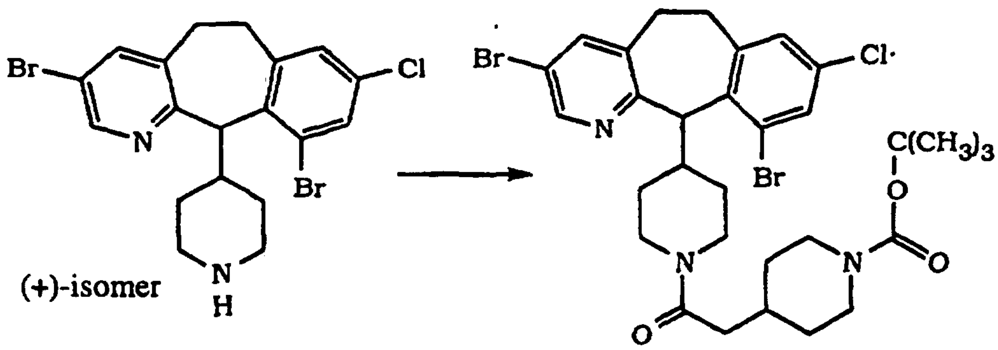

React 1.33 g of the (+)-isomer of the title compound of Preparative Example 8, Step B, with 1.37 g of 1-N-t-butoxycarbonylpiperidinyl-4-acetic acid using substantially the same procedures as described for Preparative Example 5, Step C, to give 2.78 g of the product. Mass Spec.: $MH^+$ = 694.0 (FAB); $[\alpha]_D^{25}$ = +34.1° (5.45 mg/2 mL, MeOH).

Step D:

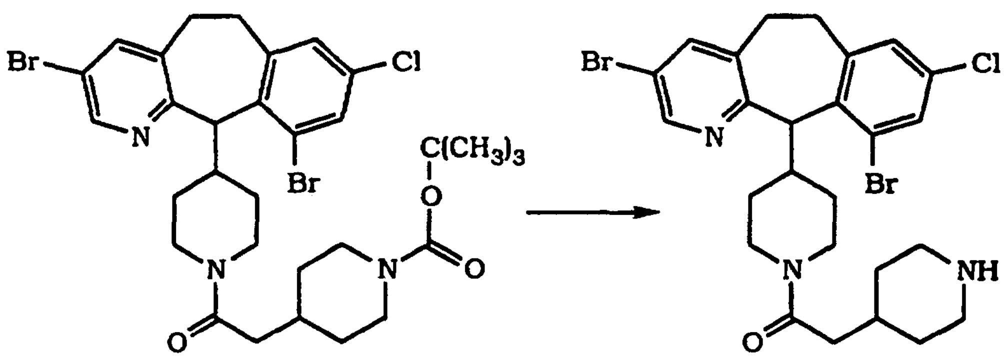

Treat 2.78 g of the product of Step C via substantially the same procedure as described for Preparative Example 5, Step D, to give 1.72 g of the product. m.p. = 104.1°C; Mass Spec.: $MH^+$ = 594; $[\alpha]_D^{25}$ = +53.4° (11.42 mg/2 mL, MeOH).

PREPARATIVE EXAMPLE 9

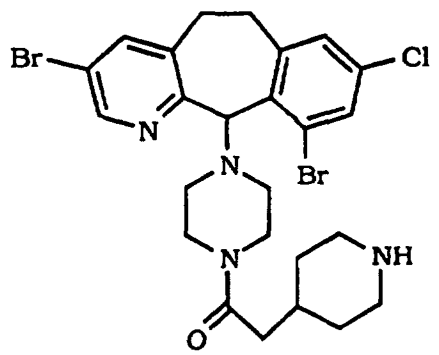

[racemic as well as (+)- and (-)-isomers]

Step A:

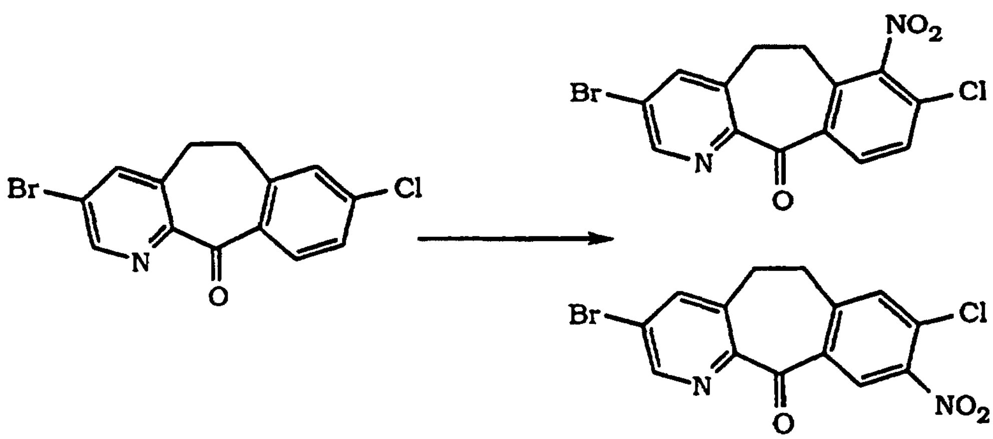

Combine 40.0 g (0.124 mole) of the starting ketone and 200 mL of $H_2SO_4$ and cool to 0°C. Slowly add 13.78 g (0.136 mole) of $KNO_3$ over a period of 1.5 hrs., then warm to room temperature and stir overnight. Work up the reaction using substantially the same procedure as described for Preparative Example 4, Step A. Chromatograph (silica gel, 20%, 30%, 40%, 50% EtOAc/hexane, then 100% EtOAc) to give 28 g of the 9-nitro product, along with a smaller quantity of the 7-nitro product and 19 g of a mixture of the 7-nitro and 9-nitro compounds.

Step B:

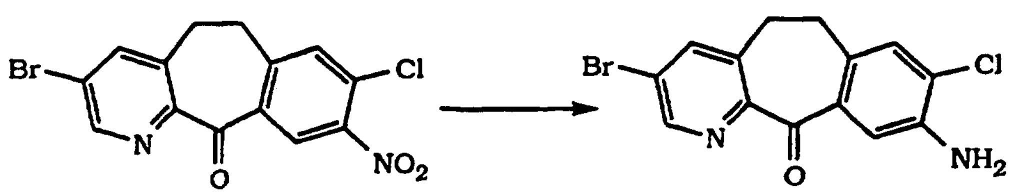

React 28 g (76.2 mmol) of the 9-nitro product of Step A, 400 mL of 85% EtOH/water, 3.8 g (34.3 mmol) of $CaCl_2$ and 38.28 g (0.685 mole) of Fe using substantially the same procedure as described for Preparative Example

4, Step C, to give 24 g of the product

Step C:

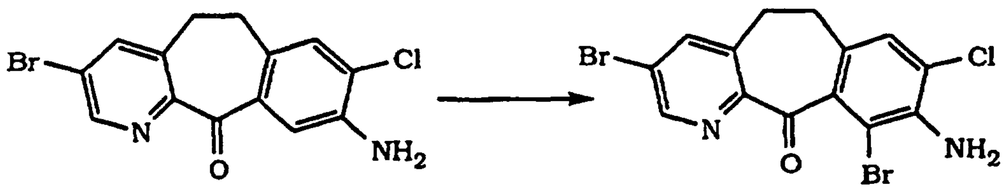

Combine 13 g (38.5 mmol) of the product of Step B, 140 mL of HOAc and slowly add a solution of 2.95 mL (57.8 mmol) of $Br_2$ in 10 mL of HOAc over a period of 20 min. Stir the reaction mixture at room temperature, then concentrate *in vacuo* to a residue. Add $CH_2Cl_2$ and water, then adjust to pH = 8-9 with 50% NaOH (aqueous). Wash the organic phase with water, then brine and dry over $Na_2SO_4$. Concentrate *in vacuo* to give 11.3 g of the product.

Step D:

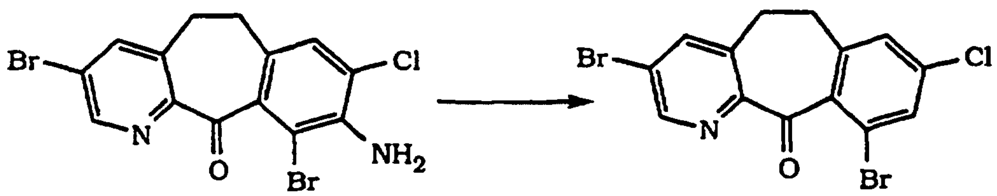

Cool 100 mL of concentrated HCl (aqueous) to 0°C, then add 5.61 g (81.4 mmol) of $NaNO_2$ and stir for 10 min. Slowly add (in portions) 11.3 g (27.1 mmol) of the product of Step C and stir the mixture at 0°-3°C for 2.25 hrs. Slowly add (dropwise) 180 mL of 50% $H_3PO_2$ (aqueous) and allow the mixture to stand at 0°C overnight. Slowly add (dropwise) 150 mL of 50% NaOH over 30 min., to adjust to pH = 9, then extract with $CH_2Cl_2$. Wash the extract with water, then brine and dry over $Na_2SO_4$. Concentrate *in vacuo* to a residue and chromatograph (silica gel, 2% EtOAc/ $CH_2Cl_2$) to give 8.6 g of the product.

Step E:

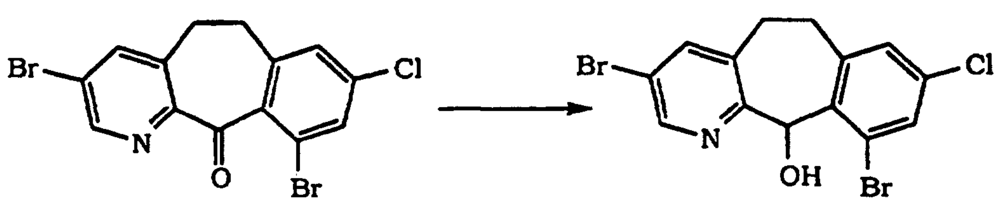

Combine 8.6 g (21.4 mmol) of the product of Step D and 300 mL of MeOH and cool to 0°-2°C. Add 1.21 g (32.1 mmol) of $NaBH_4$ and stir the mixture at ~0°C for 1 hr. Add another 0.121 g (3.21 mmol) of $NaBH_4$, stir for 2 hr. at 0°C, then let stand overnight at 0°C. Concentrate *in vacuo* to a residue then partition the residue between $CH_2Cl_2$ and water. Separate the organic phase and concentrate *in vacuo* (50°C) to give 8.2 g of the product.

Step F:

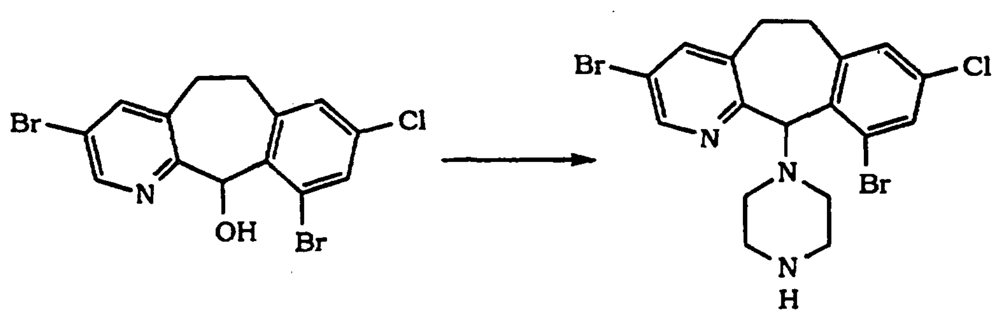

Combine 8.2 g (20.3 mmol) of the product of Step E and 160 mL of $CH_2Cl_2$, cool to 0°C, then slowly add (dropwise) 14.8 mL (203 mmol) of $SOCl_2$ over a 30 min. period. Warm the mixture to room temperature and stir for 4.5 hrs., then concentrate *in vacuo* to a residue, add $CH_2Cl_2$ and wash with 1 N NaOH (aqueous) then brine and dry over $Na_2SO_4$. Concentrate in vacuo to a residue, then add dry THF and 8.7 g (101 mmol) of piperazine and stir at room temperature overnight. Concentrate *in vacuo* to a residue, add $CH_2Cl_2$, and wash with 0.25 N NaOH (aqueous), water, then brine. Dry over $Na_2SO_4$ and concentrate *in vacuo* to give 9.46 g of the crude product. Chromatograph (silica gel, 5% MeOH/$CH_2Cl_2$ + $NH_3$) to give 3.59 g of the title compound, as a racemate. $^1$H NMR ($CDCl_3$, 200 MHz): 8.43 (d, 1H); 7.55 (d, 1H); 7.45 (d, 1H); 7.11 (d, 1H); 5.31 (s, 1H); 4.86-4.65 (m, 1H); 3.57-3.40 (m, 1H); 2.98-2.55 (m, 6H); 2.45-2.20 (m, 5H).

Step G - Separation of Enantiomers:

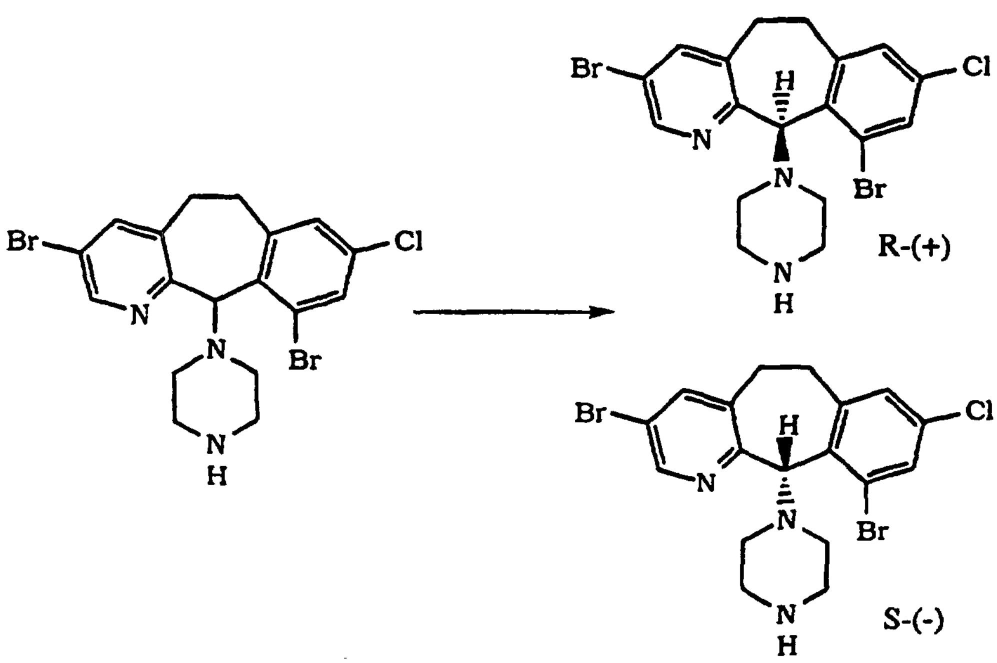


The racemic title compound from Step F (5.7 g) is chromatographed as described for Preparative Example 6, Step D, using 30% iPrOH/hexane + 0.2% diethylamine, to give 2.88 g of the R-(+)-isomer and 2.77 g of the S-(-)-isomer of the title compound.

Physical chemical data for the R-(+)-isomer: Mass Spec. $MH^+$ = 470.0; $[\alpha]_D^{25}$ = + 12.1° (10.9 mg/ 2mL MeOH).

Physical chemical data for the S-(-)-isomer: Mass Spec. $MH^+$ = 470.0; $[\alpha]_D^{25}$ = -13.2° (11.51 mg/ 2mL MeOH).

Step H:

Following essentially the same procedure as Preparative Example 5, Steps C and D, the racemic title compound of Preparative Example 9 is obtained from the racemic compound of Step F. Similarly, using the (-)- or (+)-isomer from Step G, the (-)- or (+)-isomer of the title compound of Preparative Example 9 is obtained, respectively.

PREPARATIVE EXAMPLE 10

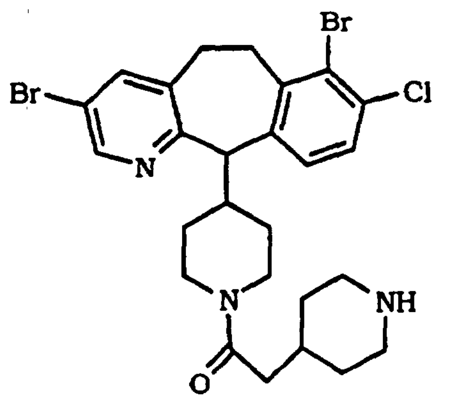

[racemic as well as (+)- and (-)-isomers]

Step A:

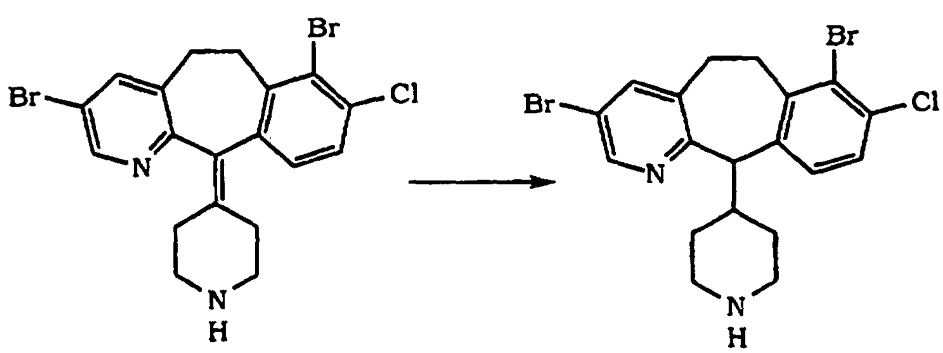

Combine 13 g (33.3 mmol) of the title compound from Preparative Example 4, Step E, and 300 mL of toluene at 20°C, then add 32.5 mL (32.5 mmol) of a 1 M solution of DIBAL in toluene. Heat the mixture at reflux for 1 hr., cool to 20°C, add another 32.5 mL of 1 M DIBAL solution and heat at reflux for 1 hr. Cool the mixture to 20°C and pour it into a mixture of 400 g of ice, 500 mL of EtOAc and 300 mL of 10% NaOH (aqueous). Extract the aqueous layer with $CH_2Cl_2$ (3 x 200 mL), dry the organic layers over $MgSO_4$, then concentrate *in vacuo* to a residue. Chromatograph (silica gel, 12% $MeOH/CH_2Cl_2$ + 4% $NH_4OH$) to give 10.4 g of the title compound as a racemate. Mass Spec.: $MH^+$ = 469 (FAB). partial $^1H$ NMR ($CDCl_3$, 400 MHz): 8.38 (s, 1H); 7.57 (s, 1H); 7.27 (d, 1H); 7.06 (d, 1H); 3.95 (d, 1H).

Step B - Separation of Enantiomers:

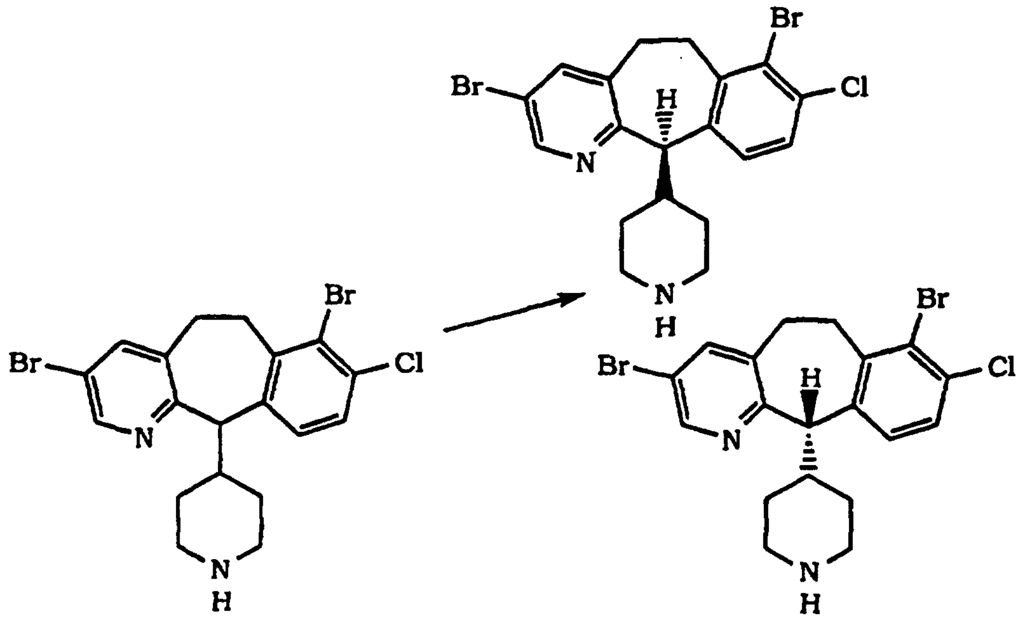

The racemic title compound of Step A is separated by preparative chiral chromatography (Chiralpack AD, 5 cm X 50 cm column, using 5% iPrOH/hexane + 0.2% diethylamine), to give the (+)-isomer and the (-)-isomer of the title compound.

Physical chemical data for (+)-isomer: Mass Spec. $MH^+$ = 469 (FAB); $[\alpha]_D^{25}$ = +43.5° (c=0.402, EtOH); partial $^1H$ NMR ($CDCl_3$, 400 MHz): 8.38 (s, 1H); 7.57 (s, 1H); 7.27 (d, 1H); 7.05 (d, 1H); 3.95 (d, 1H).

Physical chemical data for (-)-isomer: Mass Spec. $MH^+$ = 469 (FAB); $[\alpha]_D^{25}$ = -41.8° (c=0.328 EtOH); partial $^1H$ NMR ($CDCl_3$, 400 MHz): 8.38 (s, 1H); 7.57 (s, 1H); 7.27 (d, 1H); 7.05 (d, 1H); 3.95 (d, 1H).

Step C:

Following the procedure of Preparative Example 9, Step H, the racemic compound, the (+)-isomer or the (-)-isomer of the title compound of Preparative Example 10 can be obtained.

PREPARATIVE EXAMPLE 11

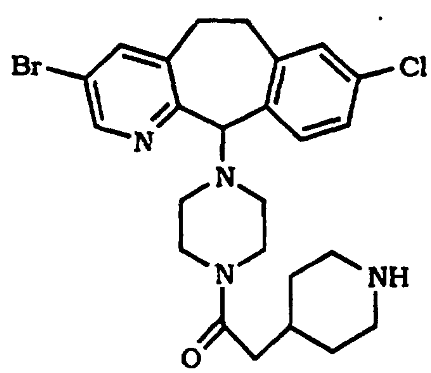

[racemic as well as R-(+)- and S-(-)-isomers]

The compound

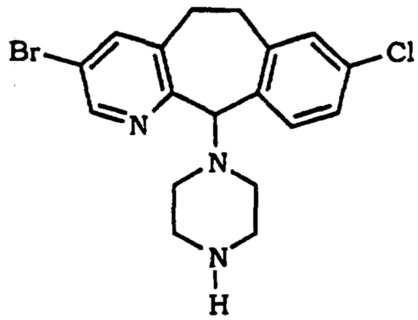

is prepared according to the procedures of Preparative Example 40 of WO 95/10516 (published April 20, 1995), by following the procedures described in Example 193 of WO 95/10516.

The (+)- and (-)-isomers can be separated by following essentially the same procedure as Step D of Preparative Example 6.

Physical chemical data for the R-(+)-isomer: $^{13}C$ NMR ($CDCl_3$): 155.8 (C); 146.4 (CH); 140.5 (CH); 140.2 (C); 136.2 (C); 135.3 (C); 133.4 (C); 132.0 (CH); 129.9 (CH); 125.6 (CH); 119.3 (C); 79.1 (CH); 52.3 ($CH_2$); 52.3 (CH); 45.6 ($CH_2$); 45.6 ($CH_2$); 30.0 ($CH_2$); 29.8 ($CH_2$). $[\alpha]_D^{25}$ = +25.8° (8.46 mg/2 mL MeOH).

Physical chemical data for the S-(-)-isomer: $^{13}C$ NMR ($CDCl_3$): 155.9 (C); 146.4 (CH); 140.5 (CH); 140.2 (C); 136.2 (C); 135.3 (C); 133.3 (C); 132.0 (CH); 129.9 (CH); 125.5 (CH); 119.2 (C); 79.1 (CH); 52.5 ($CH_2$); 52.5 (CH); 45.7 ($CH_2$); 45.7 ($CH_2$); 30.0 ($CH_2$); 29.8 ($CH_2$). $[\alpha]_D^{25}$ = -27.9° (8.90 mg/2 mL MeOH).

Following essentially the same procedure as Preparative Example 5, Steps C and D, the racemic compound, (+)-isomer or (-)-isomer of the title compound of Preparative Example 11 can be obtained from the corresponding racemic compound, (+)-isomer or (-)-isomer of the compound

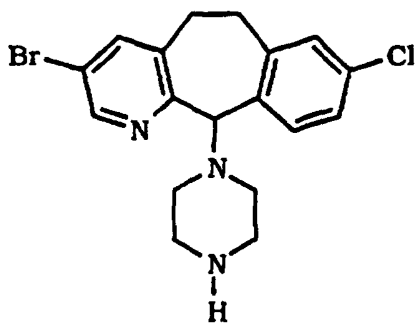

PREPARATIVE EXAMPLE 12

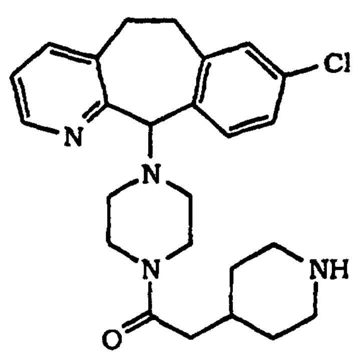

Combine 14.73 g (27.3 mmol) of the compound from Example 193 of WO 95/10516 and 125 mL of anhydrous MeOH, and add (in portions) 300 mL of a 10% solution of concentrated $H_2SO_4$ in dioxane. Stir the mixture at 25°C for 2 hours, then pour into water and adjust to pH =13 with 50% NaOH (aqueous). Extract with $CH_2Cl_2$, wash the extract

with water and dry over $MgSO_4$. Concentrate *in vacuo* to a residue and chromatograph (silica gel, 10% (10% $NH_4OH$ in MeOH)/$CH_2Cl_2$) to give 8.9 g of the title compound. Mass Spec.: $MH^+$ = 539

EXAMPLE 1

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-CYANO-1-PIPERIDINE- CARBOXIMIDATE

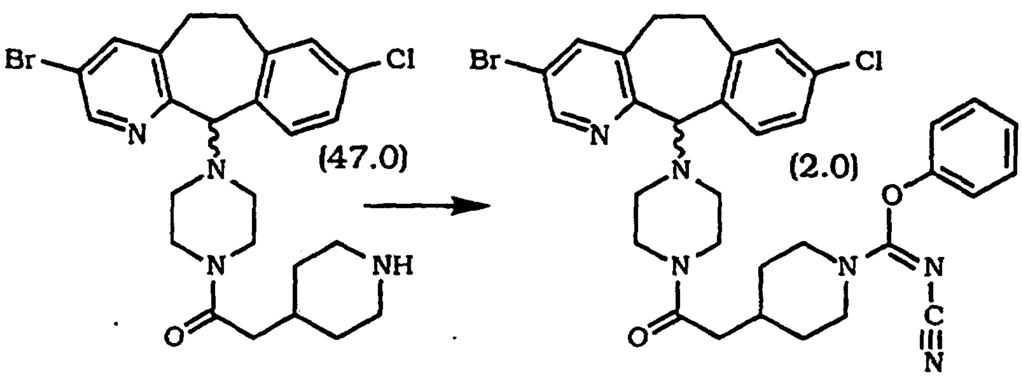


1-(3-Bromo-8-chloro-6,11-dihydro-5H-benzo[5,6]-cyclohepta[1,2-b] pyridin-11-yl)-4-[(4-piperidinyl)acetyl]-piperazine (Formula 47.0) (prepared as described in Preparative Example 11) (2.5g) (1 equivalent) and diphenylcyanocarbon-imidate (1.38g) (1.2 equivalents) were dissolved in 2-propanol (65ml) and the solution was heated at 80°C under reflux and under nitrogen for 24h. The mixture was evaporated to dryness and the product was chromatographed on a silica gel column (60X2.5cm) using neat ethyl acetate as the eluant to give the title compound (Formula 2.0) (2.7921g; 87%), FABMS: m/z 661 ($MH^+$).

| $\delta_C$ ($CDCl_3$) for Formula 2.0 | | |
|---|---|---|
| Tricyclic | $CH_2$:<br>CH:<br>C: | 30.5, 30.6<br>147.1, 141.4, 132.5, 126.3, 130.5, 79.0<br>120.1, 140.9, 134.3, 135.3, 136.8, 155.5 |
| Piperazine | $CH_2$: | 41.7, 51.4, 51.9, 45.6 |
| Piperazine N-substituent | $CH_2$:<br>CH:<br>C: | 46.4, 32.1, 32.1, 46.4, 38.8,<br>32.5, 118.5, 118.5, 130.1, 130.1, 125.7<br>113.5, 152.5, 157.4, 169.3 |

EXAMPLE 2

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-CYANO-1-PIPERIDINE-CARBOXIMIDAMIDE

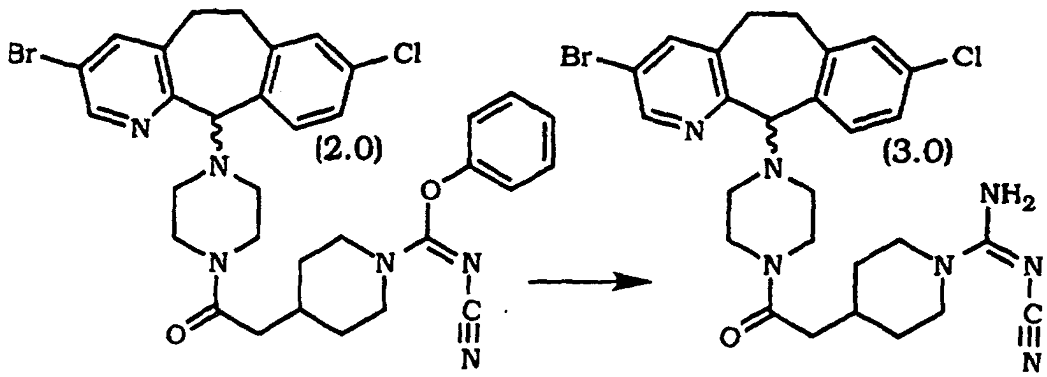

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-N-cyano-1-piperidinecarboximidate (2.62g) (Formula 2.0, prepared as described in Example 1 above) was dissolved in 2-propanol (50ml) and concentrated ammonium hydroxide (4ml) was added. The mixture was stirred at 25°C for 24h and then evaporated to dryness. The residue was triturated with diethyl ether (2X250ml) and the ether was discarded. The remaining product was chromatographed on a silica gel column (60X2.5cm) using 4% (10% concentrated ammonium hydroxide in methanol)-dichloromethane as the eluant to give the title compound (Formula 3.0) (1.967g;85%). FABMS: m/z 584.2 ($MH^+$).

| $\delta_c$ ($d_6$-DMSO) for Formula 3.0 | | |
|---|---|---|
| Tricyclic | $CH_2$:<br>CH:<br>C: | 29.0, 29.4<br>146.1, 140.7, 132.4, 125.4, 129.9, 77.7<br>119.1, 141.1, 132.4, 135.4, 136.7, 155.5 |
| Piperazine | $CH_2$: | 40.6, 50.8, 51.3, 44.6 |
| Piperazine N-substituent | $CH_2$:<br>CH:<br>C: | 44.3, 44.3, 30.9, 30.9, 38.3<br>31.9<br>118.1, 159.3, 168.8 |

EXAMPLE 3

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-1-PIPERIDINECARBOXIMIDATE

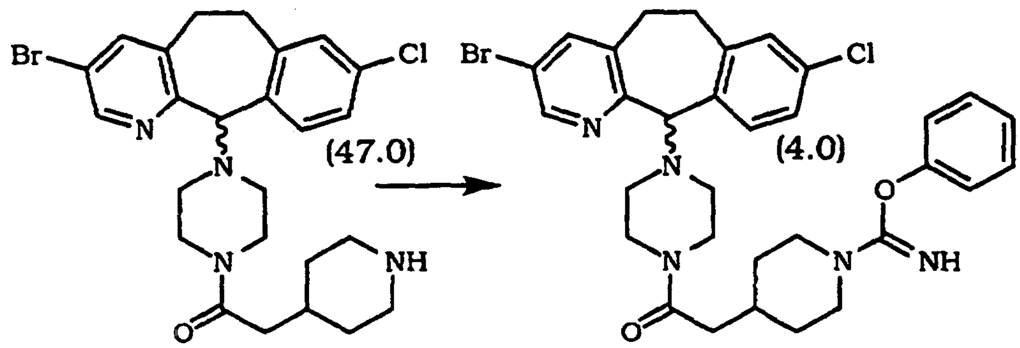
(47.0) (4.0)

1-(3-Bromo-8-chloro-6,11-dihydro-5H-benzo[5,6]cyclo-hepta[1,2-b]pyridin-11-yl)-4-[(4-piperidinyl)acetyl]piperazine (6g) (1 equivalent) (Formula 47.0 from Preparative Example 11 above) was dissolved in anhydrous dichloromethane (71.1ml). Phenyl cyanate (2.2752ml) (2 equivalents) and diisopropylethylamine (100 drops) were added and the mixture was stirred at 25°C for 15 minutes. The reaction mixture was directly introduced onto a silica gel column (15X5cm) and eluted with 10% increasing to 20% (10% concentrated ammonium hydroxide in methanol)-dichloromethane to give the title compound (6.66g; 92%), FABMS: m/z 635.9 ($MH^+$).

| $\delta_c$ ($CDCl_3$) for Formula 4.0 | | |
|---|---|---|
| Tricyclic | $CH_2$:<br>CH:<br>C: | 30.6, 30.5<br>147.1, 141.3, 132.5, 126.3, 130.5, 79.0<br>120.1, 140.9, 134.2, 135.3, 136.8, 155.6 |
| Piperazine | $CH_2$: | 41.6, 51.4, 51.9, 45.8 |
| Piperazine N-substituent | $CH_2$:<br>CH:<br>C: | 45.3, 45.3, 32.0, 32.0, 39.5,<br>32.2, 122.1, 122.1, 130.0, 130.0, 125.7<br>151.4, 159.3, 170.0 |

EXAMPLE 4

1-(3-BROMO-8-CHLORO-6,11-DIHYDRO-5H-BENZO[5,6] CYCLOHEPTA[1,2-b]PYRIDIN-11-YL)-4-[4-(1-CYANO-4-PIPERIDINYL)ACETYL]PIPERAZINE

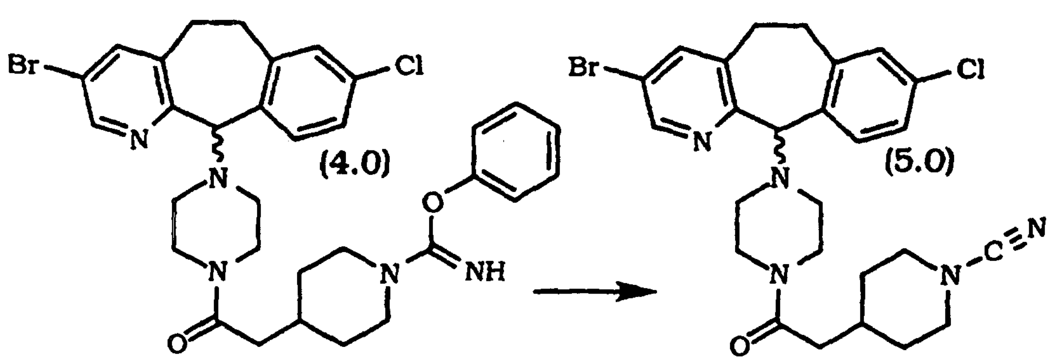
(4.0) (5.0)

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-1-piperidinecarboximidate (0.3g) (1 equivalent) (Formula 4.0, prepared as described in Example 3 above) was dissolved in anhydrous THF (11.2ml). A 60% sodium hydride dispersion in oil (0.0753g) (4 equivalents) was added and the mixture was stirred at 25°C for 2h. The mixture was diluted with dichloromethane and washed with 1.0N sodium hydroxide. The dichloromethane layer was dried over magnesium sulfate, filtered and evaporated to dryness. The product was chromatographed on a silica gel column (30X2.5cm) using 1.5% (10% concentrated ammonium hydroxide in methanol)-dichloromethane as the eluant to give the title compound (Formula 5.0) (0.1824g; 71%), FABMS: m/z 542.2 ($MH^+$).

| $\delta_C$ ($CDCl_3$) for Formula 5.0 | | |
|---|---|---|
| Tricyclic | $CH_2$:<br>CH:<br>C | 30.6, 30.5<br>147.1, 141.4, 132.5, 126.3, 130.6, 79.0<br>120.1, 140.9, 134.3, 135.3, 136.9, 155.6 |
| Piperazine | $CH_2$: | 41.6, 51.4, 51.9, 45.6 |
| Piperazine N-substituent | $CH_2$:<br>CH:<br>C: | 49.7, 49.7, 31.1, 31.1, 39.1<br>31.8<br>118.4, 169.3 |

EXAMPLE 5

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-SULFAMOYL-1-PIPERIDINE-CARBOXIMIDATE

PROCEDURE 1:

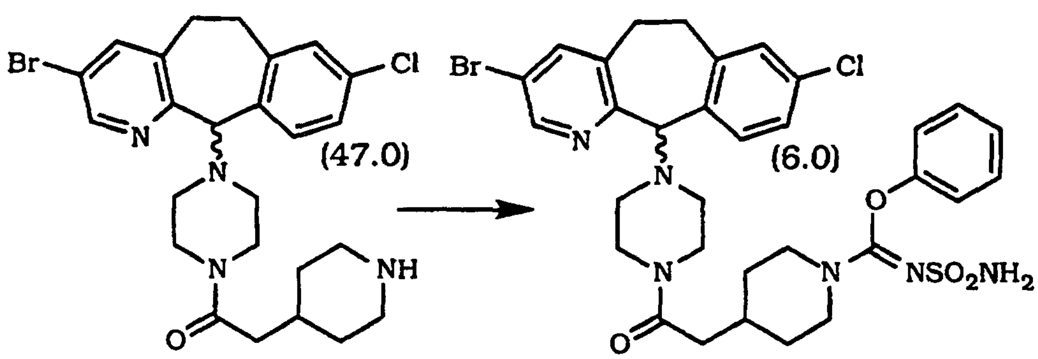

If one were to follow Procedure 1 then one would obtain the compound of Formula 6.0.

1-(3-Bromo-8-chloro-6,11-dihydro-5H-benzo[5,6]cyclo-hepta[1,2-b] pyridin-11-yl)-4-[(4-piperidinyl)acetyl] piperazine (1 equivalent) (Formula 47.0 of Preparative Example 11) and diphenylsulfamoylcarbonimidate (1.2 equivalents) [prepare as described in: M. Haake and B. Schummelfeder, Synthesis, 753-758 (1991)] are dissolved in 2-propanol and the mixture is heated as described in Example 1 above to give the title compound (Formula 6.0).

PROCEDURE 2:

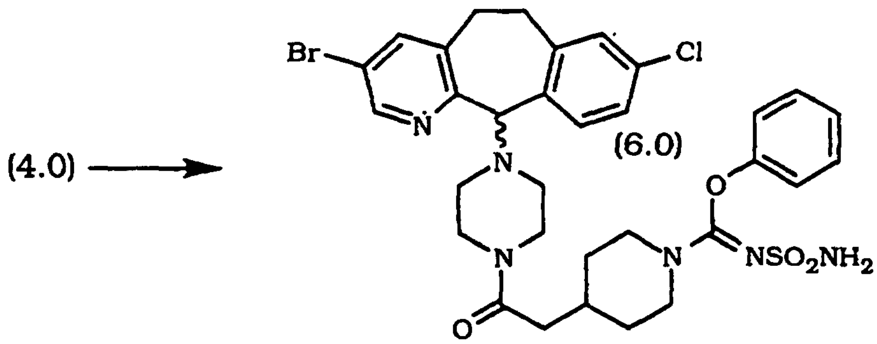

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-1-piperidinecarboximidate (0.05g)(1 equivalent) (Formula 4.0, prepared as described in Example 3 above) was dissolved in anhydrous dichloromethane (2ml) and triethylamine (0.0328ml) (3 equivalents) was added. Sulfamoyl chloride (0.0091g) (1 equivalent) [prepared as described in: R. Appel and G. Berger, Chem. Ber., 91, 1339-1341 (1958)] was added and the mixture was stirred under argon at 25°C for 18h. Additional sulfamoyl chloride (0.0091g) (1 equivalent) was added and the stirring was continued for a total of 90h. The mixture was diluted with dichloromethane and extracted with 1N sodium hydroxide. The dichloromethane layer was dried over magnesium sulfate, filtered and evaporated to dryness. The product was chromatographed on a silica gel column (15X1cm) using 2% increasing to 4% (10% concentrated ammonium hydroxide in methanol)-dichloro-methane as the eluant to give the title compound (Formula 6.0) (0.0054g; 10%), FABMS: m/z 715.4 ($MH^+$), $\delta_H$ ($CDCl_3$) 7.03 (2H, m, ArH), 7.09-7.18 (4H, m, ArH), 7.37 (1H, m, ArH), 7.39 (1H, m, ArH), 7.59 (1H, s, ArH) and 8.37 ppm (1H, s, ArH).

EXAMPLE 6

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-SULFAMOYL-1-PIPERIDINE-CARBOXIMIDAMIDE

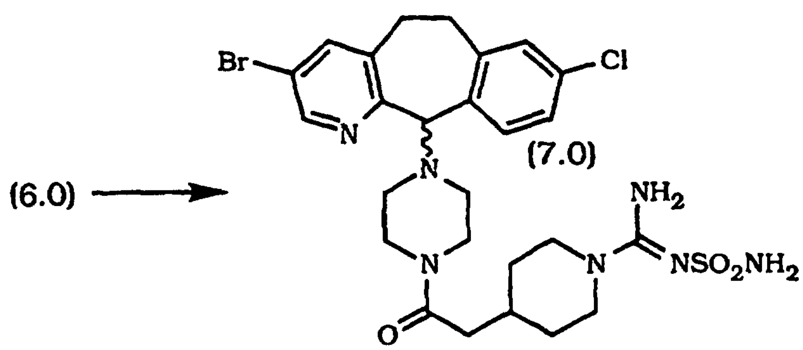

Phenyl 4-[2-[4-(3-brorno-8-chloro-6,11-dihydro-5H-benzo[5,6]cyclohepta[1,2-b]pyridin-11-yl)-1-piperazinyl]-2-oxoethyl]-N-sulfamoyl-1-piperidinecarboximidate (Formula 6.0) (prepared as described in Example 5 above) (0.089g, 0.1mmoles) was dissolved in anhydrous THF (1ml). A saturated solution of ammonia in anhydrous THF (4ml) was added and the mixture was stirred in a sealed reaction vessel at 25°C for 19h. The mixture was evaporated to dryness and chromatographed on silica gel using 2%-6% methanol in dichloromethane as the eluant to give the title compound (Formula 7.0) (0.0667g, 84%), ESIMS: m/z 638.0 ($MH^+$).

| $\delta_C$ ($CDCl_3$) | | |
|---|---|---|
| Tricyclic | $CH_2$:<br>CH:<br>C: | 30.5, 30.6<br>78.9, 126.3, 130.5, 132.6, 141.4, 147.0<br>120.1, 134.2, 135.3, 136.9, 140.9, 155.6 |
| Piperazine | $CH_2$: | 41.7, 45.7, 51.4, 51.9 |
| Piperazine N-substituent | $CH_2$:<br>CH:<br>C: | 31.8, 31.8, 39.0, 44.9, 44.9<br>32.8<br>169.8 |

EXAMPLE 7

PHENYL 4-[2-[4-(3-BROMO-8-CHLORO-6,11-DIHYDRO-5H-BENZO[5,6]CYCLOHEPTA[1,2-b]PYRIDIN-11-YL]-1-PIPERAZINYL]-2-OXOETHYL]-N-(N-METHYLSULFAMOYL)-1-PIPERIDINECARBOXIMIDATE

PROCEDURE 1:

(4.0)

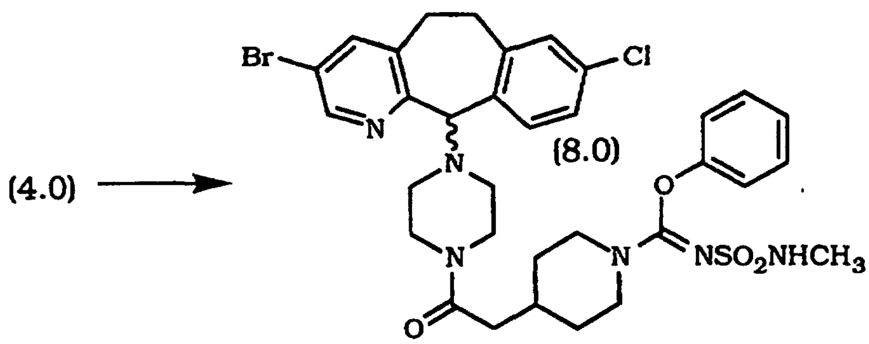

(8.0)

If one were to follow Procedure 1 then one would obtain the compound of Formula 8.0.

Phenyl 4-[2-[4-(3-bromo-8-chloro-6,11-dihydro-5H-benzo[5.6] cyclohepta[1,2-b]pyridin-11-yl)-1-piperazinyl]-2-oxoethyl]-1-piperidinecarboximidate (1 equivalent) (Formula 4.0, prepare as described in Example 3 above) is dissolved in an inert anhydrous solvent such as acetonitrile, benzene, or toluene and triethylamine (2 equivalents) is added. The solution is cooled to 0°C and N-methylsulfamoyl chloride (1.2 equivalents) [prepare as described in: J. A. Kloek and K.L. Leschinsky, J. Org. Chem., 41(25), 4028-4029 (1976)] is added. The mixture is stirred at 0°C to25°C for 3h and is worked up to give the title compound (Formula 8.0).

PROCEDURE 2:

(47.0)

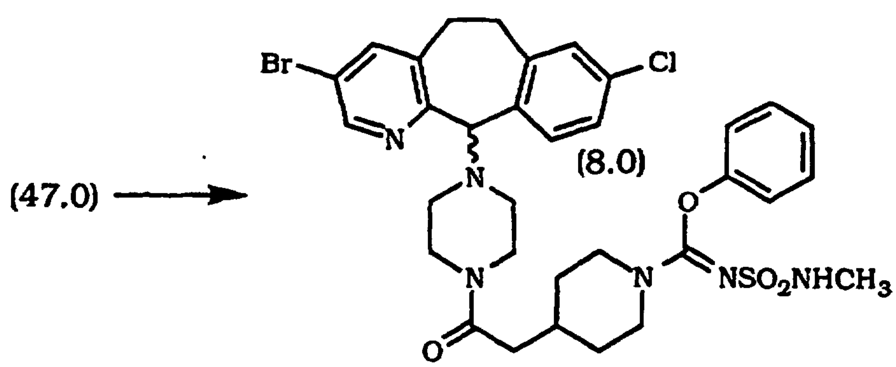

(8.0)

If one were to follow Procedure 2 then one would obtain the compound of Formula 8.0.

1-(3-Bromo-8-chloro-6,11-dihydro-5H-benzo[5,6]cyclo-hepta[1,2-b] pyridin-11-yl)-4-[(4-piperidinyl)acetyl] piperazine (1 equivalent) (Formula 47.0, prepare as described in Preparative Example 11) and diphenylmethylsulfamoylcarbonimidate (1.2 equivalents) [prepare by the same procedure, only using methylsulfamoyl chloride, as described in: A. Buschauer, Arch. Pharm., 377-378 (1987)] are dissolved in 2-propanol and the mixture is heated as described in Example 1 above to give the title compound.

EXAMPLE 8

4-[2-[4-[3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-(N-METHYLSULFAMOYL)-1-PIPERIDINECARBOXIMIDAMIDE

(8.0)

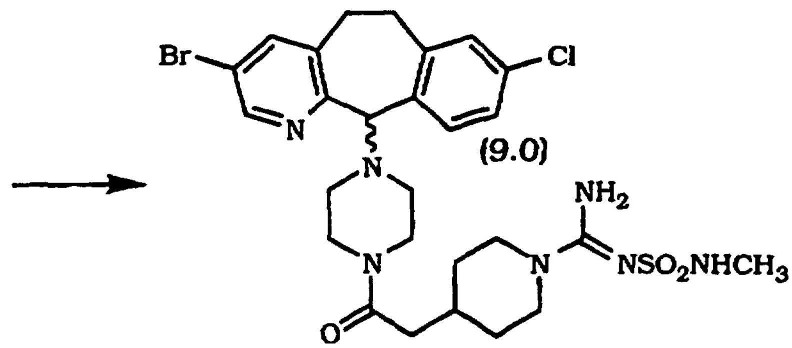

(9.0)

If one were to follow this procedure then the compound of Formula 9.0 would be obtained.

Phenyl 4-[2-[4-(3-bromo-8-chloro-6,11-dihydro-5H-benzo-[5,6]cyclohepta[1,2-b]pyridin-11-yl)-1-piperazinyl]-2-oxoethyl]-N-(N-methyl-sulfamoyl)-1-piperidinecarboximidate (Formula 8.0, prepare as described in Example 7 above) is dissolved in 2-propanol and concentrated ammonium hydroxide is added. The mixture is stirred at 25°C for 24h and then is evaporated to dryness. The residue is triturated with diethyl ether (2X250ml) and the ether is discarded. The remaining product is chromatographed on a silica gel column to give the title compound (Formula 9.0).

Alternatively, anhydrous ammonia in a suitable inert solvent such as methanol, or THF may be used in place of ammonium hydroxide in the above reaction.

EXAMPLE 9

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-(N,N-DIMETHYLSULFAMOYL)-1-PIPERIDINECARBOXIMIDATE

PROCEDURE 1:

(4.0)

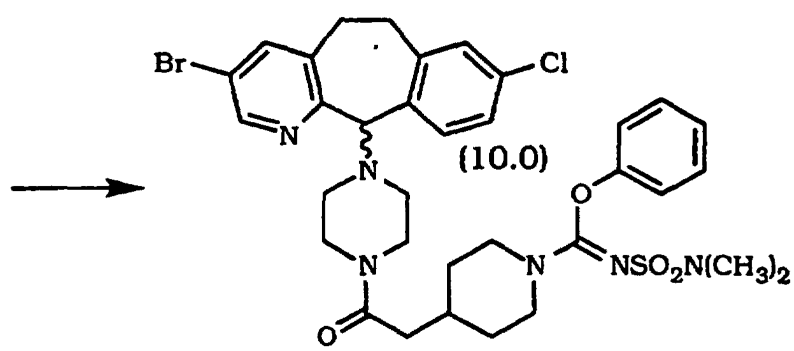

(10.0)

If one were to follow Procedure 1 then the compound of Formula 10.0 would be obtained.

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-1-piperidinecarboximidate (1 equivalent) (Formula 4.0, prepare as described in Example 3 above) is dissolved in an inert anhydrous solvent such as acetonitrile, benzene, or toluene and triethylamine (2 equivalents) is added. The solution is cooled to 0°C and N,N-dimethylsulfamoyl chloride (1.2 equivalents) is added. The mixture is stirred at 0°C to25°C for 3h and worked up to give the title compound.

PROCEDURE 2:

(47.0)

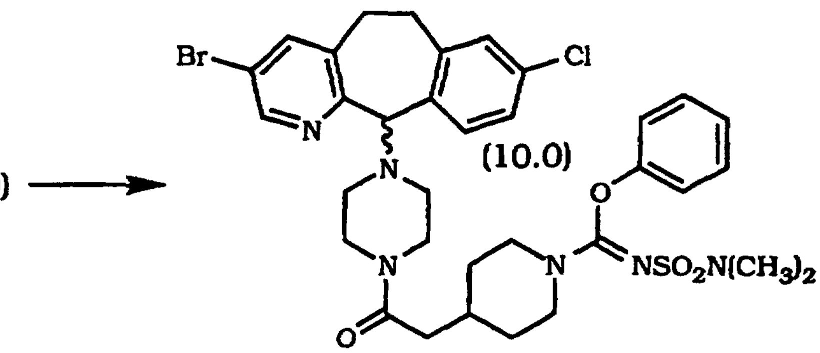

(10.0)

If one were to follow Procedure 2 then the compound of Formula 10.0 would be obtained.

1-(3-Bromo-8-chloro-6,11-dihydro-5H-benzo[5,6]cyclo-hepta[1,2-b] pyridin-11-yl)-4-[(4-piperidinyl)acetyl] piperazine (1 equivalent) (Formula 47.0, prepare as described in Preparative Example 11) and diphenyldimethylsulfamoylcarbonimidate (1.2 equivalents) [prepare by the same procedure, only using dimethylsulfamoyl chloride, as is described in: A. Buschauer, Arch. Pharm., 377-378 (1987)] are dissolved in 2-propanol and the mixture is heated as described in Example 1 above to give the title compound (Formula 10.0).

EXAMPLE 10

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-(N,N-DIMETHYLSULFAMOYL)-1-PIPERIDINECARBOXIMIDAMIDE

(10.0)

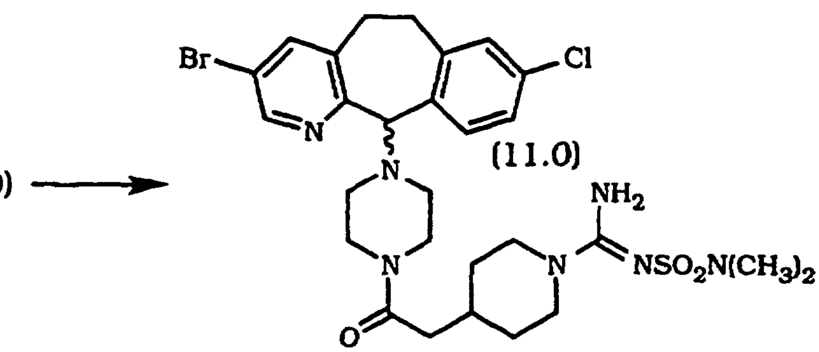

(11.0)

If one were to follow this procedure then the compound of Formula 11.0 would be obtained.

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-N-(N,N-dimethyl-sulfamoyl)-1-piperidinecarboximidate (Formula 10.0, prepare as described in Example 9 above) is dissolved in 2-propanol and concentrated ammonium hydroxide is added. The mixture is stirred at 25°C for 24h and then evaporated to dryness. The residue is triturated with diethyl ether (2X250ml) and the ether is discarded. The remaining product is chromatographed on a silica gel column to give the title compound (Formula 11.0).

Alternatively, anhydrous ammonia in a suitable inert solvent such as methanol, or THF, may be used in place

of ammonium hydroxide in the above reaction.

EXAMPLE 11

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-HYDROXY-1-PIPERIDINECARBOXIMIDATE

PROCEDURE 1:

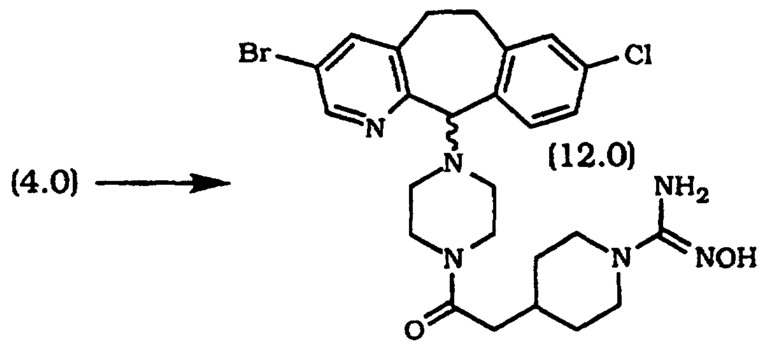
(4.0)
(12.0)

Phenyl 4-[2-[4-(3-bromo-8-chloro-6,11-dihydro-5H-benzo-[5,6]cyclohepta[1,2-b]pyridin-11-yl)-1-piperazinyl]-2-oxoethyl]-1-piperidinecarboximidate (Formula 4.0) (prepared as described in Example 3 above) (0.15g, 0.26mmoles) was dissolved in methanol (0.75ml). An aqueous solution of hydroxylamine [prepared by dissolving hydroxylamine hydrochloride (0.0164g, 0.26mmoles) in 50%(w/v) sodium hydroxide (0.0188g, 0.26mmoles) and water (0.258ml)] was added and the mixture was stirred at 25°C for 3h. Additional methanol (1.2ml) was added and the mixture was stirred at 25°C for a total of 26h. The solution was evaporated to dryness and the residue was chromatographed on silica gel using 2%-4%-5%-10%-25%-35% methanol in dichloromethane as the eluant to give the title compound (Formula 12.0) (0.0323g, 24%), which was identical with that prepared in Procedure 2 below.

PROCEDURE 2:

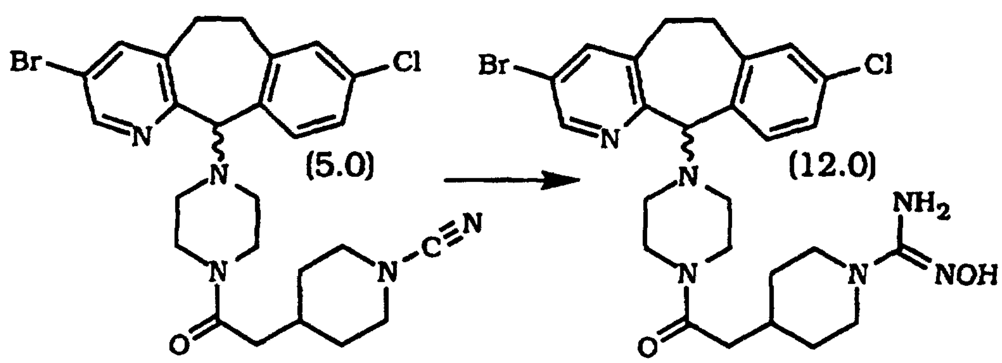
(5.0)
(12.0)

1-(3-Bromo-8-chloro-6,11-dihydro-5H-benzo[5,6]cyclo-hepta[1,2-b]pyridin-11-yl)-4-[4-(1-cyano-4-piperidinyl)acetyl]-piperazine (Formula 5.0) (prepared as described in Example 4 above) (0.4g, 0.737mmoles) was dissolved in methanol (2ml). An aqueous solution of hydroxylamine [prepared by dissolving hydroxylamine hydrochloride (0.0512g, 0.737mmoles) in 50%(w/v) sodium hydroxide (0.0592g, 0.737mmoles) and water (0.8m1)] was added and the mixture was stirred at 25°C for 3h. Additional methanol (3.2ml) was added and the mixture was stirred at 25°C for a total of 18h. The solution was evaporated to dryness and the residue was chromatographed on silica gel using 5% methanol in dichloromethane as the eluant to give the title compound (Formula 12.0) (0.2965g, 70%), FABMS: m/z 575.0 ($MH^+$).

| $\delta_C$ ($CDCl_3$) for Formula 12.0 | | |
|---|---|---|
| Tricyclic | $CH_2$:<br>CH:<br>C | 30.6, 30.6<br>147.0, 141.4, 132.6, 126.3, 130.5, 79.0<br>120.1, 140.9, 134.2, 135.3, 136.9, 155.6 |
| Piperazine | $CH_2$: | 41.7, 51.4, 51.9, 45.8 |
| Piperazine N-substituent | $CH_2$:<br>CH:<br>C | 46.8, 46.8, 31.5, 31.5, 39.2<br>32.8<br>157.6, 170.0 |

EXAMPLE 12

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-METHOXY-1-PIPERIDINE-CARBOXIMIDATE

(4.0)

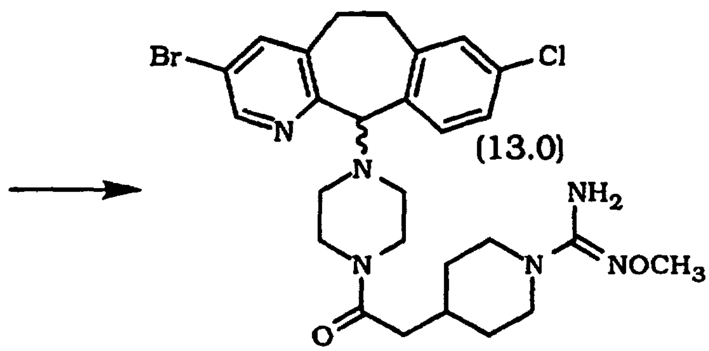

(13.0)

Phenyl 4-[2-[4-(3-bromo-8-chloro-6,11-dihydro-5H-benzo-[5.6]cyclohepta[1,2-b]pyridin-11-yl)-1-piperazinyl]-2-oxoethyl]-1-piperidinecarboximidate (Formula 4.0) (prepared as described in Example 3 above) (0.1g, 0.157mmoles) and methoxylamine hydrochloride (0.0154g. 0.157mmoles) were dissolved in anhydrous pyridine (1ml) and the mixture was stirred at 25°C for 2h. The solution was evaporated to dryness and the residue was chromatographed on silica gel using 2%-4%-10% methanol in dichloromethane as the eluant to give the title compound (Formula 13.0) (0.0741g, 80%), FABMS: m/z 589.1 ($MH^+$).

| $\delta_C$ ($CDCl_3$) | | |
|---|---|---|
| Tricyclic | $CH_2$:<br>CH:<br>C: | 30.4, 30.5<br>79.0, 126.2, 130.4, 132.4, 141.2, 147.0<br>120.0, 134.1, 135.3, 136.7, 140.8, 155.5 |
| Piperazine | $CH_2$: | 41.5, 45.7, 51.3, 51.8 |
| Piperazine N-substituent | $CH_3$:<br>$CH_2$:<br>CH:<br>C: | 61.0<br>39.4, 31.7, 31.7, 46.8, 46.8<br>33.2<br>156.7 |

EXAMPLE 13

PHENYL N-[[(AMINOCARBONYL)AMINO]CARBONYL]-4-[2-[4-(3-BROMO-8-CHLORO-6,11-DIHYDRO-5H-BENZO [5,6]CYCLOHEPTA-[1,2-b]PYRIDIN-11-YL)-1-PIPERAZINYL]-2-OXOETHYL]-1-PIPERIDINECARBOXIMIDATE

(4.0)

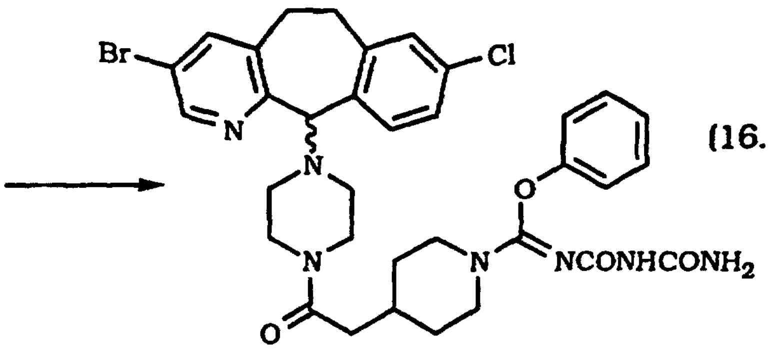

(16.1)

Phenyl 4-[2-[4-(3-bromo-8-chloro-6,11-dihydro-5H-benzo-[5,6]cyclohepta[1,2-b]pyridin-11-yl)-1-piperazinyl]-2-oxoethyl]-1-piperidinecarboximidate (Formula 4.0) (prepared as described in Example 3 above) (0.35g, 0.549mmoles) was dissolved in anhydrous dichloromethane (7ml) and trimethylsilylisocyanate (1.484ml, 10.98mmoles) was added. The mixture was stirred at 25°C for 21h. The mixture was diluted with dichloromethane, washed with saturated aqueous sodium bicarbonate, water, dried over $MgSO_4$, filtered and evaporated to dryness. The residue was chromatographed on silica gel using 2%-3% methanol in dichloromethane as the eluant to give the title compound (Formula 16.1) (0.1302g, 33%), FABMS: m/z 721.9 ($MH^+$).

| $\delta_C$ ($CDCl_3$) | | |
|---|---|---|
| Tricyclic | $CH_2$:<br>CH:<br>C: | 30.5, 30.6<br>79.0, 126.4, 130.6, 132.5, 141.4, 147.1<br>120.1, 134.3, 135.3, 136.8, 140.9, 155.5 |
| Piperazine | $CH_2$: | 41.7, 45.7, 51.4, 51.9 |
| Piperazine N-substituent | $CH_2$:<br>CH:<br>C: | 32.0, 32.0, 38.9, 46.3, 46.3<br>32.7, 119.0, 119.0, 125.0, 129.6, 129.6<br>153.0, 155.2, 157.6, 169.4 |

EXAMPLE 14

N-[[(AMINOCARBONYL)AMINO]CARBONYL]-4-[2-[4-(3-BROMO-8-CHLORO-6,11-DIHYDRO-5H-BENZO[5,6] CYCLOHEPTA[1,2-b]-PYRIDIN-11-YL)-1-PIPERAZINYL]-2-OXOETHYL]-1-PIPERIDINECARBOXIMIDAMIDE

(16.1)

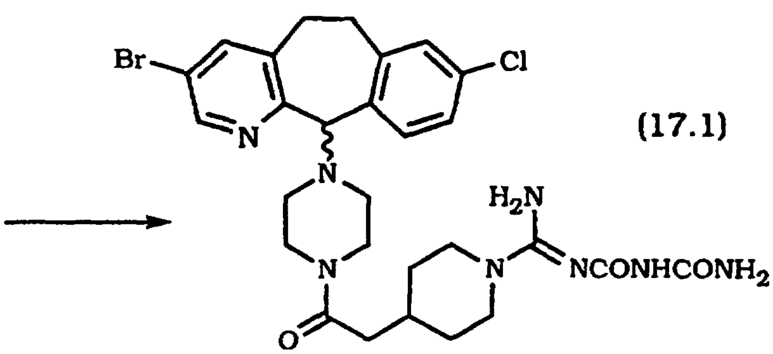

(17.1)

Phenyl N-[[(aminocarbonyl)amino]carbonyl]-4-[2-[4-(3-bromo-8-chloro-6,11-dihydro-5H-benzo[5,6]cyclohepta[1,2-b]pyridin-11-yl)-1-piperazinyl]-2-oxoethyl]-1-piperidine-carboximidate (Formula 16.1) (0.2943g, 0.462mmoles) was dissolved in a saturated solution of ammonia in anhydrous THF (80ml) and the mixture was stirred at 25°C for 19h in a sealed vessel. The solution was evaporated to dryness and the residue was chromatographed on silica gel using 3% (10% conc. ammonium hydroxide in methanol)-dichloromethane as the eluant to give the title compound (Formula 17.1) (0.2663g, 40%), ESIMS: m/z 645.2 ($MH^+$).

| $\delta_C$ ($CDCl_3$) | | |
|---|---|---|
| Tricyclic | $CH_2$:<br>CH:<br>C | 30.5, 30.6<br>79.0, 126.3, 130.6, 132.5, 141.3, 147.1<br>120.1, 134.3, 135.3, 136.8, 140.9, 155.5 |
| Piperazine | $CH_2$: | 41.7, 45.7, 51.4, 51.9 |
| Piperazine N-substituent | $CH_2$:<br>CH:<br>C: | 31.9, 31.9, 39.1, 44.3, 44.3<br>32.9<br>156.1, 159.4, 162.6, 169.6 |

EXAMPLE 15

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-CARBOXAMIDO-1-PIPERIDINECARBOXIMIDATE

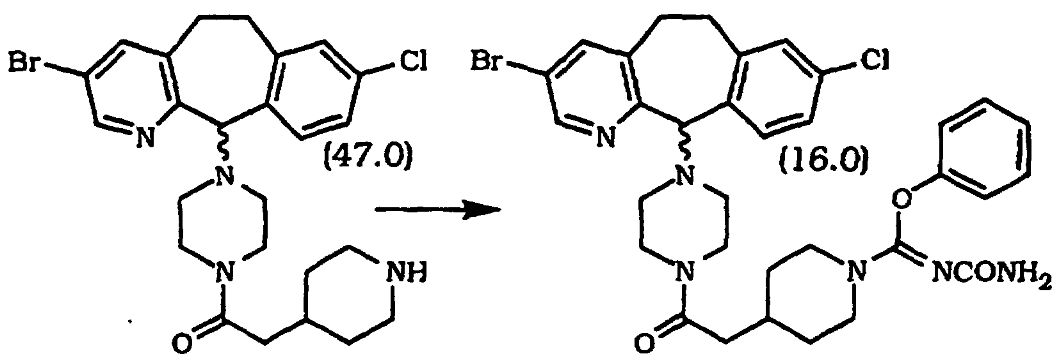
(47.0) (16.0)

If one were to follow Procedure 1 then the compound of Formula 16.0 would be obtained.

1-(3-Bromo-8-chloro-6,11-dihydro-5H-benzo[5,6]cyclo-hepta[1,2-b] pyridin-11-yl)-4-[(4-piperidinyl)acetyl] piperazine (1 equivalent) (Formula 47.0, prepare as described in Preparative Example 11) and diphenylcarboxamidocarbonimidate (1.2 equivalents) [prepare, using urea in place of sulfamide, as is described in: M. Haake and B. Schummelfeder, Synthesis, 753-758 (1991)] is dissolved in 2-propanol and the solution is heated at 80°C under reflux and under nitrogen for 24h. The mixture is evaporated to dryness and chromatographed on a silica gel column to give the title compound (Formula 16.0).

EXAMPLE 16

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-CARBOXAMIDO-1-PIPERIDINECARBOXIMIDAMIDE

(16.0)

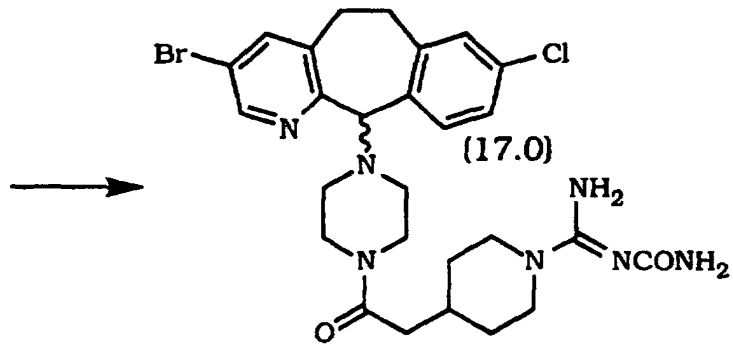

(17.0)

If one were to follow Procedure 1 then the compound of Formula 17.0 would be obtained.

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-N-carboxamido-1-piperidinecarboximidate (formula 16.0, prepare as described in Example 15 above) is dissolved in 2-propanol and concentrated ammonium hydroxide is added. The mixture is stirred at 25°C for 24h and then evaporated to dryness. The residue is triturated with diethyl ether (2X250ml) and the ether is discarded. The remaining product is chromatographed on a silica gel column to give the title compound (Formula 17.0).

Alternatively, anhydrous ammonia in a suitable inert solvent such as methanol, or THF, may be used in place of ammonium hydroxide in the above reaction.

EXAMPLE 17

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-(N'-METHYLCARBOXAMIDO)-1-PIPERIDINECARBOXIMIDATE

(4.0)

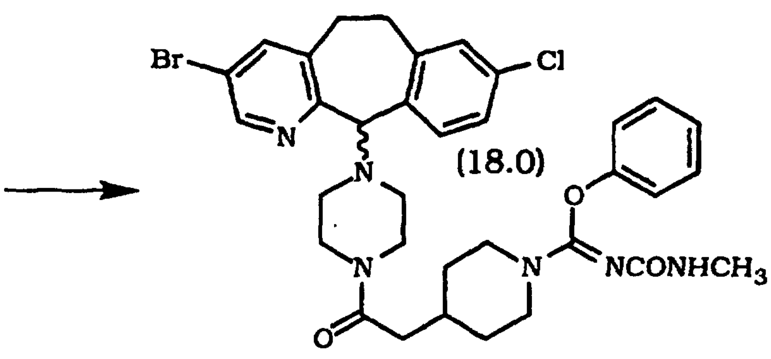

(18.0)

If one were to follow this procedure then the compound of Formula 18.0 would be obtained.

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-1-piperidinecarboximidate (1 equivalent) (Formula 4.0, prepare as described in Example 3 above) is dissolved in anhydrous dichloromethane. Methylisocyanate (2 equivalents) is added and the mixture is stirred at 25°C for 48h. The mixture is shaken with aqueous sodium bicarbonate and is extracted with dichloromethane. The latter is dried over $MgSO_4$, is filtered and is evaporated to dryness to give the title compound (Formula 18.0) after chromatography on silica gel

EXAMPLE 18

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-(N'-METHYLCARBOXAMIDO)-1-PIPERIDINECARBOXIMIDAMIDE

(18.0)

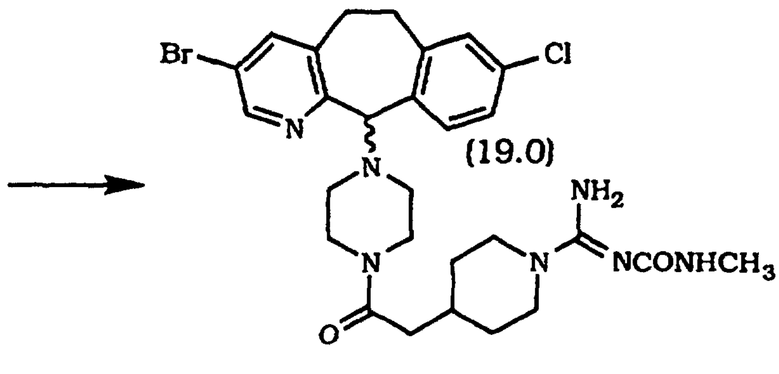

(19.0)

If one were to follow this procedure then the compound of Formula 19.0 would be obtained.

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-N-(N'-methylcarboxamido)-1-piperidinecarboximidate (Formula 18.0, prepare as described in Example 17 above) is dissolved in 2-propanol and concentrated ammonium hydroxide is added. The mixture is stirred at 25°C for 24h and then evaporated to dryness. The residue is triturated with diethyl ether (2X250ml) and the ether is discarded. The remaining product is chromatographed on a silica gel column to give the title compound (Formula 19.0).

Alternatively, anhydrous ammonia in a suitable inert solvent such as methanol, or THF, may be used in place of ammonium hydroxide in the above reaction.

EXAMPLE 19

N-[4-[2-[4-(-3-BROMO-8-CHLORO-6,11-DIHYDRO-5H-BENZO [5,6]CYCLOHEPTA[1,2-b]PYRIDIN-11-YL)-1-PIPERAZINYL]-2-OXOETHYL]-1-PIPERIDINYL]-N'-METHYL-2-NITRO-1-ETHENEAMINE

(47.0)

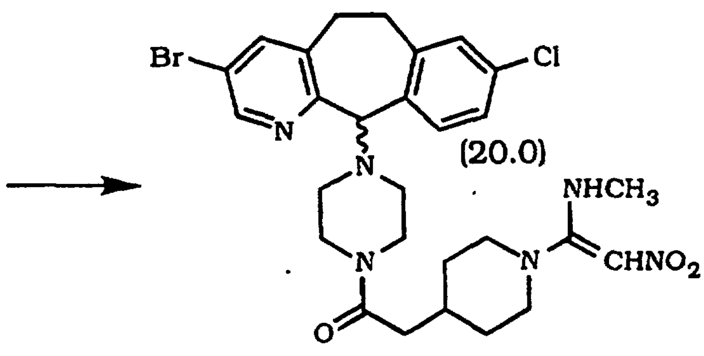

(20.0)

If one were to follow this procedure then the compound of Formula 20.0 would be obtained.

Copper(I)chloride (1 equivalent) is dissolved in anhydrous acetonitrile. To this solution, a solution of 1-(3-bromo-8-chloro-6,11-dihydro-5H-benzo[5,6]cyclohepta[1,2-b]pyridin-11-yl)-4-[(4-piperidinyl)acetyl]-piperazine (1 equivalent) (Formula 47.0, prepare as described in Preparative Example 11), 1-methylthio-1-methyl-amino-2-nitroethene (1 equivalent) (prepare as described in Canadian Patent No. 1178289 (1984)) and triethylamine in anhydrous acetonitrile is added dropwise over 10 minutes with stirring. The solid is filtered off, the volume is reduced and dichloromethane is added. The mixture is washed with aqueous sodium bicarbonate and the dichloromethane layer is dried over magnesium sulfate, filtered and evaporated to dryness. The residue is purified on silica gel to give the title compound Formula 20.0).

EXAMPLE 20

N-[4-[2-[4-(8-CHLORO-6,11-DIHYDRO-5H-BENZO [5,6]CYCLOHEPTA[1,2-b]PYRIDIN-11-YL)-1-PIPERAZINYL]-2-OXOETHYL]-1-PIPERIDINYL]-N'-METHYL-2-NITRO-1-ETHENEAMINE

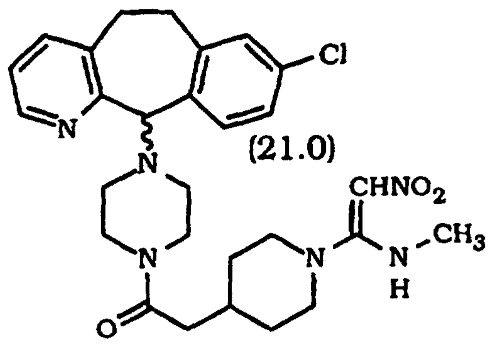

(21.0)

If one were to follow this procedure then the compound of Formula 21.0 would be obtained.

STEP A:

N-[4-[2-[4-(8-CHLORO-6,11-DIHYDRO-5H-BENZO [5,6]CYCLOHEPTA[1,2-b]PYRIDIN-11-YL)-1-PIPERAZINYL]-2-OXOETHYL]-1-PIPERIDINYL]-N'-METHYL-METHYLTHIO-CARBOXIMIDATE

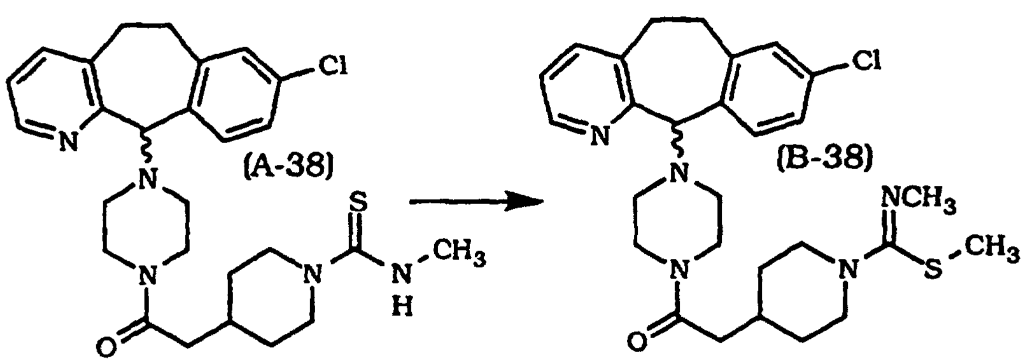

(A-38) (B-38)

4-[2-[4-(8-Chloro-6,11-dihydro-5H-benzo[5,6]cyclohepta-[1,2-b] pyridin-11-yl)-1-piperazinyl]-2-oxoethyl]-N-methyl-1-piperidinecarbothioamide (1 equivalent) (Formula A-38) is dissolved in methanol and methyl iodide (1.2 equivalents) is added over 15 minutes. The mixture is then heated under reflux for 2h. The solution is evaporated to dryness and the residue is dissolved in water and basified with 50% sodium hydroxide and then saturated with solid sodium chloride. The mixture is extracted with dichloromethane, dried over magnesium sulfate, filtered and evaporated to dryness to give the title compound (Formula B-38).

The compound of Formula A-38 is prepared by the reaction of the compound of Preparative Example 12 above with $CH_3NCS$. The reaction is conducted in anhydrous $CH_2Cl_2$, with stirring, under argon at 25°C until the reaction is complete. The reaction mixture is diluted with $CH_2Cl_2$ an is washed with saturated $NaHCO_3$ (aqueous), then water. Dry over $MgSO_4$, concentrate *in vacuo* to a residue and chromatograph (silica gel, 5% (10%$NH_4OH$ in MeOH) /$CH_2Cl_2$ to give the compound of Formula A-38.

STEP B:

N-[4-[2-[4-(8-CHLORO-6,11-DIHYDRO-5H-BENZO [5,6]CYCLOHEPTA[1,2-b]PYRIDIN-11-YL)-1-PIPERAZINYL]-2-OXOETHYL]-1-PIPERIDINYL]-N'-METHYL-2-NITRO-1-ETHENEAMINE

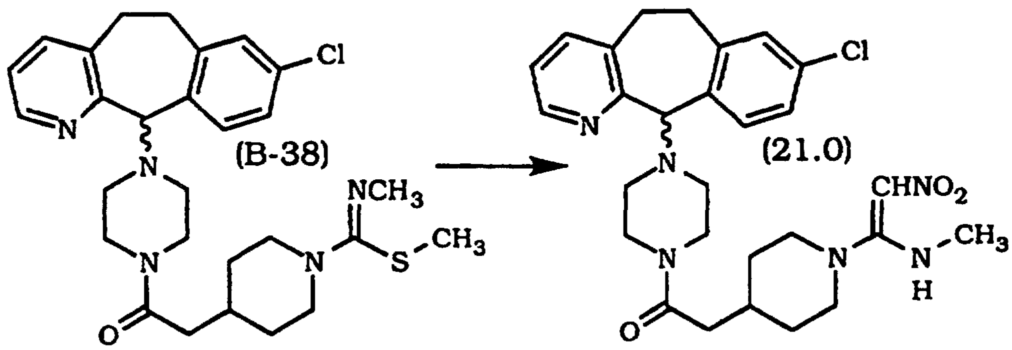

(B-38) (21.0)

The crude product from Step A above (Formula B-38) is refluxed with excess nitromethane for 16h and then is evaporated to dryness to give the title compound (Formula 21.0).

EXAMPLE 21

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-(METHYLSULFONYL)-1-PIPERIDINECARBOXIMIDATE

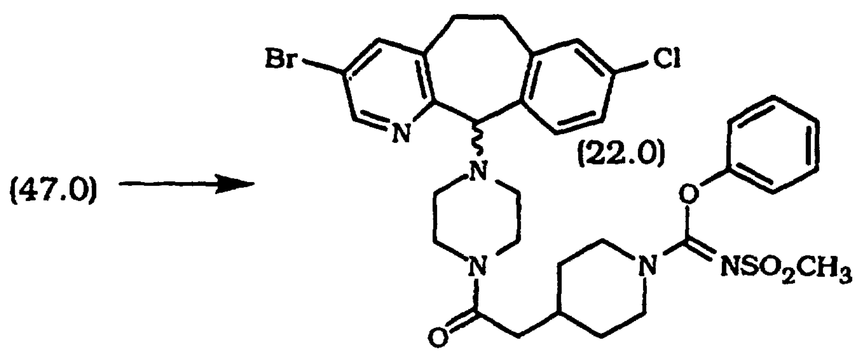

(47.0) (22.0)

If one were to follow this procedure then the compound of Formula 22.0 would be obtained.

1-(3-Bromo-8-chloro-6,11-dihydro-5H-benzo[5,6]cyclohepta[1,2-b] pyridin-11-yl)-4-[(4-piperidinyl)acetyl]piperazine (1 equivalent) (Formula 47.0, prepare as described in Preparative Example 11) and diphenylmethylsulfonylcarbonimidate (1.2 equivalents) [prepare as described in: A. Buschauer, Arch. Pharm., 377-378 (1987)] are dissolved in 2-propanol and the mixture is heated as described in Example 1 above to give the title compound (Formula 22.0).

EXAMPLE 22

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-(METHYLSULFONYL)-1PIPERIDINECARBOXIMIDAMIDE

(22.0)

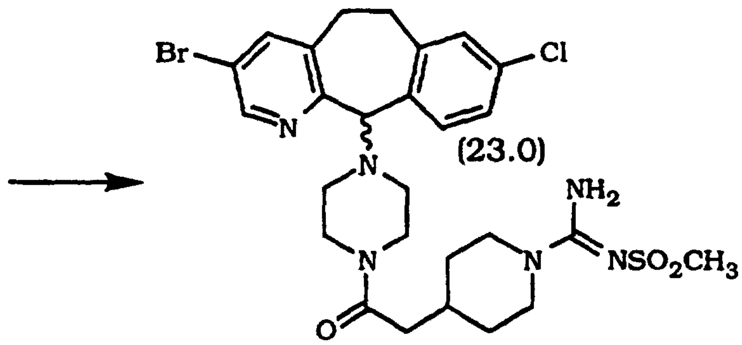

(23.0)

If one were to follow this procedure then the compound of Formula 23.0 would be obtained.

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-N-(methylsulfonyl)-1-piperidinecarboximidate (Formula 22.0, prepare as described in Example 21 above) is dissolved in 2-propanol and concentrated ammonium hydroxide is added. The mixture is stirred at 25°C for 24h and then is evaporated to dryness. The residue is triturated with diethyl ether (2X250ml) and the ether is discarded. The remaining product is chromatographed on a silica gel column to give the title compound (Formula 23.0).

Alternatively, anhydrous ammonia in a suitable inert solvent such as methanol, or THF, may be used in place of ammonium hydroxide in the above reaction.

EXAMPLE 23

PHENYL 4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-BENZOYL-1-PIPERIDINECARBOXIMIDATE

(47.0)

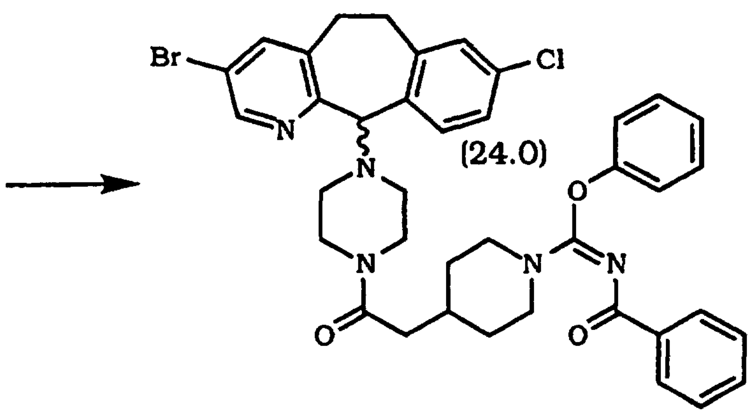

(24.0)

If one were to follow this procedure then the compound of Formula 24.0 would be obtained.

1-(3-Bromo-8-chloro-6, 11-dihydro-5H-benzo[5,6]cyclo-hepta[1,2-b] pyridin-11-yl)-4-[(4-piperidinyl)acetyl] piperazine (1 equivalent) (Formula 47.0, prepare as described in Preparative Example 11) and diphenylmethylbenzoylcarbonimidate (1.2 equivalents) [prepare as described in: A. Buschauer, Arch. Pharm., 377-378 (1987)] are dissolved in 2-propanol and the mixture is heated as described in Example 1 above to give the title compound (Formula 24.0).

EXAMPLE 24

4-[2-[4-(3-BROMO-8-CHLORO-6,11-DIHYDRO-5H-BENZO[5,6]CYCLOHEPTA[1,2-b]PYRIDIN-11-YL)-1-PIPERAZINYL]-2-OXOETHYL]-N-BENZOYL-1-PIPERIDINE-CARBOXIMIDAMIDE

(24.0)

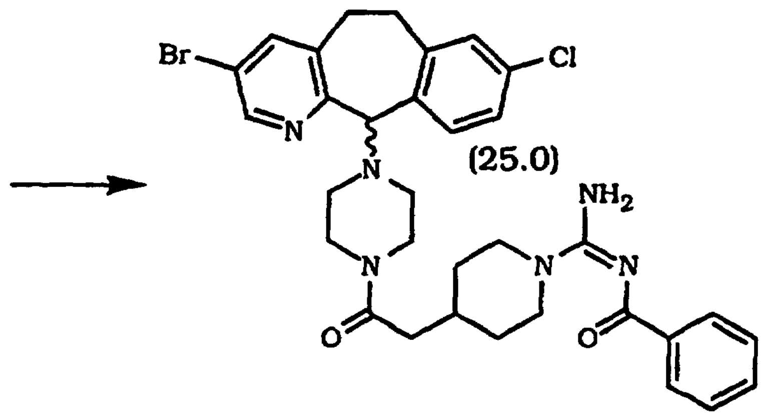

(25.0)

If one were to follow this procedure then the compound of Formula 25.0 would be obtained.

Phenyl 4-[2-[4-(3-bromo-8-chloro-6,11-dihydro-5H-benzo[5,6] cyclohepta[1,2-b]pyridin-11-yl)-1-piperazinyl]-2-oxoethyl]-N-benzoyl-1-piperidinecarboximidate (Formula 24.0, prepare as described in Example 23 above) is dissolved in 2-propanol and concentrated ammonium hydroxide is added. The mixture is stirred at 25°C for 24h and is then evaporated to dryness. The residue is triturated with diethyl ether (2X250ml) and the ether is discarded. The remaining product is chromatographed on a silica gel column to give the title compound (Formula 25.0).

Alternatively, anhydrous ammonia in a suitable inert solvent such as methanol, or THF, may be used in place of ammonium hydroxide in the above reaction.

EXAMPLE 25

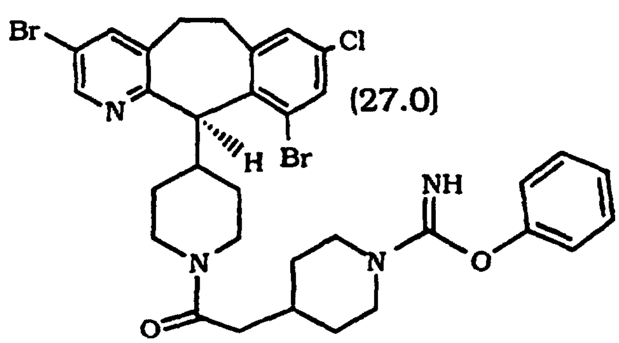

(27.0)

A solution of

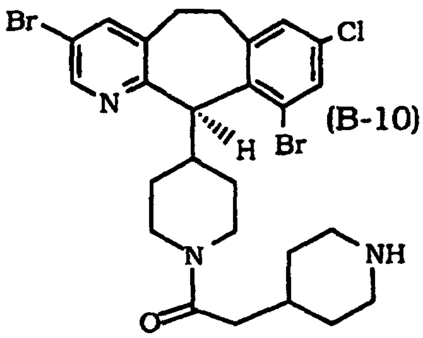

(B-10)

(Formula B-10, (+) isomer, from Step D of Preparative Example 10 above) (0.15g) in dichloromethane (1.5ml) was reacted with phenyl cyanate

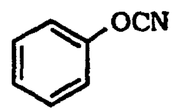

(Zweifel, *Synthesis* 150(1980)) (0.048 ml) in the presence of diisopropylethylamine (2 drops) at room temperature for one hour. The reaction mixture was then flash chromatographed on silica-gel (30 ml); elution with 10% methanol-methylene chloride followed by evaporation afforded the compound of Formula 27.0 as a colorless powder (0.15g). MS (FAB): m/e 713 ($MH^+$)

EXAMPLE 26

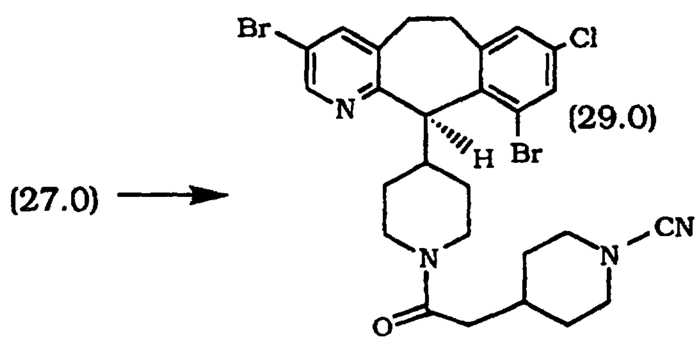

(27.0) (29.0)

A solution of the compound of Formula 27.0 (Example 25) (0.06g) in dichloromethane (2.0ml) under nitrogen was stirred with 60% sodium hydride ((0.016g) for 2hrs. at room temperature. The reaction mixture was then treated with ice-water and extracted with dichloromethane (5x5 ml); the combined extracts were dried, evaporated and the crude reaction product was chromatographed on silica-gel (20ml). Elution with 5% methanol-methylene chloride followed by evaporation afforded the compound of Formula 29.0 as a colorless powder ((0.042g). MS (FAB): m/e 619 ($MH^+$)

EXAMPLE 27

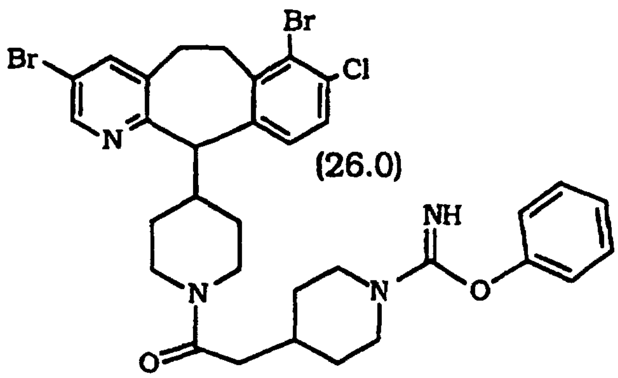

(26.0)

By following the procedure in Example 25 above, the amine

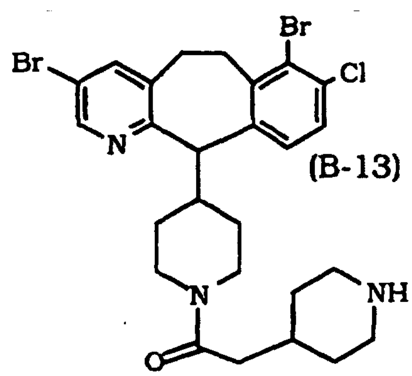

(B-13)

(Formula B-13, racemic, see Preparative Example 10 ) (0.09g) was reacted with phenyl cyanate (0.04ml) in the presence of diisopropylethylamine to afford the compound of Formula 26.0 (0.078g) as a white powder. MS(FAB): m/e 713 ($MH^+$)

EXAMPLE 28

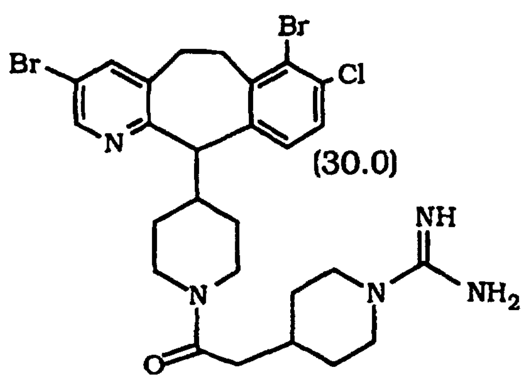

(30.0)

A solution of the compound of Formula 26.0 (Example 27) (0.24g) and ammonium chloride (0.24g) in tetrahydrofuran (2.0ml) and ammonium hydroxide (2.0ml) was heated in a pressure tube at 90°C for 1.5hrs. The reaction mixture was then cooled in ice, diluted with water and extracted with methylene chloride (3x10ml). The combined

extracts were evaporated and the crude product was chromatographed on silica-gel (20ml). Elution with 10%methanol-3%ammonia-methylene chloride afforded after evaporation the title compound (Formula 30.00 as a white solid (0.115g). MS (FAB): m/e 636 ($MH^+$)

EXAMPLE 29

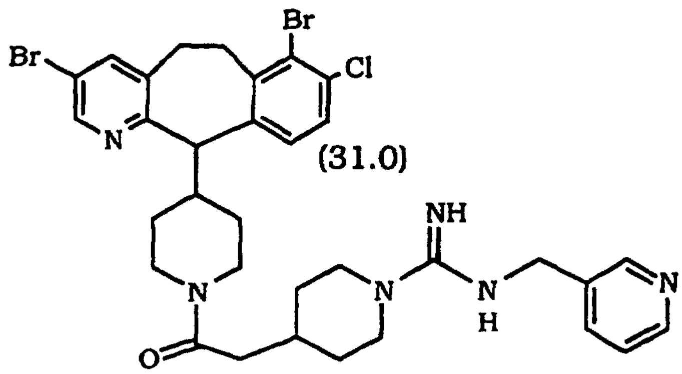

(31.0)

A solution of the compound of Formula 26.0 (Example 27) (0.25g) in tetrahydrofuran (3.0ml) was treated with a solution of 3-aminomethylpyridine (0.14ml) in water (0.5ml) containing 4M hydrochloric acid in dioxane (0.09 ml). The reaction mixture was heated at 70°C for 3.5 hrs, cooled to room temperature, diluted with ice-water; the pH of the mixture was adjusted to 10 with aqueous sodium hydroxide followed by extraction with methylene chloride (2x15ml). The crude product was then chromatographed on silica-gel (10ml); elution with 10%methanol-3%ammonia-methylene chloride afforded the title compound (Formula 31.0) as a colorless powder (0.28g). MS (FAB): m/e 727 ($MH^+$)

EXAMPLE 30

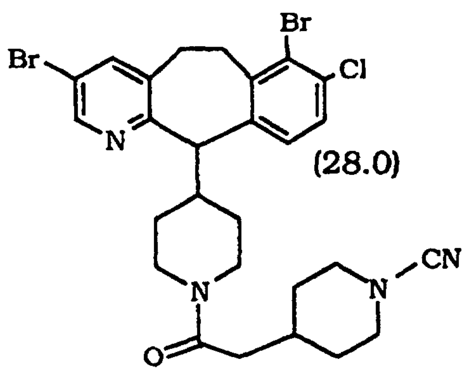

(28.0)

By following the procedure for the preparation of the compound of Formula 29.0 in described above in Example 26, the amine 26.0 (Example 27) (0.2g) was reacted with 60% sodium-hydride (.022g) to afford the title compound (Formula 28.0) (0.087g) as a white powder. MS(FAB): m/e 619 ($MH^+$)

EXAMPLE 31

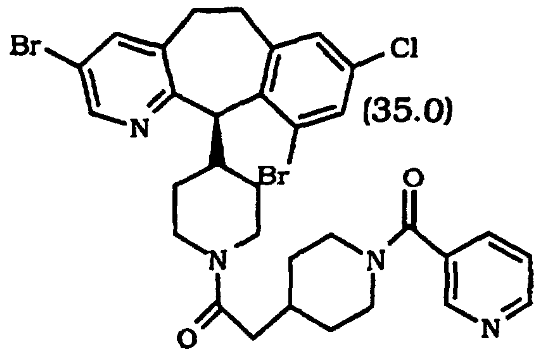

(35.0)

The compound of Formula B-10

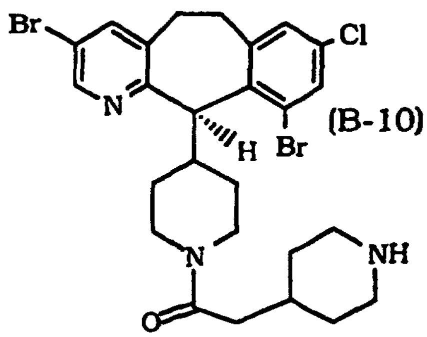

(B-10)

(+-isomer, see Example 25 above) (100 mg, 1 eq), HOBT (25 mg, 1.1 eq), DEC (35 mg, 1.1 eq), DMF (5 ml) and the carboxylic acid

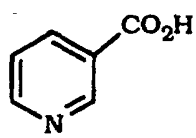

(23 mg, 1.1 eq) were added to a flask and stirred at 25°C for 18 h. When TLC showed that the reaction was complete, water was added and the resulting mixture was stirred for about 5 minutes. The mixture was filtered and the precipitate was washed with water. The resulting solids were diluted in $CH_2Cl_2$, washed with 1M NaOH (aqueous), dried over anhydrous $MgSO_4$, filtered and the solvent was removed under vacuum. Formula 35.0 was obtained as a white solid (52 mg).

EXAMPLE 32

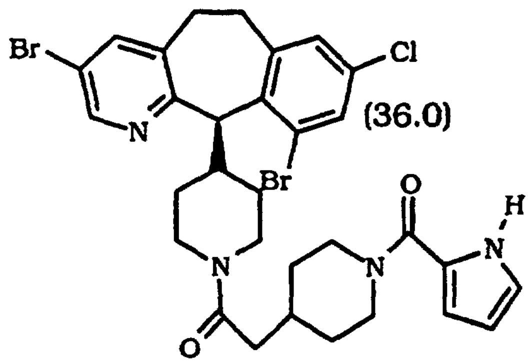

(36.0)

Following the procedure of Example 31, but using

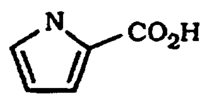

as the carboxylic acid (21 mg, 1.1 eq), yielded the compound of Formula 36.0 as a white solid (86 mg).

EXAMPLE 33

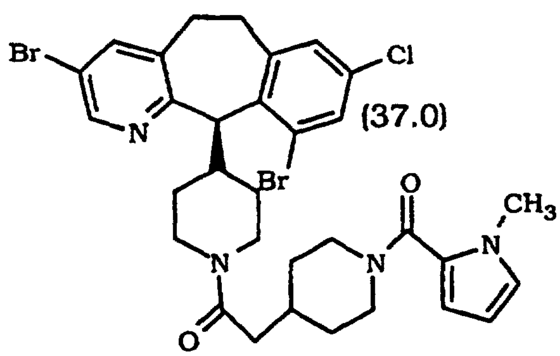

(37.0)

Following the procedure of Example 31, but using

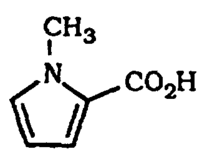

as the carboxylic acid (23 mg, 1.1 eq) yielded the compound of Formula 37.0 as a white solid (84.4 mg).

EXAMPLE 34

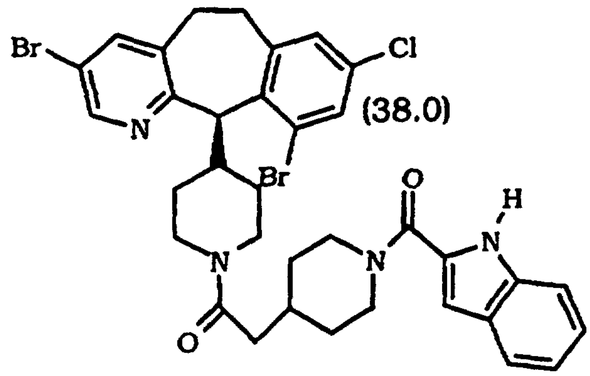

(38.0)

Following the procedure of Example 31, but using

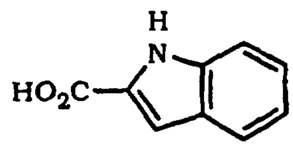

as the carboxylic acid (30 mg, 1.1 eq) yielded the compound of Formula 38.0 as an off-white solid (0.105 g).

EXAMPLE 35

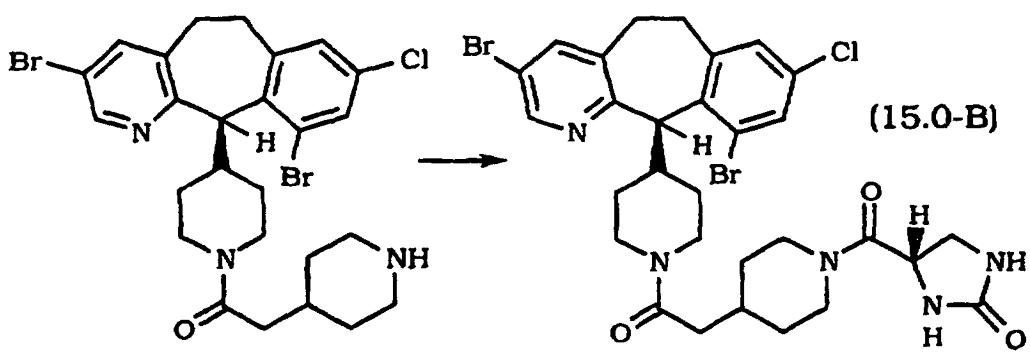

(15.0-B)

Dissolve the (+) product of Preparative Example 8, Step D (0.139 g, 0.233 mmol) in 1.3 mL of DMF, stir at room temperature and add 55.2 mg (0.288 mmol) of DEC, 41.5 mg (0.307 mmol) of HOBT and 42.2 mg (0.324 mmole) of

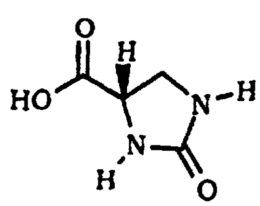

and 80µL (0.73 mmol) N-methylmorpholine. Stir the mixture at room temperature for 1 hr. Add the mixture to 10 mL $H_2O$, filter the resulting precipitate and wash with water. Dry at 10 mm Hg 40°C 18h to give 123 mg of the product (m.

p. = 170.3-177.2°C, heating 2-3°C per minute).

EXAMPLES 36-53

Follow the procedure of Example 35 but use the acid shown in Table 1 below instead of the acid used in Example 35 to obtain the compounds of Formula 1.7

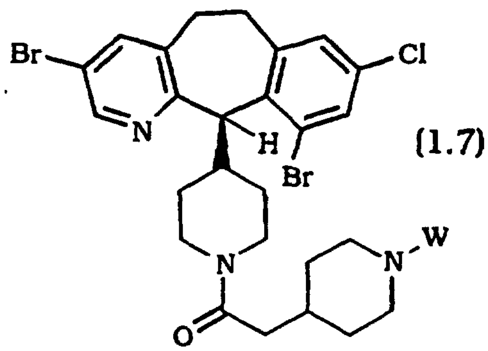

(1.7)

wherein W is defined in Table 1. The Formula number of the compound formed is given in parenthesis below the W substituent.

TABLE 1

| EX. | Acid | W | mp (°C) |
|---|---|---|---|
| 36 | 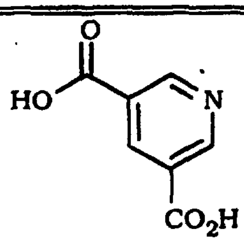 | 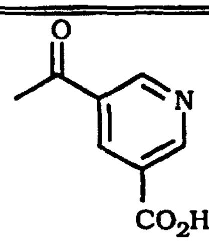 (25.0-B) | 183.7-185.9 |
| 37 | 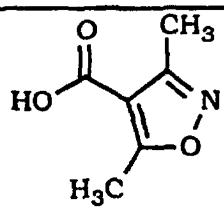 | 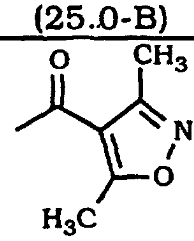 (27.0-B) | 117.6-127.4 |
| 38 | 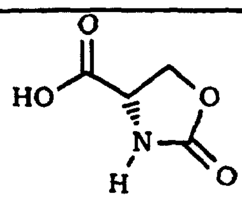 | 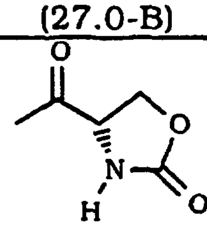 (28.0-B) | 154.5-157.1 |

TABLE 1 - continued

| EX. | Acid | W | mp (°C) |
|---|---|---|---|
| 39 | 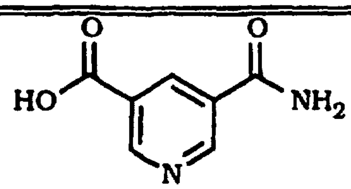 | 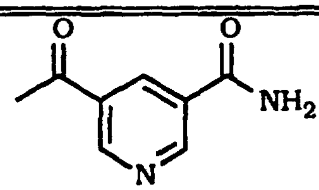 (29.0-B) | 81.9-83.9 |
| 40 | 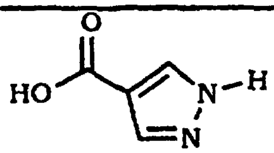 | 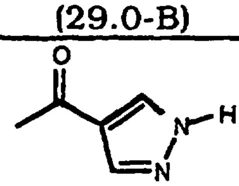 (31.0-B) | 117.1-120 |
| 41 | 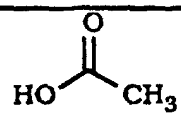 | 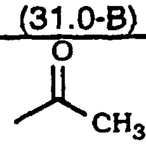 (36.0-B) | 123-124 |
| 42 | 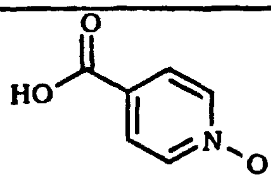 | 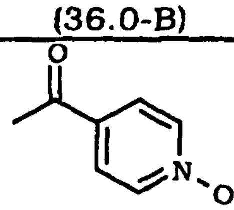 (45.0-B) | 151.3 |
| 43 | 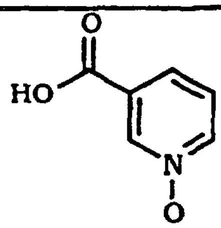 | 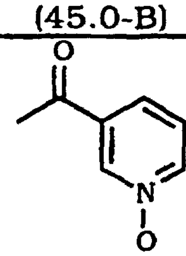 (46.0-B) | 157.2 |
| 44 | 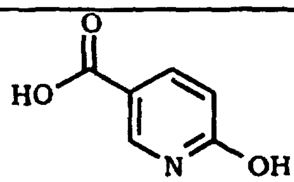 | 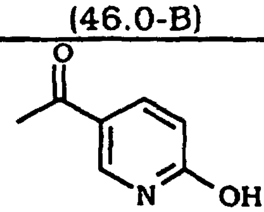 (47.0-B) | 184.8-190.0 |
| 45 | 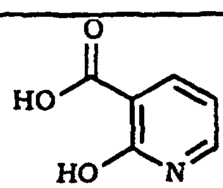 | 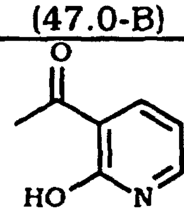 (48.0-B) | 206.5 |

TABLE 1 - continued

| EX. | Acid | W | mp (°C) |
|---|---|---|---|
| 46 | 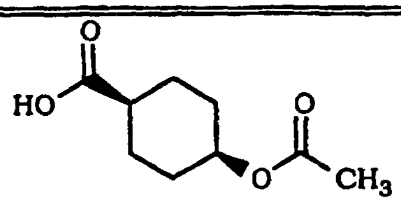 J. Chem. Soc. (1950), 1379 | 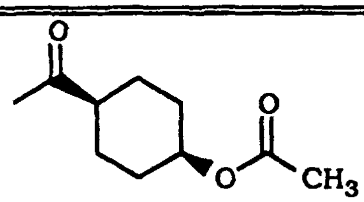 (66.0-B) | 120.3-127.1 |
| 47 | 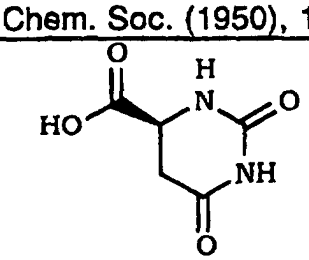 | 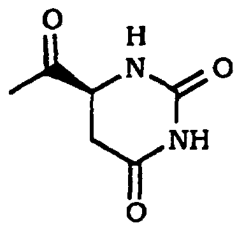 (78.0-B) | 192-203 |
| 48 | 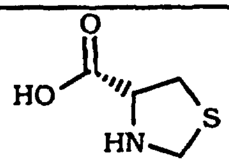 | 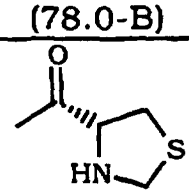 (80.0-B) | 162-172 |
| 49 | 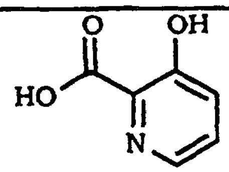 | 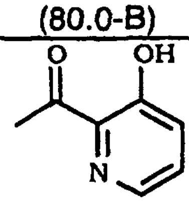 (83.0-B) | 138-151 |
| 50 | 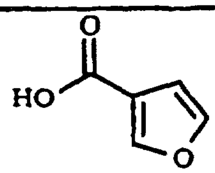 | 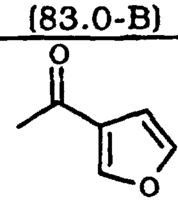 (84.0-B) | 134-148 |
| 51 | 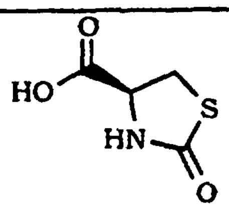 | 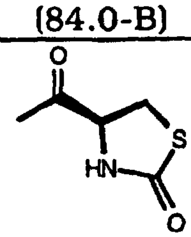 (114.1-B) | 176-191 |

TABLE 1 - continued

| EX. | Acid | W | mp (°C) |
|---|---|---|---|
| 52 | 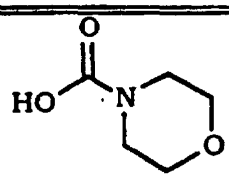 | 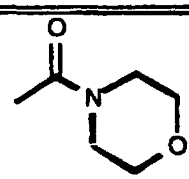 (100.0-B) | 111-119 |
| 53 | 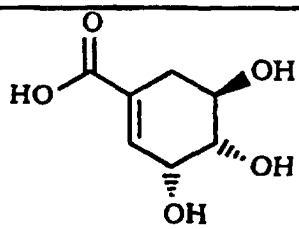 | 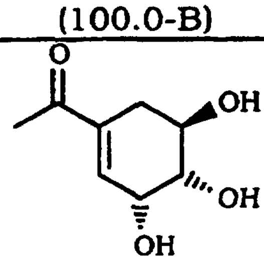 (113.0-B) | 230 (d) |

EXAMPLE 54

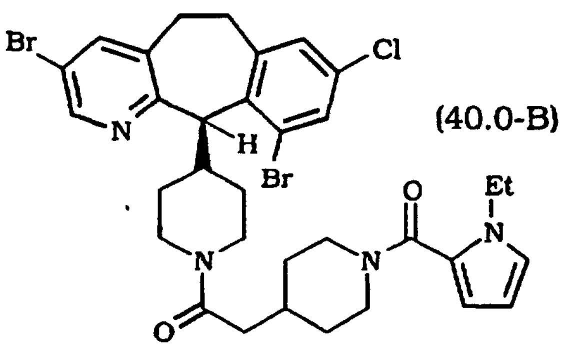

(40.0-B)

Dissolve 1.0 equivalent of the product of Example 32 in anhydrous DMF and add 2.0 equivalents of NaH (60% in mineral oil). Stir for 0.5 hr and add 1.5 equivalent of ethyliodide and stir for 18 hr and adjust to pH7.0 with 1N HCl. Concentrate under vacuum and partition between water and dichloromethane. Dry the organic layer over magnesium sulfate and concentrate under vacuum. Chromatograph the residue on silica gel using methanol-dichloromethane, saturated with ammonia (5-95) to give the product as a white solid, mp = 112.6°C.

EXAMPLE 55

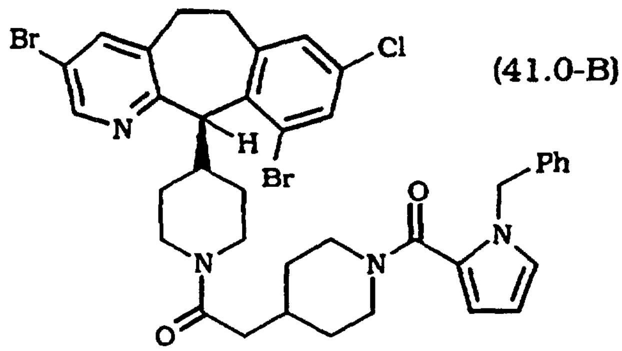

(41.0-B)

Ph = phenyl

Follow the procedure of Example 54 (Compound 40.0-B), but use benzylbromide instead of ethyliodide to obtain the product as a white solid, mp = 108°C.

EXAMPLE 56

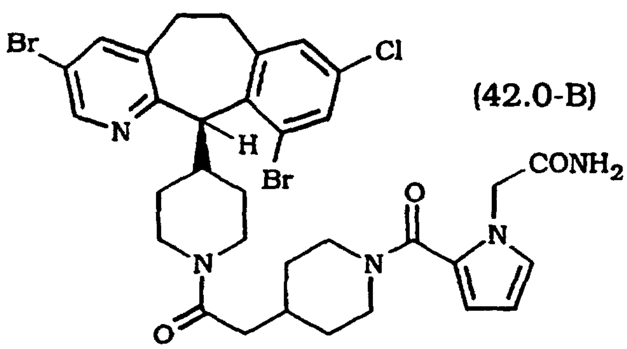

(42.0-B)

Follow the procedure of Example 54, but use bromoacetamide instead of ethyliodide to obtain the product as a white solid, mp = 138.3°C.

EXAMPLE 57

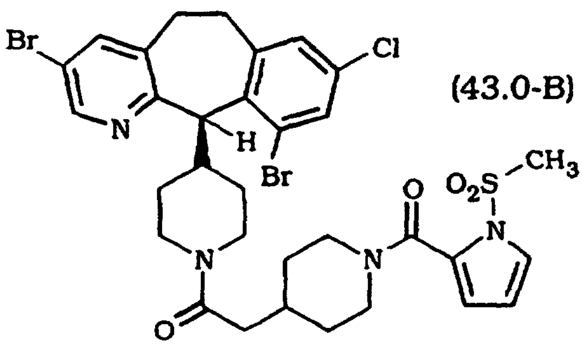

(43.0-B)

Follow the procedure of Example 54, but use methane-sulfonylchloride instead of ethyliodide to obtain the

product as a white solid, mp = 136.3°C.

EXAMPLE 58

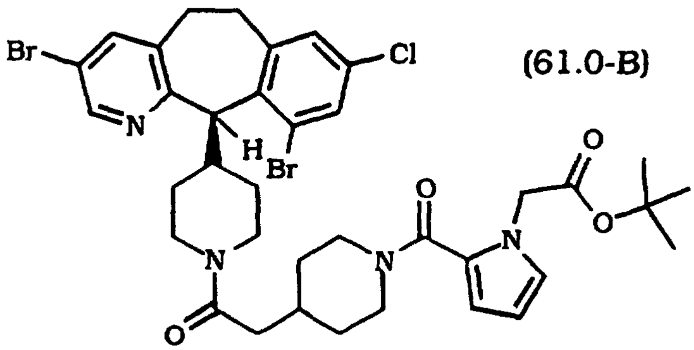

(61.0-B)

Follow the procedure of Example 54, but use tert-butyl-bromo acetate instead of ethyliodide to obtain the product as a yellow solid, mp = 120-127°C.

EXAMPLE 59

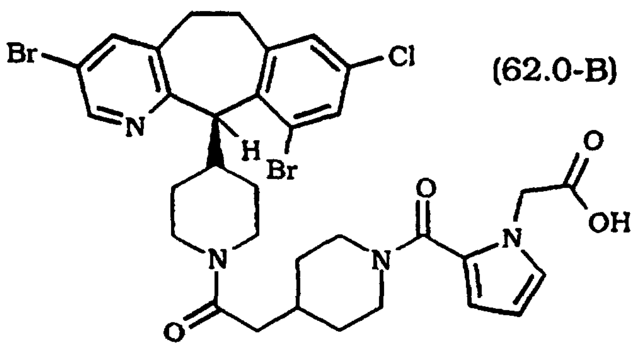

(62.0-B)

Dissolve the product of Example 58 in dichloromethane and add trifluoroacetic acid and allow to stir for 1 hr. Concentrate under vacuum. Chromatograph the residue by preparative silica gel TLC using methanol-dichloromethane (10-90) to obtain the product as a pink solid, mp = 198°C.

EXAMPLE 60

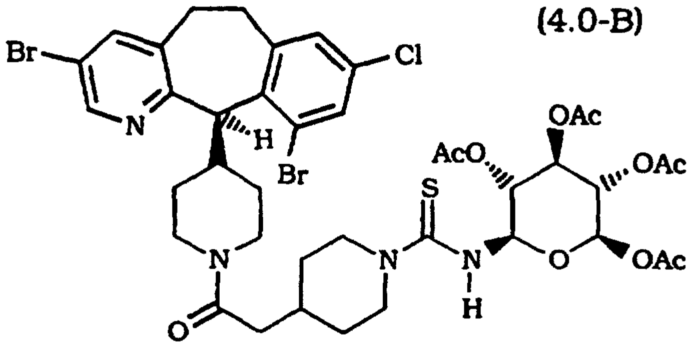

(4.0-B)

Dissolve 1.0 equivalent of the (+) product of Preparative Example 8, Step D, in dichloromethane containing 1.0 equivalent of 2,3,4,6-tetra-O-acetyl-beta-D-glucopyranosyl isothiocyanate. Stir for 6 days then wash with 1N HCl followed by 1N NaOH. Dry the organic layer over magnesium sulfate and concentrate under vacuum. Chromatograph the residue on silica gel using methanol-dichloromethane saturated with ammonia (2-98) to obtain the product as a white solid, mp = 156.7-157.9°C.

EXAMPLE 61

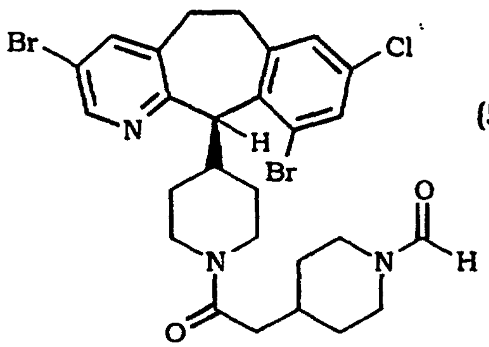

(5.0-B)

Dissolve1.0 equivalent of the (+) product of Preparative Example 8, Step D, in DMF containing 2.5 equivalent of anhydrous sodium carbonate and 2.2 equivalents of 4-chloropyridine hydrochloride and heat at 100°C for 5 days. Cool to 25°C, add water and filter the precipitate. Dissolve the precipitate in dichloromethane and wash with 1N HCl followed by 1N NaOH. Dry the organic layer over magnesium sulfate and concentrate under vacuum. Chromatograph the residue on silica gel to obtain the produce as a white solid, mp = 128.6°C.

EXAMPLE 62

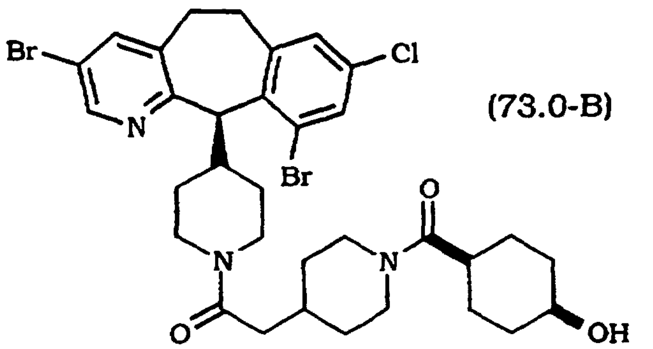

(73.0-B)

Dissolve 471.4 mg, (0.617 mmol) of the product of Example 46 in 2 mL of 6 M HCl and the solution stirred at room temperature overnight. The reaction mixture was added to 20 mL water, and the resulting precipitate was filtered and washed with 0.1 M HCl. Purify by flash chromatoghrapy (C-18 reverse phase silica, gradient of 50% $MeOH/H_2O$ to 90% $MeOH/H_2O$). The resulting material was dissolved in MeOH and added to water to give the title compound as a white solid after filtration and drying (334.7 mg, mp 147.1° - 154.3° C, heating 2° - 3° C/min).

EXAMPLE 63

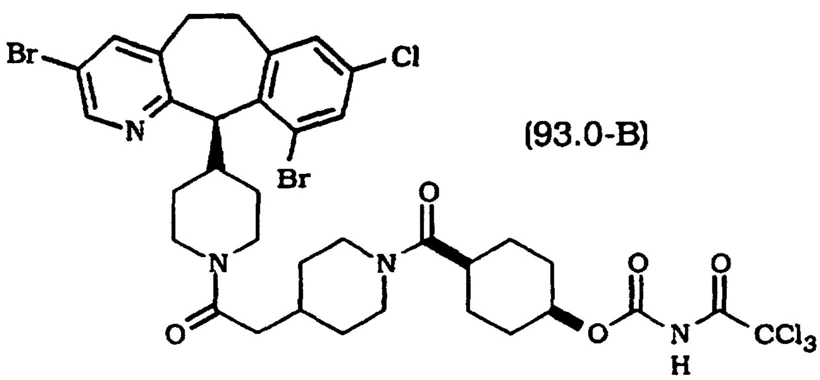

(93.0-B)

The product of Example 62 (63.8 mg, 0.0884 mmol) was dissolved in $CH_2Cl_2$ and trichloroacetyl isocyanate (12 μL) was added. After 24 h trichloroacetyl isocyanate (12 μL) was added. After 24 h the reaction mixture was purified by preparative TLC (5% $EtOH/CH_2Cl_2$). The isolate material was dissolved in $CH_2Cl_2$ and added to hexane and the resulting suspension evaporated to give the title compound as a white solid (51.6 mg, mp 165.4° - 178.5° C, heating 2° - 3° C/min).

EXAMPLE 64

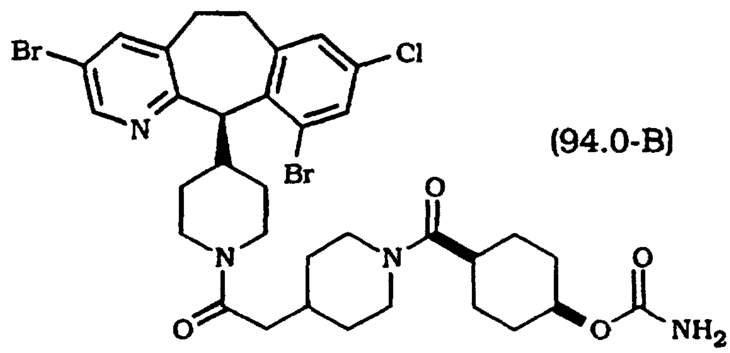

(94.0-B)

The product of Example 62 (151.4 mg, 0.2097 mmol) was dissolved in $CH_2Cl_2$ and trichloroacetyl isocyanate (40 μL) was added. After 15 min trichloroacetyl isocyanate (6 μL) was added. After an additional 15 min MeOH was added and the mixture evaporated to dryness. The residue was suspended in MeOH/THF and $K_2CO_3$ (anhydrous, 15.9 mg, 0.115 mmol) was added. After 3 h the reaction mixture was purified by flash chromatography (5% EtOH/ $CH_2Cl_2$). The resulting material was dissolved in MeOH and added to water to give the title compound as a white solid after filtration and drying (mp 165.4° - 178.5° C, heating 2° - 3° C/min).

ASSAYS

FPT $IC_{50}$ (inhibition of farnesyl protein transferase, in vitro enzyme assay), GGPT $IC_{50}$ (inhibition of geranylgeranyl protein transferase, in vitro enzyme assay) and COS Cell $IC_{50}$ (Cell-Based Assay) were determined following the assay procedures described in WO 95/10516, published April 20, 1995. Cell Mat Assay, and anti-tumor activity (in vivo anti-tumor studies) could be determined by the assay procedures described in WO 95/10516. The disclosure of WO 95/10516 is incorporated herein by reference thereto.

Additional assays can be carried out by following essentially the same procedure as described above, but with substitution of alternative indicator tumor cell lines in place of the T24-BAG cells. The assays can be conducted using either DLD-1-BAG human colon carcinoma cells expressing an activated K-ras gene or SW620-BAG human colon carcinoma cells expressing an activated K-ras gene. Using other tumor cell lines known in the art, the activity of the compounds of this invention against other types of cancer cells could be demonstrated.

Soft Agar Assay:

Anchorage-independent growth is a characteristic of tumorigenic cell lines. Human tumor cells are suspended in growth medium containing 0.3% agarose and an indicated concentration of a farnesyl transferase inhibitor. The solution is overlayed onto growth medium solidified with 0.6% agarose containing the same concentration of farnesyl transferase inhibitor as the top layer. After the top layer is solidified, plates are incubated for 10-16 days at 37°C under 5% $CO_2$ to allow colony outgrowth. After incubation, the colonies are stained by overlaying the agar with a solution of MTT (3-[4,5-dimethylthiazol-2-yl]-2,5-diphenyltetrazolium bromide, Thiazolyl blue) (1 mg/mL in PBS). Colonies can be counted and the $IC_{50}$'s can be determined.

Compounds 2.0, 3.0, 4.0, 5.0, 7.0, 12.0, 13.0, 16.1, 17.1, 27.0, 29.0, 26.0, 28.0, 30.0, 31.0, 4.0-B, 5.0-B, 15.0-B, 25.0-B, 27.0-B, 28.0-B, 29.0-B, 31.0-B, 36.0-B, 40.0-B, 41.0-B, 42.0-B, 43.0-B, 45.0-B, 46.0-B, 47.0-B, 48.0-B, 61.0-B, 62.0-B, 66.0-B, 73.0-B, 78.0-B, 80.0-B, 83.0-B, 84.0-B, 91.0-B, 93.0-B, 94.0-B, 98.0-B and 100.0-B had an FPT $IC_{50}$ (H-ras) within the range of 0.8 to >300 nM (nanomolar).

Compounds 27.0, 28.0, 31.0, 4.0-B, 5.0-B and 98.0-B had an FPT $IC_{50}$ (K-ras) within the range of 16 to 782 nM.

Compounds 27.0, 28.0, 29.0, 30.0 and 31.0 had a Cos Cell $IC_{50}$ within the range of 12 to >1000nM.

The compound of Formula 31.0 (Example 29) had a GGPT $IC_{50}$ of >7.5μM and the compound of Formula 28.0 (Example 30) had a GGPT $IC_{50}$ of >9.6μM.

Compounds 27.0, 4.0-B, 5.0-B, 15.0-B, 25.0-B, 27.0-B, 28.0-B, 29.0-B, 31.0-B, 36.0-B, 40.0-B, 41.0-B, 42.0-B, 43.0-B, 45.0-B, 46.0-B, 47.0-B, 48.0-B, 62.0-B, 66.0-B, 73.0-B, 78.0-B, 83.0-B, 84.0-B, 91.0-B, 93.0-B, 94.0-B, 98.0-B and 100.0-B had a Cos Cell $IC_{50}$ within the range of 7 to >1000nM.

Compounds 27.0, 5.0-B, 15.0-B, 25.0-B, 27.0-B, 28.0-B, 29.0-B, 31.0-B, 36.0-B, 40.0-B, 41.0-B, 42.0-B,

43.0-B, 45.0-B, 46.0-B, 47.0-B, 48.0-B, 62.0-B, 66.0-B, 73.0-B, 78.0-B, 80.0-B, 83.0-B, 84.0-B, 91.0-B, 93.0-B, 94.0-B, 98.0-B and 100.0-B had a Soft Agar $IC_{50}$ within the range of 90 to >500nM.

For preparing pharmaceutical compositions from the compounds described by this invention, inert, pharmaceutically acceptable carriers can be either solid or liquid. Solid form preparations include powders, tablets, dispersible granules, capsules, cachets and suppositories. The powders and tablets may be comprised of from about 5 to about 70 percent active ingredient. Suitable solid carriers are known in the art, e.g. magnesium carbonate, magnesium stearate, talc, sugar, lactose. Tablets, powders, cachets and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection.

Liquid form preparations may also include solutions for intranasal administration.

Aerosol preparations suitable for inhalation may include solutions and solids in powder form, which may be in combination with a pharmaceutically acceptable carrier, such as an inert compressed gas.

Also included are solid form preparations which are intended to be converted, shortly before use, to liquid form preparations for either oral or parenteral administration. Such liquid forms include solutions, suspensions and emulsions.

The compounds of the invention may also be deliverable transdermally. The transdermal compositions can take the form of creams, lotions, aerosols and/or emulsions and can be included in a transdermal patch of the matrix or reservoir type as are conventional in the art for this purpose.

Preferably the compound is administered orally.

Preferably, the pharmaceutical preparation is in unit dosage form. In such form, the preparation is subdivided into unit doses containing appropriate quantities of the active component, e.g., an effective amount to achieve the desired purpose.

The quantity of active compound in a unit dose of preparation may be varied or adjusted from about 0.1 mg to 1000 mg, more preferably from about 1 mg. to 300 mg, according to the particular application.

The actual dosage employed may be varied depending upon the requirements of the patient and the severity of the condition being treated. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The amount and frequency of administration of the compounds of the invention and the pharmaceutically acceptable salts thereof will be regulated according to the judgment of the attending clinician considering such factors as age, condition and size of the patient as well as severity of the symptoms being treated. A typical recommended dosage regimen is oral administration of from 10 mg to 2000 mg/day preferably 10 to 1000 mg/day, in two to four divided doses to block tumor growth. The compounds are non-toxic when administered within this dosage range.

The following are examples of pharmaceutical dosage forms which contain a compound of the invention. The scope of the invention in its pharmaceutical composition aspect is not to be limited by the examples provided.

| Pharmaceutical Dosage Form Examples | | | |
|---|---|---|---|
| EXAMPLE A - Tablets | | | |
| No. | Ingredients | mg/tablet | mg/tablet |
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| | Total | 300 | 700 |

Method of Manufacture

Mix Item Nos. 1 and 2 in a suitable mixer for 10-15 minutes. Granulate the mixture with Item No. 3. Mill the

damp granules through a coarse screen (e.g., 1/4", 0.63 cm) if necessary. Dry the damp granules. Screen the dried granules if necessary and mix with Item No. 4 and mix for 10-15 minutes. Add Item No. 5 and mix for 1-3 minutes. Compress the mixture to appropriate size and weigh on a suitable tablet machine.

| EXAMPLE B - Capsules | | | |
|---|---|---|---|
| No. | Ingredient | mg/capsule | mg/capsule |
| 1. | Active compound | 100 | 500 |
| 2. | Lactose USP | 106 | 123 |
| 3. | Corn Starch, Food Grade | 40 | 70 |
| 4. | Magnesium Stearate NF | 7 | 7 |
| Total | | 253 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a . suitable encapsulating machine.

While the present invention has been described in conjunction with the specific embodiments set forth above, many alternatives, modifications and variations thereof will be apparent to those of ordinary skill in the art. All such alternatives, modifications and variations are intended to fall within the spirit and scope of the present invention.

## Claims

**1.** A compound of the formula:

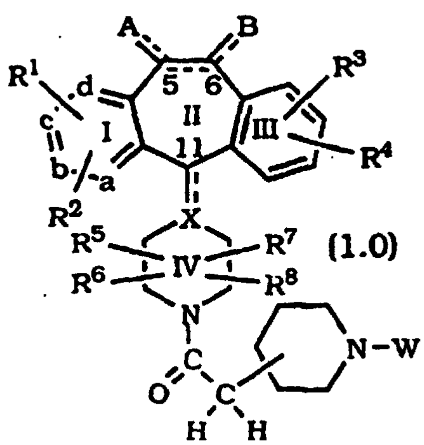

or a pharmaceutically acceptable salt or solvate thereof, wherein: one of a, b, c and d represents N or $NR^9$ wherein $R^9$ is $O^-$, $-CH_3$ or $-(CH_2)_nCO_2H$ wherein n is 1 to 3, and the remaining a, b, c and d groups represent $CR^1$ or $CR^2$; or each of a, b, c, and d are independently selected from $CR^1$ or $CR^2$;

$R^2$ is H and $R^1$, $R^3$ and $R^4$ are halo;

$R^5$, $R^6$, $R^7$ and $R^8$ each independently represents H, $-CF_3$, $-COR^{10}$, alkyl or aryl, said alkyl or aryl optionally being substituted with $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$, $OPO_3R^{10}$, or $R^5$ is combined with $R^6$ to represent =O or =S and/or $R^7$ is combined with $R^8$ to represent =O or =S;

$R^{10}$ represents H, alkyl, aryl, or aralkyl;

$R^{11}$ represents alkyl or aryl;

X represents N, CH or C, which C may contain an optional double bond (represented by the dotted line) to carbon atom 11;

the dotted line between carbon atoms 5 and 6 represents an optional double bond, such that when a double bond is present, A and B independently represent $-R^{10}$, halo, $-OR^{11}$, $-OCO_2R^{11}$ or $-OC(O)R^{10}$, and when no double

bond is present between carbon atoms 5 and 6, A and B each independently represent $H_2$, $-(OR^{11})_2$; H and halo, dihalo, alkyl and H, $(alkyl)_2$, - H and $-OC(O)R^{10}$, H and $-OR^{10}$, =O, aryl and H, $=NOR^{10}$ or $-O-(CH_2)_p-O-$ wherein p is 2, 3 or 4; and

W is selected from the group consisting of:

(1) cyano;

(2) $-C(O)R^{12}$ wherein $R^{12}$ is selected from:

(a) a heteroaryl group;

(b) H;

(c) alkyl; or

(d) a substituent of the formula:

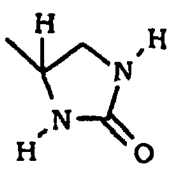 , 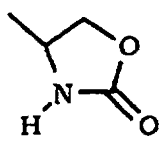 , 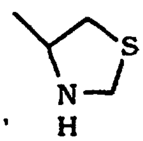 , 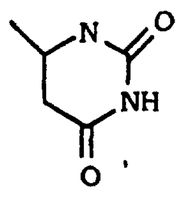 , 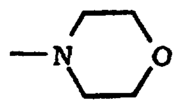 ,

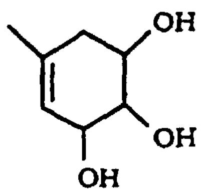 or 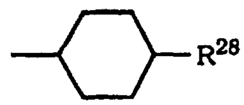 ,

wherein $R^{28}$ is selected from $-OC(O)R^{29}$, -OH, $-OC(O)NHC(O)CCl_3$, or $-OC(O)NH_2$, and wherein $R^{29}$ is alkyl;

(3) an imidate represented by the formula:

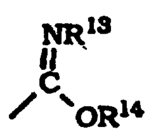

wherein $R^{13}$ is selected from the group consisting of: (a) H, (b) CN, (c) $-SO_2$-alkyl, (d) -C(O)-aryl, (e) $-SO_2NR^{10}R^{15}$, (f) $-C(O)NR^{10}R^{15}$, (g) $-OR^{10}$ and (h) $-C(O)NR^{10}C(O)NR^{10}R^{15}$; $R^{14}$ is aryl; and $R^{10}$ and $R^{15}$ are independently selected from the group consisting of: H, alkyl, aryl and aralkyl;

(4) an imidamido represented by the formula:

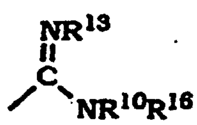

wherein $R^{13}$ is selected from the group consisting of (a)H, (b) CN, (c) $-SO_2$-alkyl, (d) -C(O)-aryl, (e) $-SO_2NR^{10}R^{15}$, (f) $-C(O)NR^{10}R^{15}$, (g) $-OR^{10}$ and (h) $-C(O)NR^{10}C(O)NR^{10}R^{15}$; $R^{16}$ is selected from the group consisting of: alkyl, aralkyl, aryl, cycloalkyl, heteroaryl, heteroaralkyl and heterocycloalkyl; and $R^{10}$ and $R^{15}$ are as defined above; and $R^{10}$ and $R^{16}$ are independently selected from the above defined groups;

(5) 1-amino-2-nitroethylene derivatives of the formula:

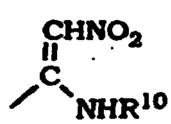

wherein $R^{10}$ is as defined above; and

(6) a substituent of the formula:

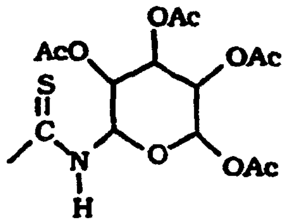, e.g., 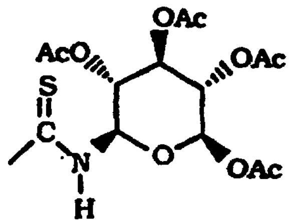

wherein unless otherwise indicated:

alkenyl-represents straight and branched carbon chains having at least one carbon to carbon double bond and containing from 2 to 12 carbon atoms;

alkynyl-represents straight and branched carbon chains having at least one carbon to carbon triple bond and containing from 2 to 12 carbon atoms;

alkyl-(including the alkyl portions of alkoxy, aralkyl and heteroarylalkyl)-represents straight and branched carbon chains and contains from one to twenty carbon atoms;

aralkyl-represents an aryl group, as defined below, bound to an alkyl group, as defined above,

aryl (including the aryl portion of aralkyl, aryloxy and arylalkyloxy)-represents a carbocyclic group containing from 6 to 15 carbon atoms and having at least one aromatic ring,

with all available substitutable carbon atoms of the carbocyclic group being intended as possible points of attachment, said carbocyclic group being optionally substituted (e.g., 1 to 3) with one or more of halo, alkyl, hydroxy, alkoxy, phenoxy, $CF_3$, amino, alkylamino, dialkylamino, $-COOR^{10}$ or $-NO_2$;

cycloalkyl-represents saturated carbocyclic rings branched or unbranched of from 3 to 20 carbon atoms;

halo-represents fluoro, chloro, bromo and iodo;

heteroaryl-represents cyclic groups, optionally substituted with $R^3$ and $R^4$, having at least one heteroatom selected from O, S or N, said heteroatom interrupting a carbocyclic ring structure and having a sufficient number of delocalized pi electrons to provide aromatic character, with the aromatic heterocyclic groups containing from 2 to 14 carbon atoms;

heteroarylalkyl (heteroaralkyl)-represents a heteroaryl group, as defined above, bound to an alkyl group, as defined above;

heterocycloalkyl-represents a saturated, branched or unbranched carbocylic ring containing from 3 to 15 carbon atoms, which carbocyclic ring is interrupted by 1 to 3 hetero groups selected from -O-, -S-, - $NR^{10}$- (wherein $R^{10}$ is as defined above).

**2.** The compound of Claim 1 wherein a is N and b, c and d are carbon; A and B are each $H_2$; the optional bond between $C_5$ and $C_6$ is absent; X is CH; and $R^5$, $R^6$, $R^7$ and $R^8$ are H.

**3.** The compound of any of Claims 1 or 2 wherein W is selected from the group consisting of:

(1) -CN;

(2) $-C(O)R^{12}$ wherein $R^{12}$ is selected from the group consisting of: H, alkyl,

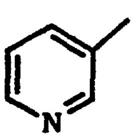, 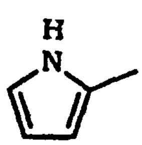, 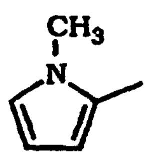, 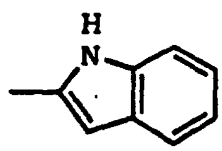, 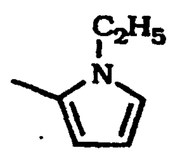,

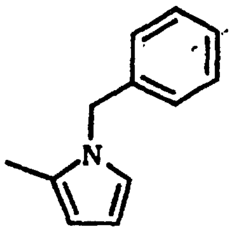 , 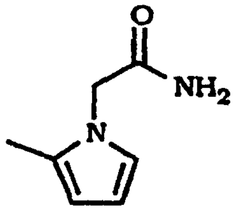 , 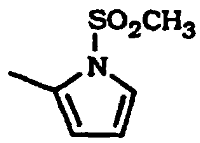 , 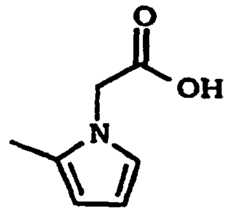 ,

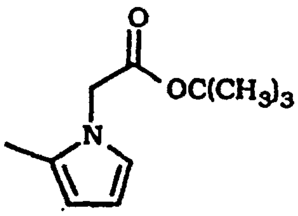 , 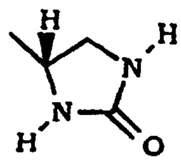 , 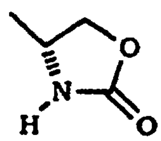 , 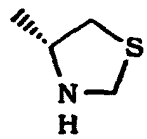 , 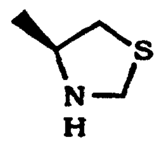 ,

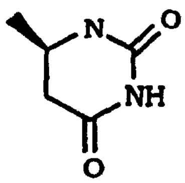 , 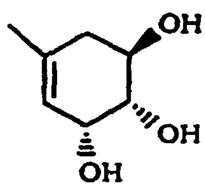 , 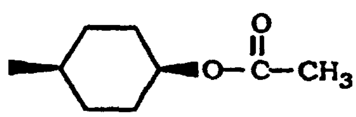 , 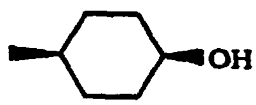 ,

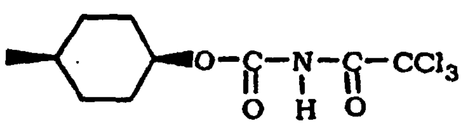 , 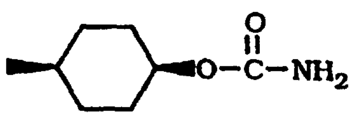 , 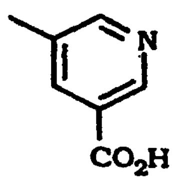 ,

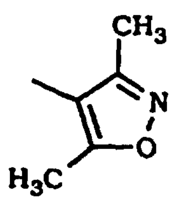 , 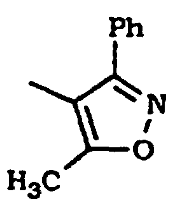 , 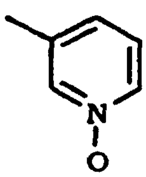 , 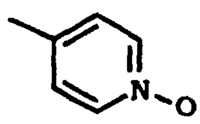 , 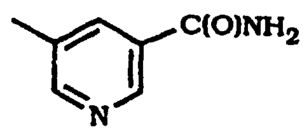 ,

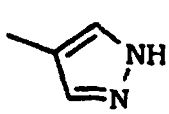 , 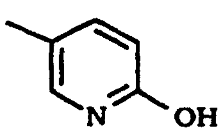 , 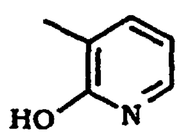 , 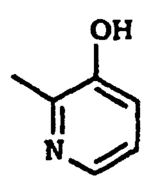 and 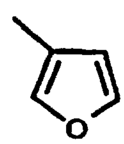 ;

(3) an imidate wherein $R^{13}$ is selected from the group consisting of:

(a) -CN;
(b) H;
(c) $-SO_2NR^{10}R^{15}$ wherein $R^{10}$ and $R^{15}$ are selected from the group consisting of: H and alkyl;
(d) $-C(O)NR^{10}R^{15}$ wherein $R^{10}$ and $R^{15}$ are selected from the group consisting of: H and alkyl;
(e) $-SO_2$-alkyl; and
(f) -C(O)-aryl;

(4) an imidamido wherein $R^{13}$ is selected from the group consisting of:

(a) CN;
(b) H;
(c) $-OR^{14}$;
(d) $-NR^{10}R^{15}$ wherein $R^{10}$ and $R^{15}$ are independently selected from the group consisting of: H and alkyl;
(e) $-SO_2NR^{10}R^{15}$ wherein $R^{10}$ and $R^{15}$ are independently selected from the group consisting of: H and alkyl;
(f) $-C(O)NR^{10}R^{15}$ wherein $R^{10}$ and $R^{15}$ are independently selected from the group consisting of: H and alkyl;
(g) $-SO_2$-alkyl; and
(h) -C(O)-aryl; and

(5) a 1-amino-2-nitroethylene derivative wherein $R^{10}$ is alkyl.

**4.** The compound of any of Claims 1-3 wherein $R^{14}$ for said imidate group is phenyl; and $R^{10}$ and $R^{16}$ for said imidamido group is selected from the group consisting of: H and heteroaralkyl.

**5.** The compound of any of Claims 1-4 wherein $R^4$ is selected from the group consisting of: Cl or Br.

**6.** The compound of any of Claims 1-5 wherein X is CH.

**7.** The compound of any of Claims 1-6 wherein X is N.

**8.** The compound of any of Claims 1-7 wherein $R^1$ is Br and $R^3$ is Cl.

**9.** The compound of any of Claims 1-8 selected from:

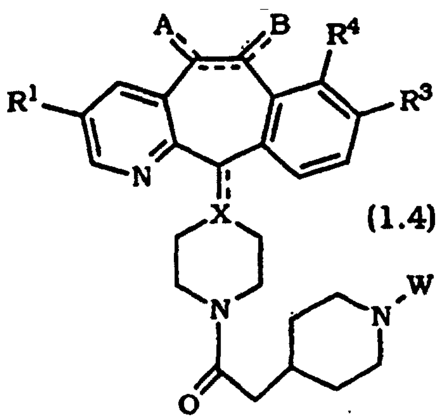
(1.4) or

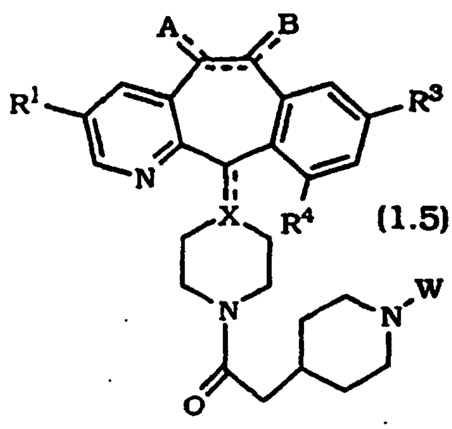
(1.5)

wherein $R^1$, $R^3$, $R^4$, A, B, X and W are as defined in Claim 1.

**10.** The compound of any of Claims 1-9 wherein $R^1$ is Br; $R^3$ is Cl; and $R^4$ is Br.

**11.** The compound of any of Claims 1-10 wherein W is selected from the group consisting of:

(1) $-C(O)R^{12}$ wherein $R^{12}$ is selected from the group consisting of:H, $-CH_3$,

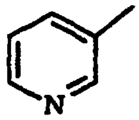 , 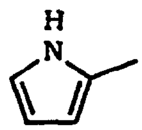 , 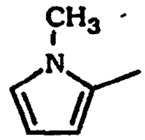 , 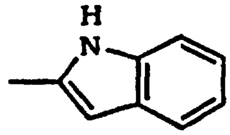 , 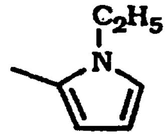 ,

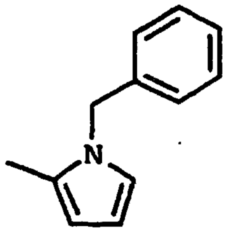 , 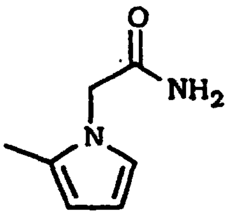 , 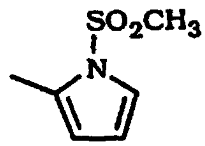 , 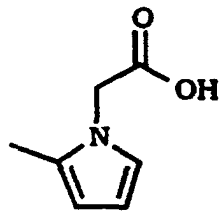 ,

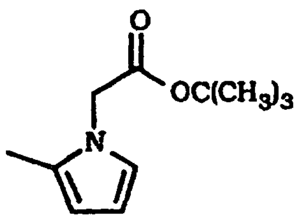 , 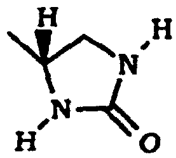 , 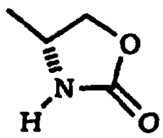 , 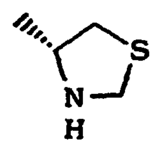 , 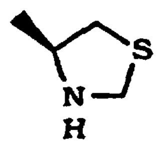 ,

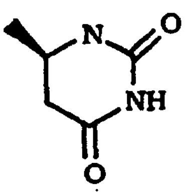 , 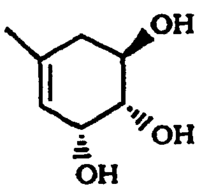 , 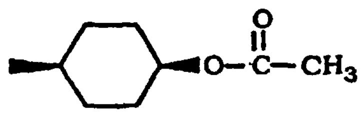 , 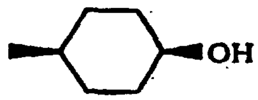 ,

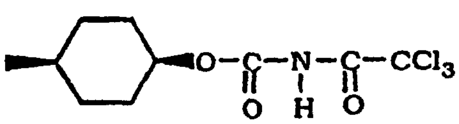 , 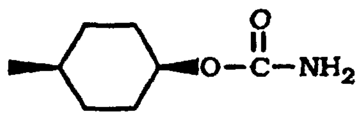 , 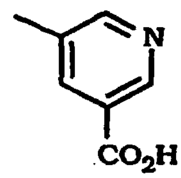 ,

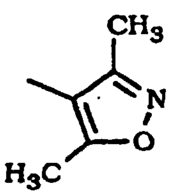 , 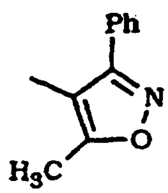 , 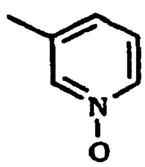 , 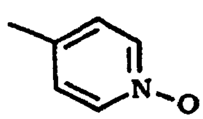 , 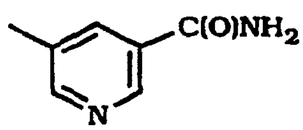 ,

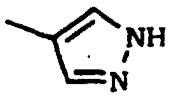, 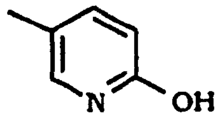, 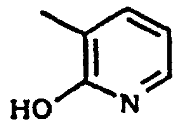, 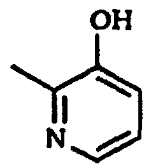 and 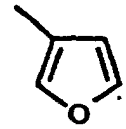;

(2) an imidate wherein $R^{13}$ is selected from the group consisting of: (a) CN; (b) $-C(O)NH_2$; (c) H; (d) $-SO_2NH_2$; (e) - $SO_2NHCH_3$; (f) $-SO_2N(CH_3)_2$; (g) $-C(O)NHCH_3$; (h) $-SO_2CH_3$ and (i) $-C(O)C_6H_5$;
(3) an imidamido wherein $R^{13}$ is selected from the group consisting of: (a) CN; (b) H; (c) $-OCH_3$; (d) -OH; (e) $-NH_2$; (f) $-N(CH_3)_2$; (g) $-SO_2NH_2$; (h) $-SO_2NHCH_3$; (i) $-SO_2N(CH_3)_2$; (j) $-C(O)NH_2$; (k) $-C(O)NHCH_3$; (l) $-SO_2CH_3$ and (m) $-C(O)C_6H_5$; and
(4) a 1-amino-2-nitroethylene derivative wherein $R^{10}$ is $-CH_3$.

**12.** The compound of any of Claims 1-11 wherein for said imidate group $R^{14}$ is phenyl; and for said imidamido groups $R^{10}$ and $R^{16}$ are independently selected from the group consisting essentially of: H and

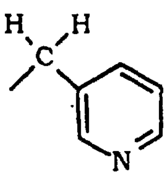.

**13.** The compound of any of Claims 1-3 having the structure

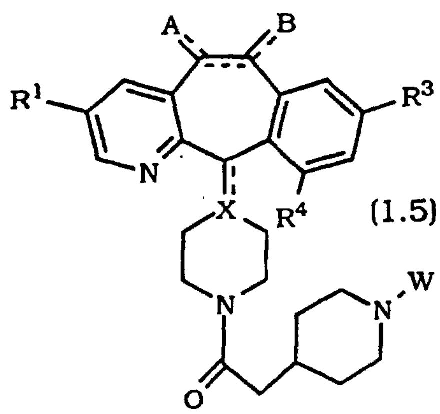

(1.5)

**14.** The compound of Claim 1 selected from:

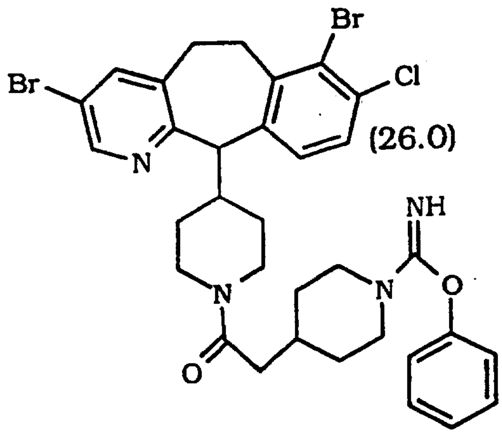

(26.0)

;

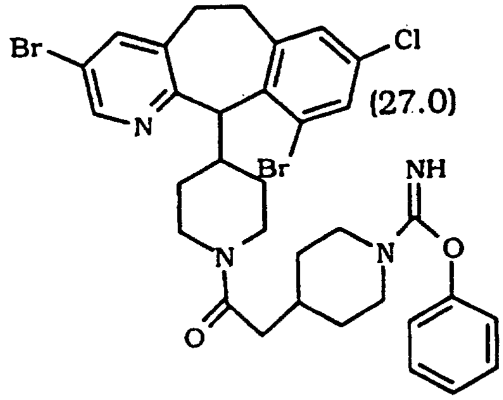

(27.0)

;

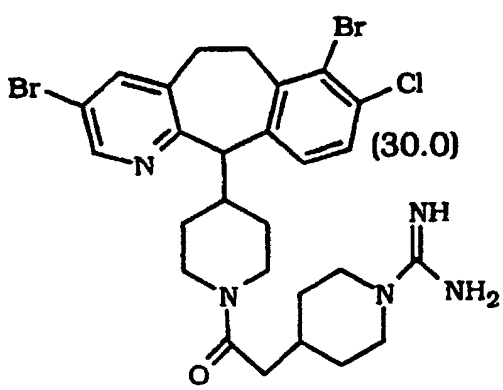

(30.0)

;

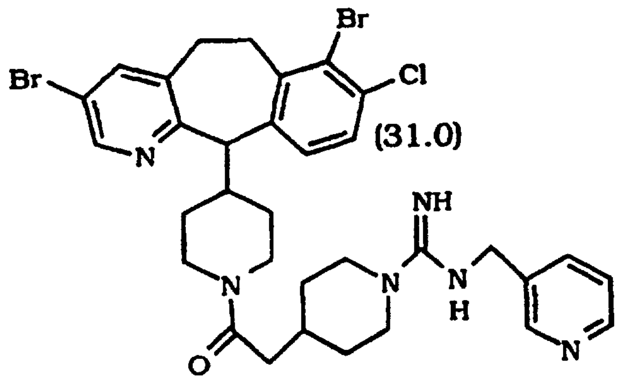

(31.0)

;

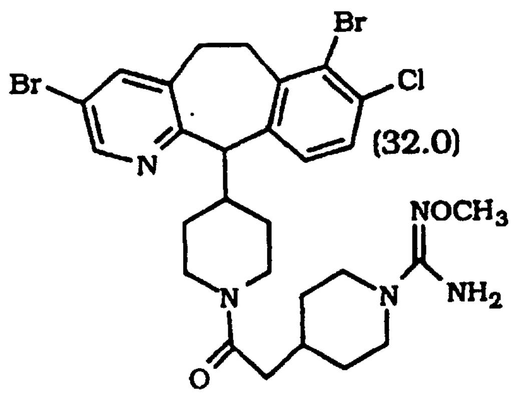

(32.0)

;

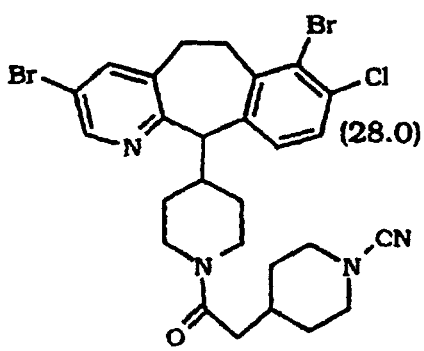

(28.0)

;

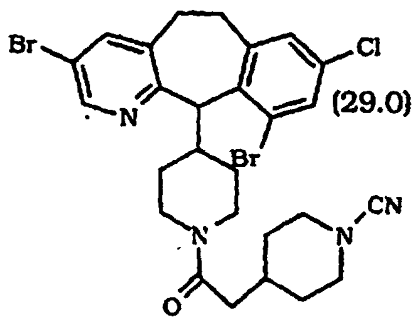

(29.0)

;

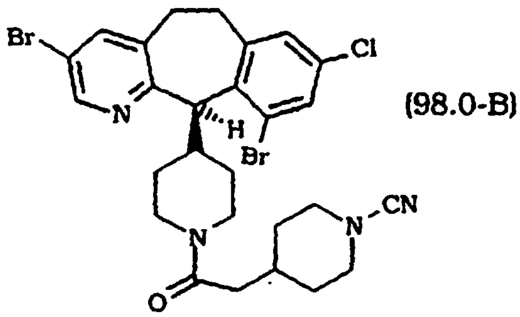

(98.0-B)

;

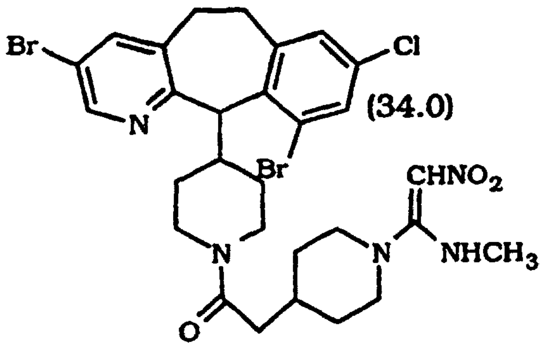

(34.0)

;

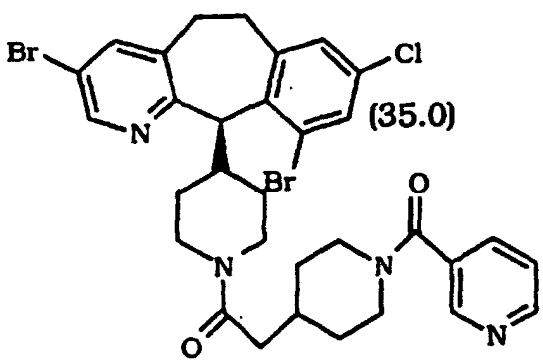

(35.0)

;

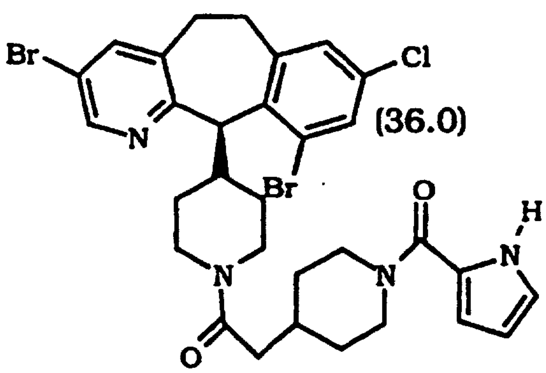

(36.0)

;

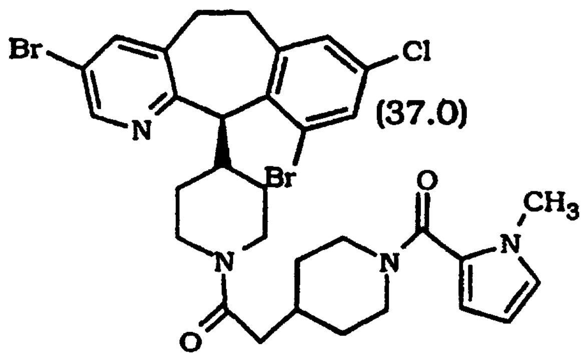

(37.0)

;

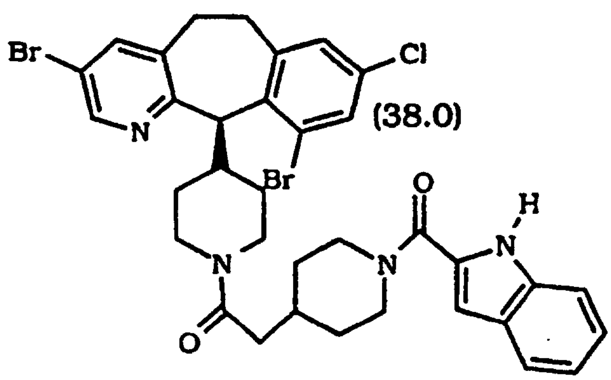

(38.0)

;

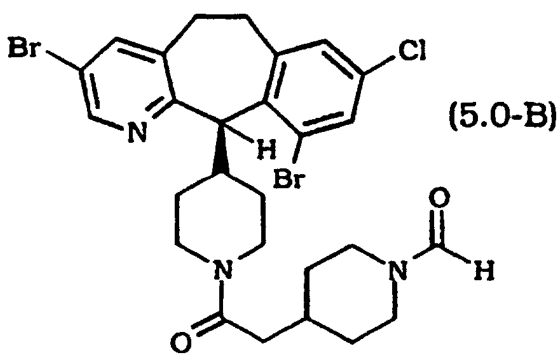

(5.0-B)

;

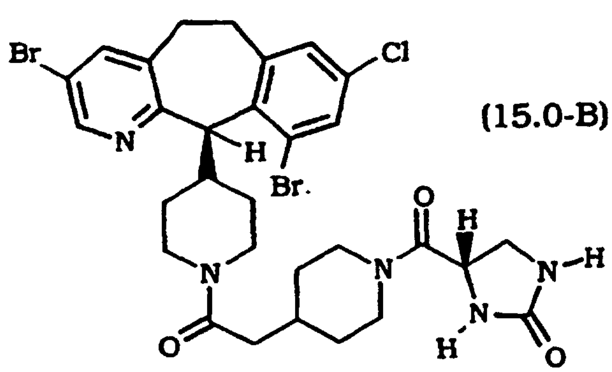

(15.0-B)

;

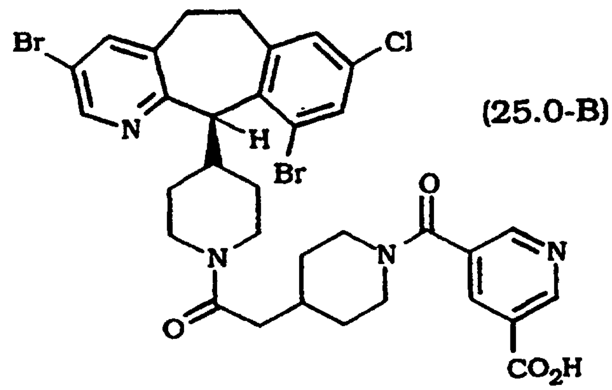

(25.0-B)

;

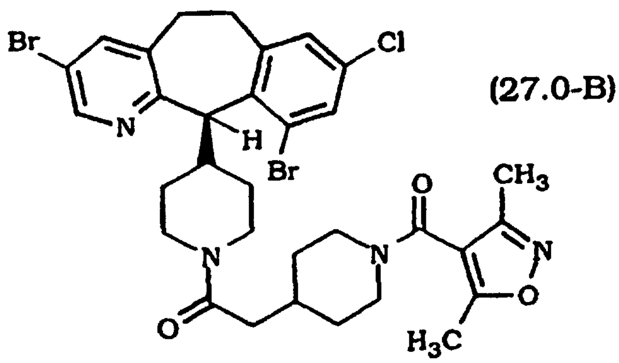

(27.0-B)

;

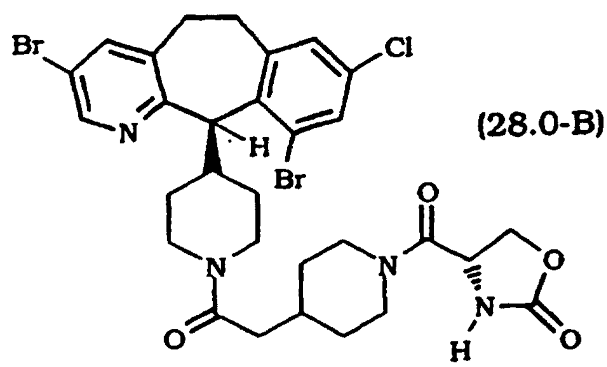

(28.0-B)

;

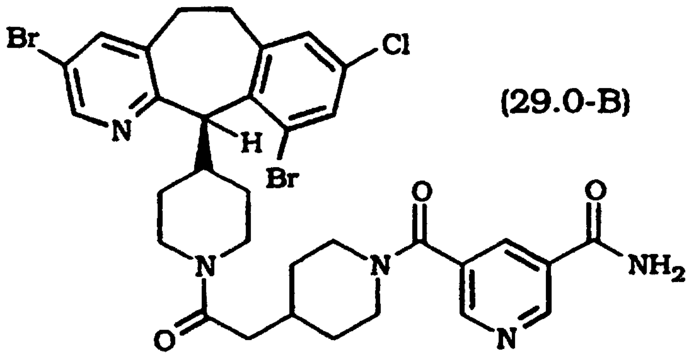

(29.0-B)

;

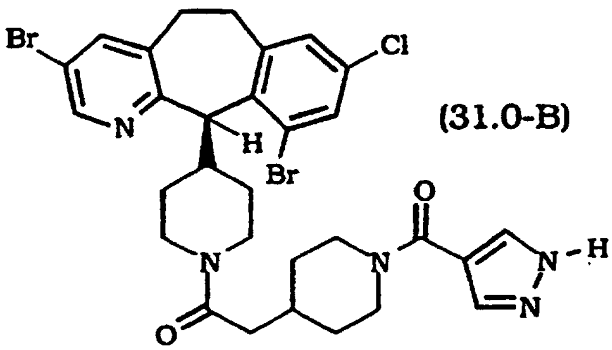

(31.0-B)

;

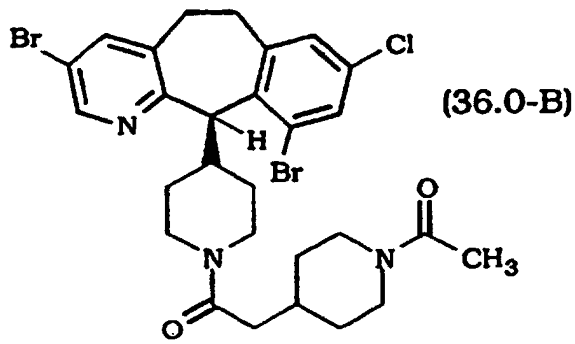

(36.0-B)

;

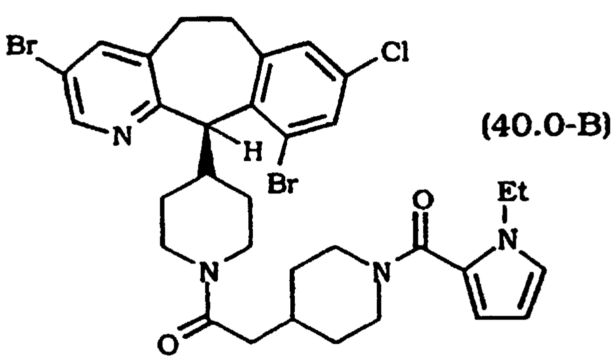

(40.0-B)

;

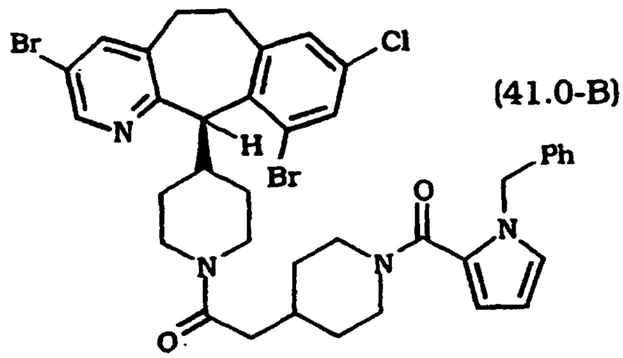

(41.0-B)

;

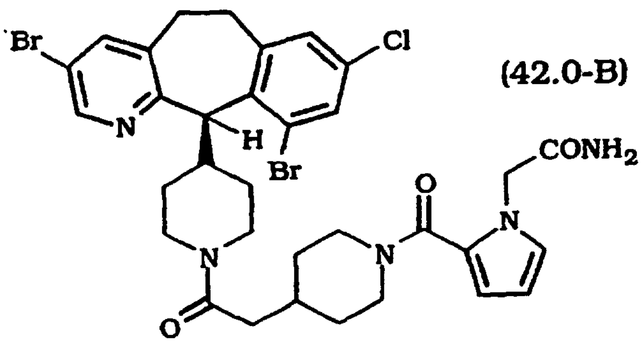

(42.0-B)

;

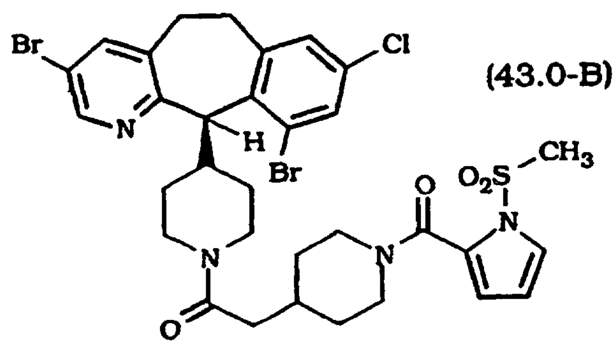

(43.0-B)

;

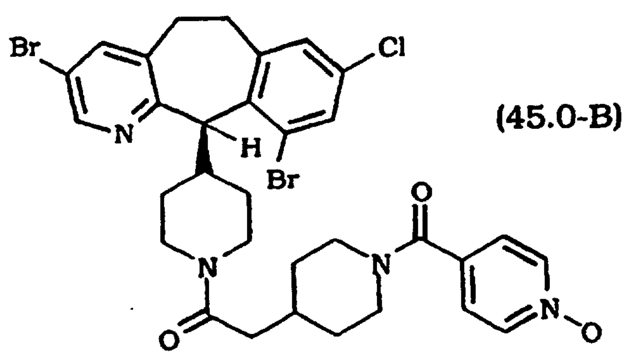

(45.0-B)

;

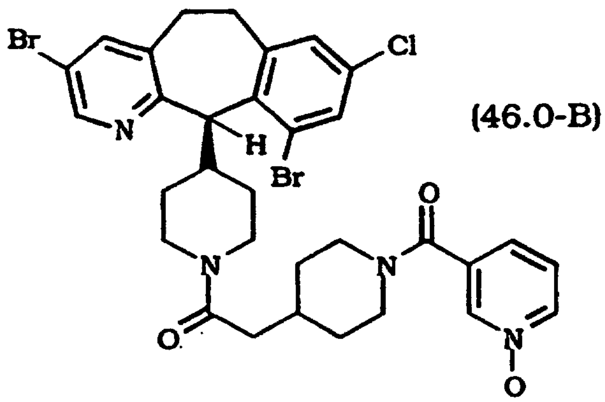

(46.0-B)

;

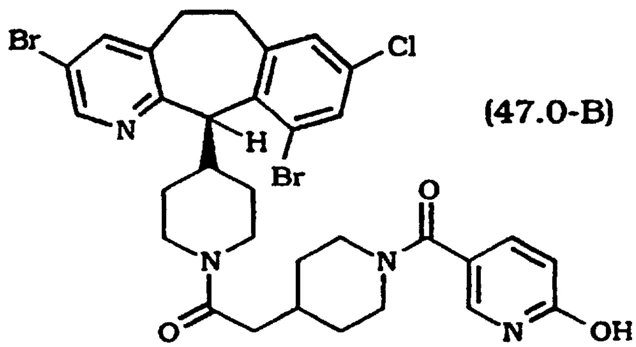

(47.0-B)

;

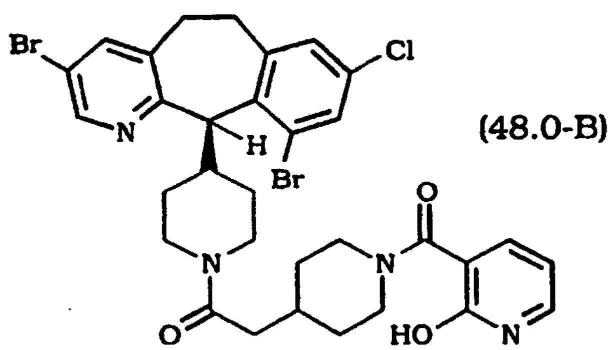

(48.0-B)

;

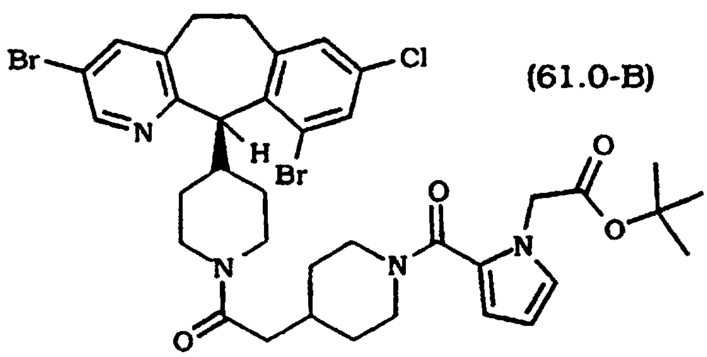

(61.0-B)

;

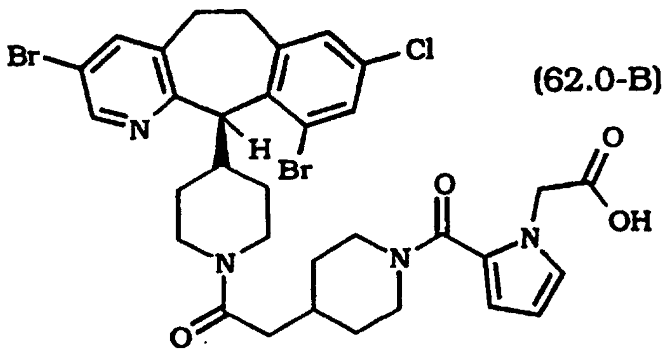

(62.0-B)

;

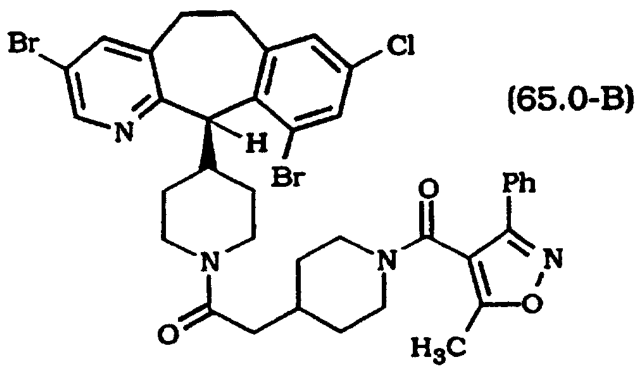

(65.0-B)

;

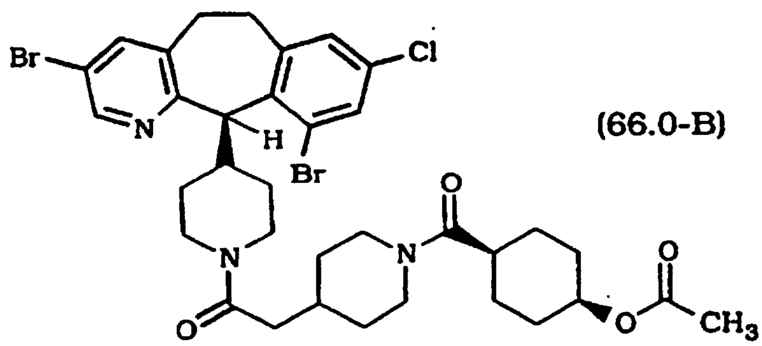

(66.0-B)

;

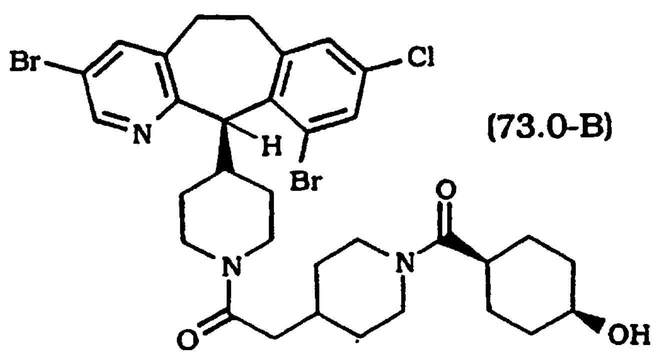

(73.0-B)

;

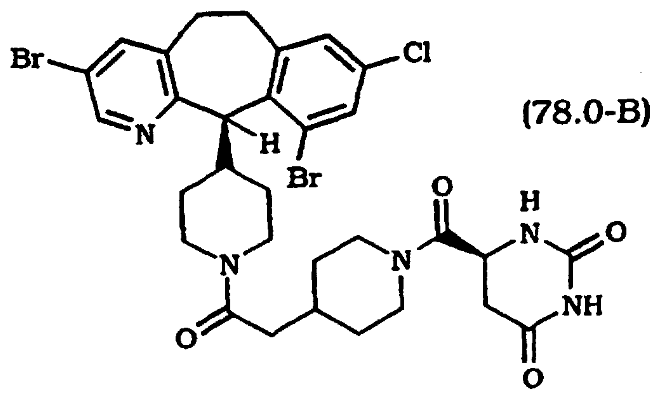

(78.0-B)

;

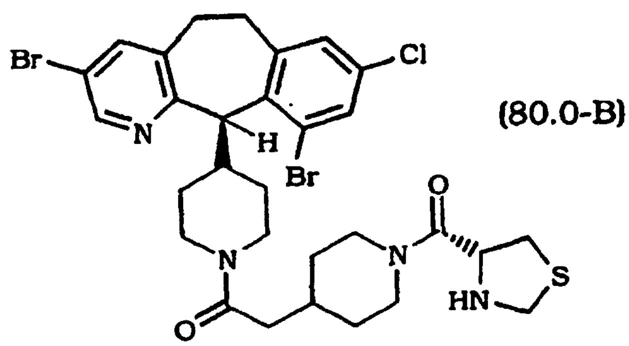

(80.0-B)

;

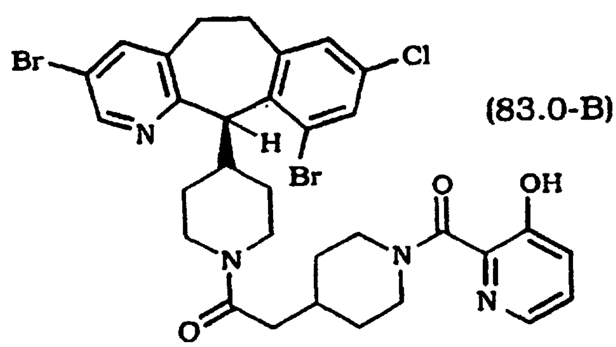

(83.0-B)

;

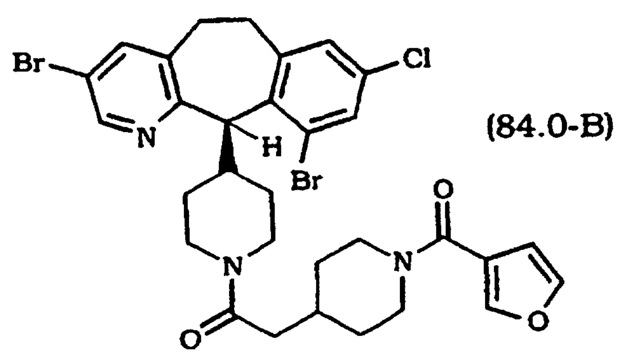

(84.0-B)

;

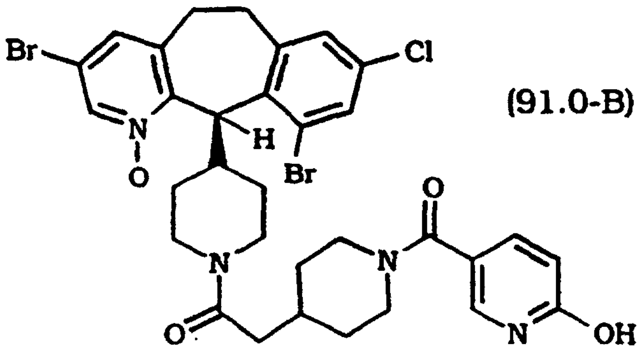

(91.0-B)

;

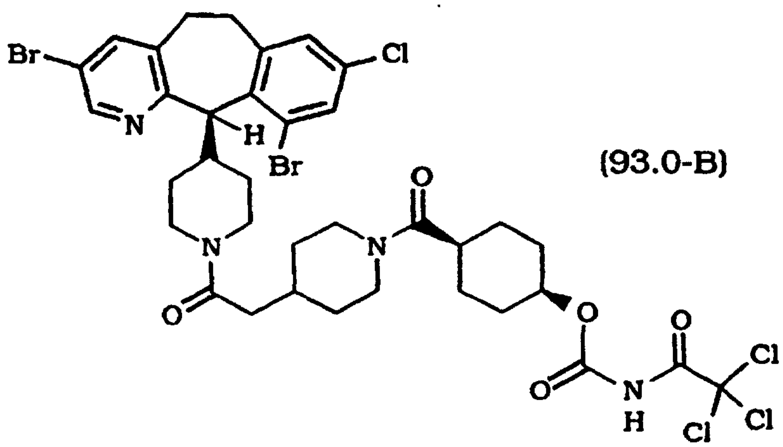

(93.0-B)

;

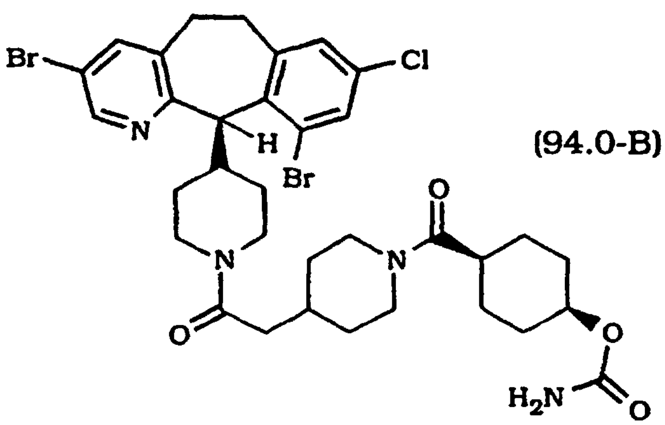

(94.0-B)

;

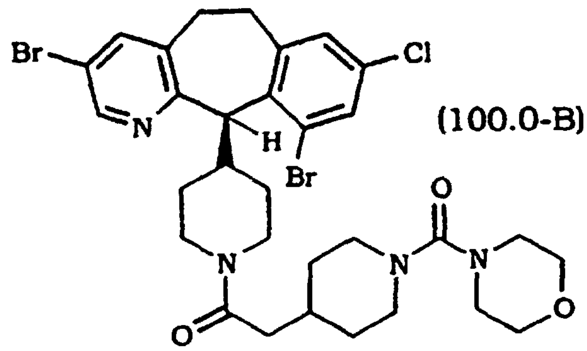

(100.0-B)

;

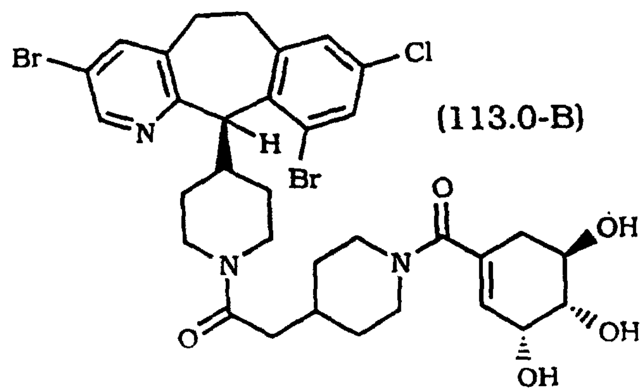

(113.0-B)

or

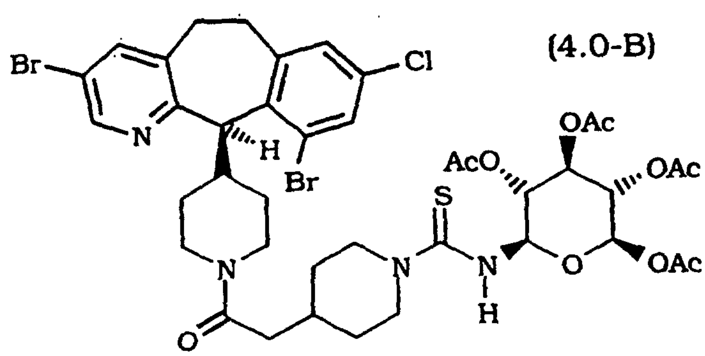

(4.0-B)

.

**15.** A pharmaceutical composition comprising an effective amount of compound of any of Claims 1-14 in combination with a pharmaceutically acceptable carrier.

**16.** The use of a compound of any of Claims 1-14 for the manufacture of a medicament for the treatment of tumor cells.

**17.** The use according to Claim 16 wherein the cells treated are pancreatic tumor cells, lung cancer cells, myeloid leukemia tumor cells, thyroid follicular tumor cells, myelodysplastic tumor cells, epidermal carcinoma tumor cells, bladder carcinoma tumor cells, colon tumors cells, breast tumor cells or prostate tumor cells.

**18.** The use of a compound of any of Claims 1-14 for the manufacture of a medicament for the inhibition of farnesyl protein transferase.

**Patentansprüche**

**1.** Verbindung der Formel:

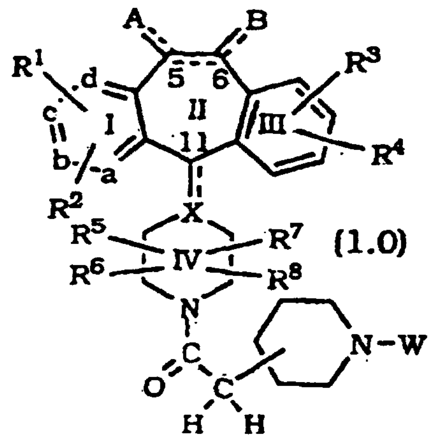

(1.0)

oder ein pharmazeutisch annehmbares Salz oder Solvat davon, wobei:

eines von a, b, c und d N oder $NR^9$ repräsentiert, wobei $R^9$ $O^-$, $-CH_3$ oder $-(CH_2)_nCO_2H$ ist, wobei n 1 bis 3 ist, und die übrigen Gruppen a, b, c und d $CR^1$ oder $CR^2$ repräsentieren; oder

jedes a, b, c und d unabhängig ausgewählt ist aus $CR^1$ oder $CR^2$;

$R^2$ H ist und $R^1$, $R^3$ und $R^4$ Halogen sind;

$R^5$, $R^6$, $R^7$ und $R^8$ jeweils unabhängig H, $-CF_3$, $-COR^{10}$, Alkyl oder Aryl repräsentieren, wobei das Alkyl oder Aryl gegebenenfalls mit $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$, $OPO_3R^{10}$ substituiert ist, oder $R^5$ mit $R^6$ kombiniert ist, um =O oder =S zu repräsentieren und/oder $R^7$ mit $R^8$ kombiniert ist, um =O oder =S zu repräsentieren;

$R^{10}$ H, Alkyl, Aryl oder Aralkyl repräsentiert;

$R^{11}$ Alkyl oder Aryl repräsentiert;

X N, CH oder C repräsentiert, wobei C gegebenenfalls eine Doppelbindung zu Kohlenstoffatom 11 enthalten kann (durch eine gestrichelte Linie dargestellt);

die gestrichelte Linie zwischen den Kohlenstoffatomen 5 und 6 eine optionale Doppelbindung darstellt, so dass wenn eine Doppelbindung vorhanden ist, A und B unabhängig $-R^{10}$, Halogen, $-OR^{11}$, $-OCO_2R^{11}$ oder $-OC(O)R^{10}$ repräsentieren, und wenn keine Doppelbindung zwischen Kohlenstoffatomen 5 und 6 vorhanden ist, A und B jeweils unabhängig $H_2$, $-(OR^{11})_2$; H und Halogen, Dihalogen, Alkyl und H, $(Alkyl)_2$, -H und $-OC(O)R^{10}$, H und $-OR^{10}$, =O, Aryl und H, $=NOR^{10}$ oder $-O-(CH_2)_p-O-$ repräsentieren, wobei p 2, 3 oder 4 ist; und

W ist ausgewählt aus der Gruppe bestehend aus:

(1) Cyan;

(2) $-C(O)R^{12}$ wobei $R^{12}$ ausgewählt ist aus:

(a) einer Heteroarylgruppe;
(b) H;
(c) Alkyl oder
(d) einem Substituenten der Formel:

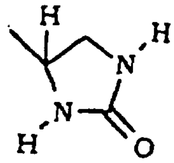, 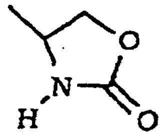, 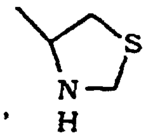, 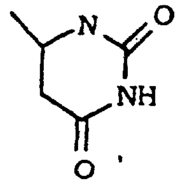, 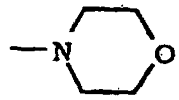,

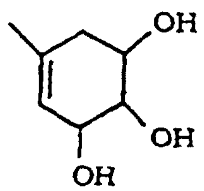 oder 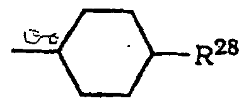,

wobei $R^{28}$ ausgewählt ist aus -OC (O)$R^{29}$, -OH, -OC(O)NHC(O)$CCl_3$ oder -OC(O)$NH_2$, wobei $R^{29}$ Alkyl ist;

(3) einem Imidat repräsentiert durch die Formel:

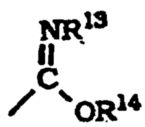,

wobei $R^{13}$ ausgewählt ist aus der Gruppe bestehend aus: (a) H, (b) CN, (c) -$SO_2$-Alkyl, (d) -C(O)-Aryl, (e) -$SO_2NR^{10}R^{15}$, (f) -C(O)$NR^{10}R^{15}$, (g) -$OR^{10}$ und (h) -C(O)$NR^{10}$C(O)$NR^{10}R^{15}$, $R^{14}$ Aryl ist und $R^{10}$ und $R^{15}$ unabhängig ausgewählt sind aus der Gruppe bestehend aus: H, Alkyl, Aryl und Aralkyl;

(4) einen Imidamid, repräsentiert durch die Formel:

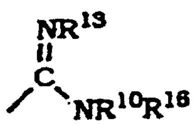,

wobei $R^{13}$ ausgewählt ist aus der Gruppe bestehend aus: (a) H, (b) CN, (c) -$SO_2$-Alkyl, (d) -C(O)-Aryl, (e) -$SO_2NR^{10}R^{15}$, (f) -C(O)$NR^{10}R^{15}$, (g) -$OR^{10}$ und (h) -C(O)$NR^{10}$C(O)$NR^{10}R^{15}$, $R^{16}$ ausgewählt ist aus der Gruppe bestehend aus: Alkyl, Aralkyl, Aryl, Cycloalkyl, Heteroaryl, Heteroaralkyl und Heterocycloalkyl und $R^{10}$ und $R^{15}$ wie oben definiert sind sowie $R^{10}$ und $R^{16}$ unabhängig ausgewählt sind aus den oben definierten Gruppen;

(5) 1-Amino-2-nitroethylenderivaten der Formel:

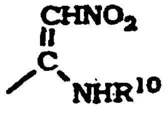,

wobei $R^{10}$ wie oben definiert ist und

(6) einem Substituenten der Formel:

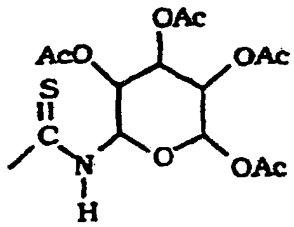, z.B. 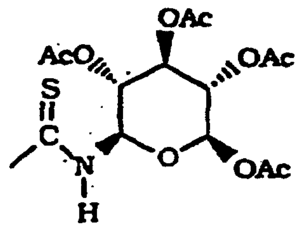,

wobei, sofern nicht anders angegeben:

Alkenyl geradkettige und verzweigte Kohlenstoffketten mit mindestens einer Kohlenstoff-Kohlenstoff-Doppelbindung repräsentiert und 2 bis 12 Kohlenstoffatome enthält;

Alkinyl geradkettige und verzweigte Kohlenstoffketten mit mindestens einer Kohlenstoff-Kohlenstoff-Dreifachbindung repräsentiert und 2 bis 12 Kohlenstoffatome enthält;

Alkyl (einschließlich der Alkylanteile von Alkoxy, Aralkyl und Heteroarylalkyl) geradkettige oder verzweigte Kohlenstoffketten repräsentiert und 1 bis 20 Kohlenstoffatome enthält;

Aralkyl eine Arylgruppe repräsentiert, wie unten definiert, die an eine Alkyl-Gruppe, wie oben definiert, gebunden ist;

Aryl (einschließlich der Arylanteile von Aralkyl, Aryloxy und Arylalkyloxy) eine carbocyclische Gruppe repräsentiert, die 6 bis 15 Kohlenstoffatome enthält und mindestens einen aromatischen Ring aufweist, wobei alle verfügbaren substituierbaren Kohlenstoffatome der carbocyclischen Gruppe als mögliche Anknüpfungspunkte vorgesehen sind, wobei die carbocyclische Gruppe gegebenenfalls mit einem oder mehreren (z.B. 1 bis 3) von Halogen, Alkyl, Hydroxy, Alkoxy, Phenoxy, $CF_3$, Amino, Alkylamino, Dialkylamino, $-COOR^{10}$ oder $-NO_2$ substituiert ist;

Cycloalkyl gesättigte carbocylische Ringe repräsentiert, verzweigt oder unverzweigt, mit 3 bis 20 Kohlenstoffatomen;

Halogen Fluor, Chlor, Brom und Iod repräsentiert;

Heteroaryl cyclische Gruppen repräsentiert, die gegebenenfalls mit $R^3$ und $R^4$ substituiert sind, die mindestens ein Heteroatom aufweisen, das ausgewählt ist aus O, S oder N, wobei das Heteroatom eine carbocyclische Ringstruktur unterbricht und eine ausreichende Anzahl von delokalisierten pi-Elektronen aufweist, um aromatischen Charakter zu verleihen, wobei die aromatischen heterocyclischen Gruppen 2 bis 14 Kohlenstoffatome enthalten;

Heteroarylakyl (Heteroaralkyl) eine Heteroarylgruppe repräsentiert, wie oben definiert, die an eine Alkylgruppe, wie oben definiert, gebunden ist;

Heterocycloalkyl einen gesättigten, verzweigten oder unverzweigten carbocyclischen Ring repräsentiert, der 3 bis 15 Kohlenstoffatome enthält, wobei der carbocyclische Ring durch 1 bis 3 Hetero-Gruppen unterbrochen ist, die ausgewählt sind aus -O-, -S-, $-NR^{10}-$ (wobei $R^{10}$ wie oben definiert ist).

**2.** Verbindung nach Anspruch 1, bei der a N ist und b, c und d Kohlenstoff sind; A und B jeweils $H_2$ sind, die optionale Bindung zwischen C5 und C6 nicht vorhanden ist; X CH ist und $R^5$, $R^6$, $R^7$ und $R^8$ H sind.

**3.** Verbindung nach Anspruch 1 oder 2, bei der W ausgewählt ist aus der Gruppe bestehend aus:

(1) -CN;
(2) $-C(O)R^{12}$, wobei $R^{12}$ ausgewählt ist aus der Gruppe bestehend aus: H, Alkyl,

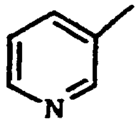, 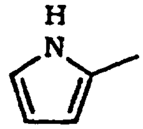, 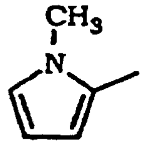, 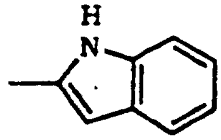, 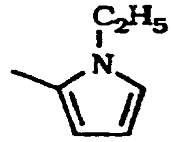,

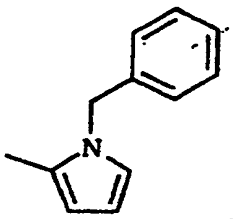, 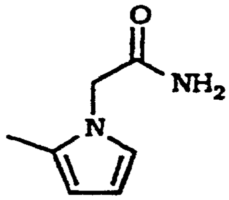, 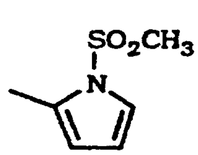, 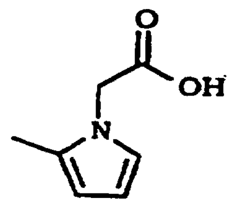,

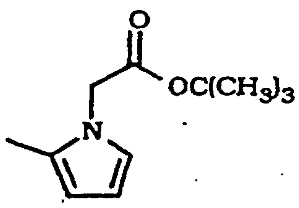, 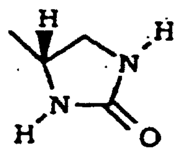, 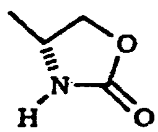, 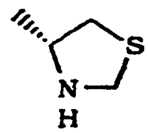, 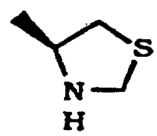,

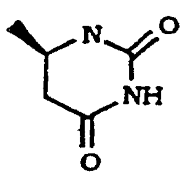, 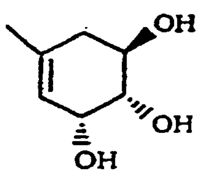, 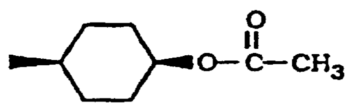, 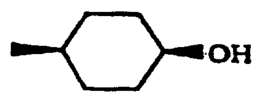,

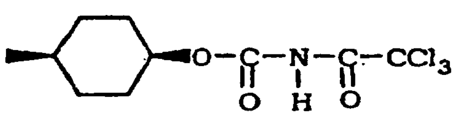, 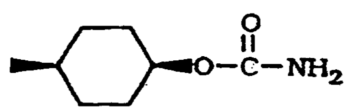, 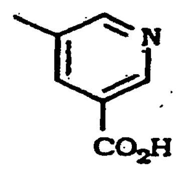,

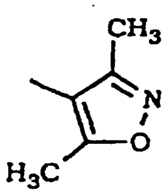, 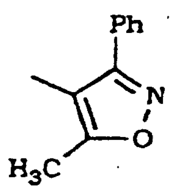, 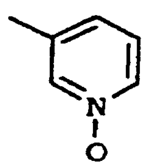, 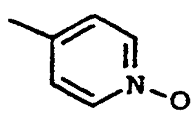, 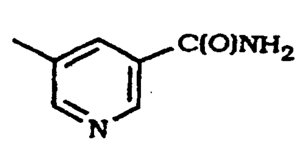.

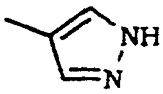, 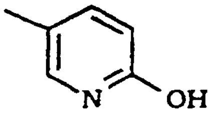, 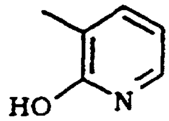, 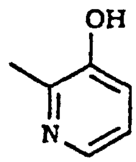 und 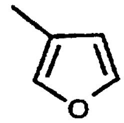

(3) einem Imidat, wobei $R^{13}$ ausgewählt ist aus der Gruppe bestehend aus:

(a) -CN;
(b) H;
(c) $-SO_2NR^{10}R^{15}$, wobei $R^{10}$ und $R^{15}$ ausgewählt sind aus der Gruppe bestehend aus: H und Alkyl;
(d) $-C(O)NR^{10}R^{15}$, wobei $R^{10}$ und $R^{15}$ ausgewählt sind aus der Gruppe bestehend aus: H und Alkyl;
(e) $-SO_2$-Alkyl und
(f) -CO(O)-Aryl;

(4) einem Imidamid, wobei $R^{13}$ ausgewählt ist aus der Gruppe bestehend aus:

(a) CN;
(b) H;
(c) $-OR^{14}$;
(d) $-NR^{10}R^{15}$, wobei $R^{10}$ und $R^{15}$ unabhängig ausgewählt sind aus der Gruppe bestehend aus H und Alkyl;
(e) $-SO_2NR^{10}R^{15}$, wobei $R^{10}$ und $R^{15}$ unabhängig ausgewählt sind aus der Gruppe bestehend aus: H und Alkyl;
(f) $-C(O)NR^{10}R^{15}$, wobei $R^{10}$ und $R^{15}$ unabhängig ausgewählt sind aus der Gruppe bestehend aus: H und Alkyl;
(g) $-SO_2$-Alkyl und
(h) -C(O)-Aryl und

(5) einem 1-Amino-2-nitroethylenderivat, wobei $R^{10}$ Alkyl ist.

**4.** Verbindung nach einem der Ansprüche 1 bis 3, bei der $R^{14}$ für die Imidatgruppe Phenyl ist und $R^{10}$ und $R^{16}$ für die Imidamidgruppe ausgewählt ist aus der Gruppe bestehend aus: H und Heteroaralkyl.

**5.** Verbindung nach einem der Ansprüche 1 bis 4, bei der $R^4$ ausgewählt ist aus der Gruppe bestehend aus: Cl oder Br.

**6.** Verbindung nach einem der Ansprüche 1 bis 5, bei der X CH ist.

**7.** Verbindung nach einem der Ansprüche 1 bis 6, bei der X N ist.

**8.** Verbindung nach einem der Ansprüche 1 bis 7, bei der $R^1$ Br und $R^3$ Cl ist.

**9.** Verbindung nach einem der Ansprüche 1 bis 8, die ausgewählt ist aus:

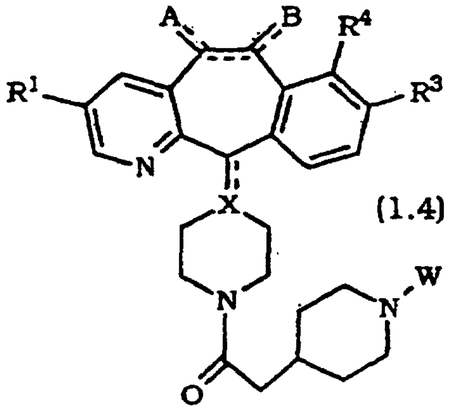
(1.4)

oder

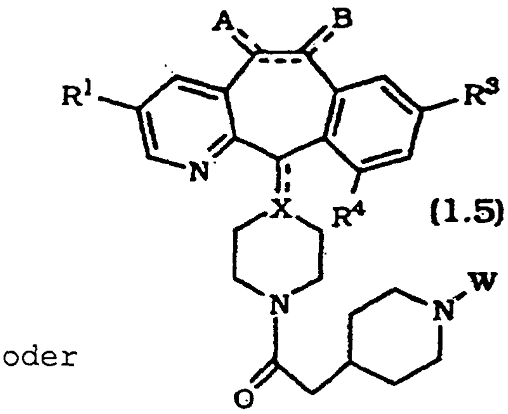
(1.5)

wobei $R^1$, $R^3$, $R^4$ A, B, X und W wie in Anspruch 1 definiert sind.

**10.** Verbindung nach einem der Ansprüche 1 bis 9, bei der $R^1$ Br, $R^3$ Cl und $R^4$ Br ist.

**11.** Verbindung nach einem der Ansprüche 1 bis 10, bei der W ausgewählt ist aus der Gruppe bestehend aus:

(1) -C(O)$R^{12}$, wobei $R^{12}$ ausgewählt ist aus der Gruppe bestehend aus H, -$CH_3$,

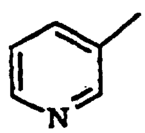
,
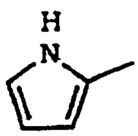
,
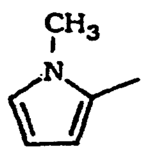
,
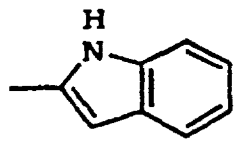
,
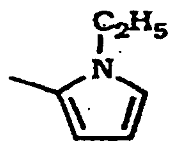
,

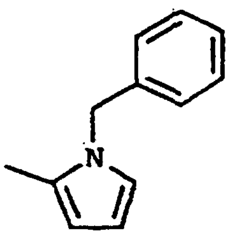
,
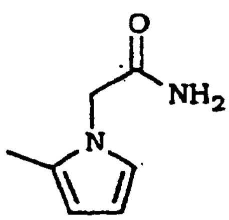
,
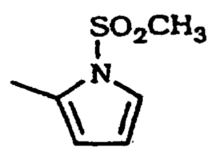
,
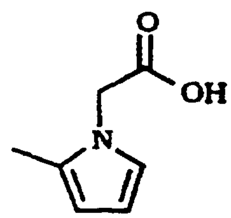
,

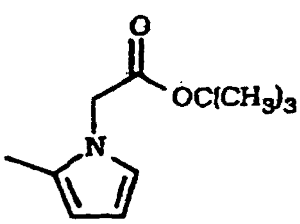
,
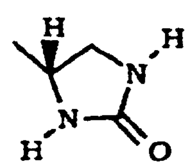
,
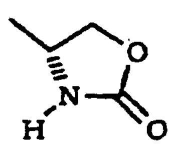
,
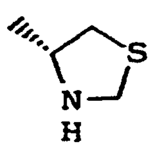
,
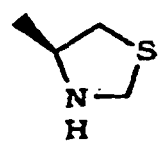
,

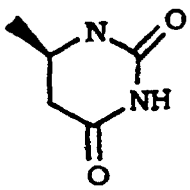 , 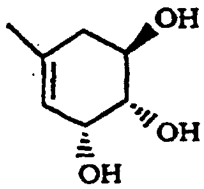 , 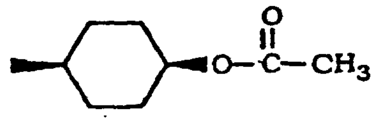 , 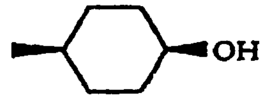 ,

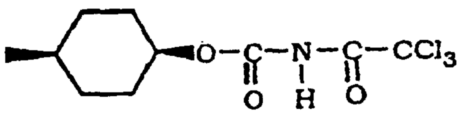 , 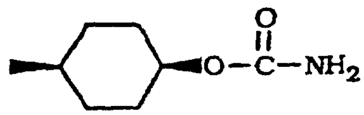 , 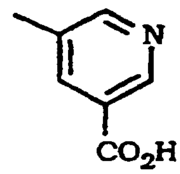 ,

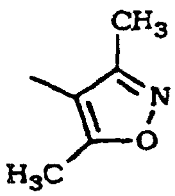 , 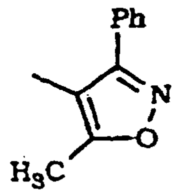 , 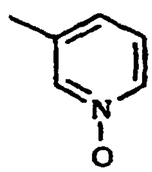 , 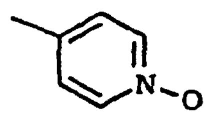 , 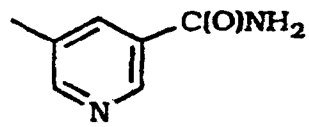 ,

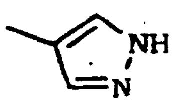 , 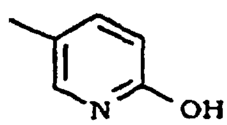 , 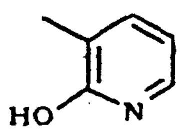 , 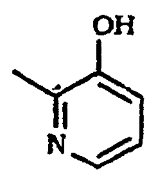 und 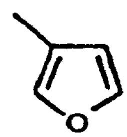 ,

(2) einem Imidat, wobei $R^{13}$ ausgewählt ist aus der Gruppe bestehend aus: (a) CN, (b) $-C(O)NH_2$, (c) H, (d) - $SO_2NH_2$, (e) $-SO_2NHCH_3$, (f) $-SO_2N(CH_3)_2$, (g) $-C(O)NHCH_3$, (h) $-SO_2CH_3$ und (i) $-C(O)C_6H_5$;

(3) einem Imidamid, wobei $R^{13}$ ausgewählt ist aus der Gruppe bestehend aus: (a) CN; (b) H, (c) $-OCH_3$; (d) -OH, (e) $-NH_2$, (f) $-N(CH_3)_2$, (g) $-SO_2NH_2$, (h) - $SO_2NHCH_3$, (i) $-SO_2N(CH_3)_2$, (j) $-C(OH)NH_2$, (k) $-OC(O)NHCH_3$, (1) $-SO_2CH_3$ und (m) $-C(O)C_6H_5$ und

(4) einem 1-Amino-2-nitroethylenderivat, wobei $R^{10}$ $CH_3$ ist.

**12.** Verbindung nach einem der Ansprüche 1 bis 11, bei der für die Imidatgruppe $R^{14}$ Phenyl ist und für die Imidamidgruppen $R^{10}$ und $R^{16}$ unabhängig ausgewählt sind aus der Gruppe im wesentlichen bestehend aus: H und

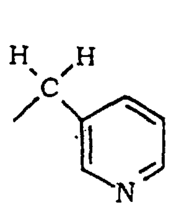 .

**13.** Verbindung nach einem der Ansprüche 1 bis 3, die die Struktur

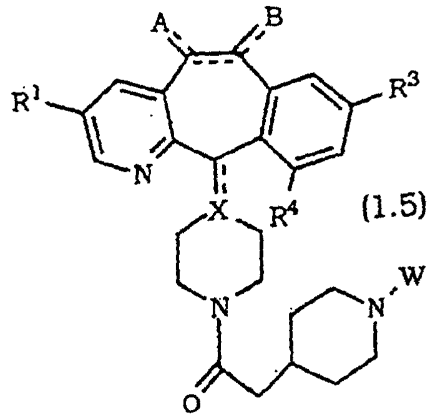

(1.5)

aufweist.

**14.** Verbindung nach Anspruch 1, die ausgewählt ist aus:

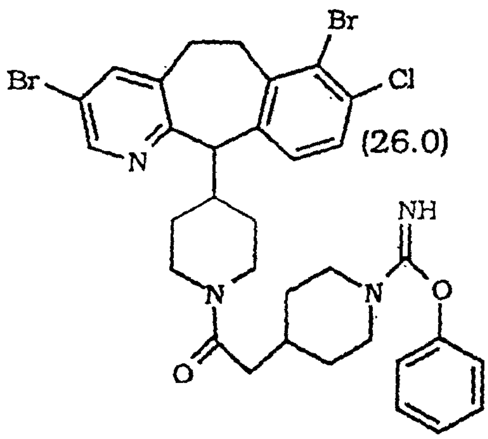

(26.0)

;

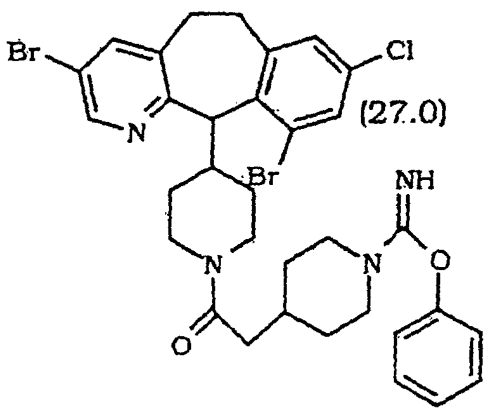

(27.0)

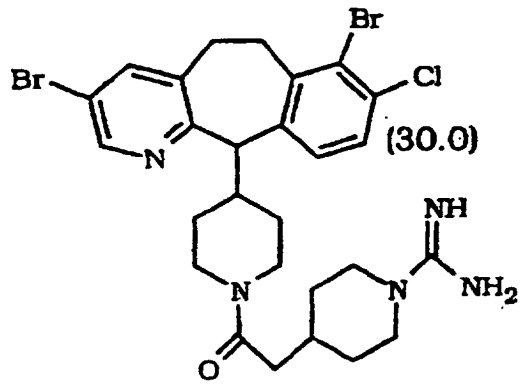

(30.0)

;

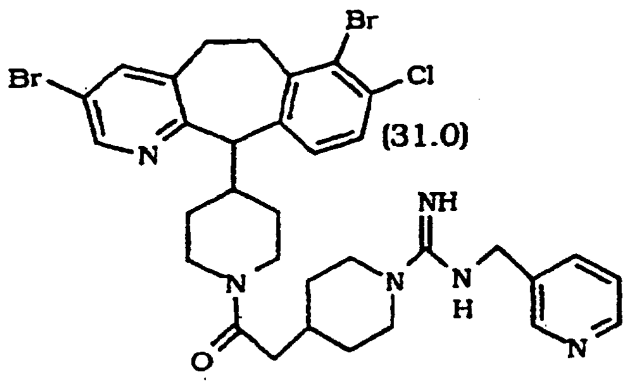

(31.0)

;

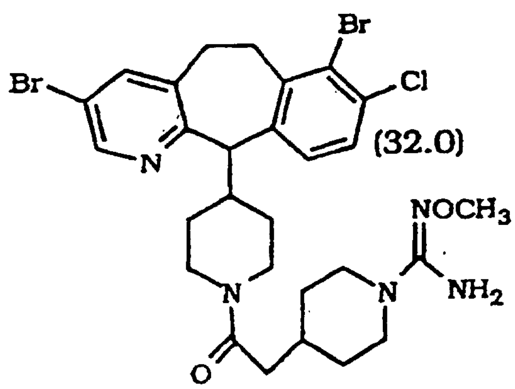

(32.0)

;

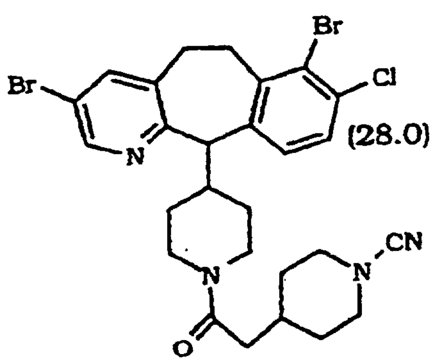

(28.0)

;

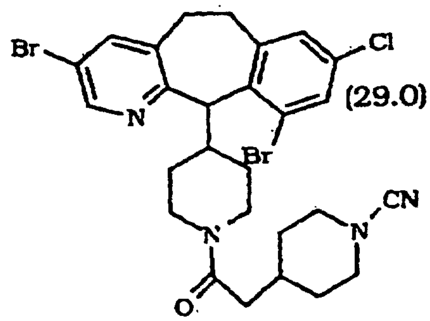

(29.0) ;

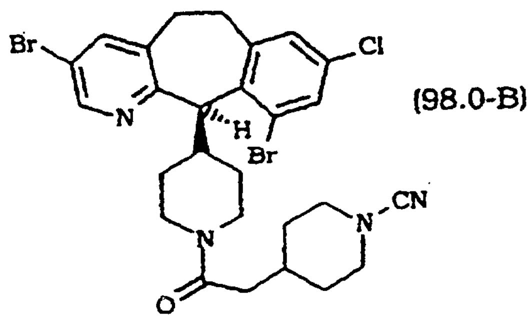

(98.0-B) ;

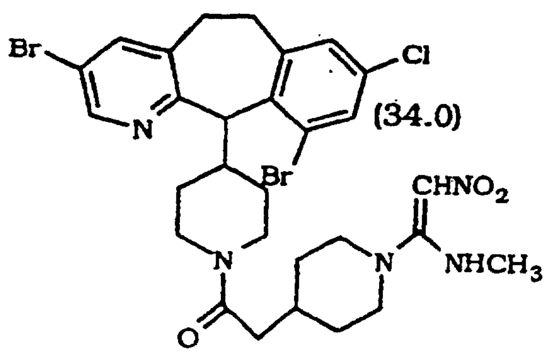

(34.0) ;

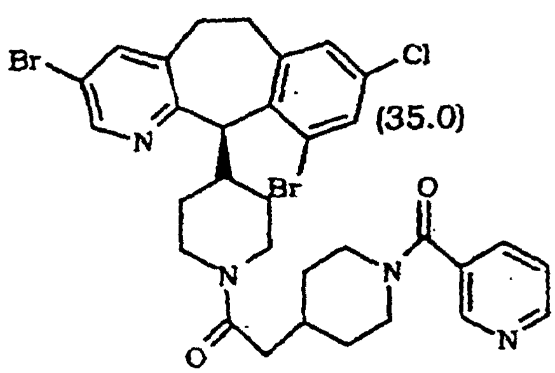

(35.0) ;

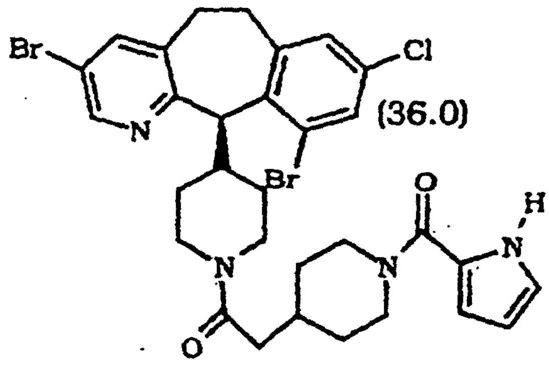

(36.0)

;

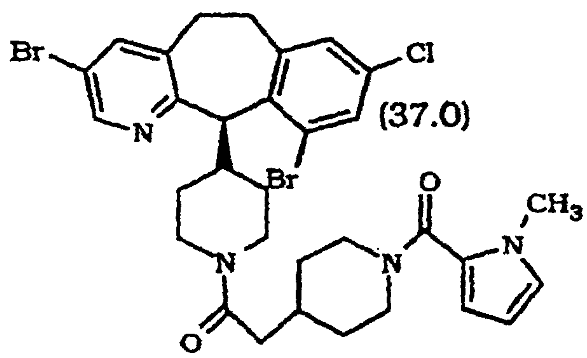

(37.0)

;

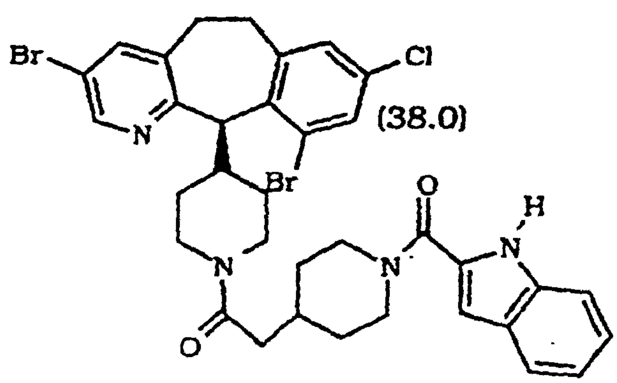

(38.0)

;

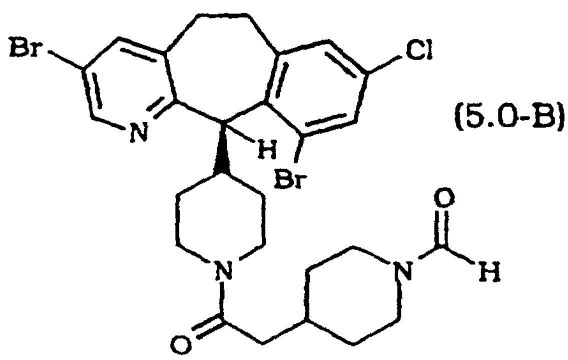

(5.0-B)

;

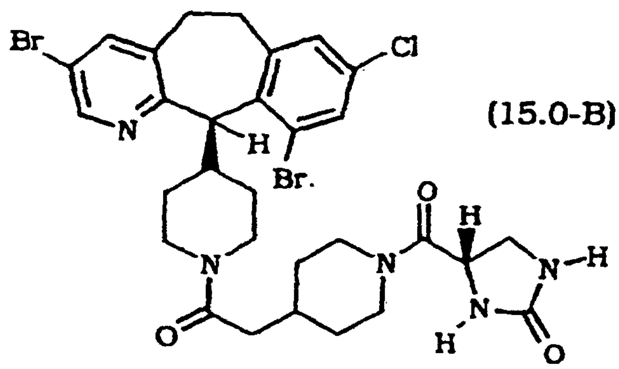

(15.0-B)

;

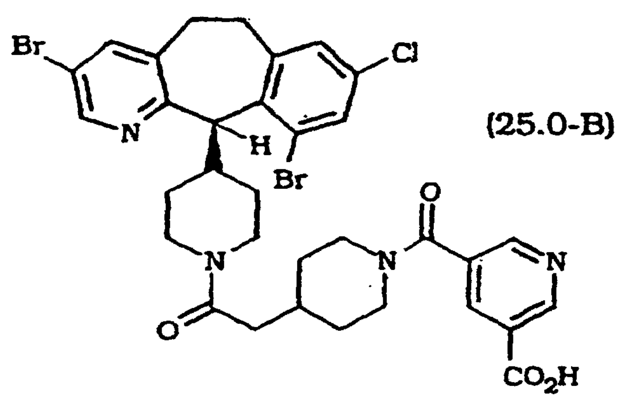

(25.0-B)

;

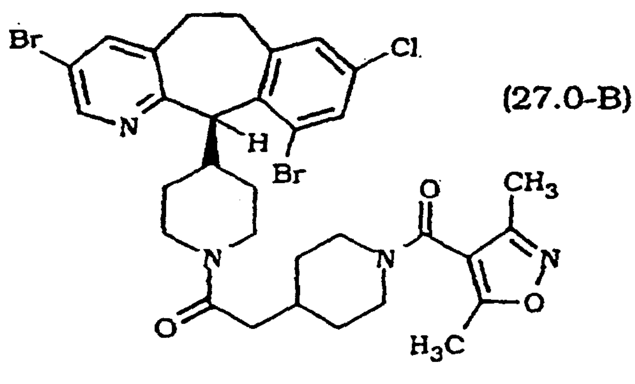

(27.0-B)

;

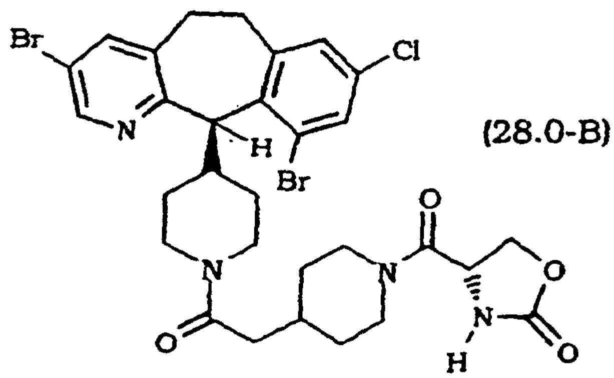

(28.0-B) ;

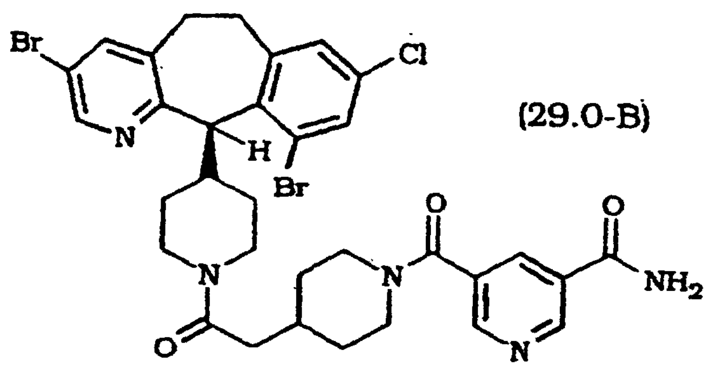

(29.0-B) ;

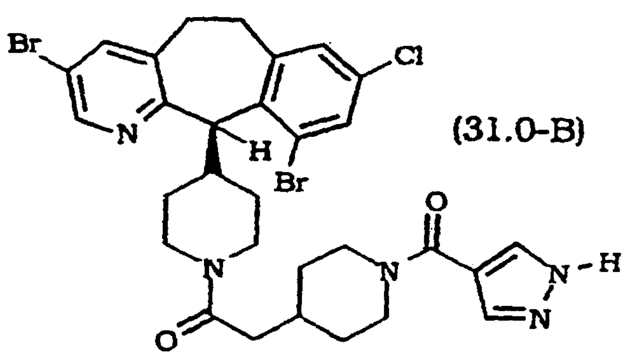

(31.0-B) ;

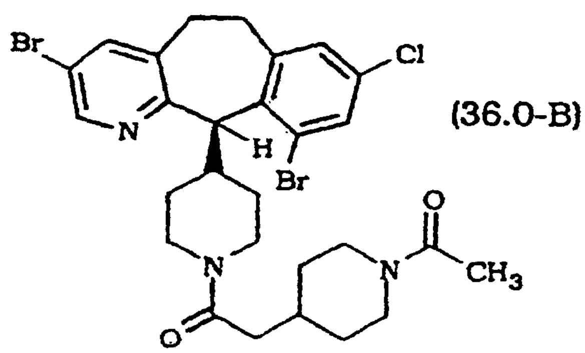

(36.0-B) ;

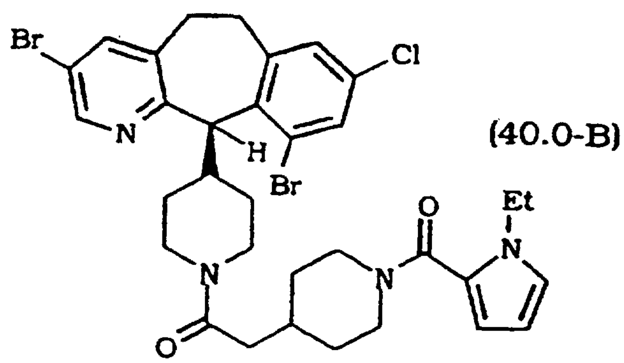

(40.0-B)

;

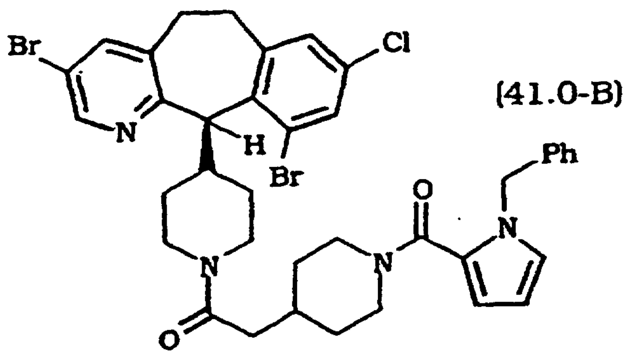

(41.0-B)

;

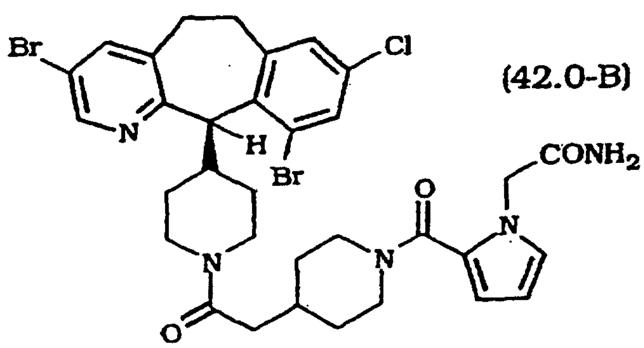

(42.0-B)

;

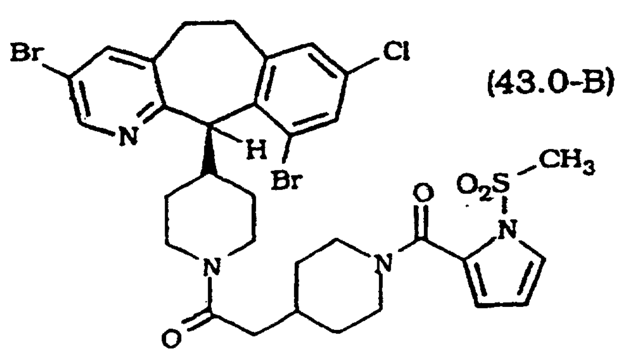

(43.0-B)

;

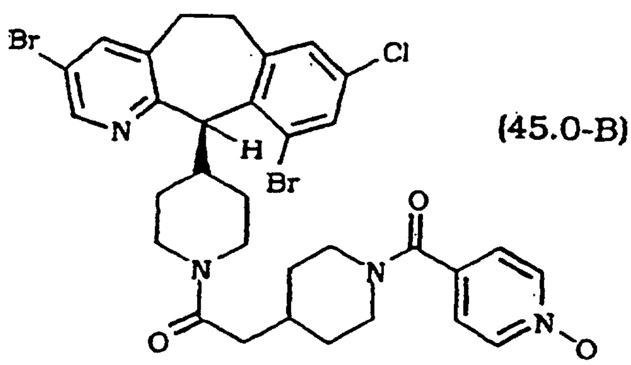

(45.0-B)

;

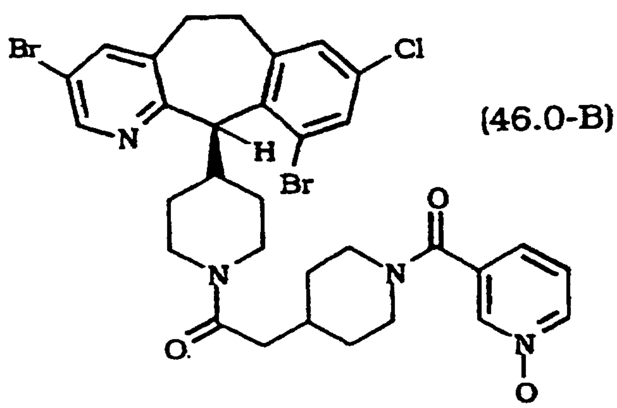

(46.0-B)

;

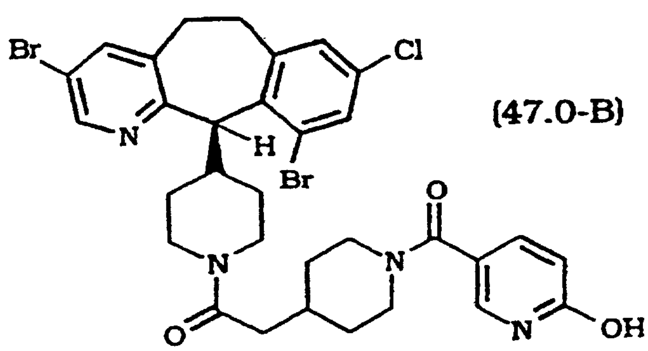

(47.0-B)

;

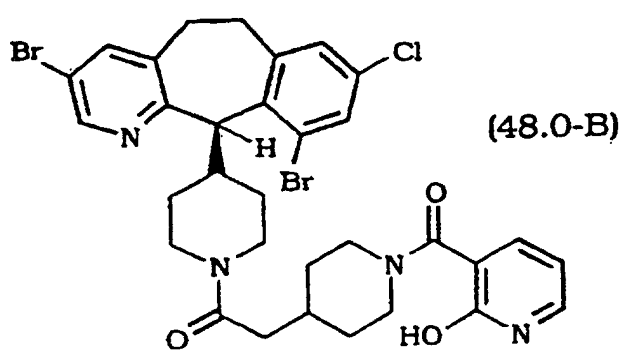

(48.0-B)

;

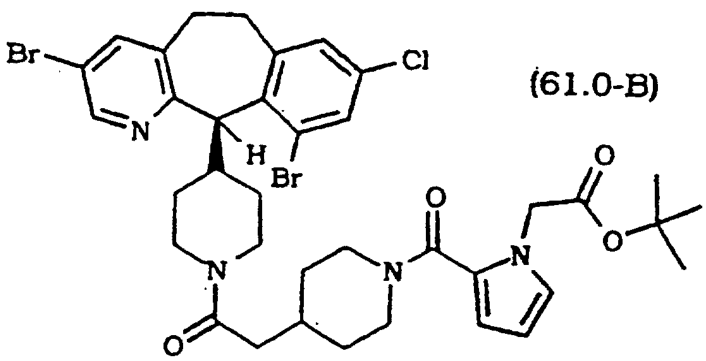

(61.0-B)

;

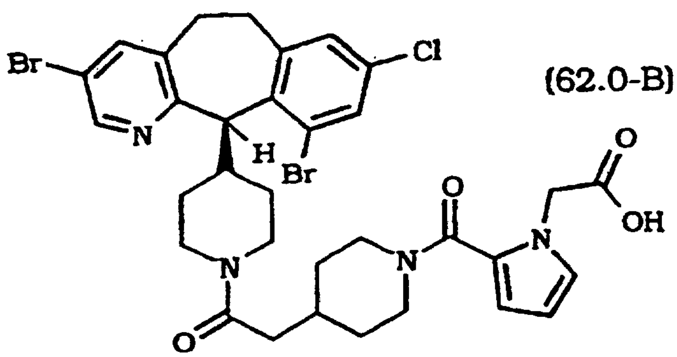

(62.0-B)

;

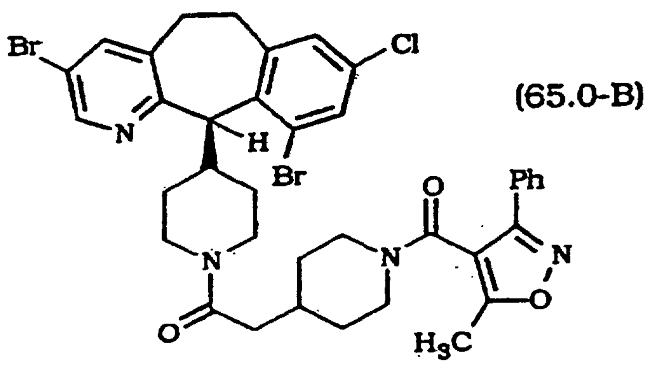

(65.0-B)

;

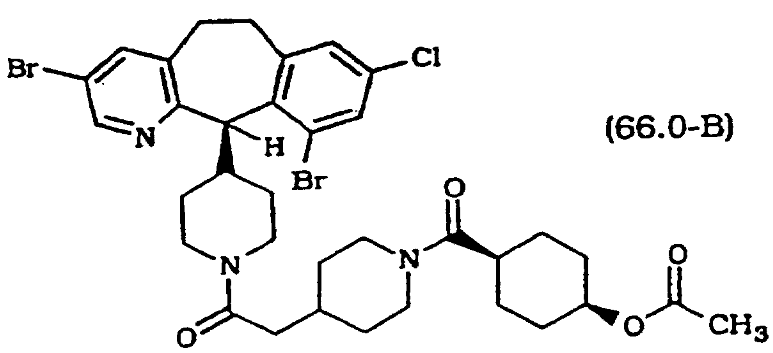

(66.0-B)

;

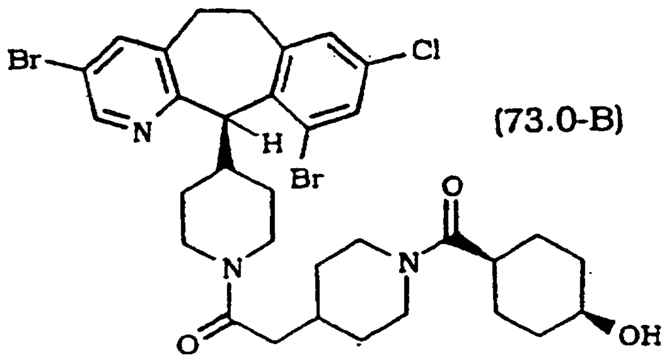

(73.0-B)

;

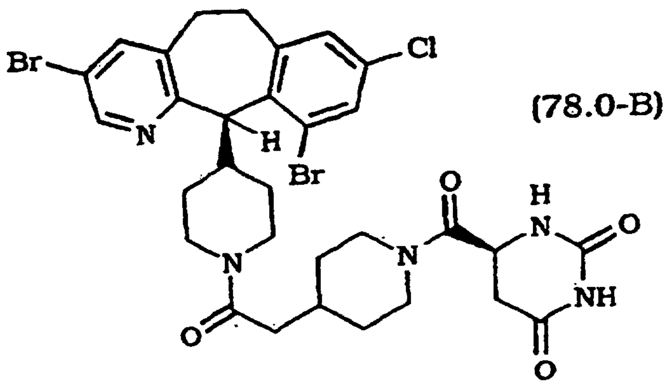

(78.0-B)

;

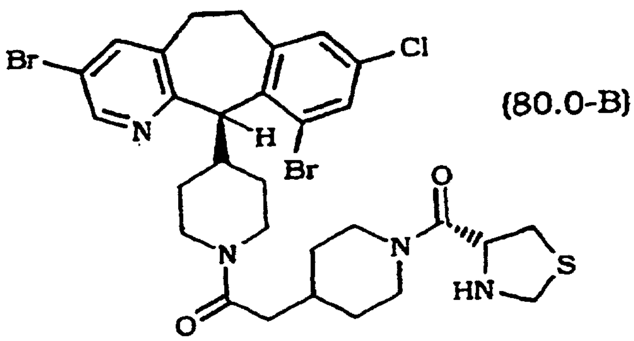

(80.0-B)

;

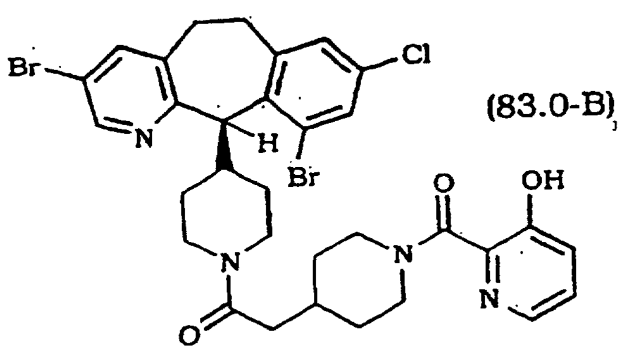

(83.0-B),

;

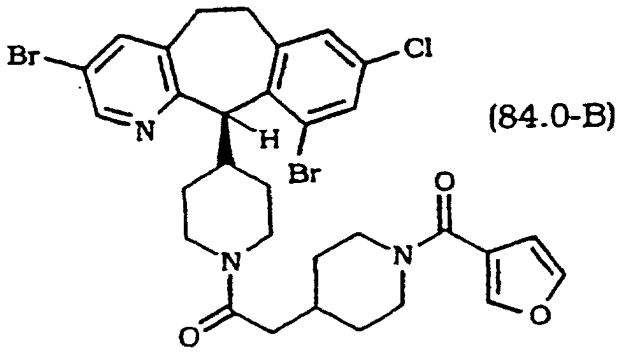

(84.0-B)

;

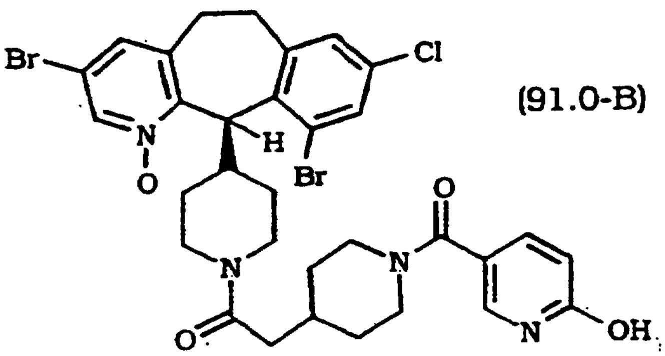

(91.0-B)

;

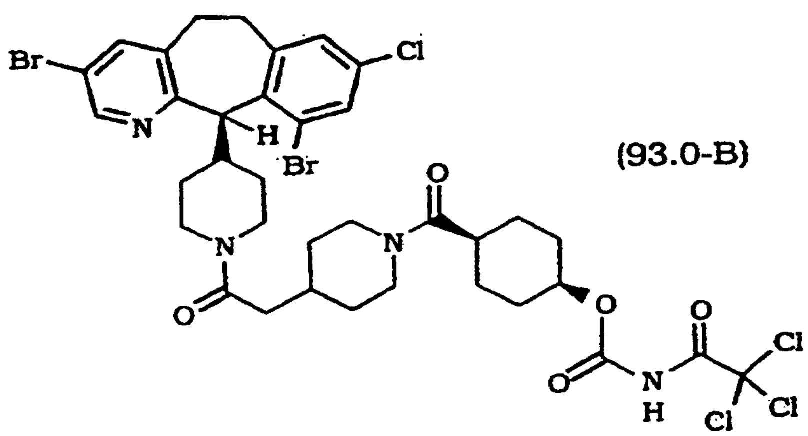

(93.0-B)

;

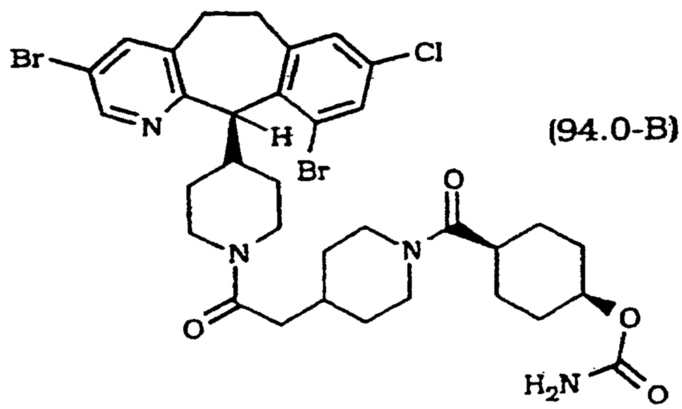

(94.0-B) ;

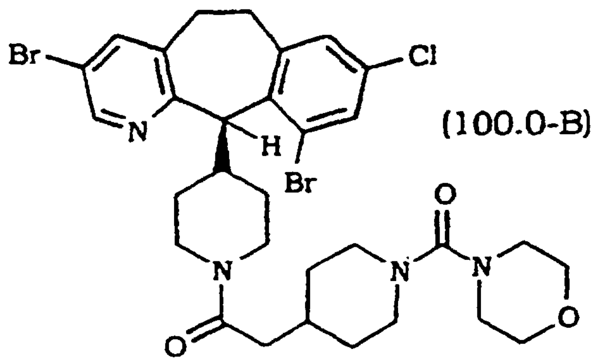

(100.0-B) ;

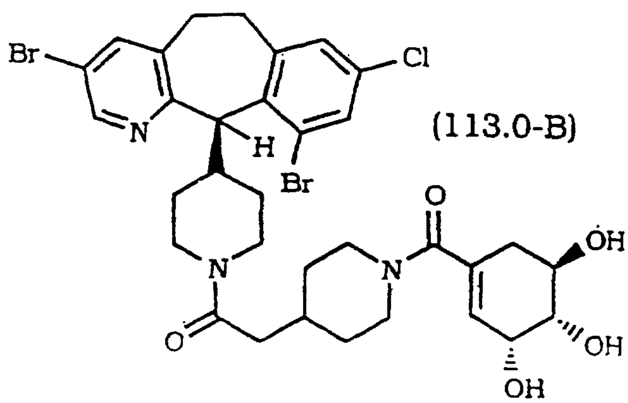

(113.0-B)

oder

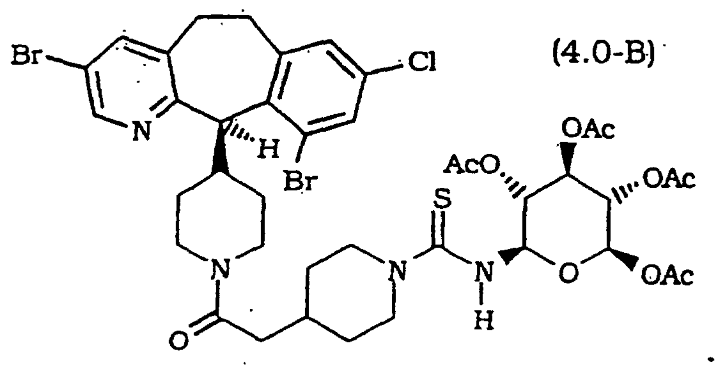

(4.0-B)

**15.** Pharmazeutische Zusammensetzung, die eine wirksame Menge einer Verbindung nach einem der Ansprüche 1 bis 14 in Kombination mit einem pharmazeutisch annehmbaren Träger enthält.

**16.** Verwendung einer Verbindung nach einem der Ansprüche 1 bis 14 zur Herstellung eines Medikaments für die Behandlung von Tumorzellen.

**17.** Verwendung nach Anspruch 16, bei der die behandelten Zellen Pankreastumorzellen, Lungenkrebszellen, myeloide Leukämietumorzellen, thyroide Follikeltumorzellen, myelodysplastische Tumorzellen, epidermale Krebstumorzellen, Blasenkrebstumorzellen, Darmtumorzellen, Brusttumorzellen oder Prostataturmozellen sind.

**18.** Verwendung einer Verbindung gemäß einem der Ansprüche 1 bis 14 zur Herstellung eines Medikaments für die Hemmung von Farnesylproteintransferase.

**Revendications**

**1.** Composé de formule :

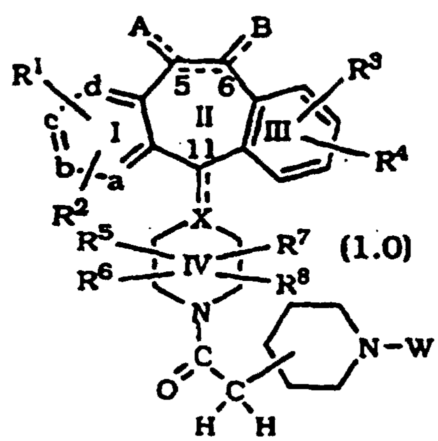

(1.0)

dans laquelle l'un de a, b, c et d représente N ou $NR^9$ où $R^9$ représente $O^-$, $-CH_3$ ou $-(CH_2)_nCO_2H$ où n est un nombre de 1 à 3, et les groupes a, b, c et d restants représentent $CR^1$ ou $CR^2$, ou bien chacun de a, b, c et d représente indépendamment $CR^1$ ou $CR^2$,
$R^2$ représente H et $R^1$, $R^3$ et $R^4$ représentent des atomes d'halogène,
$R^5$, $R^6$, $R^7$ et $R^8$ représentent chacun indépendamment H, $-CF_3$, $-COR^{10}$,
ou un groupe alkyle ou aryle, éventuellement substitué par $-OR^{10}$, $-SR^{10}$, $-S(O)_tR^{11}$, $-NR^{10}COOR^{11}$, $-N(R^{10})_2$, $-NO_2$, $-COR^{10}$, $-OCOR^{10}$, $-OCO_2R^{11}$, $-CO_2R^{10}$ ou $-OPO_3R^{10}$, ou bien $R^5$ et $R^6$ sont combinés pour représenter =O ou =S et/ou $R^7$ et $R^8$ sont combinés pour représenter =O ou =S,
$R^{10}$ représente H ou un groupe alkyle, aryle ou aralkyle,
$R^{11}$ représente un groupe alkyle ou aryle,

X représente N, CH ou C qui peut porter une deuxième liaison éventuelle (représentée par la ligne en pointillés) avec l'atome de carbone 11,
la ligne en pointillés entre les atomes de carbone 5 et 6 représente une deuxième liaison éventuelle, de sorte que, lorsqu'il y a une double liaison, A et B représentent chacun indépendamment $-R^{10}$, un atome d'halogène, $-OR^{11}$, $-OCO_2R^{11}$ ou $-OC(O)R^{10}$, et, lorsqu'il n'y a pas de double liaison entre les atomes de carbone 5 et 6, A et B représentent chacun indépendamment $H_2$, $-(OR^{11})_2$, H et un atome d'halogène, deux atomes d'halogène, un groupe alkyle et H, deux groupes alkyle, H et
$- OC(O)R^{10}$, H et $-OR^{10}$, =O, un groupe aryle et H, $=NOR^{10}$ ou $-O-(CH_2)_p-O-$ où p est égal à 2, 3 ou 4,
et W représente un groupe choisi parmi :

(1) le groupe cyano,
(2) les groupes $-C(O)R^{12}$ où $R^{12}$ représente :

(a) un groupe hétéroaryle,
(b) H,
(c) un groupe alkyle, ou
(d) un substituant de formule :

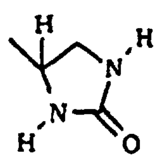 , 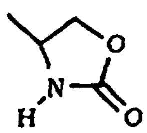 , 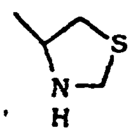 , 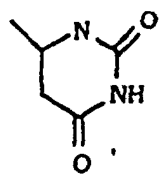 , 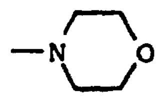 ,

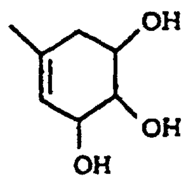 ou 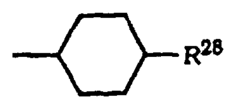 ,

où $R^{28}$ représente $-OC(O)R^{29}$, -OH, $-OC(O)NHC(O)CCl_3$ ou $-OC(O)NH_2$, $R^{29}$ désignant un groupe alkyle,
(3)les groupes imidate de formule :

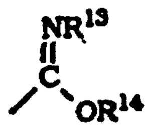

dans laquelle $R^{13}$ représente H ou un groupe CN, $-SO_2$-alkyl, -C(O)-aryl, $-SO_2NR^{10}R^{15}$, $-C(O)NR^{10}R^{15}$, $-OR^{10}$ ou $-C(O)NR^{10}C(O)NR^{10}R^{15}$, $R^{14}$ représente un groupe aryle, et $R^{10}$ et $R^{15}$ représentent chacun indépendamment H ou un groupe alkyle, aryle ou aralkyle,
(4)les groupes imidamido de formule :

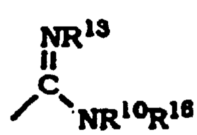

dans laquelle $R^{13}$ représente H ou un groupe CN, $-SO_2$-alkyl, -C(O)-aryl, $-SO_2NR^{10}R^{15}$, $-C(O)NR^{10}R^{15}$, $-OR^{10}$ ou $-C(O)NR^{10}C(O)NR^{10}R^{15}$, $R^{16}$ représente un groupe alkyle, aralkyle, aryle, cycloalkyle, hétéroaryle, hété-

roaralkyle ou hétérocycloalkyle, $R^{10}$ et $R^{15}$ ont les définitions indiquées précédemment, et $R^{10}$ et $R^{16}$ sont choisis indépendamment parmi les groupes définis précédemment,
(5)les groupes 1-amino-2-nitroéthylène de formule :

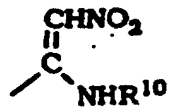

dans laquelle $R^{10}$ a les définitions indiquées précédemment, et
(6)les substituants de formule :

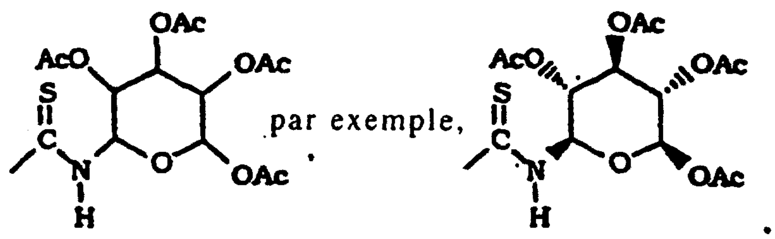

par exemple,

les termes employés précédemment ayant, sauf indication contraire, les significations suivantes :

alcényle représente une chaîne carbonée, droite ou ramifiée, ayant au moins une double liaison carbone-carbone et contenant 2 à 12 atomes de carbone,
alcynyle représente une chaîne carbonée, droite ou ramifiée, ayant au moins une triple liaison carbone-carbone et contenant 2 à 12 atomes de carbone,
alkyle (y compris la partie alkyle des groupes alcoxy, aralkyle et hétéroarylalkyle) représente une chaîne carbonée, droite ou ramifiée, contenant 1 à 20 atomes de carbone,
aralkyle représente un groupe aryle tel que défini ci-après, lié à un groupe alkyle tel que défini précédemment,
aryle (y compris la partie aryle des groupes aralkyle, aryloxy et arylalkyloxy) représente un groupe carbocyclique contenant 6 à 15 atomes de carbone et ayant au moins un noyau aromatique, tous les atomes de carbone substituables disponibles du groupe carbocyclique étant des points possibles de rattachement et ledit groupe carbocyclique étant éventuellement substitué par un ou plusieurs substituants (par exemple 1 à 3) pris parmi les atomes d'halogène, les groupes alkyle, le groupe hydroxyle, les groupes alcoxy, le groupe phénoxy, $CF_3$, le groupe amino, les groupes alkylamino, les groupes dialkylamino, les groupes $-COOR^{10}$ et le groupe $-NO_2$,
cycloalkyle représente un noyau carbocyclique saturé, ramifié ou non ramifié, ayant 3 à 20 atomes de carbone,
halogène représente le fluor, le chlore, le brome et l'iode,
hétéroaryle représente un groupe cyclique, éventuellement substitué par $R^3$ et $R^4$, ayant au moins un hétéroatome pris parmi O, S et N, interrompant une structure carbocyclique, et ayant un nombre suffisant d'électrons pi délocalisés pour avoir le caractère aromatique, ledit groupe hétérocyclique aromatique contenant 2 à 14 atomes de carbone, hétéroarylalkyle (ou hétéroaralkyle) représente un groupe hétéroaryle tel que défini précédemment, lié à un groupe alkyle tel que défini précédemment,
hétérocycloalkyle représente un noyau carbocyclique saturé, ramifié ou non ramifié, contenant 3 à 15 atomes de carbone, qui est interrompu par 1 à 3 hétérogroupes pris parmi -O-, -S- et $-NR^{10}-$ (où $R^{10}$ a les définitions indiquées précédemment),

ou un sel ou produit de solvatation pharmaceutiquement acceptable d'un tel composé.

**2.** Composé selon la revendication 1, pour lequel a représente N, b, c et d représentent un groupe carboné, A et B représentent chacun $H_2$, la deuxième liaison éventuelle entre C5 et C6 est absente, X représente CH, et $R^5$, $R^6$, $R^7$ et $R^8$ représentent des atomes d'hydrogène.

**3.** Composé selon la revendication 1 ou 2, pour lequel W représente un groupe choisi parmi :

(1) le groupe -CN,

(2) les groupes $-C(O)R^{12}$ où $R^{12}$ représente un atome d'hydrogène ou un groupe alkyle,

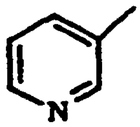 , 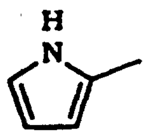 , 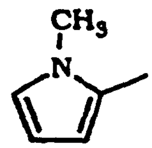 , 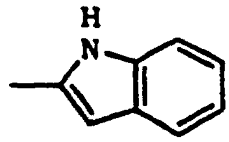 , 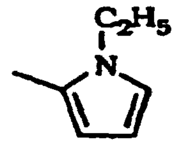 ,

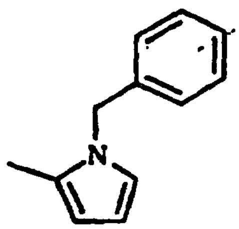 , 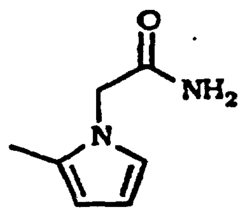 , 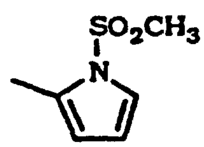 , 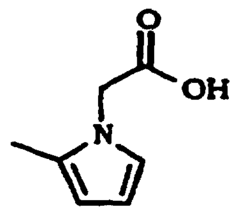 ,

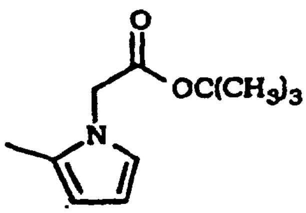 , 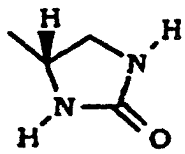 , 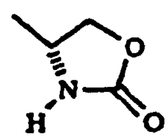 , 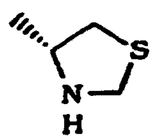 , 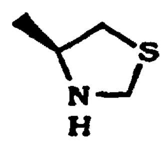 ,

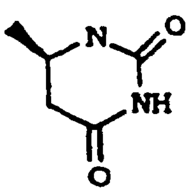 , 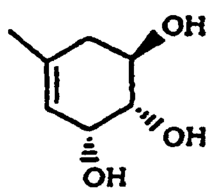 , 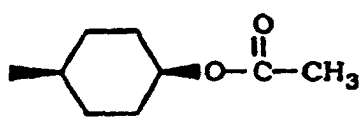 , 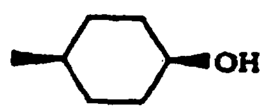 ,

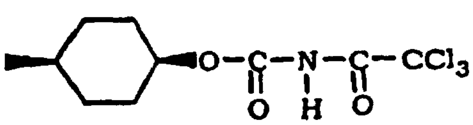 , 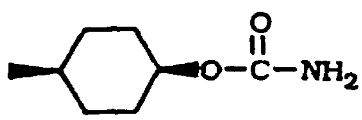 , 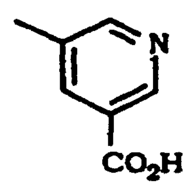 ,

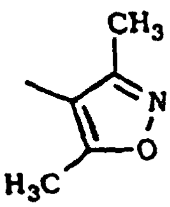, 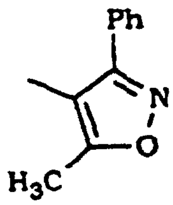, 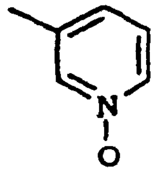, 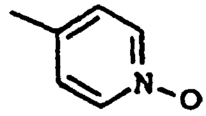, 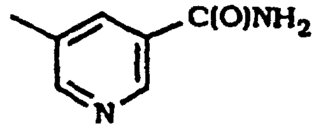, 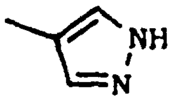, 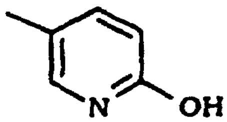, 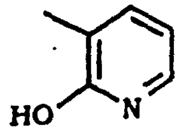, 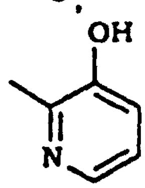 ou 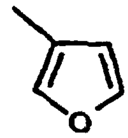 ;

(3)les groupes imidate où $R^{13}$ est choisi parmi :

(a) -CN,
(b) H,
(c) $-SO_2NR^{10}R^{15}$ où $R^{10}$ et $R^{15}$ représentent chacun H ou un groupe alkyle,
(d)-$C(O)NR^{10}R^{15}$ où $R^{10}$ et $R^{15}$ représentent chacun H ou un groupe alkyle,
(e)-$SO_2$-alkyl, et
(f)-C(O)-aryl,

(4)les groupes imidamido où $R^{13}$ représente un groupe choisi parmi :

(a)CN,
(b)H,
(c)-$OR^{14}$,
(d)-$NR^{10}R^{15}$ où $R^{10}$ et $R^{15}$ représentent chacun indépendamment H ou un groupe alkyle,
(e)-$SO_2NR^{10}R^{15}$ où $R^{10}$ et $R^{15}$ représentent chacun indépendamment H ou un groupe alkyle,
(f) -$C(O)NR^{10}R^{15}$ où $R^{10}$ et $R^{15}$ représentent chacun indépendamment H ou un groupe alkyle,
(g)-$SO_2$-alkyl, et
(h)-C(O)-aryl, et

(5)les groupes 1-amino-2-nitroéthylène où $R^{10}$ représente un groupe alkyle.

**4.** Composé selon l'une quelconque des revendications 1 à 3, pour lequel $R^{14}$ dudit groupe imidate est un groupe phényle, et $R^{10}$ et $R^{16}$ dudit groupe imidamido sont choisis parmi H et les groupes hétéroaralkyle.

**5.** Composé selon l'une quelconque des revendications 1 à 4, pour lequel $R^4$ représente Cl ou Br.

**6.** Composé selon l'une quelconque des revendications 1 à 5, pour lequel X représente CH.

**7.** Composé selon l'une quelconque des revendications 1 à 5, pour lequel X représente N.

**8.** Composé selon l'une quelconque des revendications 1 à 7, pour lequel $R^1$ représente Br et $R^3$ représente Cl.

**9.** Composé selon l'une quelconque des revendications 1 à 8, qui est choisi parmi les composés de formule :

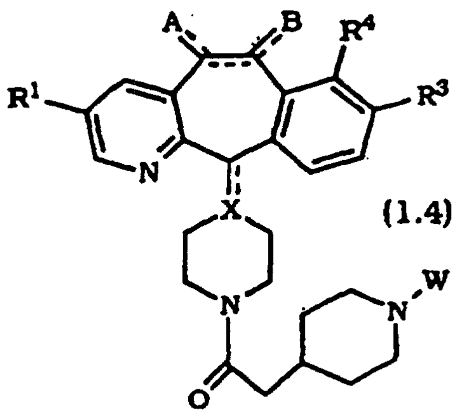

(1.4)

ou

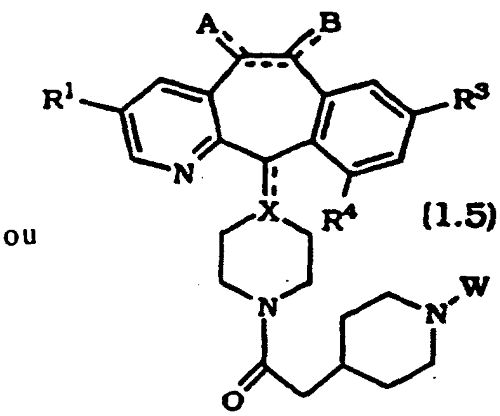

(1.5)

dans laquelle $R^1$, $R^3$, $R^4$, A, B, X et W sont tels que définis dans la revendication 1.

**10.** Composé selon l'une quelconque des revendications 1 à 9, pour lequel $R^1$ représente Br, $R^3$ représente Cl, et $R^4$ représente Br.

**11.** Composé selon l'une quelconque des revendications 1 à 10, pour lequel W représente un groupe choisi parmi :

(1)les groupes $-C(O)R^{12}$ où $R^{12}$ représente H, $-CH_3$,

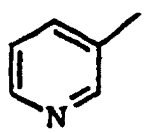,
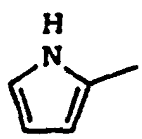,
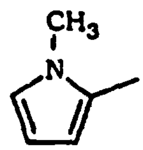,
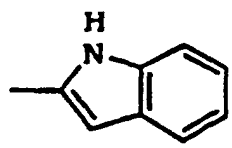,
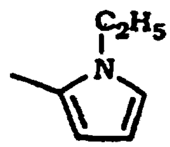,

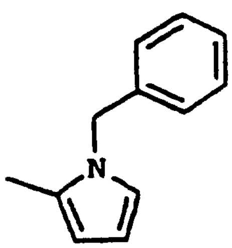,
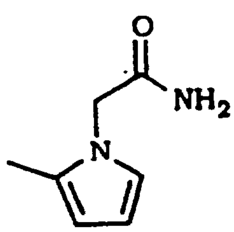,
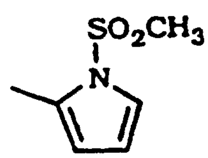,
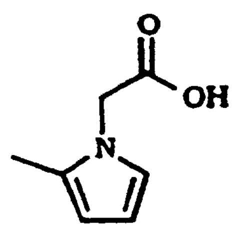,

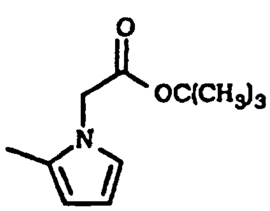,
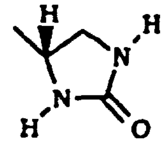,
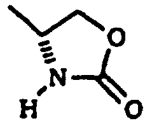,
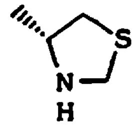,
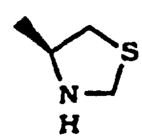,

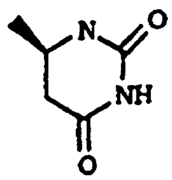, 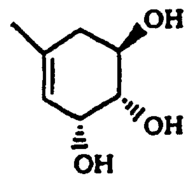, 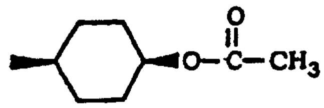, 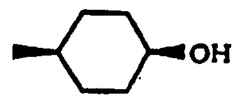,

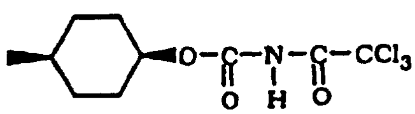, 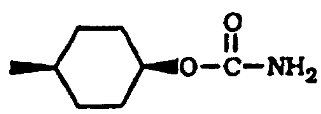, 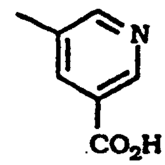,

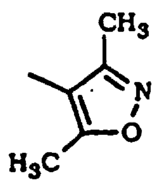, 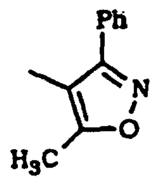, 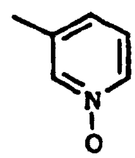, 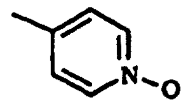, 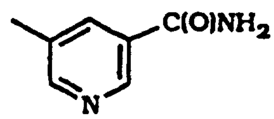,

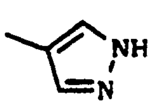, 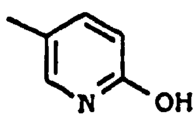, 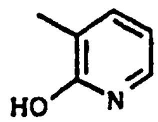, 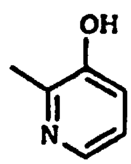 ou 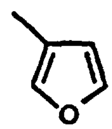 ;

(2)les groupes imidate pour lesquels $R^{13}$ représente CN, $-C(O)NH_2$, H, $-SO_2NH_2$, $-SO_2NHCH_3$, $-SO_2N(CH_3)_2$, $-C(O)NHCH_3$, $-SO_2CH_3$ ou $-C(O)C_6H_5$,
(3)les groupes imidamido pour lesquels $R^{13}$ représente CN, H, $-OCH_3$, -OH, $-NH_2$, $-N(CH_3)_2$, $-SO_2NH_2$, $-SO_2NHCH_3$, $-SO_2N(CH_3)_2$, $-C(O)NH_2$, $-C(O)NHCH_3$, $-SO_2CH_3$ ou $-C(O)C_6H_5$, et
(4)le groupe 1-amino-2-nitroéthylène où $R^{10}$ représente $-CH_3$.

**12.** Composé selon l'une quelconque des revendications 1 à 11, pour lequel le groupe $R^{14}$ dudit groupe imidate est le groupe phényle, et les groupes $R^{10}$ et $R^{16}$ dudit groupe imidamido sont choisis indépendamment parmi H et

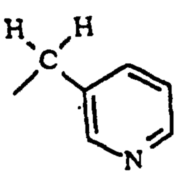.

**13.** Composé selon l'une quelconque des revendications 1 à 3, qui a la structure suivante :

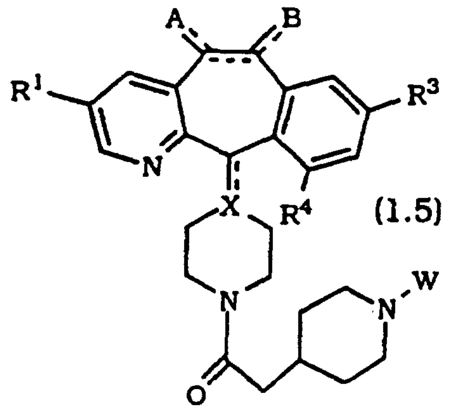

(1.5)

.

**14.** Composé selon la revendication 1, qui est choisi parmi les composés suivants :

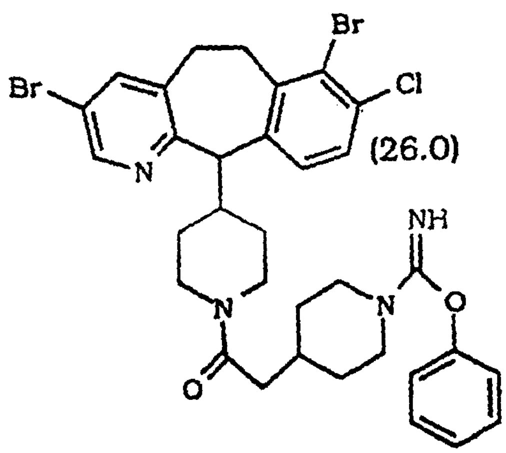

(26.0)

;

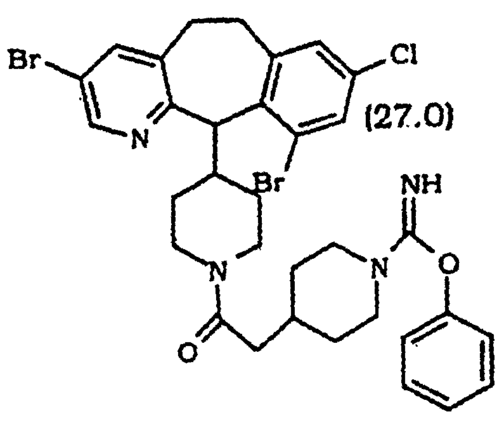

(27.0)

;

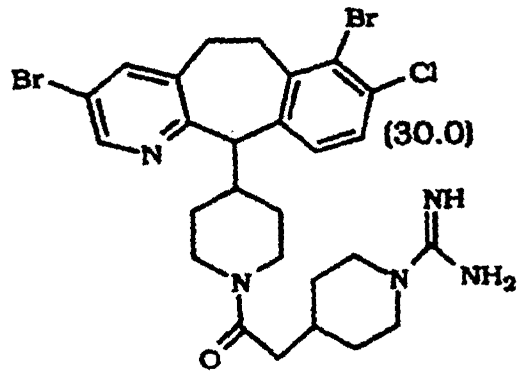

(30.0)

;

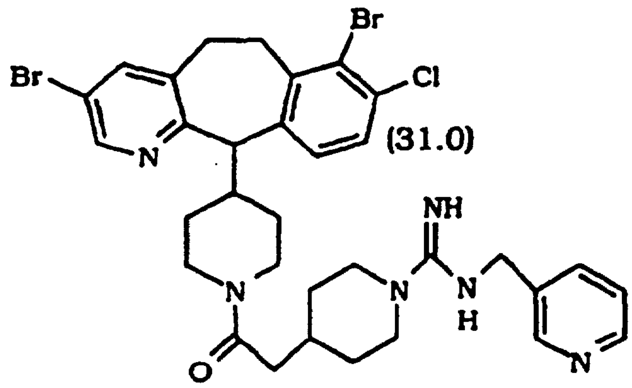

(31.0)

;

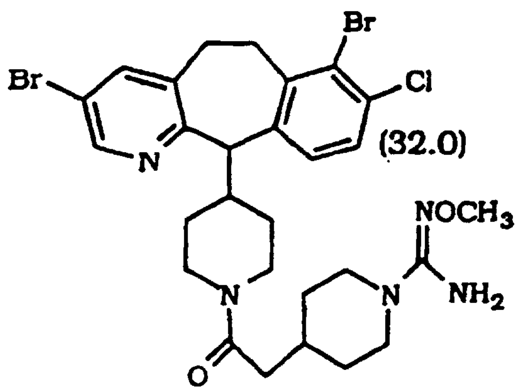

(32.0)

;

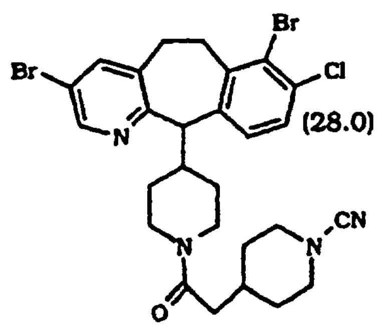

(28.0)

;

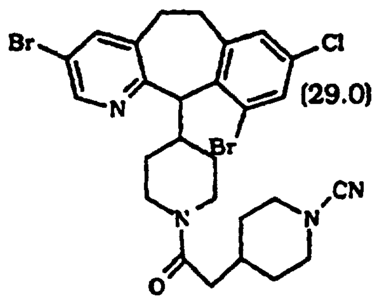

(29.0)

;

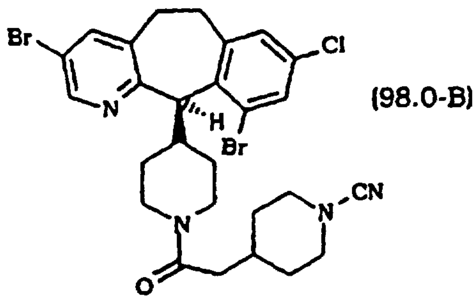

(98.0-B)

;

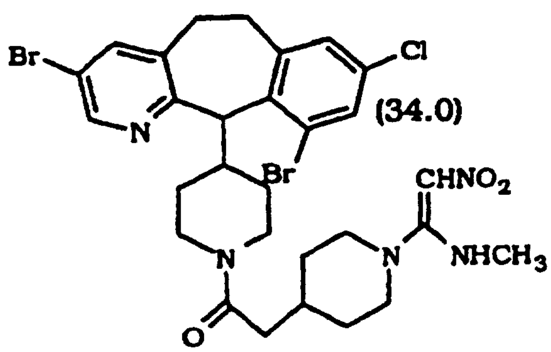

(34.0)

;

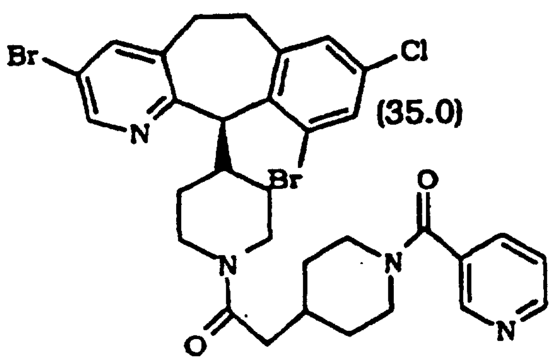

(35.0)

;

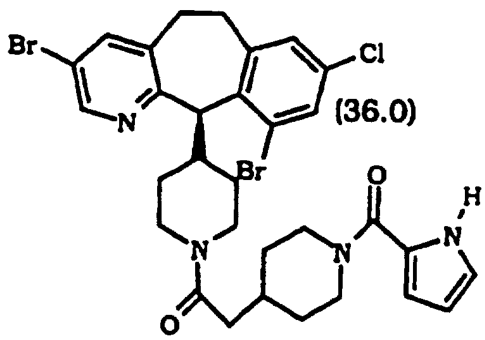

(36.0)

;

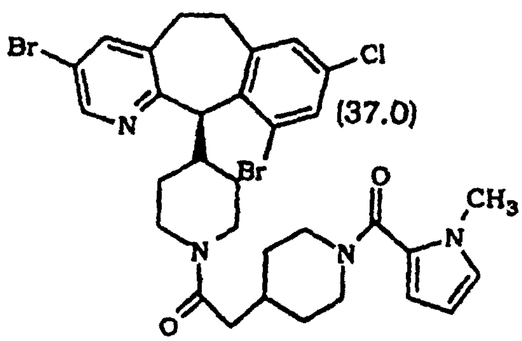

(37.0)

;

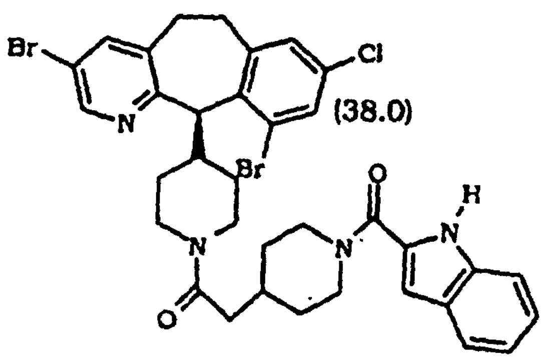

(38.0)

;

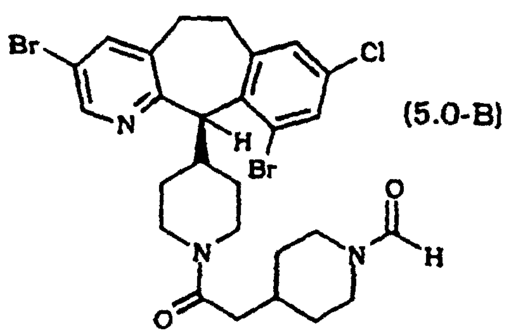

(5.0-B)

;

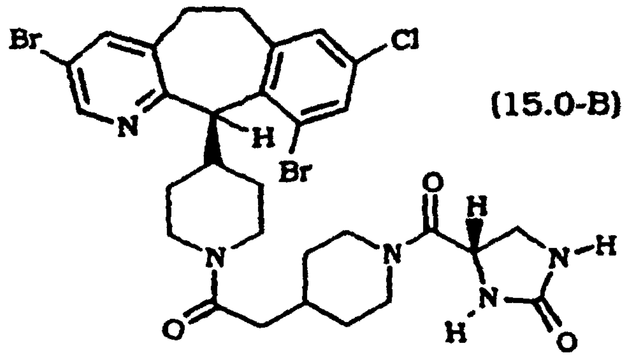

(15.0-B)

;

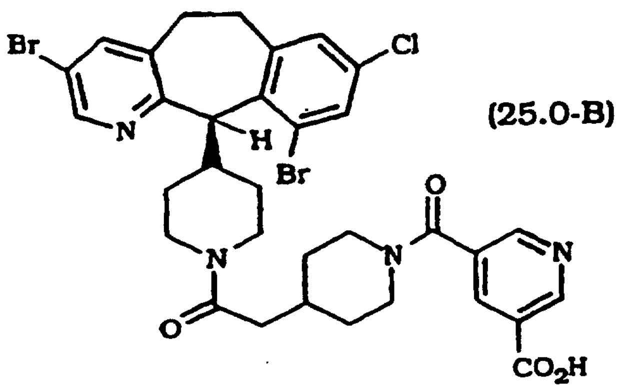

(25.0-B)

;

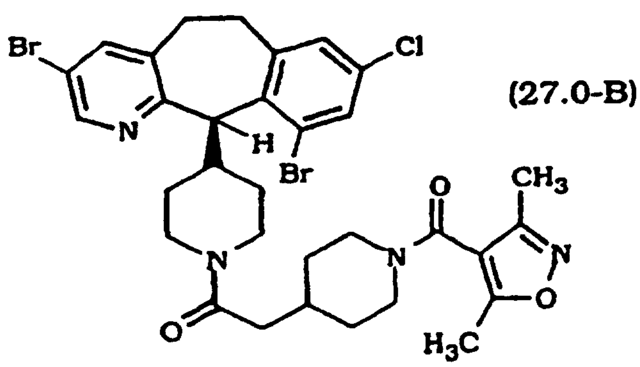

(27.0-B)

;

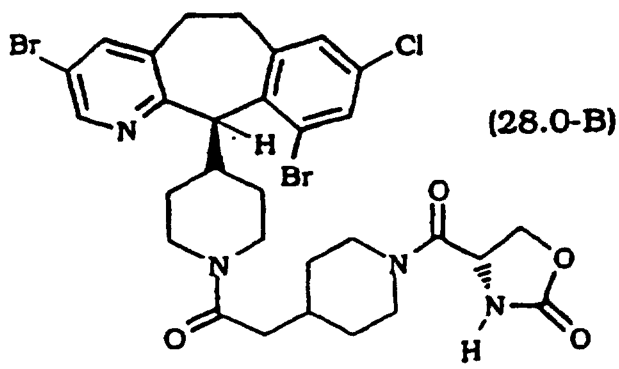

(28.0-B)

;

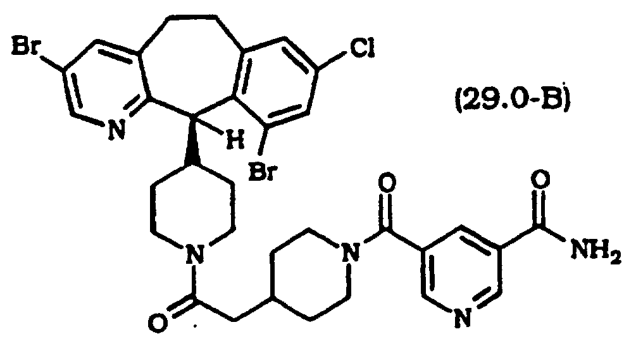

(29.0-B)

;

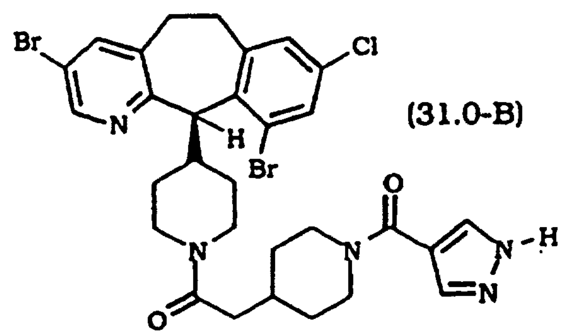

(31.0-B)

;

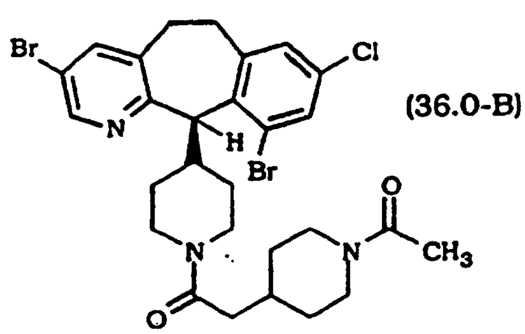

(36.0-B)

;

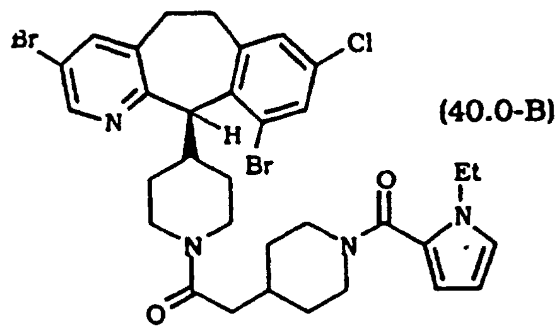

(40.0-B)

;

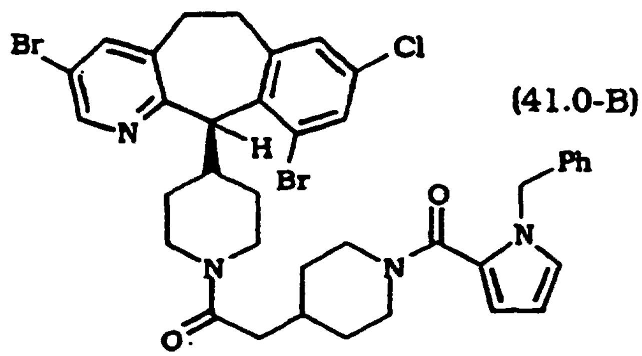

(41.0-B)

;

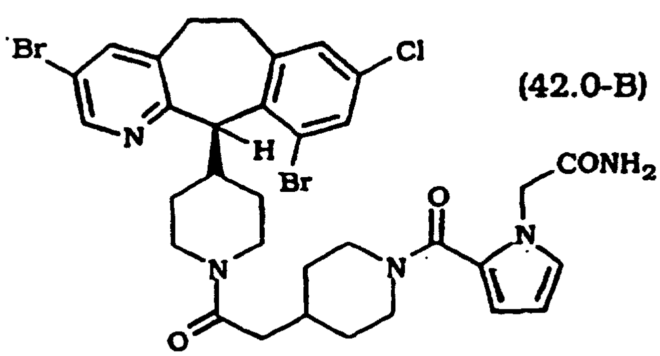

(42.0-B)

;

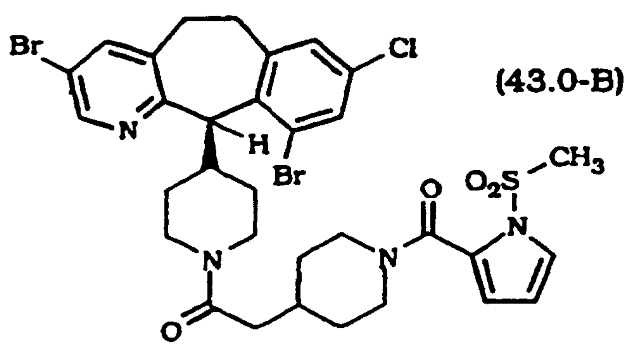

(43.0-B)

;

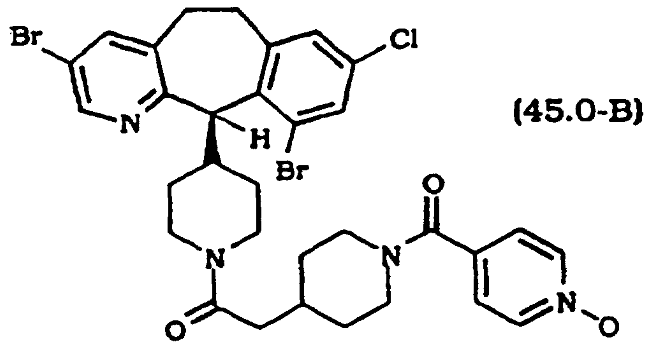

(45.0-B)

;

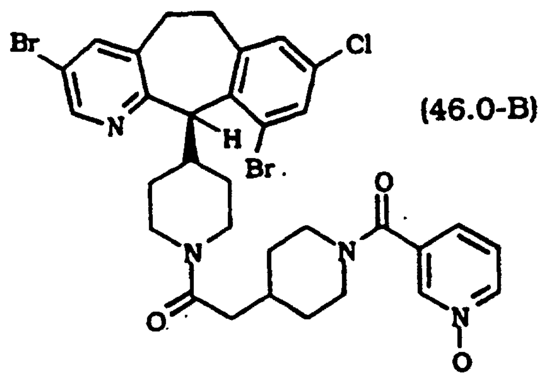

(46.0-B)

;

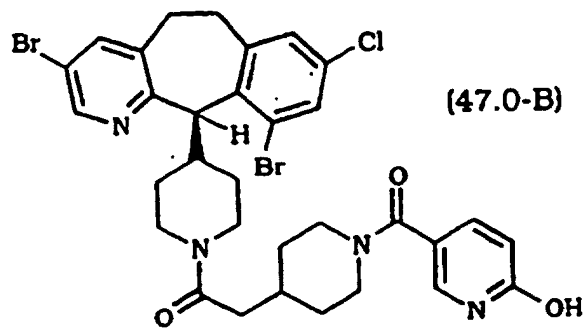

(47.0-B)

;

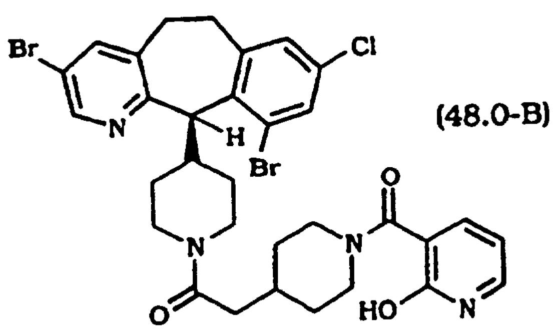

(48.0-B)

;

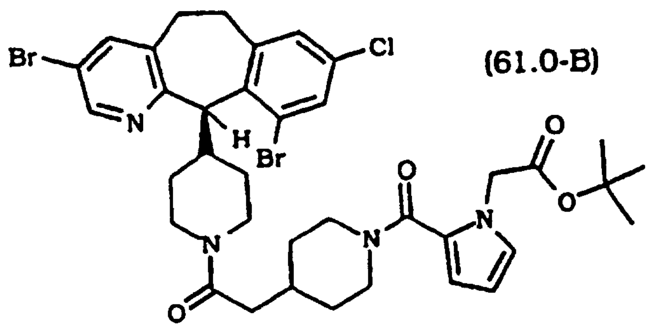

(61.0-B)

;

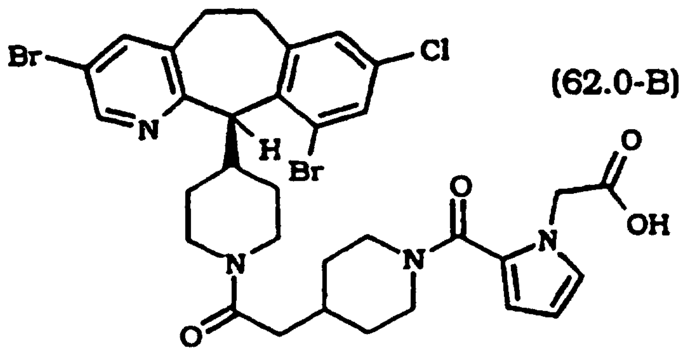

(62.0-B)

;

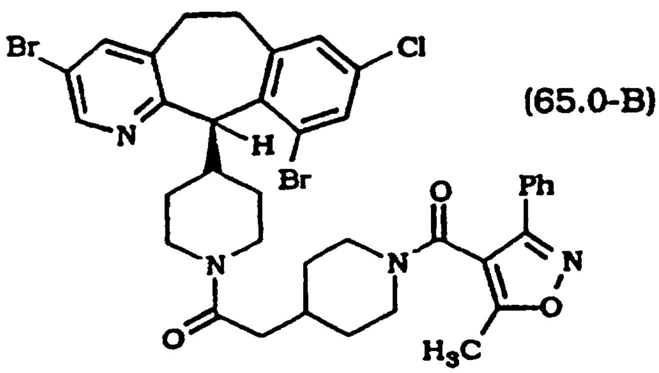

(65.0-B)

;

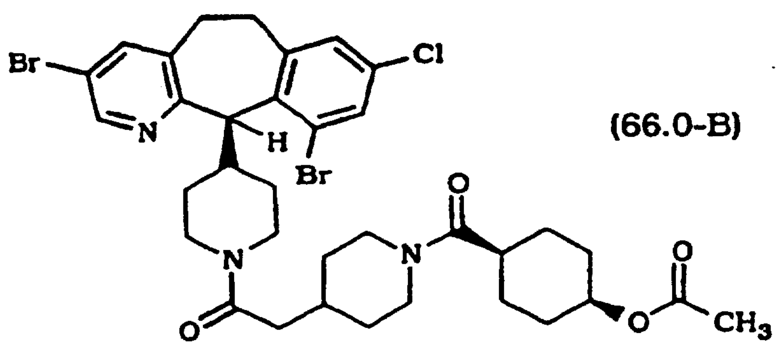

(66.0-B)

;

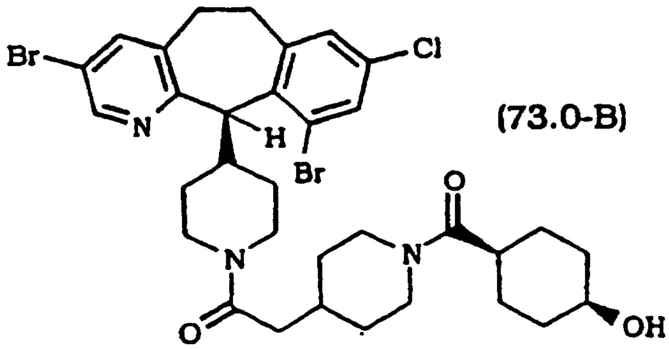

(73.0-B)

;

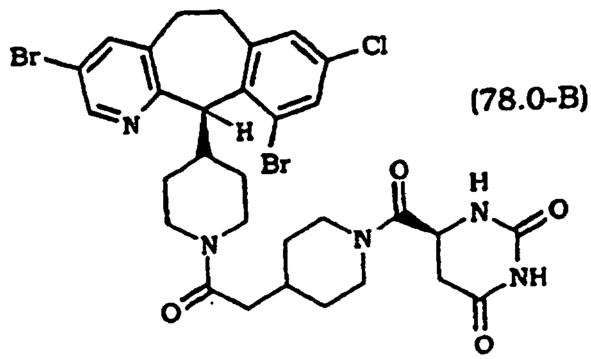

(78.0-B)

;

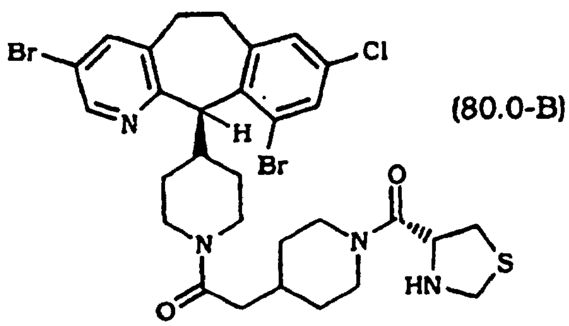

(80.0-B)

;

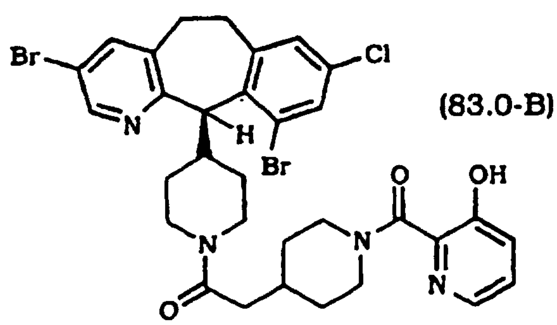

(83.0-B)

;

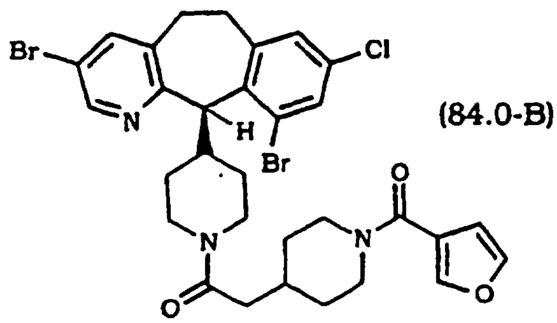

(84.0-B)

;

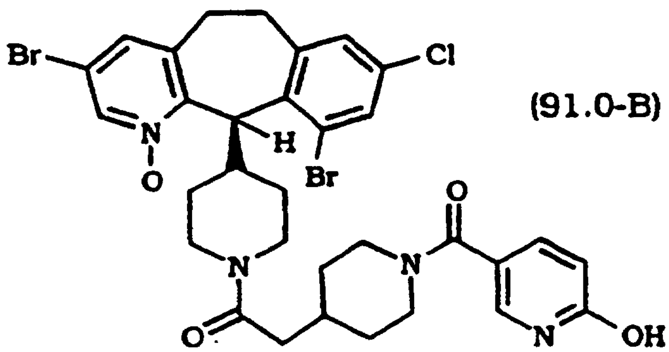

(91.0-B)

;

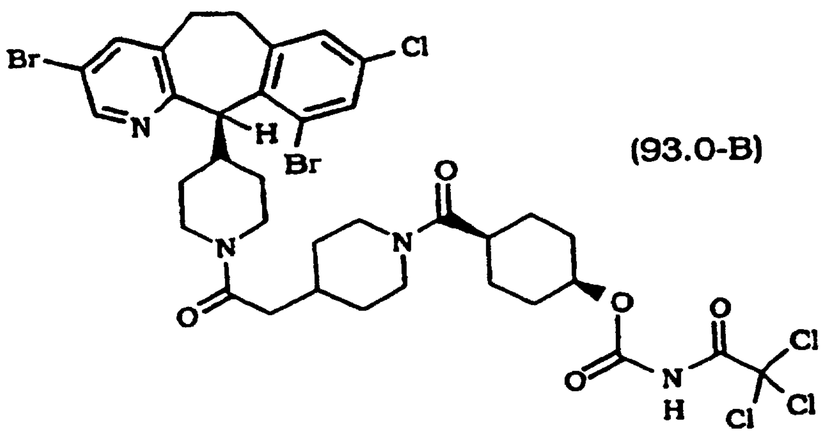

(93.0-B)

;

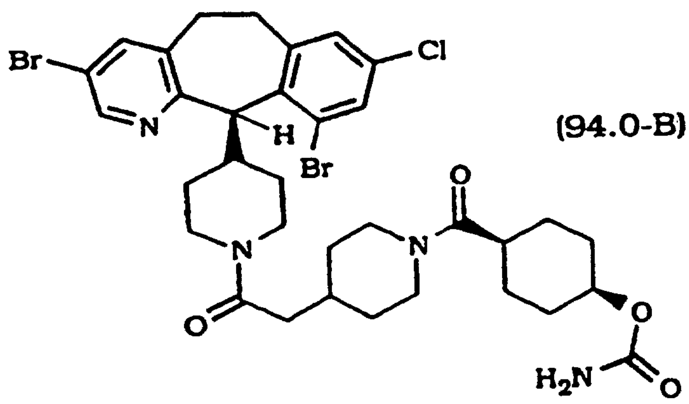

(94.0-B) ;

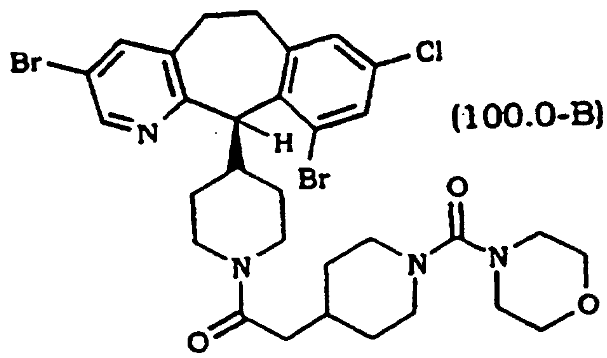

(100.0-B) ;

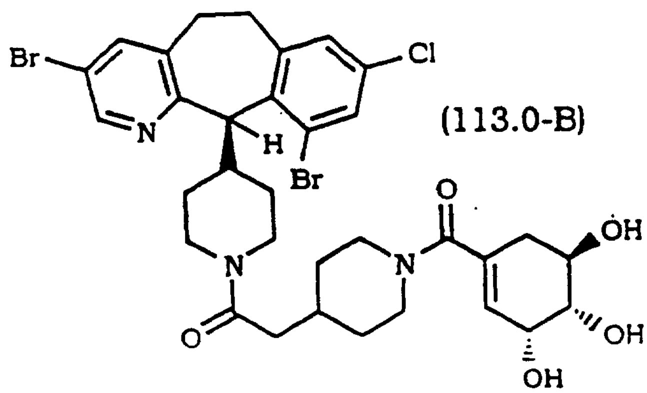

(113.0-B)

ou

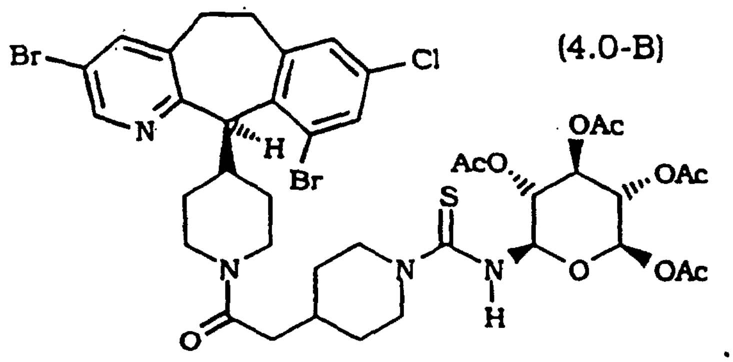

(4.0-B)

**15.** Composition pharmaceutique qui comprend une quantité efficace d'un composé selon l'une quelconque des revendications 1 à 14, en combinaison avec un support pharmaceutiquement acceptable.

**16.** Utilisation d'un composé selon l'une quelconque des revendications 1 à 14 pour la préparation d'un médicament destiné au traitement de cellules tumorales.

**17.** Utilisation selon la revendication 16, dans laquelle les cellules traitées sont des cellules de tumeur pancréatique, des cellules de cancer du poumon, des cellules de tumeur de leucémie myéloïde, des cellules de tumeur folliculaire de la thyroïde, des cellules de tumeur myélodysplasique, des cellules de tumeur de carcinome de l'épiderme, des cellules de tumeur de carcinome de la vessie, des cellules de tumeur du côlon, des cellules de tumeur du sein ou des cellules de tumeur de la prostate.

**18.** Utilisation d'un composé selon l'une quelconque des revendications 1 à 14 pour la préparation d'un médicament pour l'inhibition de la farnésyl protéine transférase.